Europäisches Patentamt

European Patent Office

Office européen des brevets

(19)

(11) EP 1 510 943 A1

(12) EUROPEAN PATENT APPLICATION
published in accordance with Art. 158(3) EPC

(43) Date of publication:
02.03.2005 Bulletin 2005/09

(51) Int Cl.⁷: $G06F\ 17/30$, $C07K\ 1/00$

(21) Application number: 03733232.7

(86) International application number:
PCT/JP2003/006952

(22) Date of filing: 02.06.2003

(87) International publication number:
WO 2003/107218 (24.12.2003 Gazette 2003/52)

(84) Designated Contracting States:
AT BE BG CH CY CZ DE DK EE ES FI FR GB GR
HU IE IT LI LU MC NL PT RO SE SI SK TR
Designated Extension States:
AL LT LV MK

(30) Priority: 31.05.2002 JP 2002160781
31.05.2002 JP 2002160782
20.09.2002 JP 2002275300
20.12.2002 JP 2002371038

(71) Applicant: Celestar Lexico-Sciences, Inc.
Chiba-shi, Chiba 261-8501 (JP)

(72) Inventors:
• SAITO, Seiji c/o Celestar Lexico-Sciences, Inc.
Chiba-shi, Chiba 261-8501 (JP)
• ONO, Kazuki c/o Celestar Lexico-Sciences, Inc.
Chiba-shi, Chiba 261-8501 (JP)

• WADA, Mitsuhito
c/o Celestar Lexico-Scienc., Inc.
Chiba-shi, Chiba 261-8501 (JP)
• IMAI, Kensaku
c/o Celestar Lexico-Sciences, Inc.
Chiba-shi, Chiba 261-8501 (JP)
• HOSOGI, Shinya
c/o Celestar Lexico-Sciences, Inc.
Chiba-shi, Chiba 261-8501 (JP)
• SHIMADA, Takashi
c/o Celestar Lexico-Scienc.,Inc.
Chiba-shi, Chiba 261-8501 (JP)

(74) Representative: HOFFMANN - EITLE
Patent- und Rechtsanwälte
Arabellastrasse 4
81925 München (DE)

(54) **INTERACTION PREDICTING DEVICE**

(57) Objective sequence data (10) which is primary sequence information on an objective protein is entered in an interaction site predicting device by the user. A secondary structure prediction simulation is executed on the objective sequence data (10) entered for secondary structure prediction programs (20a to 20d) that predict a secondary structure of a protein from primary sequence information of the protein. Results of secondary structure prediction (30a to 30d) from the respective secondary structure prediction programs (20a to 20d) are compared (60). Based on the comparison result, frustration of a local portion in the primary sequence information of the objective protein is calculated (70). An interaction site of the objective protein is predicted from the calculated frustration of the local portion (80).

FIG.1

EP 1 510 943 A1

**Description**

TECHNICAL FIELD

The present invention relates to interaction site predicting devices, interaction site predicting methods, programs and recording media, and more particularly to an interaction site predicting device, an interaction site predicting method, a program and a recording medium that predict an interaction site based on frustration of a local site.

Also the present invention relates to active site predicting devices, active site predicting methods, programs and recording media, and more particularly to an active site predicting device, an active site predicting method, a program and a recording medium that estimate an active site of a physiologically active polypeptide or protein with high accuracy.

Also the present invention relates to protein interaction information processing devices, protein interaction information processing methods, programs and recording media, and more particularly to a protein interaction information processing device, a protein interaction information processing method, a program and a recording medium capable of, for example, identifying an interaction site by determining a site which is highly unstable when a protein is in a single substance based on hydrophobic interaction and electrostatic interaction calculated from structure data of the protein.

Also the present invention relates to binding site predicting devices, binding site predicting methods, programs and recording media, and more particularly to a binding site predicting device, a binding site predicting method, a program and a recording medium capable of, for example, efficiently predicting a binding site or a binding partner of a protein or a physiologically active polypeptide by predicting an electrostatically unstable portion using three-dimensional structure information (information about spatial distance between amino acid residues) which is predicted from amino acid sequence data or experimentally obtained and information about electric charge.

Also the present invention relates to protein structure optimizing devices, protein structure optimizing methods, programs and recording media, and more particularly to a protein structure optimizing device, a protein structure optimizing method and a program and a recording medium capable of optimizing a desired atomic coordinate while splitting structure of a protein.

BACKGROUND ART

(I) A protein should have some sort of interaction with other protein, substrate or the like to act, or carry out a certain function. Therefore, determining an interaction site in a protein is a very important research theme in the field of drug discovery or the like, and conventionally developed was a technique to analyze an interaction site of a protein by executing motif retrieving on primary sequence information (amino acid sequence information) of a protein in the field of bioinformatics or the like. To be more specific, an interaction site of a protein is predicted through retrieving of amino acid sequences specifically existing in known interaction sites.

Although the conventional analysis for an interaction site by motif retrieving or the like enabled analysis of known interaction sites, it had a fundamental problem regarding system structure that unknown interaction sites cannot be analyzed. In the following, the problem will be described more specifically.

In a conventional method for analyzing an interaction site, primary sequences which are known to be specific to interaction sites are registered in a motif database or the like, and an interaction site is predicted using the registered information. Therefore, it is impossible to analyze interaction sites that have not been found at the time. Accordingly, in predicting unfound and unknown interaction sites on a computer using the bioinformatics technique, it is necessary to use a completely different approach, however no effective approaches have been established.

In a native state, a protein is folded into a three-dimensional structure that gives as little frustration as possible on interactions between amino acids. In other words, it is believed that an energy curved surface of a protein is designed in a funnel shape toward the whole structure (native structure) where there is no frustration (folding funnel). Although "native structure" is a structure where frustration is small, it does not mean that frustration is perfectly removed, from the view points of complexity of interaction between elements, degree of freedom, evolutionary process and the like.

Recent computational experiments have proved that the funnel-shaped energy surface of a protein which is a product of evolution is not essentially isotropic, but has two directions of large frustration and small frustration (has anisotropy) (anisotropic funnel). This structurally represents that local structures include structures having large frustration and structures having small frustration. Local structure portions having large frustration are structure portions that are scarified for stabilization of the entire structure. These portions are in such a situation that they inevitably have distorted conformation for stabilization of the entire structure and hence are so-called unstable portions in the entire structure.

Protein interaction may be described as a process that allows further stabilization through interaction between two proteins each having a stable entire structure. In further description of structural change during protein interaction, when Protein A and Protein B interact with each other, a part of structure of Protein A and a part of structure of Protein B will change and achieve binding.

Now a local site that appears to be a part of the structure where a change occurs will be considered. First, as to a local structure which is locally and globally stable, there is no need to stabilize more than as it is. On the other hand, as to a portion which is globally stable but locally unstable, the site may possibly be stabilized as a result of binding with other protein or the like and the entire structure may further be stabilized as the result of the binding. In brief, a structure region which is locally unstable is relatively likely to be a protein interaction site. Prediction of a locally unstable portion from a primary sequence as described above may make it possible to provide a candidate for an interaction site.

In prediction of a secondary structure of a protein, a pattern of locally stable structure is predicted from a primary sequence. As such a prediction method, a variety of approaches have been proposed. A secondary structure can be predicted by using a variety of different approaches including early Chou-Fasman's method based on secondary structure attribution information of amino acid, as well as recent so-called 3rd generation approaches which take sequences related with evolution into account such as (1) approach using a neural network, (2) approach using linear statistics and (3) approach using nearest neighbor method.

These secondary structure predicting approaches basically consider a local sequence of a part of primary sequence information for prediction. However, since a secondary structure is eventually determined in relation with the entire structure of the protein, the result of the secondary structure prediction is often incorrect in a portion where mismatch arises between the global scale and the local scale, in other words, in a portion having large frustration (Limit of Secondary Structure Prediction).

In prediction of a secondary structure for such a local site having large frustration, differences in the processing manner in the aforementioned various approaches may largely influence. In other words, the portion where errors are large among different approaches, or the portion where accuracy is poor is very likely to be a local site having large frustration. Thus by comparing the results of secondary structure prediction obtained by various approaches, it would be possible to predict a local site where frustration is relatively large.

As to a protein whose three-dimensional structure is known, or a protein whose three-dimensional data is registered in an existing protein data bank (PDB), it is possible to find a local site having frustration (site which is very likely to be an interaction site) more accurately by considering differences between prediction results obtained by various secondary structure predicting approaches and the real structure because the entire structure of the protein is known.

Therefore, it is an object of the present invention to provide an interaction site predicting device, an interaction site predicting method, a program and a recording medium capable of effectively predicting an interaction site by finding a local site having frustration in primary sequence information of protein.

(II) A variety of methods of estimating an active site of a physiologically active polypeptide or protein have been proposed which are generally classified into two groups: one using only an amino acid sequence and a gene sequence, and the other using information about three-dimensional structure.

However, these conventional predicting methods of active site had a problem of poor prediction accuracy.

Now, this problem will be explained more specifically.

As a typical technique of the above predicting methods belonging to the former group using only a gene sequence, a method of predicting a functional site using frequency of appearance of oligopeptide as disclosed in, for example, Japanese Patent Application Laid-open Publication No. 11-213003, entitled "Method and apparatus for predicting functional site of protein" is recited. These methods belonging to the former group are superior in time and calculation cost, and can be advantageously used in analysis of a protein whose information about three-dimensional structure is not available. However, these methods are inferior in accuracy to the cases where information about three-dimensional structure is available.

On the other hand, a most commonly used method in the active site predicting methods belonging to the latter group using three-dimensional structure is a method of finding a major groove of a protein. Most of active sites are located in a groove of protein which is called a binding pocket. The above method predicts an active site of an enzyme by finding the groove. However, it is often the case that a plurality of grooves are found, or an active site does not coincide with a position of a groove, which deteriorates the accuracy. Additionally, this method has a problem that it is impossible to distinguish an amino acid residue that is required for the activity from amino acid residues just existing in the vicinity of the active site.

Therefore, many researchers have attempted to improve the prediction accuracy by utilizing computational chemistry rather than just relying on the topological information. For example, Ondrechen et al. discloses a system for predicting an active site utilizing the fact that a dissociative amino acid residue in an active site tends to show an abnormal pH titration curve (Proc. Natl. Acad. Sci. USA, Vol.98, Issue 22, 12473-12478, October 23, 2001). However, this method essentially has a drawback that the calculation accuracy is poor because it employs calculations according to the classical theory. Another problem is that a dissociative amino acid residue exhibiting an abnormal pH titration curve is not always an active site as can be seen from the data disclosed in the reference paper.

Elock et al. shows that an amino acid residue that destabilizes the protein calculated according to classical theory is likely to form a binding site or an active site ("Journal of Molecular Biology" Vol.312, No.4, 885-896, September 28, 2001). However, this method confronts the problems of insufficient calculation accuracy due to use of the classical theory as is the case with the above method, and lack of theoretical basis that an amino acid residue destabilizing the protein becomes an active site.

In summary, the problems associated with the conventional predicting methods are that these active site predicting methods have poor theoretical support, and that accuracy of the employed calculation is insufficient. These problems limit prediction accuracy of an active site according to the conventional methods.

Therefore, it is an object of the present invention to provide an active site predicting device, an active site predicting method, a program and a recording medium capable of predicting an active site of a protein from information of energy or extension of a molecular orbital obtained by molecular orbital calculation.

(III) A protein should have some sort of interaction with other protein, substrate or the like, to act, or carry out a certain function. Therefore, determining an interaction site in a protein is a very important research theme in the field of drug discovery or the like, and conventionally developed was a technique to analyze an interaction site of a protein by executing motif retrieving on primary sequence information (amino acid sequence information) of a protein in the field of bioinformatics or the like. To be more specific, an interaction site of a protein is predicted through retrieving of amino acid sequences specifically existing in known interaction sites.

Although the conventional analysis for an interaction site by motif retrieving or the like enabled analysis of known interaction sites, it had a fundamental problem regarding system structure that unknown interaction sites cannot be analyzed.

In a conventional method for analyzing an interaction site, primary sequences which are known to be specific to interaction sites are registered in a motif database or the like, and an interaction site is predicted using the registered information. Therefore, it is impossible to analyze interaction sites that have not been found at the time. Accordingly, in predicting unfound and unknown interaction sites on a computer using the bioinformatics technique, it is necessary to use a completely different approach, however no effective approaches have been established.

Protein interaction may be described as a process that allows further stabilization through interaction between two proteins each having a stable entire structure. In further description of structural change during protein interaction, when Protein A and Protein B interact with each other, a part of structure of Protein A and a part of structure of Protein B will change and achieve binding.

Now a local site that appears to be a part of the structure where a change occurs will be considered. First, as to a local structure which is locally and globally stable, there is no need to stabilize more than as it is. On the other hand, as to a portion which is globally stable but locally unstable, the site may possibly be stabilized as a result of binding with other protein or the like and the entire structure may further be stabilized as the result of the binding. In brief, a structure region which is locally unstable is relatively likely to be a protein interaction site. Prediction of a locally unstable portion from a primary sequence as described above may make it possible to provide a candidate for an interaction site.

Therefore, it is an object of the invention to provide a protein interaction information processing device, a protein interaction information processing method, a program and a recording medium capable of, for example, identifying an interaction site by determining a site that is highly unstable when a protein is in a single substance, based on hydrophobic interaction and electrostatic interaction calculated from structure data of the protein.

(IV) Furthermore, it is important for a protein or physiologically active polypeptide to interact with other protein or the like to carry out a certain function. A substance that inhibits or enhances interaction of a specific protein has the potential for becoming a medical drug. Therefore, it is a very meaningful issue in the biological, medical and pharmaceutical fields to predict an interaction site of a protein and an interaction partner of a protein. To achieve this, in the field of bioinformatics, many attempts have been made to predict an interaction partner of a protein in various manners.

However, known approaches for predicting protein interaction based on the bioinformatics suffer from great calculating load, long processing time and poor prediction accuracy, so that there is a need to develop an approach achieving higher accuracy and shorter processing time.

Now, this problem will be explained more specifically.

For example, with regard to interaction site prediction in the bioinformatics field, prediction techniques based on the motif retrieving or the like have been developed. Although the motif retrieving allows analysis of known interaction sites, it has a problem that it fails to analyze unknown interaction sites.

Also developed are methods of predicting a biding site utilizing amino acid frequency analysis. These are disclosed in, for example, Japanese Patent Application Laid-open Publications Nos. 11-213003, 10-222486 and 10-045795. These prediction methods, however, have a problem of poor prediction accuracy.

In addition to the above, for example, there is a method that obtains a composite body with utmost stability by docking three-dimensional structures of two proteins. Although this method achieves high prediction accuracy, it has some problems. First, proteins whose three-dimensional structures are known are very limited, so that the above method cannot be applied to most of proteins. Secondly, since these approaches suffer from great calculating load and long processing time, it is difficult to execute exhaustive calculation.

Furthermore, no effective means have been established for prediction of interaction partner which is more difficult than prediction of interaction site. That is, no effective means have been established, although a fully new approach is needed for predicting a completely unknown interaction site, and an interaction partner with high accuracy.

Therefore, it is an object of the present invention to provide a binding site predicting device, a binding site predicting method, a program and a recording medium that enables prediction of protein interaction based on the bioinformatics thorough calculation in a very short time and through exhaustive analysis.

(V) In conducting drug design based on a three-dimensional structure of a protein, generally a crystalline structure is often used as a starting structure (See, for example, "Molecular modeling" by H.-D. Höltje and G. Folkers, translated into Japanese by Toshiyuki Ezaki, Chijinshokan, 1998). However, this is accompanied with two problems. The first problem lies in disability of X-ray crystal diffraction to determine positions of hydrogens (See, for example, "Introduction to crystal analysis for life science" by Noriaki Hirayama, MARUZEN CO., LTD., 1996). Missing hydrogens can automatically be added using some modeling software (for example, "WebLab Viewer Pro 4.2 (trade name)" and "Insight II (trade name)" manufactured by Accelrys Inc. (www.accelrys.com), "SYBYL 6.7 (trade name)" manufactured by Tripos, Inc. (www.tripos.com), "Chem3D 7.0 (trade name)" manufactured by CambridgeSoft Corporation (www.camsoft.com) and the like), however they do not necessarily take an orientation which is stable in terms of energy. Another problem lies in that a molecule packed in a crystal structure is in a state just like "dry food", so that the crystal structure does not necessarily reflect the structure functioning in a biological body. In order to bring such a structure closer to "fresh state", it is necessary to make at least side chain portions relaxed. Therefore, it is necessary to optimize the structure for stabilizing a local atomic structure (See for example, "Molecular modeling" by H.-D. Höltje and G. Folkers, translated into Japanese by Toshiyuki Ezaki, Chijinshokan, 1998).

As a method of calculating an electron state of protein, "MOZYME method" implemented by "MOPAC 2000 ver.1.0 (trade name) manufactured by Fujitu Limited) which is a semi empirical molecular orbital calculating program can be exemplified (See, for example, "J.J.P. Stewart, Int. J. Quant. Chem., 58, 133, 1996"). Using this method, one can calculate in a practical level of about 20,000 atoms, or a protein composed of 1,000 residues. This applies only when structural optimization such as "EF (Eigenvector Following) method" (see, for example, "J. Baker, J. Comp. Chem., 7, 385, 1986) and "BFGS (Broyden-Fletcher-Goldfarb-Shanno) method" (see, for example, "C.G.Broyden, Computer Journal, 13, 317, 1970.", "R.Fletcher, J. Inst. Math. Appl., 6, 222, 1970", "D.Goldfarb, Mathematics of Computation, 24, 23, 1970", "D.F.Shanno, Mathematics of Computation, 24, 647, 1970") is not conducted. Generally, the MOPAC2000 uses the EF method achieving high reliability for lower molecules, while using the BEGS method which shows fast convergence and hence reduces the required memory amount for higher molecules.

It is also important to consider a solvent effect in calculation of biological molecule (See, for example, "Molecular modeling" by H.-D. Höltje and G. Folkers, translated into Japanese by Toshiyuki Ezaki, Chijinshokan, 1998, and "Biological engineering basic course - Introduction to computational chemistry" edited by Minoru Sakurai and Atsushi Ikai, MARUZEN CO., LTD., 1999").

However, a practical optimizing calculation used in conducting structure optimization on all atoms of a protein using any one of approaches as described above had a problem regarding system structure that it can handle about 800 residues at most in the case of optimizing only hydrogen atoms, and about 500 residues at most in the case of optimizing side chains.

The above problem mainly arises from steric hindrance of neighboring atoms, so that it is not necessary to consider all the atoms at once in calculation, but a locally stable structure should be determined for each site. In other words, this problem can be solved by means of practical calculation sources by splitting the general structure into partial structures and repeating local structure optimization. However, in the conventional optimizing calculation, no approach has split a structure of a protein for conducting accurate optimization.

Various documents have pointed out the significance of solvent effect in calculation of biological molecule (See, for example, "Molecular modeling" by H.-D. Höltje and G. Folkers, translated into Japanese by Toshiyuki Ezaki, Chijinshokan, 1998, and "Biological engineering basic course - Introduction to computational chemistry" edited by Minoru Sakurai and Atsushi Ikai, MARUZEN CO., LTD., 1999"), however, no conventional methods have enabled structural optimization of protein which takes solvent effect into account.

Therefore, it is an object of the present invention to provide a protein structure optimizing device, a protein structure optimizing method, a program and a recording medium capable of optimizing a desired atomic coordinate while splitting the structure of a protein.

DISCLOSURE OF THE INVENTION

(I) In order to achieve the above object, an interaction site predicting device, an interaction site predicting method and a program according to the present invention include: an inputting unit (inputting step) that inputs primary sequence information of an objective protein; a secondary structure prediction program executing unit (secondary structure prediction program executing step) that makes a secondary structure prediction program to execute a secondary structure prediction simulation for the primary sequence information inputted by the inputting unit (inputting step), the secondary structure prediction program predicting a secondary structure of a protein from primary sequence information of the protein; a prediction result comparing unit (prediction result comparing step) that compares prediction results of secondary structure obtained by the secondary structure prediction program executed by the secondary structure prediction program executing unit (secondary structure prediction program executing step); a frustration calculating unit (frustration calculating step) that calculates frustration of a local site of the primary sequence information of the objective protein based on a comparison result made by the prediction result comparing unit (prediction result comparing step); and an interaction site predicting unit (interaction site predicting step) that predicts an interaction site of the objective protein from the frustration of the local site calculated by the frustration calculating unit (frustration calculating step).

According to the present device, method and program, since primary sequence information of an objective protein is inputted; a secondary structure prediction program which predicts a secondary structure of a protein from primary sequence information of the protein is made to execute a secondary structure prediction simulation for inputted primary sequence information; prediction results of secondary structure obtained by the secondary structure prediction program are compared; frustration of a local site of the primary sequence information of the objective protein is calculated based on the comparison result; and an interaction site of the objective protein is predicted from the calculated frustration of the local site, it is possible to effectively predict an interaction site by finding a local site where frustration is observed in primary sequence information of the protein.

An interaction site predicting device, an interaction site predicting method and a program according to another aspect of the invention include: an inputting unit (inputting step) that inputs primary sequence information of an objective protein; a secondary structure data acquiring unit (secondary structure data acquiring step) that acquires secondary structure data of the objective protein; a secondary structure prediction program executing unit (secondary structure prediction program executing step) that makes a secondary structure prediction program to execute a secondary structure prediction simulation for the primary sequence information inputted by the inputting unit (inputting step), the secondary structure prediction program predicting a secondary structure of a protein from primary sequence information of the protein; a prediction result comparing unit (prediction result comparing step) that compares a prediction result of secondary structure obtained by the secondary structure prediction program executed by the secondary structure prediction program executing unit (secondary structure prediction program executing step), with the secondary structure data acquired by the secondary structure data acquiring unit (secondary structure data acquiring step); a frustration calculating unit (frustration calculating step) that calculates frustration of a local site of the primary sequence information of the objective protein based on a comparison result made by the prediction result comparing unit (prediction result comparing step); and an interaction site predicting unit (interaction site predicting step) that predicts an interaction site of the objective protein from the frustration of the local site calculated by the frustration calculating unit (frustration calculating step).

According to the present device, method and program, since primary sequence information of an objective protein is inputted; secondary structure data of the objective protein is obtained; a secondary structure prediction program which predicts a secondary structure of a protein from primary sequence information of the protein is made to execute a secondary structure prediction simulation for inputted primary sequence information; a prediction result of secondary structure obtained by the secondary structure prediction program is compared with the acquired secondary structure data; frustration of a local site of the primary sequence information of the objective protein is calculated based on the comparison result; and an interaction site of the objective protein is predicted from the calculated frustration of the local site, it is possible to find a local site having frustration (site which is very likely to be an interaction site) more accurately by considering difference between the prediction result of the secondary structure predicting program and the actual secondary structure of the objective protein.

In an interaction site predicting device, an interaction site predicting method and a program according to another aspect of the invention, the interaction site predicting device, the interaction site predicting method and the program as described above further include a certainty factor information setting unit (certainty factor information setting step) that sets certainty factor information representing certainty factor for the prediction result of secondary structure obtained by the secondary structure prediction program, wherein the frustration calculating unit (frustration calculating step) calculates the frustration of the local site based on the certainty factor information set by the certainty factor information setting unit (certainty factor information setting step) and the comparison result.

This shows an exemplary frustration calculation more specifically. According to the present device, method and program, since certainty factor information representing certainty factor for the prediction result of secondary structure obtained by the secondary structure prediction program is set, and frustration of the local site is calculated based on the set certainty factor information and the comparison result, it is possible to reflect certainty factor for the simulation result in the frustration calculation by increasing the weight to the secondary structure prediction result data by the program whose certainty factor information is high (that is, exhibiting high simulation accuracy).

The present invention also relates to a recording medium, and a recording medium according to the present invention records the above program.

According to the present recording medium, by making a computer read the program recorded on the recording medium to execute the same, it is possible to implement the program using a computer and hence to obtain similar effects with these methods.

(II) Under such circumstances, the inventors of the present invention diligently researched for a simple and accurate method of estimating a functional site (active site) of protein, and found the following two facts 1) and 2) to finally complete the present invention: 1) there is a relationship between a position of HOMO (HOMO; highest occupied molecular orbital) or LUMO (LUMO; lowest unoccupied molecular orbital) calculated by the molecular orbital method and their peripheral orbitals, and a position of an active site; and 2) there is a relationship between an amino acid residue whose orbital energy of the molecular orbital distributed in a main chain atom of a protein is relatively high, and an active site.

Since the present invention 1) utilizes molecular orbital calculation which is said to be accurate, and 2) applies the relationship between a position of frontier orbital and a reactive site that was suggested by Kenichi Fukui et al., and demonstrated by many scientists, into the system of protein, as will be described later, it has a feature that accurate prediction is expected owing to the two theoretical grounds.

That is, the active site predicting device, the active site predicting method, the program and recording medium of the present invention were devised on the basis of the following concept. According to the frontier orbital theory advocated by Kenichi Fukui, the highest occupied molecular orbital (HOMO) is responsible for electron giving reaction of a chemical substance and the lowest unoccupied molecular orbital (LUMO) is responsible for electron accepting reaction of a chemical substance. This theory is well demonstrated with regard to low molecular compounds. From these facts, the inventors assumed that a similar theory also applies to a macromolecule such as protein. This possibility is presented by an approach based on the computational chemistry (Journal of the American Chemical Society; 2001; 123(33);8161-8162). Then the inventors of the present invention improved calculating conditions, changed the abstract concept of frontier orbital and its peripheral orbitals into a specific definition, examined the calculating condition in detail, and increased the number of embodiments, to finally complete the present invention that reversely predicts an active site from the electron state.

In order to achieve the above object, in an active site predicting method according to the present invention, an electron state of a protein or physiologically active polypeptide is calculated by molecular orbital calculation to determine a frontier orbital and its peripheral orbital, and/or an orbital energy localized in a heavy atom of a main chain, and to predict an amino acid residue which serves as an active site of the protein or physiologically active polypeptide is predicted based on the frontier orbital and its peripheral orbital, and/or the orbital energy.

According to the present method, since an electron state of a protein or physiologically active polypeptide is calculated by molecular orbital calculation to determine a frontier orbital and its peripheral orbital, and/or an orbital energy localized in a heavy atom of a main chain, and based on the frontier orbital and its peripheral orbital, and/or the orbital energy, an amino acid residue which serves as an active site of the protein or physiologically active polypeptide is predicted, it is possible to accurately predict an active site because molecular orbital calculation which is said to be accurate is used, and relationship between a position of frontier orbital or a position of high orbital energy, and a reactive site is applied for a system of protein or physiologically active polypeptide.

An active site predicting device, an active site predicting method and a program according to another aspect of the invention include: a structure data acquiring unit (structure data acquiring step) that acquires structure data of an objective protein or physiologically active polypeptide; a frontier orbital calculating unit (frontier orbital calculating step) that calculates an electron state of the protein or physiologically active polypeptide by molecular orbital calculation based on the structure data acquired by the structure data acquiring unit (structure data acquiring step) to determine a frontier orbital; a peripheral orbital determining unit (peripheral orbital determining step) that determines a molecular orbital having a predetermined energy gap from the frontier orbital, as a peripheral orbital of the frontier orbital; a candidate amino acid residue determining unit (candidate amino acid residue determining step) that determines as candidate amino acid residues for an active site, amino acid residues in which the frontier orbital and the peripheral orbital distribute; and an active site predicting unit (active site predicting step) that predicts an active site by selecting an active site from the candidate amino acid residues determined by the candidate amino acid residue determining unit (candidate amino acid residue determining step).

According to the present device, method and program, since structure data of an objective protein or physiologically active polypeptide is acquired; an electron state of the protein or physiologically active polypeptide is calculated by molecular orbital calculation based on the acquired structure data to determine a frontier orbital; a molecular orbital having a predetermined energy gap from the frontier orbital is determined, as a peripheral orbital of the frontier orbital; amino acid residues in which the frontier orbital and the peripheral orbital distribute are determined as candidate amino acid residues for an active site; and an active site is predicted by selecting an active site from the determined candidate amino acid residues, it is possible to accurately predict an active site because molecular orbital calculation which is said to be accurate is used, and relationship between a position of frontier orbital and a reactive site is applied for a system of protein or physiologically active polypeptide.

An active site predicting device, an active site predicting method and a program according to another aspect of the invention include: a structure data acquiring unit (structure data acquiring step) that acquires structure data of an objective protein or physiologically active polypeptide; an orbital energy calculating unit (orbital energy calculating step) that calculates an electron state of the protein or physiologically active polypeptide by molecular orbital calculation based on the structure data acquired by the structure data acquiring unit (structure data acquiring step) to determine an orbital energy localized in a heavy atom of a main chain; and a candidate amino acid residue determining unit (candidate amino acid residue determining step) that determines as a candidate amino acid residue for an active site, amino acid residues in which a molecular orbital having an orbital energy exceeding a predetermined level and/or a molecular orbital having a relatively high orbital energy in the orbital energy determined by the orbital energy calculating unit (orbital energy calculating step) distributes.

According to the present device, method and program, since structure data of an objective protein or physiologically active polypeptide is acquired; an electron state of the protein or physiologically active polypeptide is calculated by molecular orbital calculation based on the acquired structure data to determine an orbital energy localized in a heavy atom of a main chain; and amino acid residues in which a molecular orbital having an orbital energy exceeding a predetermined level and/or a molecular orbital having a relatively high orbital energy in the determined orbital energy distribute are determined as a candidate amino acid residue for an active site, it is possible to accurately predict an active site because molecular orbital calculation which is said to be accurate is used, and relationship between a position of high orbital energy and a reactive site is applied for a system of protein or physiologically active polypeptide.

An active site predicting device, an active site predicting method and a program according to another aspect of the invention include: a structure data acquiring unit (structure data acquiring step) that acquires structure data of an objective protein or physiologically active polypeptide; a frontier orbital calculating unit (frontier orbital calculating step) that calculates an electron state of the protein or physiologically active polypeptide by molecular orbital calculation based on the structure data acquired by the structure data acquiring unit (structure data acquiring step) to determine a frontier orbital; an orbital energy calculating unit (orbital energy calculating step) that calculates an electron state of the protein or physiologically active polypeptide by molecular orbital calculation based on the structure data acquired by the structure data acquiring unit (structure data acquiring step) to determine an orbital energy localized in a heavy atom of a main chain; a peripheral orbital determining unit (peripheral orbital determining step) that determines a molecular orbital having a predetermined energy gap from the frontier orbital, as a peripheral orbital of the frontier orbital; a candidate amino acid residue determining unit (candidate amino acid residue determining step) that determines as candidate amino acid residues for an active site, amino acid residues in which the frontier orbital and the peripheral orbital distribute and/or amino acid residues in which a molecular orbital having an orbital energy exceeding a predetermined level and/or a molecular orbital having a relatively high orbital energy in the orbital energy determined by the orbital energy calculating unit (orbital energy calculating step) distributes; an active site predicting unit (active site predicting step) that predicts an active site by selecting an active site from the candidate amino acid residues determined by the candidate amino acid residue determining unit (candidate amino acid residue determining step).

According to the present device, method and program, since structure data of an objective protein or physiologically active polypeptide is acquired; an electron state of the protein or physiologically active polypeptide is calculated by molecular orbital calculation based on the acquired structure data to determine a frontier orbital; an electron state of the protein or physiologically active polypeptide is calculated by molecular orbital calculation based on the acquired structure data to determine an orbital energy localized in a heavy atom of a main chain; a molecular orbital having a predetermined energy gap from the frontier orbital is determined as a peripheral orbital of the frontier orbital; amino acid residues in which the frontier orbital and the peripheral orbital distribute and/or amino acid residues in which a molecular orbital having an orbital energy exceeding a predetermined level and/or a molecular orbital having a relatively high orbital energy in the determined orbital energy are determined as candidate amino acid residues for an active site; and an active site is predicted by selecting an active site from the determined candidate amino acid residues, it is possible to accurately predict an active site because molecular orbital calculation which is said to be accurate is used, and relationship between a position of frontier orbital or a position of high orbital energy and a reactive site is applied for a system of protein or physiologically active polypeptide.

In an active site predicting device, an active site predicting method and a program according to another aspect of the invention, the active site predicting device, the active site predicting method and the program as described above further include: a calculating condition setting unit (calculating condition setting step) that sets at least one of the following calculating conditions 1) to 3) in the molecular orbital calculation: 1) generating water molecules around the protein or physiologically active polypeptide; 2) placing continuous dielectric materials around the protein or physiologically active polypeptide; and 3) bringing dissociative amino acid residues on a surface of the protein or physiologically active polypeptide into a non-charged state while bringing embedded inside dissociative amino acids into a charged state.

This shows one example of molecular orbital calculation more specifically. According to the present device, method and program, since at least one of the following calculating conditions 1) to 3) is set in the molecular orbital calculation: 1) generating water molecules around the protein or physiologically active polypeptide; 2) placing continuous dielectric materials around the protein or physiologically active polypeptide; and 3) bringing dissociative amino acid residues on a surface of the protein or physiologically active polypeptide into a non-charged state while bringing embedded inside dissociative amino acids into a charged state, it is possible to efficiently execute the molecular orbital calculation by appropriately setting the three calculating conditions, and to significantly improve the prediction accuracy of active site.

The present invention also relates to a recording medium, and a recording medium according to the present invention records the above program.

According to the present recording medium, by making a computer read the program recorded on the recording medium to execute the same, it is possible to implement the program using a computer and hence obtain similar effects with these methods.

(III) Further, to achieve the above object, a protein interaction information processing device, a protein interaction information processing method and a program according to the present invention include: a structure data acquiring unit (structure data acquiring step) that acquires structure data including primary structure data of a plurality of interacting proteins and three-dimensional structure data thereof when they are single substances and/or when they form a composite body; a hydrophobic surface determining unit (hydrophobic surface determining step) that determines a hydrophobic interaction energy for each of amino acid residues constituting the primary structure data, according to the structure data acquired by the structure data acquiring unit (structure data acquiring step); an electrostatic interaction determining unit (electrostatic interaction determining step) that determines an electrostatic interaction energy for each of amino acid residues constituting the primary structure data, according to the structure data acquired by the structure data acquiring unit (structure data acquiring step); and an interaction site determining unit (interaction site determining step) that determines an interaction site by determining a portion in the amino acid residues which is highly unstable, based on the hydrophobic interaction energy determined by the hydrophobic surface determining unit (hydrophobic surface determining step) and the electrostatic interaction energy determined by the electrostatic interaction site determining unit (electrostatic interaction determining step).

According to the present device, method and program, since structure data including primary structure data of a plurality of interacting proteins and three-dimensional structure data thereof when they are single substances and/or when they form a composite body is acquired; a hydrophobic interaction energy for each of amino acid residues constituting the primary structure data is determined, according to the acquired structure data; an electrostatic interaction energy for each of amino acid residues constituting the primary structure data is determined, according to the acquired structure data; and an interaction site is determined by determining a portion in the amino acid residues which is highly unstable, based on the determined hydrophobic interaction energy and electrostatic interaction energy, it is possible to readily determine an interaction site of protein from the structure data.

In a protein interaction information processing device, a protein interaction information processing method and a program according to another aspect of the invention, the protein interaction information processing device, the protein interaction information processing method and the program as described above further include: a solvent contact face determining unit (solvent contact face determining step) that determines a solvent contact face for each of amino acid residues constituting the primary structure data, according to the structure data acquired by the structure data acquiring unit (structure data acquiring step); wherein the interaction site determining unit (interaction site determining step) determines an interaction site by determining a site in the amino acid residues which is highly unstable, based on the solvent contact face determined by the solvent contact face determining unit (solvent contact face determining step), the hydrophobic interaction energy determined by the hydrophobic surface determining unit (hydrophobic surface determining step) and the electrostatic interaction energy determined by the electrostatic interaction site determining unit (electrostatic interaction site determining step).

According to the present device, method and program, since a solvent contact face for each of amino acid residues constituting the primary structure data is determined according to the acquired structure data, and an interaction site is determined by determining a site in the amino acid residues which is highly unstable, based on the determined solvent contact face, hydrophobic interaction energy, and electrostatic interaction energy, it is possible to determine an interaction site of protein more accurately and readily when structure data in the state of composite body is available.

In a protein interaction information processing device, a protein interaction information processing method and a program according to another aspect of the invention, the protein interaction information processing device, the protein interaction information processing method and the program as described above further include: a candidate protein retrieving unit (candidate protein retrieving step) that determines a primary sequence of an interacting partner for the interaction site determined by the interaction site determining unit (interaction site determining step) and retrieves for a candidate protein having a primary structure including the determined primary sequence, wherein with respect to the candidate protein retrieved out by the candidate protein retrieving unit (candidate protein retrieving step), whether a part of the primary sequence of the partner is identified as an interaction site of the candidate protein is confirmed.

According to the present device, method and program, since a primary sequence of an interacting partner is determined for the interaction site determined by the interaction site determining unit (interaction site determining step) and a candidate protein having a primary structure including the determined primary sequence is retrieved for, and with respect to the retrieved out candidate protein, whether a part of the primary sequence of the partner is identified as an interaction site of the candidate protein is confirmed by executing the above structure data acquiring unit (structure data acquiring step), solvent contact face determining unit (solvent contact face determining step) (when structure data in the state of composite body is available), hydrophobic surface determining unit (hydrophobic surface determining step), electrostatic interaction site determining unit (electrostatic interaction site determining step) and interaction site determining unit (interaction site determining step), it is possible to readily predict an unknown interaction.

The present invention also relates to a recording medium, and a recording medium according to the present invention records the above program.

According to the present recording medium, by making a computer read the program recorded on the recording medium to execute the same, it is possible to implement the program using a computer and hence obtain similar effects with these methods.

(IV) Furthermore, in order that two proteins may automatically interact with each other, the energy of the entire system needs to decrease as a result of binding. In other words, an unstable portion in a protein may possibly be stabilized as a result of binding, so that such portion is considered as being likely to bind. In addition, an interaction partner is expected to have higher binding ability compared with other proteins. Hence, to predict an interaction partner, it is necessary to search for those having greater ability to interact than others, in addition to exhaustive calculation of interaction. In order to achieve this, interaction of not only one-to-one but also interaction of many-to-many should be calculated, so that it is necessary to significantly improve the calculation cost.

Central concept of the present invention is that a region which is less stable than other regions is more likely to be a binding site from the view point of the protein structure. That is, the present invention predicts a binding site by determining a locally unstable region through a comparatively simple calculation.

Thus, the present invention is mainly featured by enabling a binding site to be accurately predicted basically only from sequence information of a protein (three-dimensional structure information may be added as necessary), and enabling calculation in very short time and exhaustive analysis.

Therefore, the present invention relates to a binding site predicting device, a binding site predicting method, a program and a recording medium capable of, for example, predicting a binding site and a binding partner by predicting three-dimensional structure information (spatial distance between amino acids) from amino acid information of a protein to predict an electrostatically unstable portion from the information of three-dimensional structure and electric charge, and/or by calculating an electrostatic energy when two proteins bind with each other.

In order to achieve the above object, in a binding site predicting method according to the present invention, from amino acid sequence data of a protein or physiologically active polypeptide, spatial distance data between each amino acid residue in three-dimensional structure of the protein or physiologically active polypeptide is calculated, and a binding site is predicted by determining an amino acid residue which is electrostatically unstable according to the distance data and an electric charge of each amino acid.

According to the present method, since from amino acid sequence data of a protein or physiologically active polypeptide, spatial distance data between each amino acid residue in three-dimensional structure of the protein or physiologically active polypeptide is calculated, and a binding site is predicted by determining an amino acid residue which is electrostatically unstable according to the distance data and an electric charge of each amino acid, it is possible to predict a binding site rapidly and accurately by utilizing the fact that an amino acid residue which is appeared to be electrostatically unstable from an amino acid sequence of a protein or physiologically active peptide is likely to be a binding site.

A binding site predicting device, a binding site predicting method and a program according to another aspect of the present invention include: an amino acid sequence data acquiring unit (amino acid sequence data acquiring step) that acquires amino acid sequence data of an objective protein or physiologically active polypeptide; a spatial distance determining unit (spatial distance determining step) that determines a spatial distance between each amino acid residue contained in the amino acid sequence data acquired by the amino acid sequence data acquiring unit (amino acid sequence data acquiring step); an electric charge determining unit (electric charge determining step) that determines an electric charge possessed by each amino acid residue included in the amino acid sequence data; an energy calculating unit (energy calculating step) that calculates an energy of each amino acid residue, according to the spatial distance of each amino acid residue determined by the spatial distance determining unit (spatial distance determining step) and an electric charge possessed by each amino acid residue determined by the electric charge determining unit (electric charge determining step); and a candidate amino acid residue determining unit (candidate amino acid residue determining step) that determines a candidate amino acid residue which serves as a binding site, according to the energy calculated by the energy calculating unit (energy calculating step).

According to the present device, method and program, since amino acid sequence data of an objective protein or physiologically active polypeptide is acquired; a spatial distance between each amino acid residue contained in the acquired amino acid sequence data is determined; an electric charge possessed by each amino acid residue included in the amino acid sequence data is determined; an energy of each amino acid residue is calculated, according to the determined spatial distance of each amino acid residue and the determined electric charge possessed by each amino acid residue; and a candidate amino acid residue which serves as a binding site is determined, according to the calculated energy, it is possible to predict a binding site rapidly and accurately by utilizing the fact that an amino acid residue which is appeared to be electrostatically unstable from an amino acid sequence of a protein or physiologically active peptide is likely to be a binding site.

A binding site predicting device, a binding site predicting method and a program according to another aspect of the present invention include: an amino acid sequence data acquiring unit (amino acid sequence data acquiring step) that acquires amino acid sequence data of a plurality of objective proteins or physiologically active polypeptides; a composite body structure generating unit (composite body structure generating step) that generates three-dimensional structure information of a composite body resulting from binding of the objective proteins or physiologically active polypeptides; a spatial distance determining unit (spatial distance determining step) that determines a spatial distance between each amino acid residue contained in the amino acid sequence data acquired by the amino acid sequence data acquiring unit (amino acid sequence data acquiring step), according to the three-dimensional structure information of the composite body generated by the composite body structure generating unit (composite body structure generating step); an electric charge determining unit (electric charge determining step) that determines an electric charge possessed by each amino acid residue contained in the amino acid sequence data; an energy calculating unit (energy calculating step) that calculates an energy of each amino acid residue, according to the spatial distance of each amino acid residue determined by the spatial distance determining unit (spatial distance determining step) and an electric charge possessed by each amino acid residue determined by the electric charge determining unit (electric charge determining step); an energy minimization unit (energy minimization step) that generates three-dimensional structure information of the composite body while changing the biding site for the composite body by the composite body structure generating unit (composite body structure generating step), calculates an energy of each amino acid residue by the energy calculating unit (energy calculating step), and determines a binding site where a sum total of the energies is minimum; and a candidate amino acid residue determining unit (candidate amino acid residue determining step) that determines a binding site where a sum total of energies is determined as being minimum by the energy minimization unit (energy minimization step), as a candidate amino acid residue of a binding site.

According to the present device, method and program, since amino acid sequence data of a plurality of objective proteins or physiologically active polypeptides is acquired; three-dimensional structure information of a composite body resulting from binding of the objective proteins or physiologically active polypeptides is generated; a spatial distance between each amino acid residue contained in the acquired amino acid sequence data is determined, according to the generated three-dimensional structure information of the composite body; an electric charge possessed by each amino acid residue contained in the amino acid sequence data is determined; an energy of each amino acid residue is calculated, according to the determined spatial distance of each amino acid residue and the determined electric charge possessed by each amino acid residue; three-dimensional structure information of the composite body is generated while changing the biding site for the composite body, an energy of each amino acid residue is calculated and a binding site where a sum total of the energies is minimum is determined; and a binding'site where a sum total of energies is determined as being minimum is determined as a candidate amino acid residue of a binding site, it is possible to predict a binding site rapidly and accurately by utilizing the fact that an amino acid residue which appears to be electrostatically unstable from an amino acid sequence of a protein or physiologically active polypeptide is likely to be a binding site.

A binding site predicting device, a binding site predicting method and a program according to another aspect of the present invention include: an amino acid sequence data acquiring unit (amino acid sequence data acquiring step) that acquires amino acid sequence data of an objective protein or physiologically active polypeptide and amino acid sequence data of one or more candidate protein(s) or physiologically active polypeptide(s) for a binding site; a composite body structure generating unit (composite body structure generating step) that generates three-dimensional structure information of a composite body resulting from binding of the objective protein or physiologically active polypeptide and the candidate protein or physiologically active polypeptide; a spatial distance determining unit (spatial distance determining step) that determines a spatial distance between each amino acid residue contained in the objective amino acid sequence data and the candidate amino acid sequence data acquired by the amino acid sequence data acquiring unit (amino acid sequence data acquiring step), according to the three-dimensional structure information of the composite body generated by the composite body structure generating unit (composite body structure generating step); an electric charge determining unit (electric charge determining step) that determines an electric charge possessed by each amino acid residue contained in the objective amino acid sequence data and the candidate amino acid sequence data; an energy calculating unit (energy calculating step) that calculates an energy of each amino acid residue, according to the spatial distance of each amino acid residue determined by the spatial distance determining unit (spatial distance determining step) and an electric charge possessed by each amino acid residue determined by the electric charge determining unit (electric charge determining step); an energy minimization unit (energy minimization step) that generates three-dimensional structure information of the composite body while changing the biding site for the composite body by the composite body structure generating unit (composite body structure generating step), calculates an energy of each amino acid residue by the energy calculating unit (energy calculating step), and determines a binding site where a sum total of the energies is minimum; and a binding candidate determining unit (binding candidate determining step) that determines a binding candidate having a binding site where a sum total of energies is minimum as a result of execution of the energy minimization unit (energy minimization step) for every binding candidate.

According to the present device, method and program, amino acid sequence data of an objective protein or physiologically active polypeptide and amino acid sequence data of one or more candidate protein(s) or physiologically active polypeptide(s) for a binding site are acquired; three-dimensional structure information of a composite body resulting from binding of the objective protein or physiologically active polypeptide and the candidate protein or physiologically active polypeptide is generated; a spatial distance between each amino acid residue contained in the objective amino acid sequence data and the acquired candidate amino acid sequence data is determined, according to the generated three-dimensional structure information of the composite body; an electric charge possessed by each amino acid residue contained in the objective amino acid sequence data and the candidate amino acid sequence data is determined; an energy of each amino acid residue is calculated, according to the determined spatial distance of each amino acid residue and the determined electric charge possessed by each amino acid residue; three-dimensional structure information of the composite body is generated while changing the biding site for the composite body, an energy of each amino acid residue is calculated, and a binding site where a sum total of the energies is minimum is determined; the energy minimization process is performed for every binding candidate and a binding candidate having a binding site where a sum total of energies is minimum is determined, hence, it is possible to predict a binding site rapidly and accurately by utilizing the fact that an amino acid residue which appears to be electrostatically unstable from an amino acid sequence of a protein or physiologically active polypeptide is likely to be a binding site.

The present invention also relates to a recording medium, and a recording medium according to the present invention records the above program.

According to the present recording medium, by making a computer read the program recorded on the recording medium to execute the same, it is possible to implement the program using a computer and hence obtain similar effects with these methods.

(V) In order to achieve the above object, a protein structure optimizing device, a protein structure optimizing method and a program according to the present invention include: a coordinate data acquiring unit (coordinate data acquiring step) that acquires coordinate data of a protein; a neighboring amino acid residue group extracting unit (neighboring amino acid residue group extracting step) that extracts a coordinate of neighboring amino acid residue group located within a certain distance from a specific amino acid residue, with respect to the coordinate data of a protein; a cap adding unit (cap adding step) that adds a capping substituent for a cutting portion of the neighboring amino acid residue group; an electric charge calculating unit (electric charge calculating step) that calculates an electric charge of the whole of the neighboring amino acid residue group for which the capping substituent is added by the cap adding unit (cap adding step); a structure optimizing unit (structure optimizing step) that executes structure optimization on an atomic coordinate of the specific amino acid residue using the electric charge calculated by the electric charge calculating unit (electric charge calculating step) for the neighboring amino acid residue group to which the capping substituent is added by the cap adding unit (cap adding step); and an atomic coordinate substituting unit (atomic coordinate substituting step) that substitutes the atomic coordinate optimized by the structure optimizing unit (structure optimizing step) for a corresponding atomic coordinate on the coordinate data of the protein.

According to the present device, method and program, since coordinate data of a protein is acquired; a coordinate of neighboring amino acid residue group located within a certain distance from a specific amino acid residue is acquired, with respect to the coordinate data of a protein; a capping substituent is added for a cutting portion of the neighboring amino acid residue group; an electric charge of the whole of the neighboring amino acid residue group for which the capping substituent is added is calculated; structure optimization is executed on an atomic coordinate of the specific amino acid residue using the calculated electric charge for the neighboring amino acid residue group to which the capping substituent is added; and the optimized atomic coordinate is substituted for a corresponding atomic coordinate on the coordinate data of the protein, it is possible to solve the problems of determination of hydrogen position and packing using practical calculation sources.

Furthermore, according to the present device, method and program, it is possible to speed up the optimization process without making any modification on the existing calculation program. In other words, it is possible to execute the present device using input/output files of an existing molecular orbital calculation program or molecular dynamic calculation program. An algorithm of the present device may be incorporated into the existing molecular orbital calculation program or molecular dynamic calculation program.

According to the present device, method and program, structure optimization of protein taking solvent effect into account, which was impossible in the conventional method can be achieved.

In a protein structure optimizing device, a protein structure optimizing method and a program according to another aspect of the present invention, the capping substituent is a hydrogen atom (H) or a methyl group (CH$_3$) in the protein structure optimizing device, the protein structure optimizing method and the program.

This shows one example of a capping substituent more specifically. According to the present device, method and program, since the capping substituent is a hydrogen atom (H) or a methyl group (CH$_3$), it is possible to easily prevent a cutting face resulting from mechanical cutting of coordinates of the neighboring amino acid residue group from becoming a radical to disturb the calculation.

In a protein structure optimizing device, a protein structure optimizing method and a program according to another aspect of the present invention, the neighboring amino acid residue group extracting unit (neighboring amino acid residue group extracting step), when cysteine (CYS) is included in the extracted neighboring amino acid residue group, judges whether another cysteine (CYS) that forms a disulfide bond with the cysteine (CYS) in question but not included in the neighboring amino acid residue group, and when there is another cysteine (CYS), said another cysteine (CYS) is added to the neighboring amino acid residue group, in the protein structure optimizing device, the protein structure optimizing method and the program as described above.

This shows one example of the neighboring amino acid residue group extracting unit (neighboring amino acid residue group extracting step) more specifically. According to the present device, method and program, since the neighboring amino acid residue group extracting unit (neighboring amino acid residue group extracting step) judges, when cysteine (CYS) is included in the extracted neighboring amino acid residue group, whether another cysteine (CYS) that forms a disulfide bond with the cysteine (CYS) in question but not included in the neighboring amino acid residue group, and when there is another cysteine (CYS), another cysteine (CYS) is added to the neighboring amino acid residue group, it is possible to optimize the structure while taking a disulfide bond between cysteines into account.

The present invention also relates to a recording medium, and a recording medium according to the present invention records the above program.

According to the present recording medium, by making a computer read the program recorded on the recording medium to execute the same, it is possible to implement the program using a computer and hence obtain similar effects with these methods.

BRIEF DESCRIPTION OF THE DRAWINGS

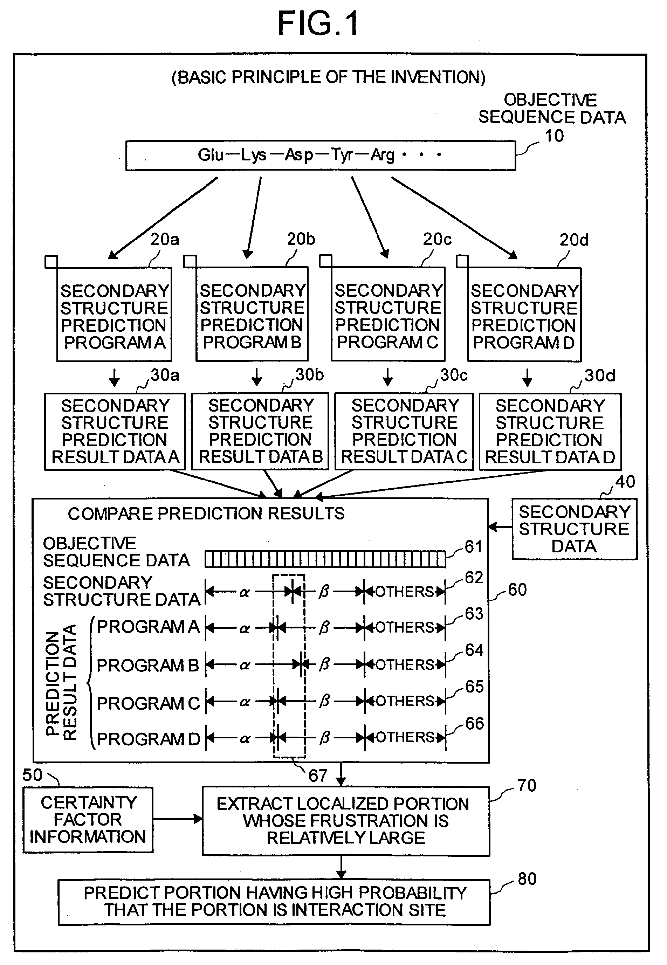

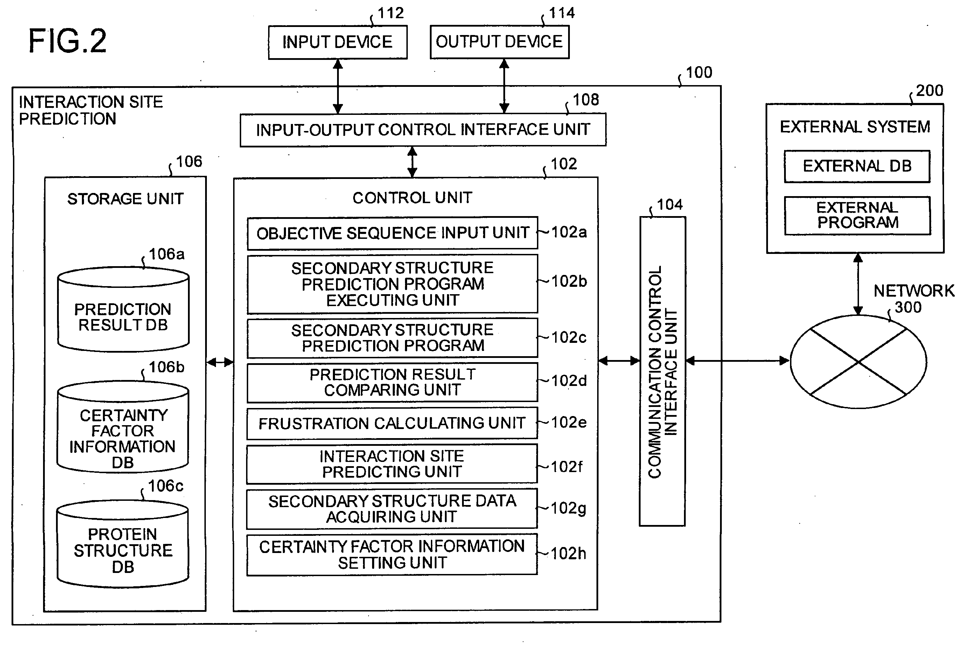

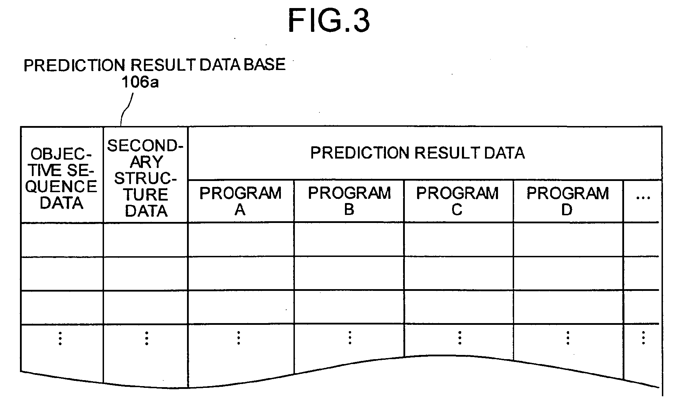

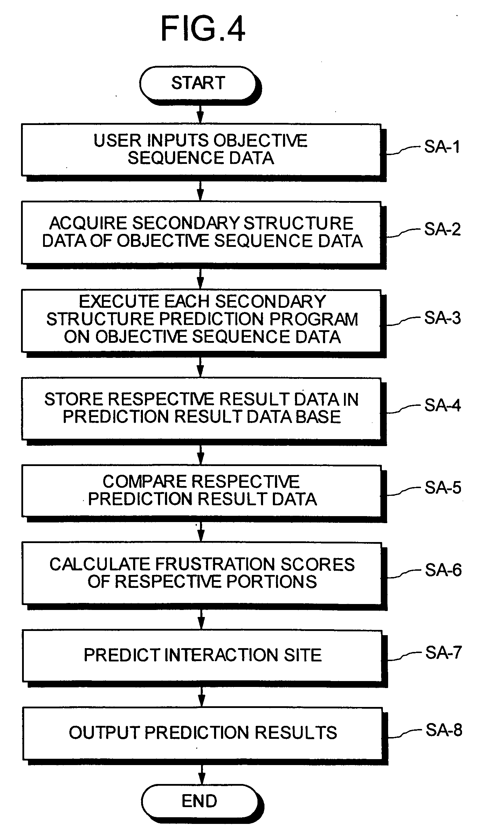

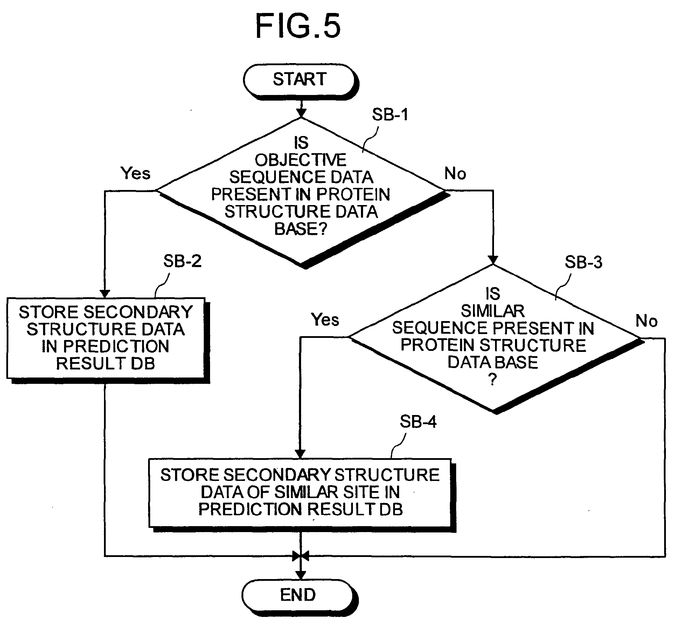

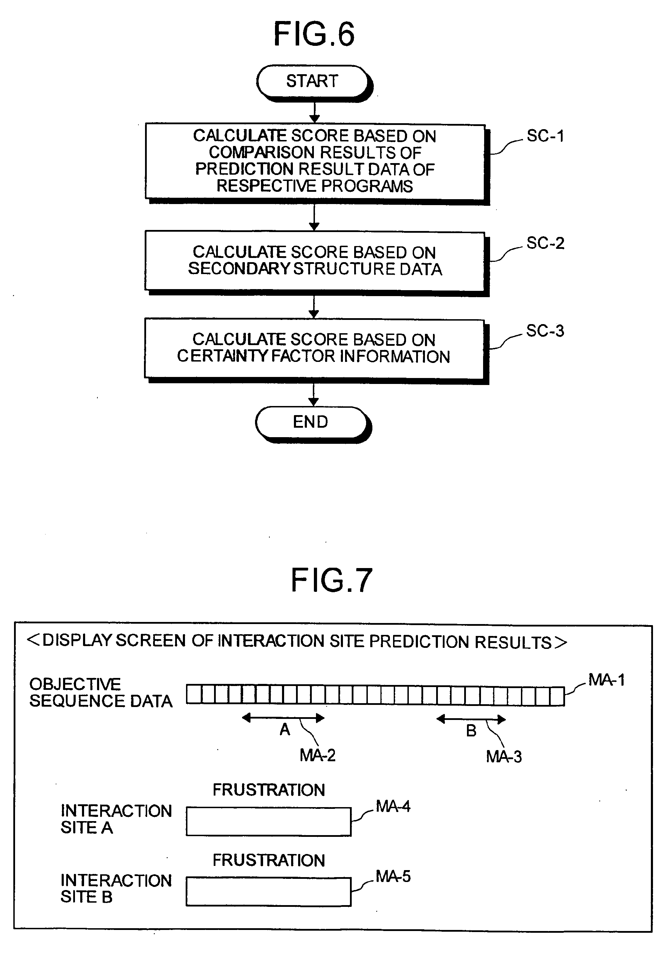

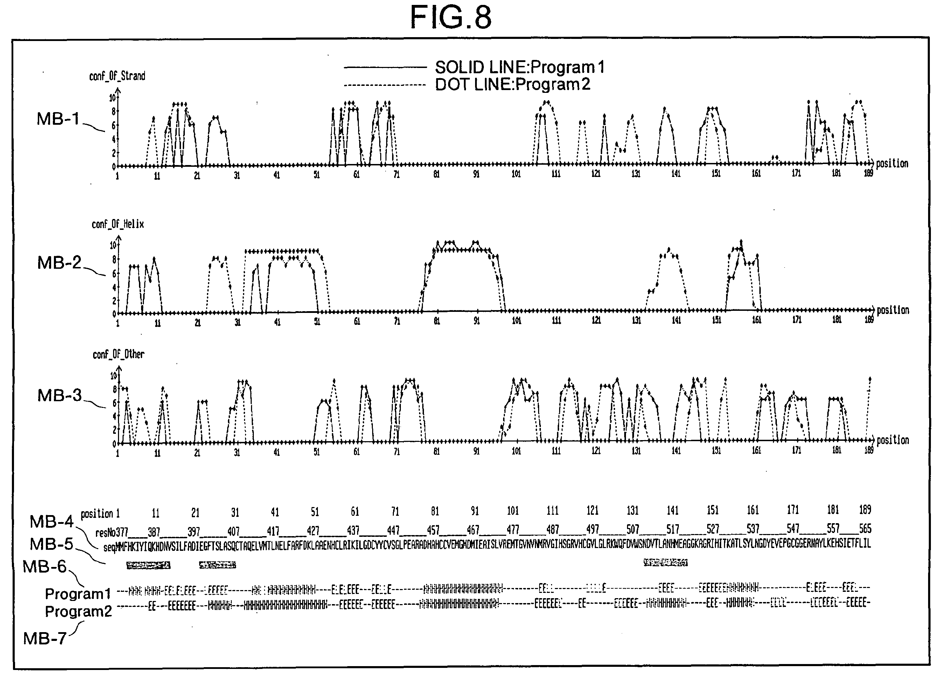

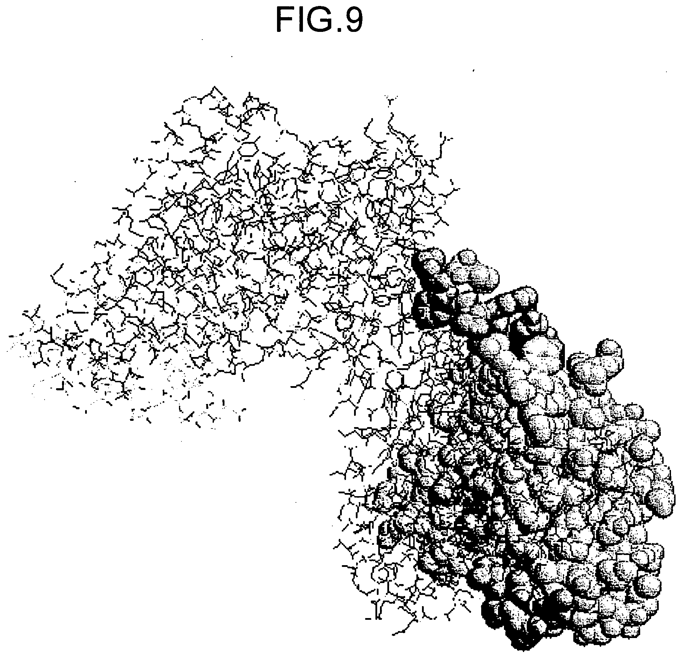

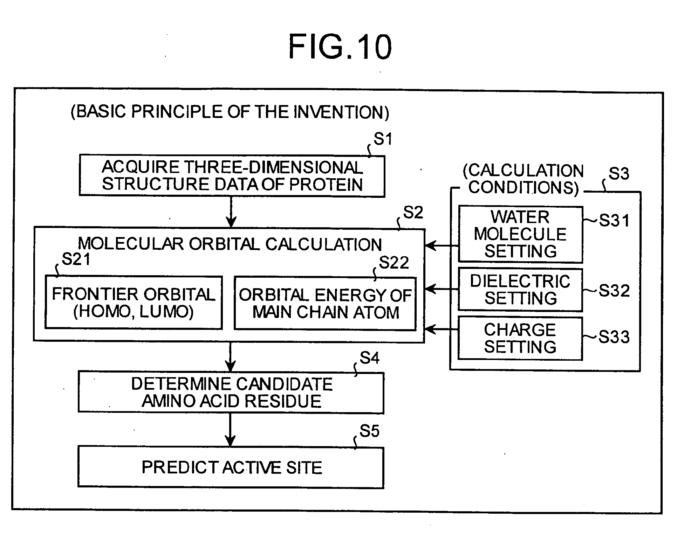

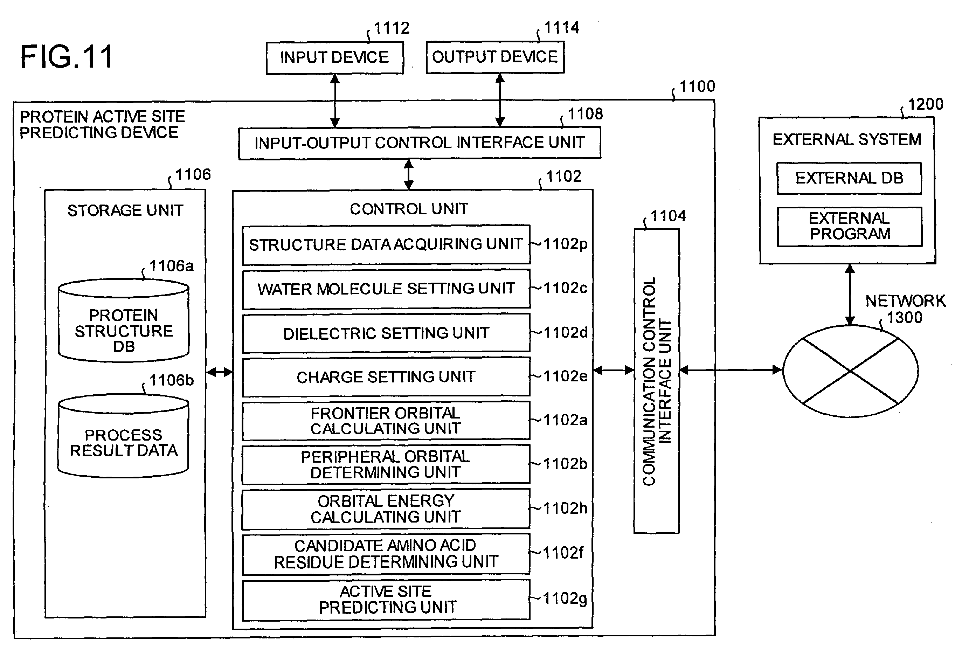

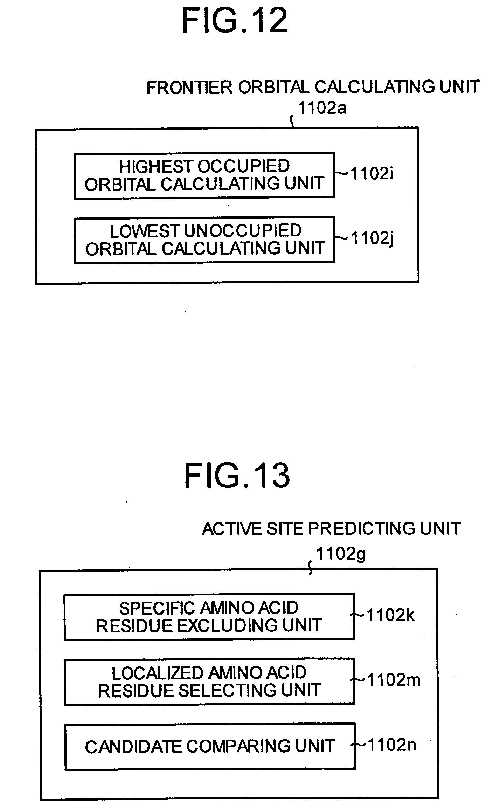

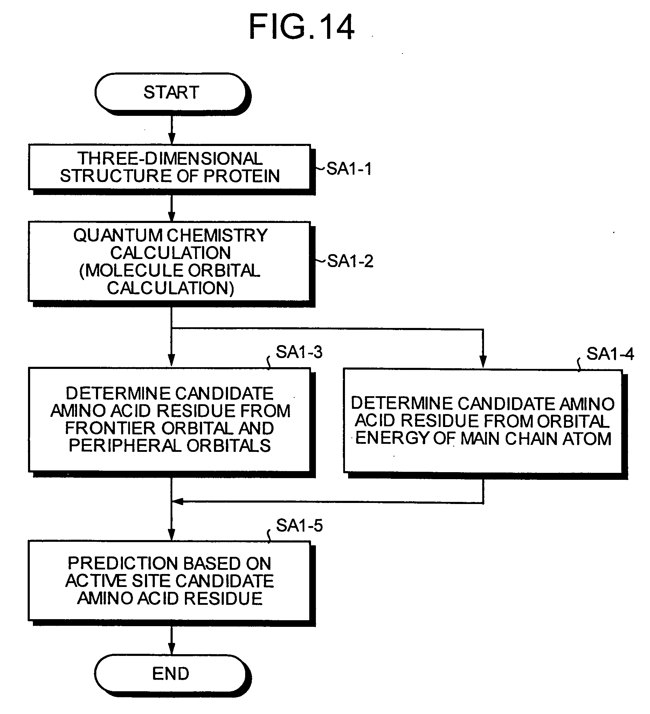

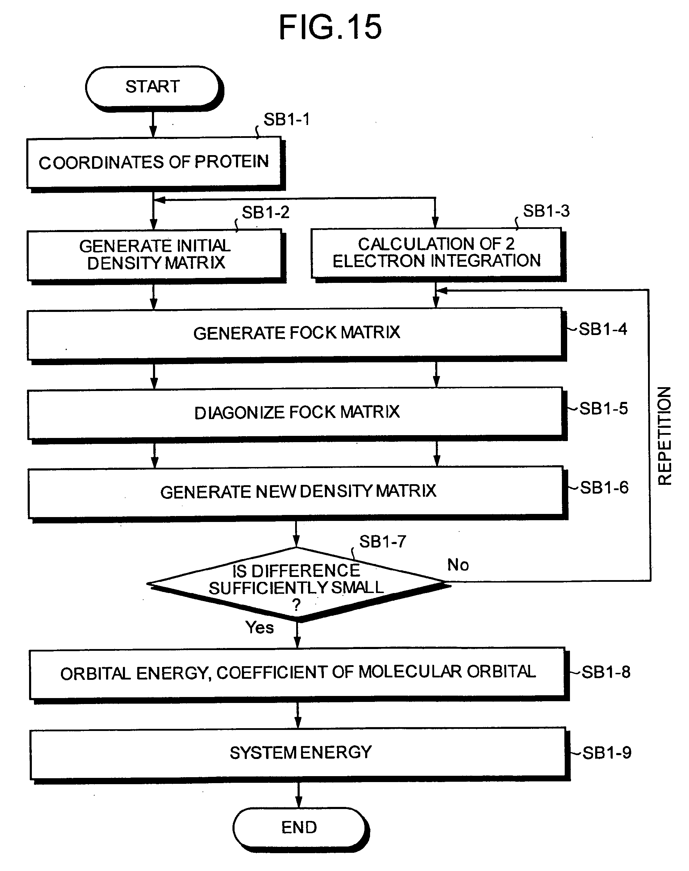

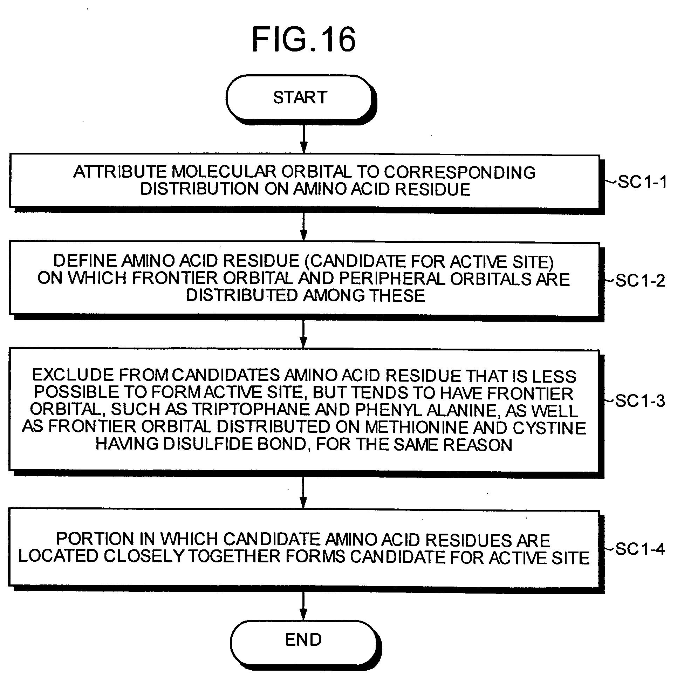

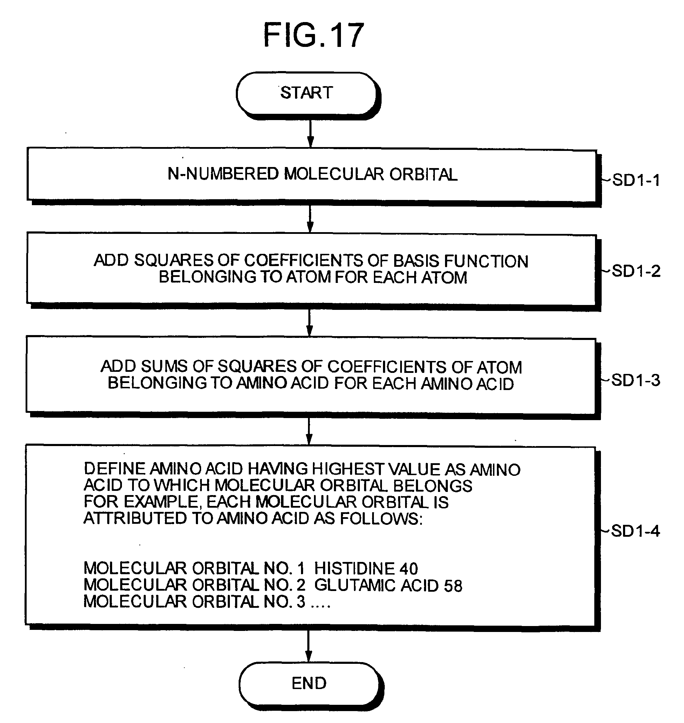

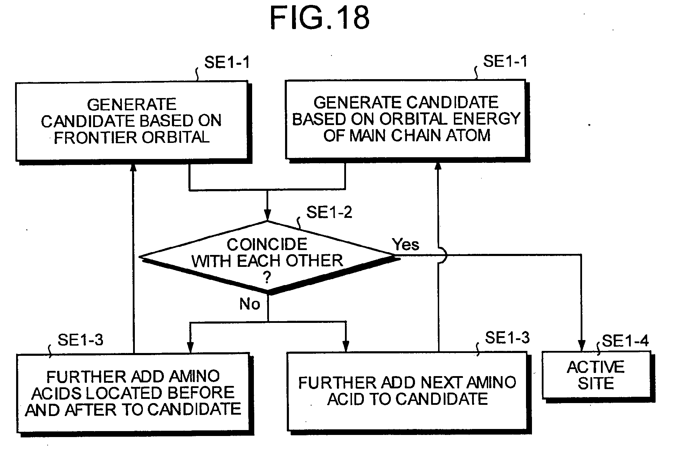

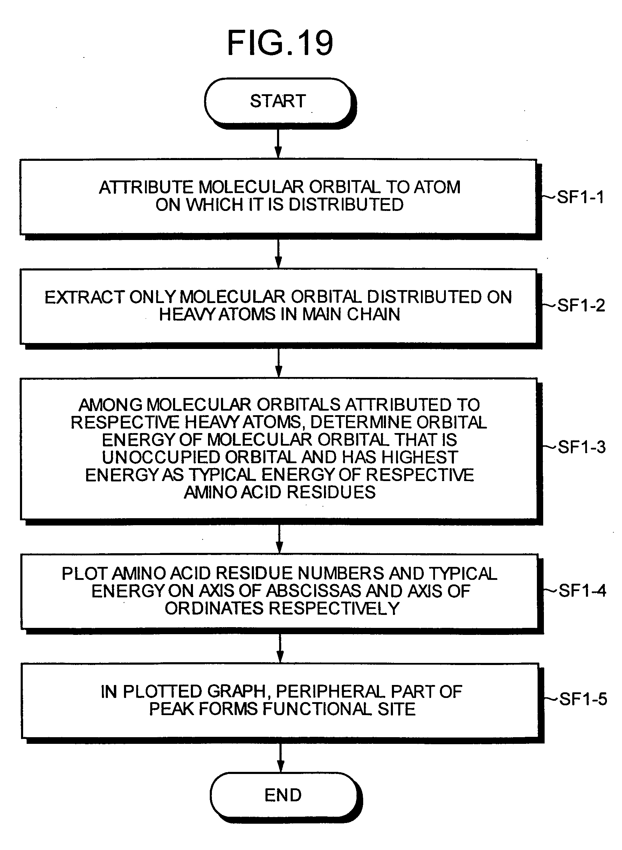

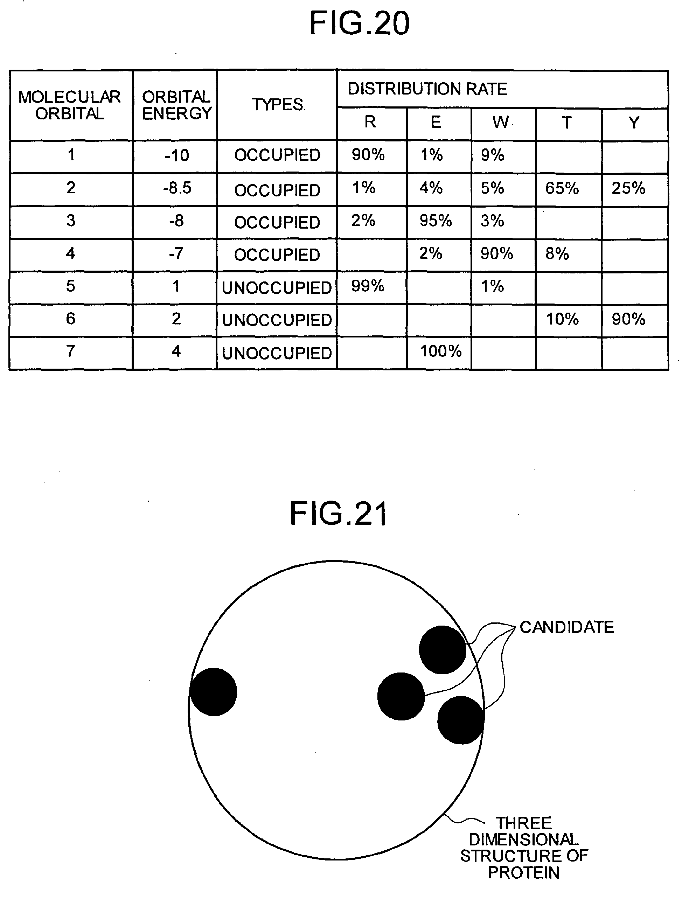

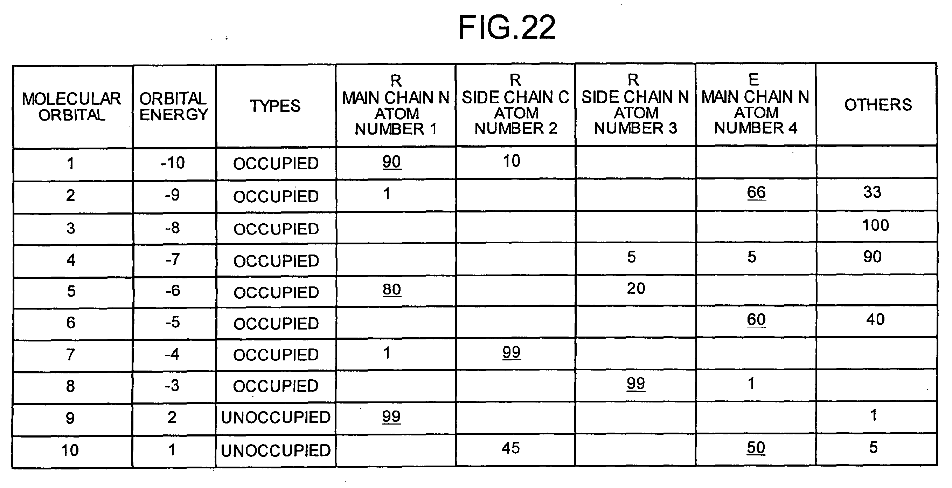

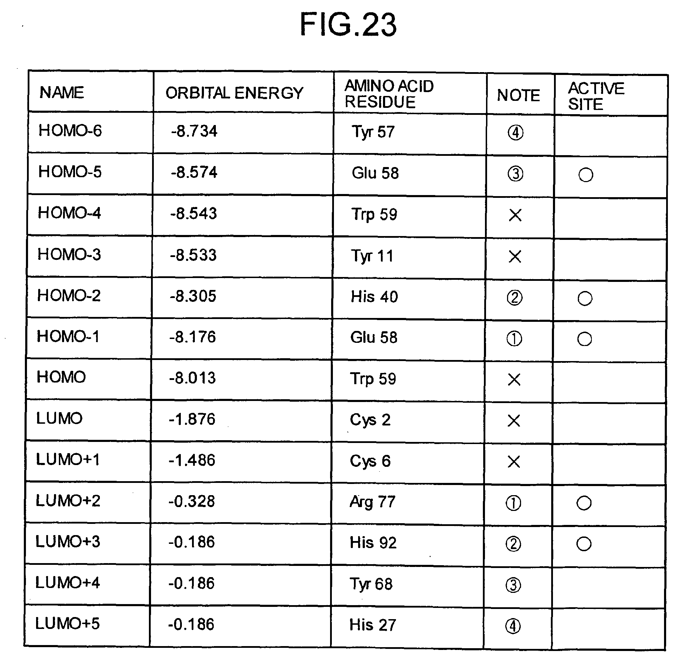

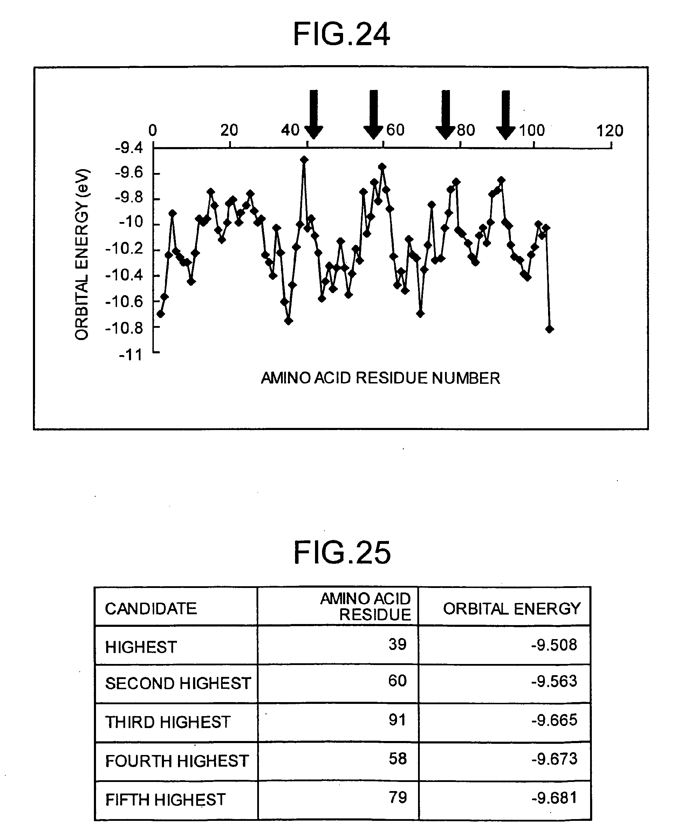

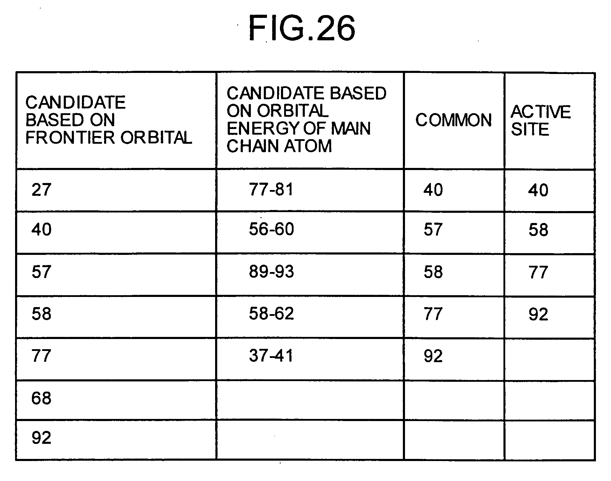

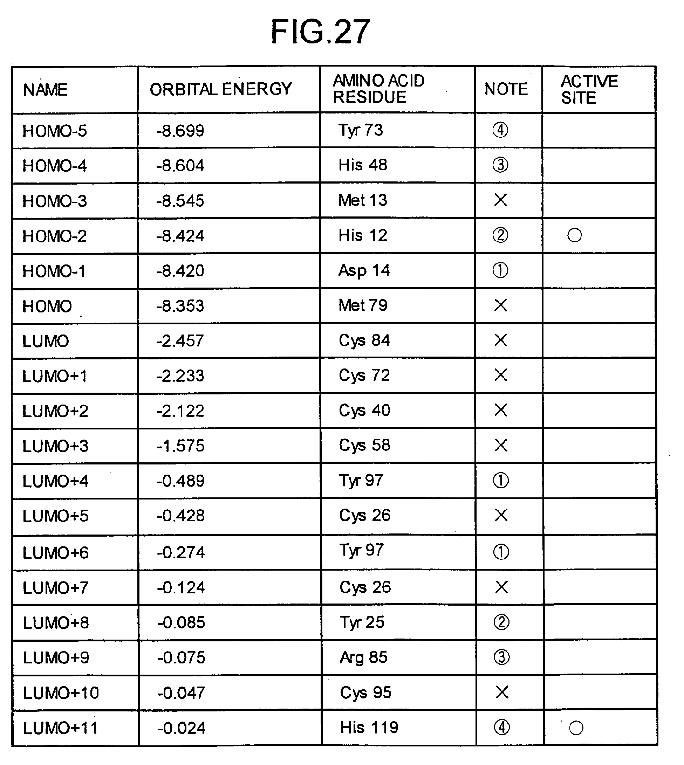

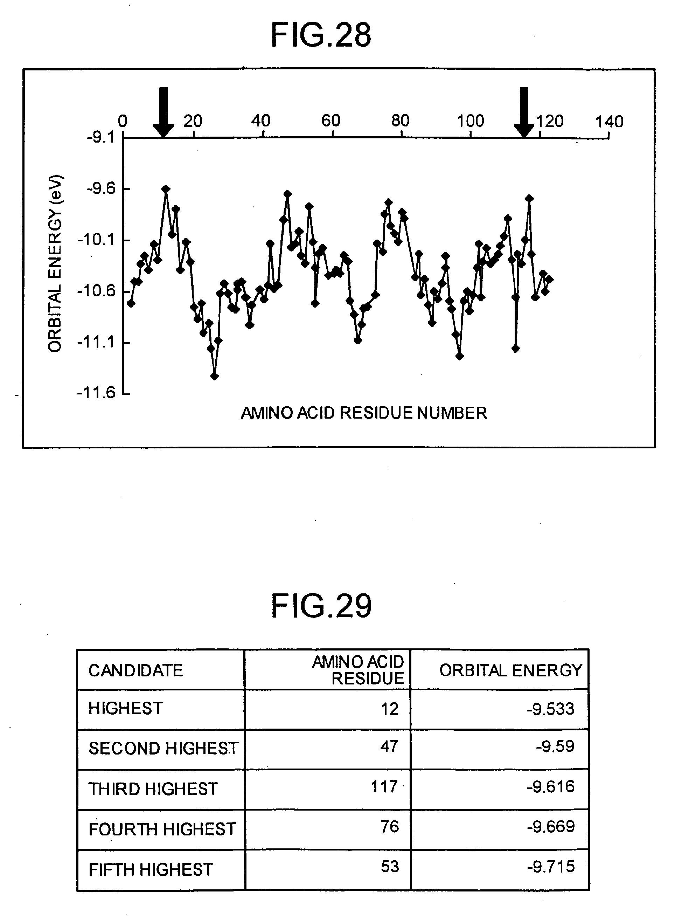

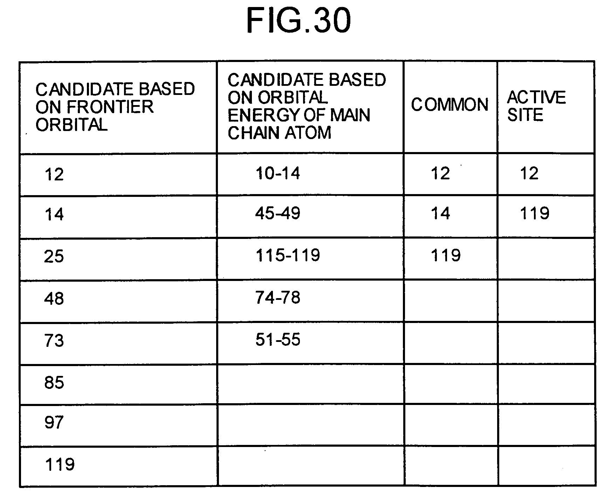

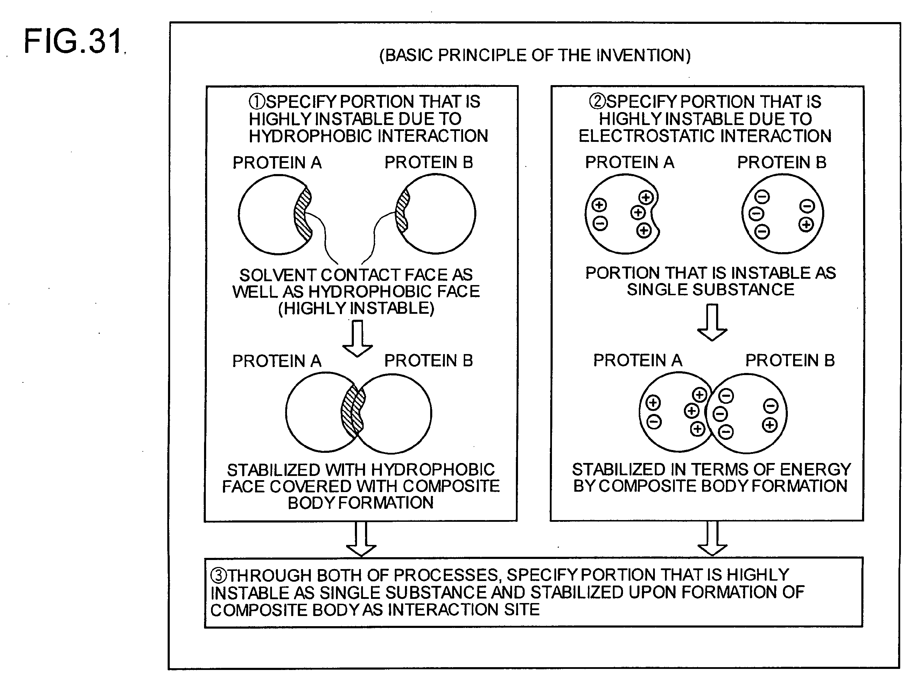

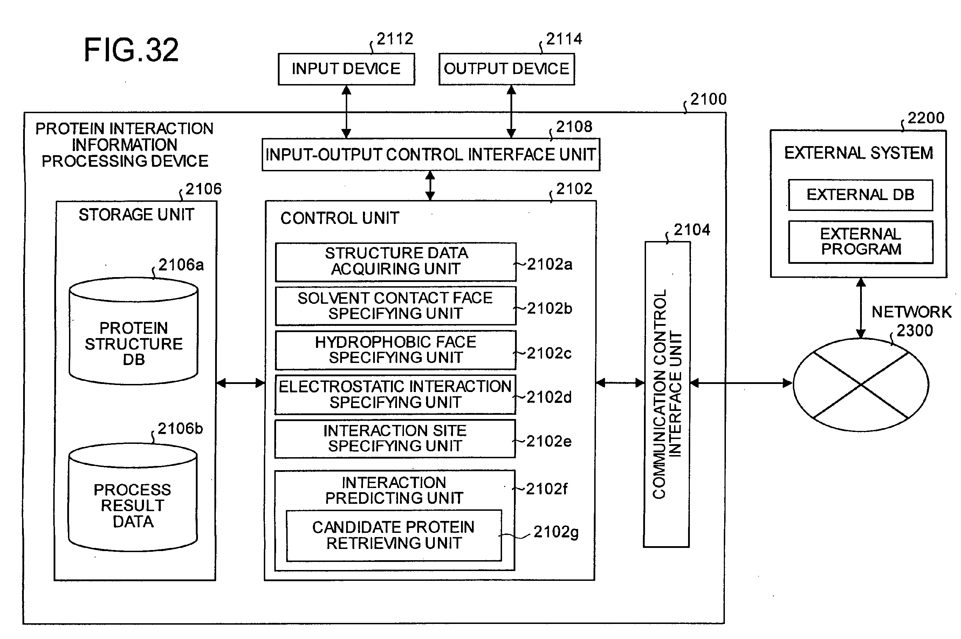

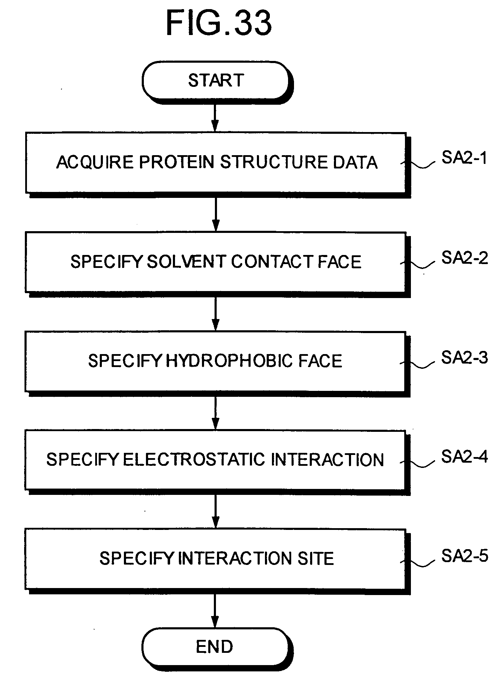

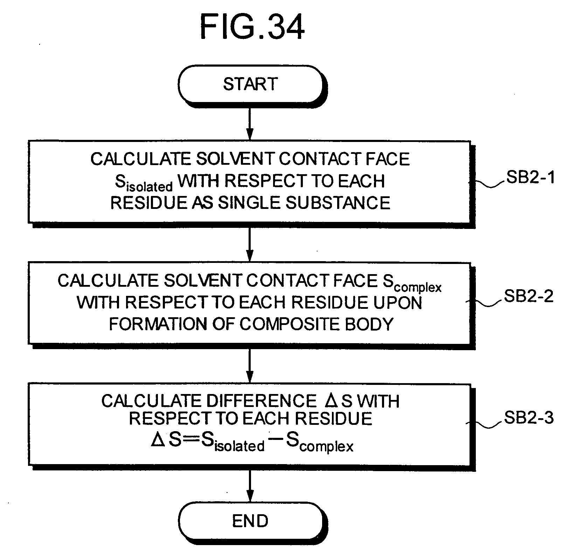

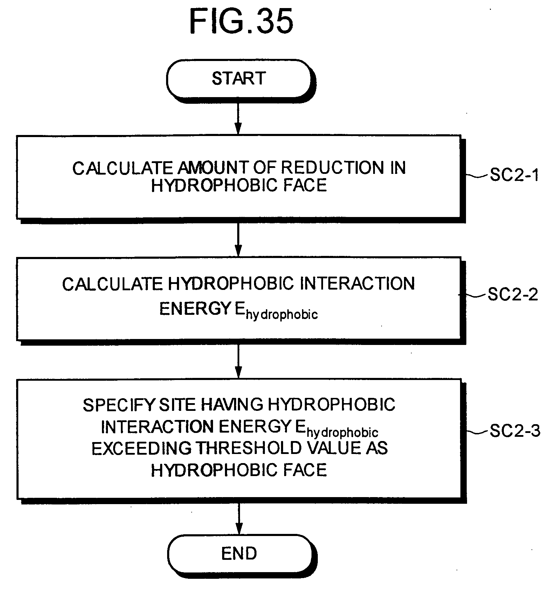

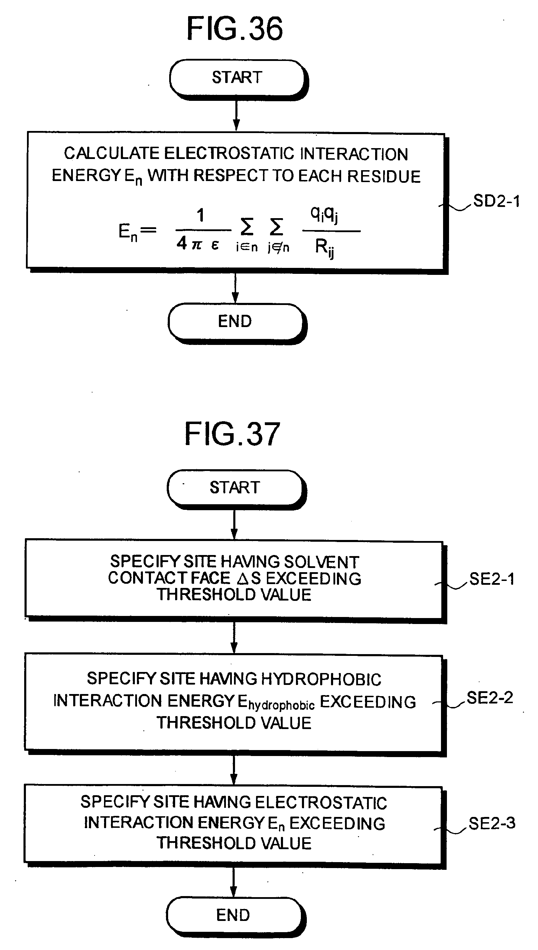

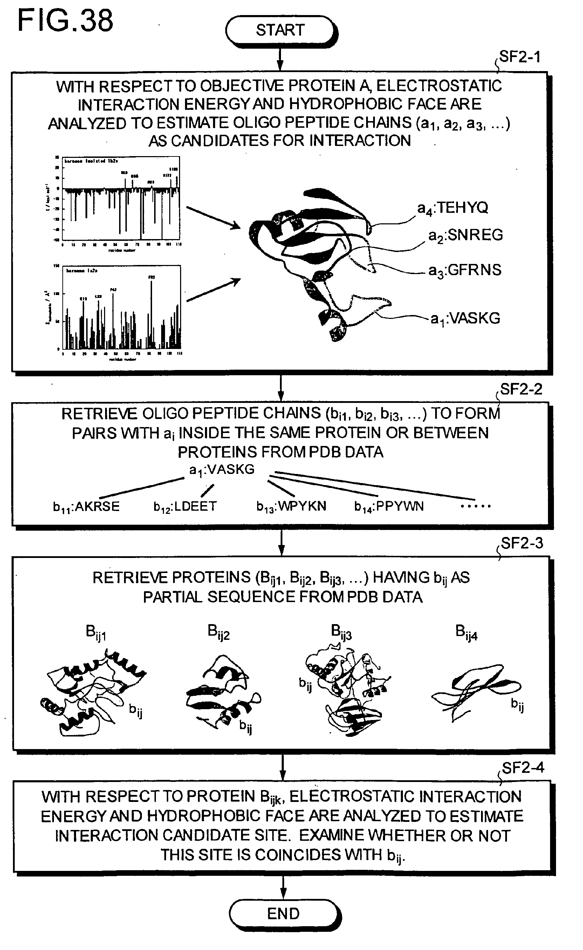

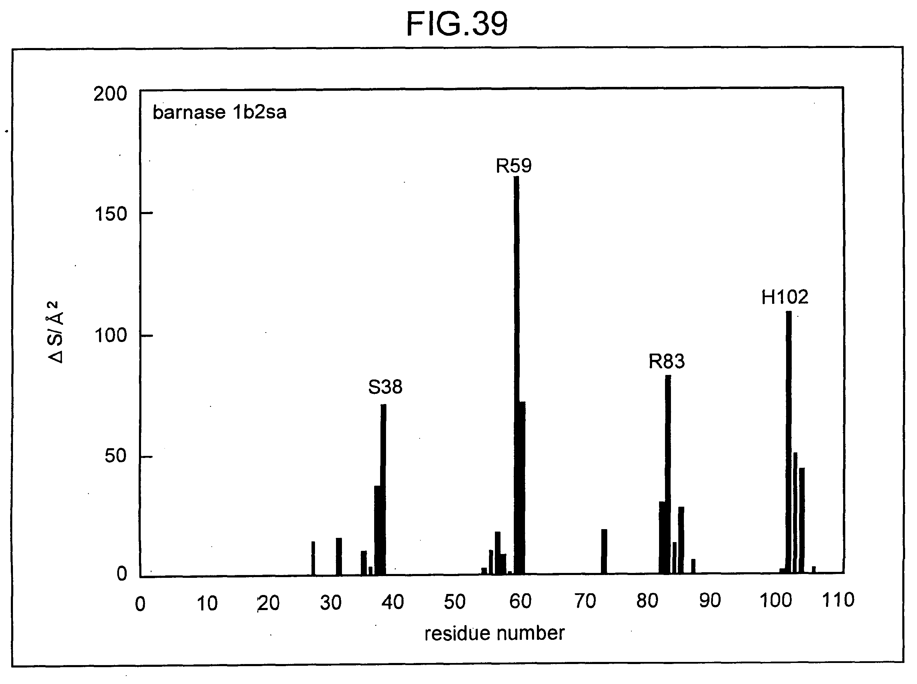

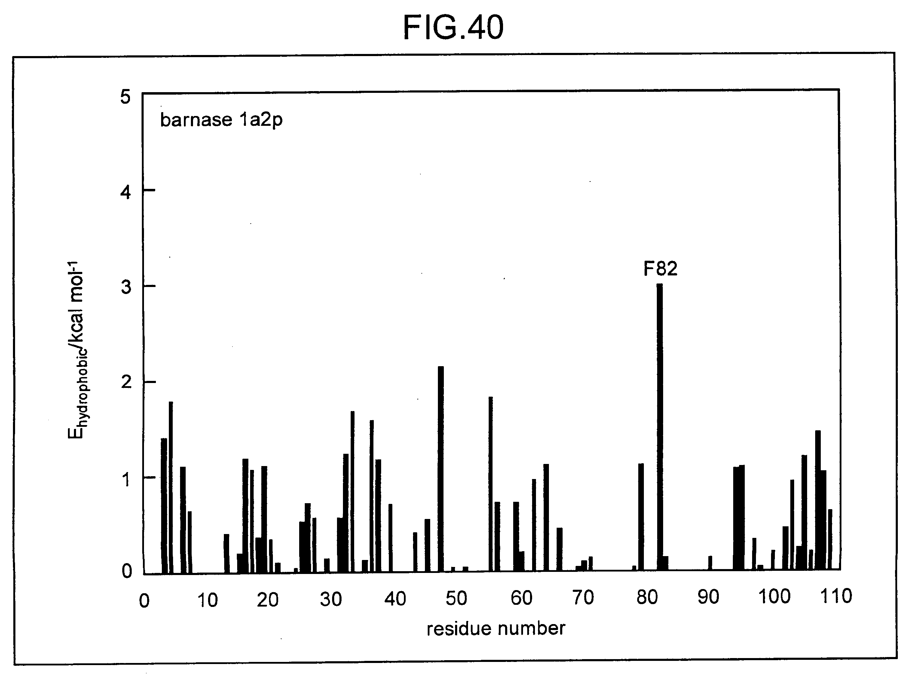

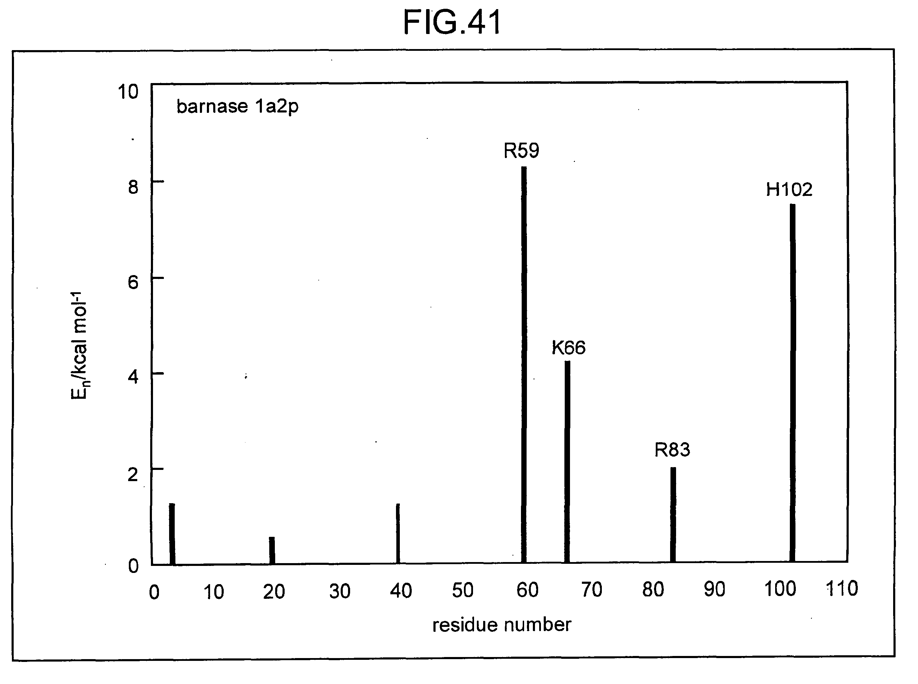

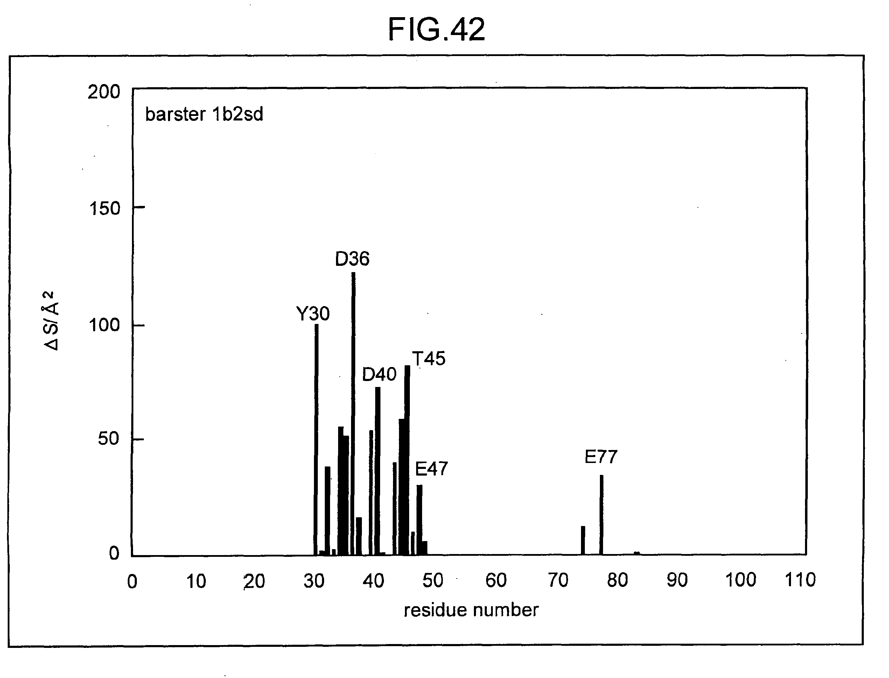

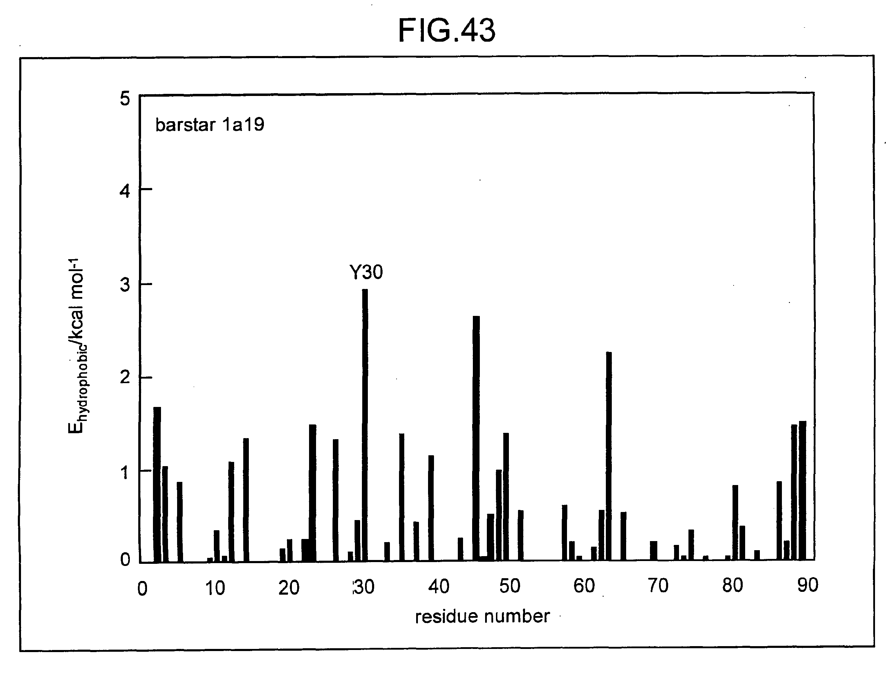

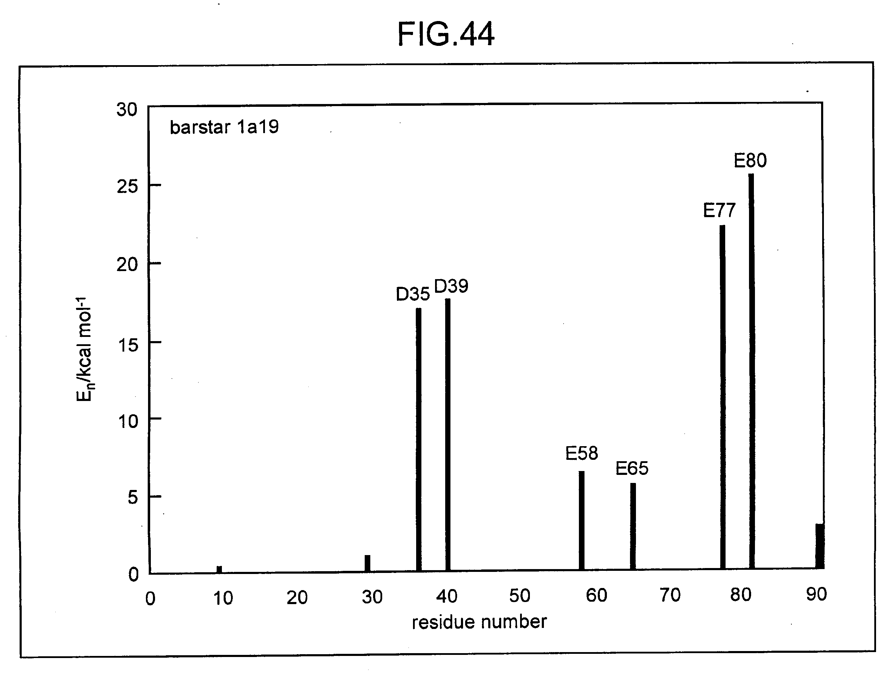

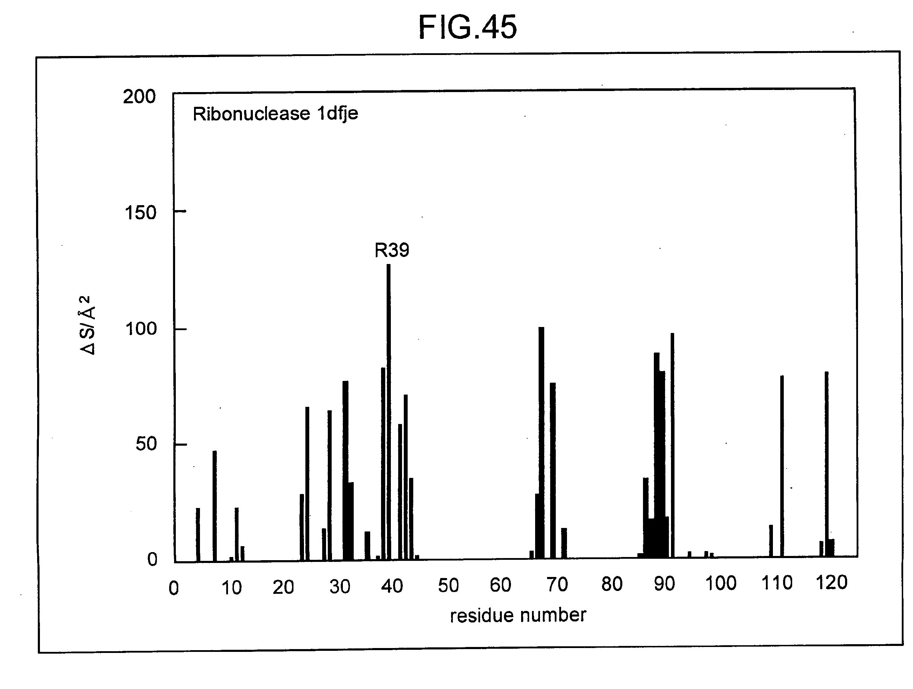

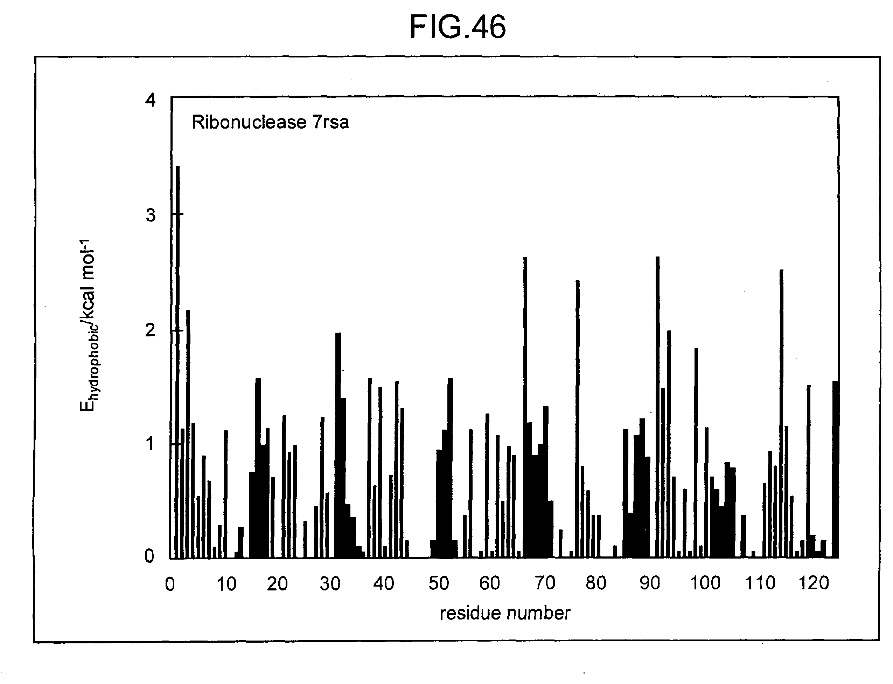

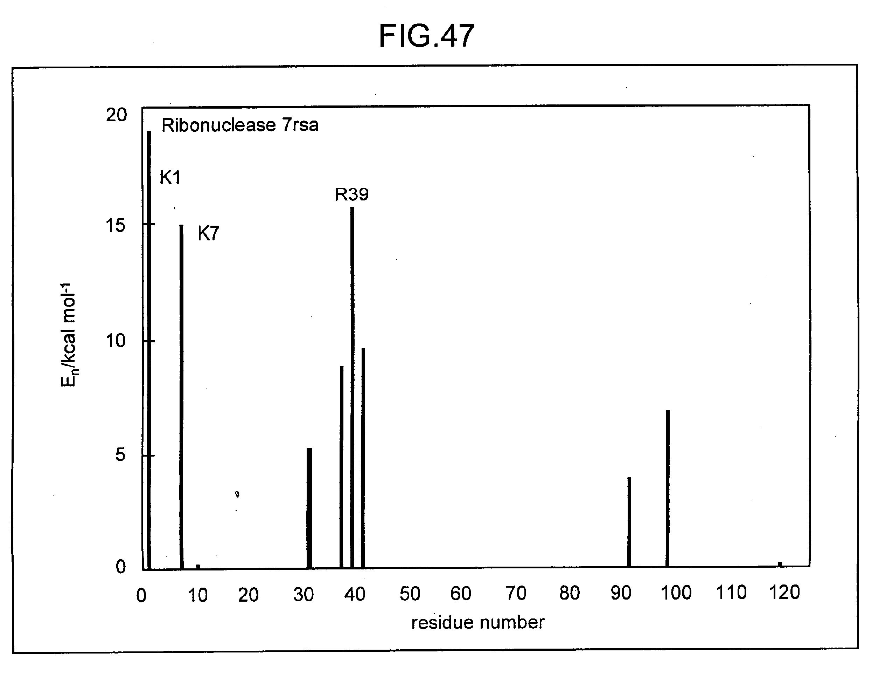

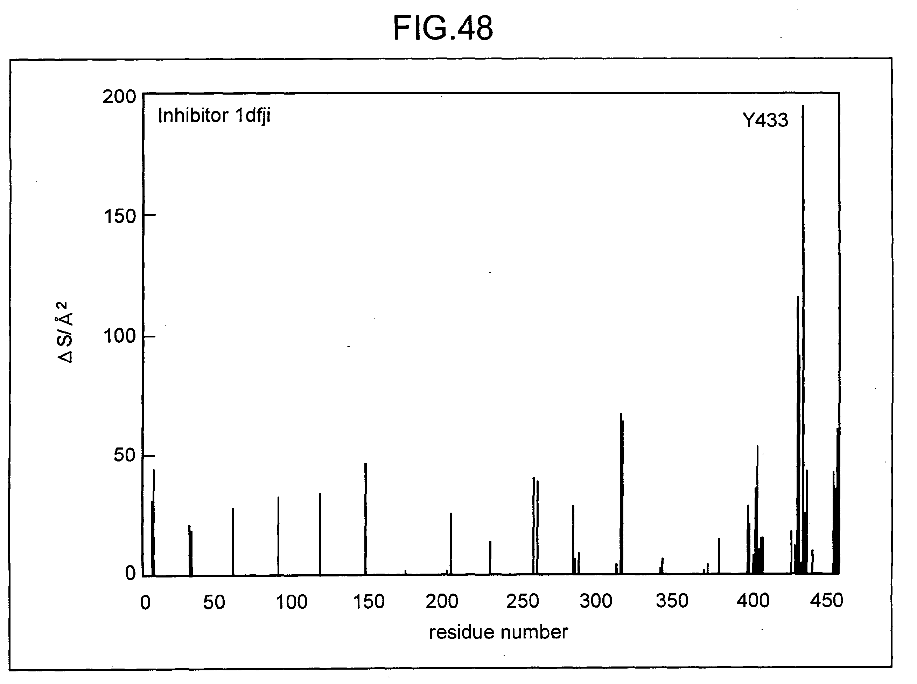

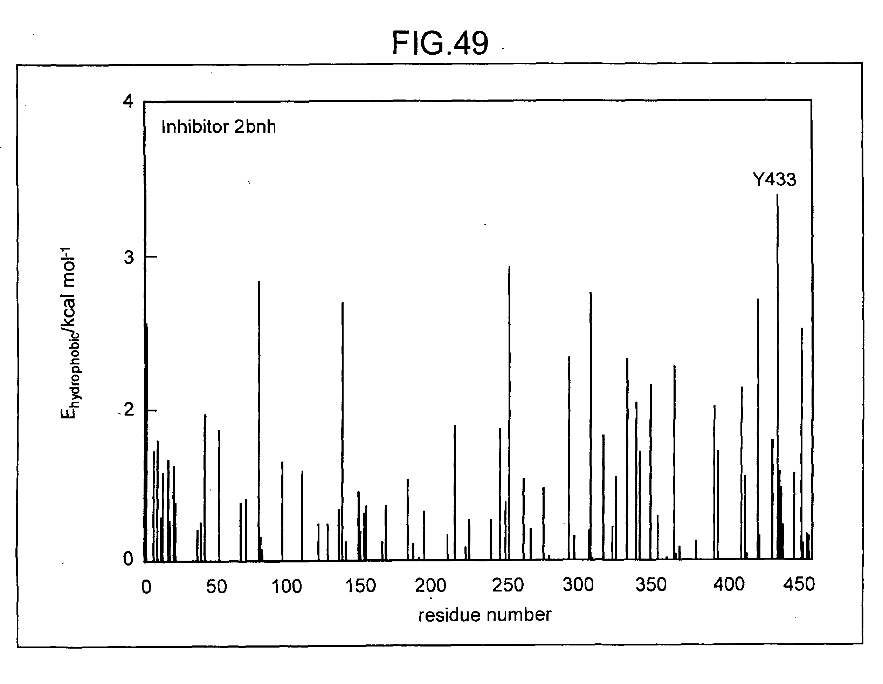

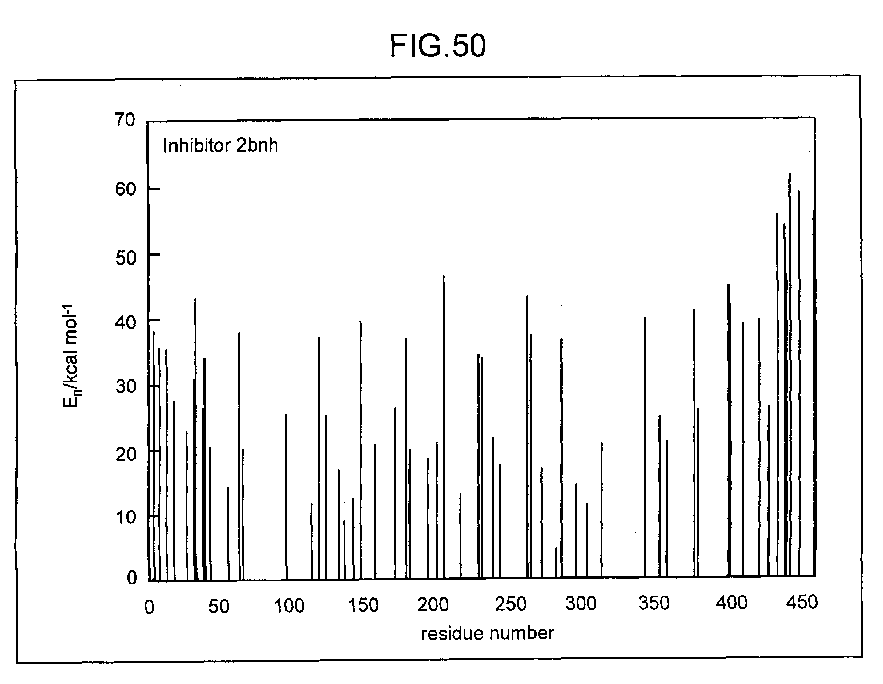

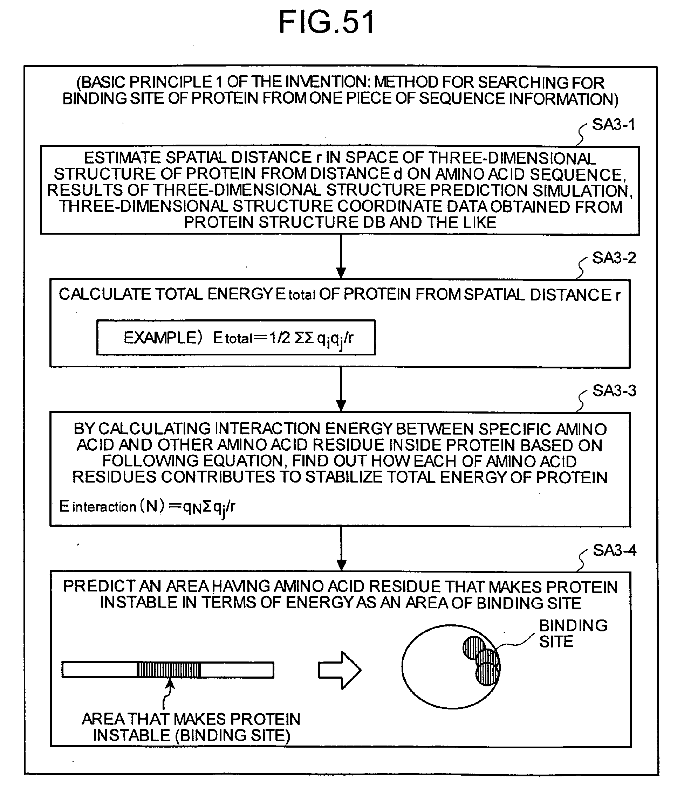

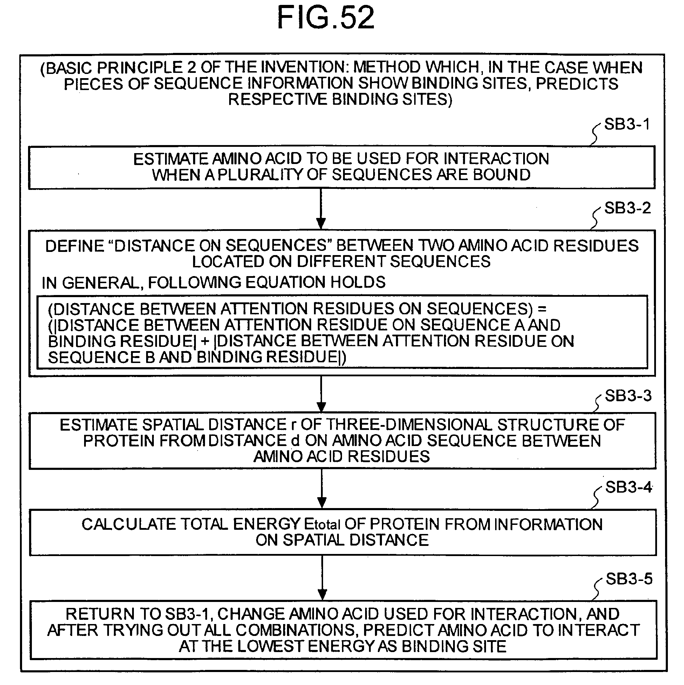

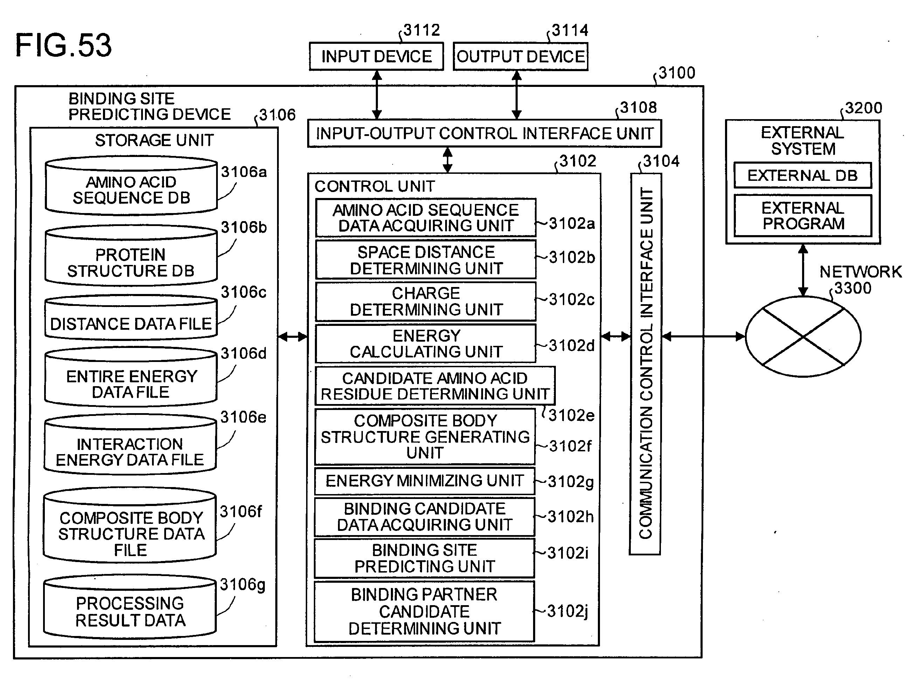

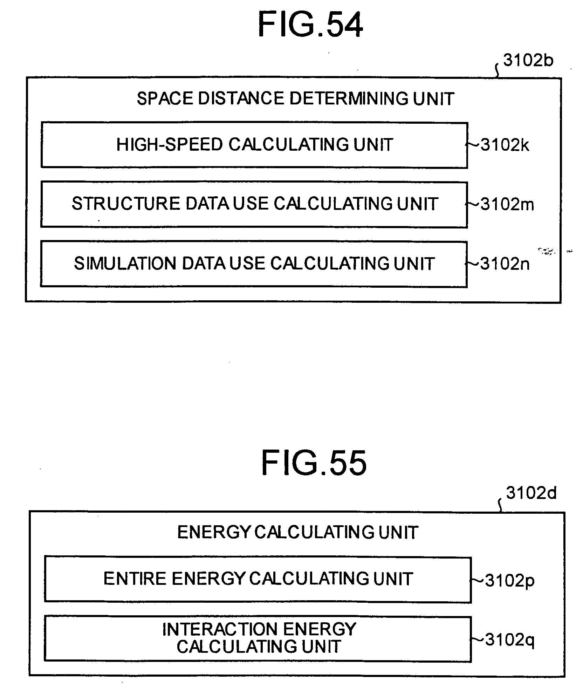

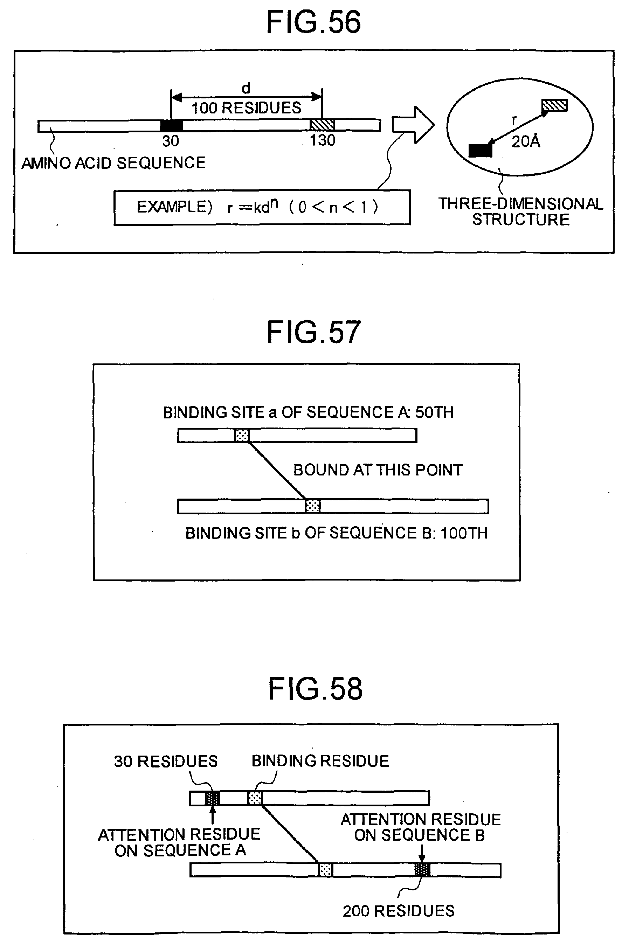

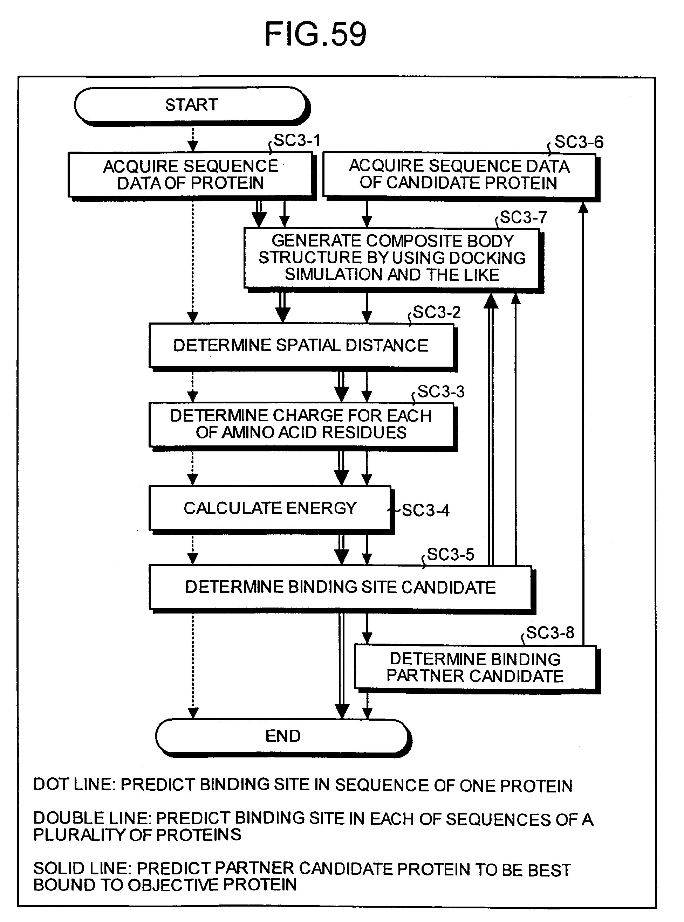

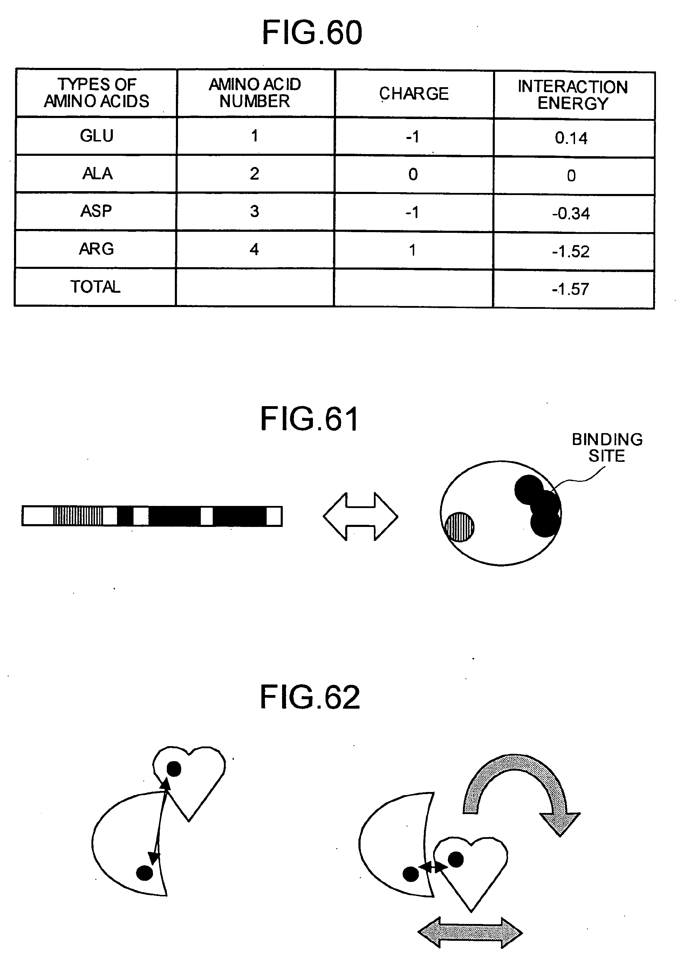

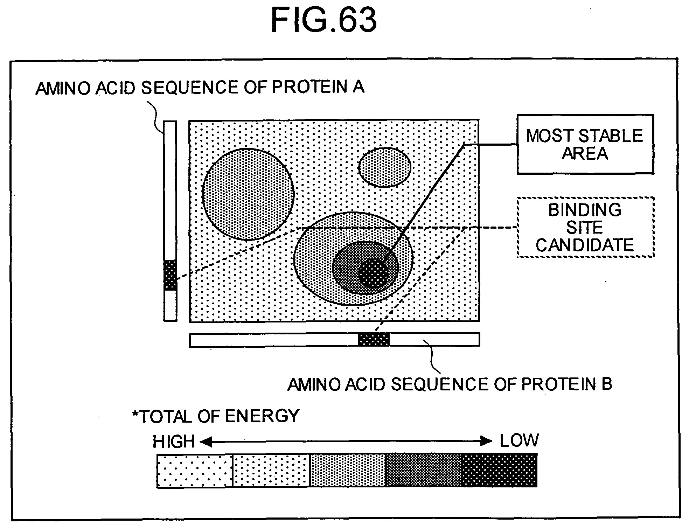

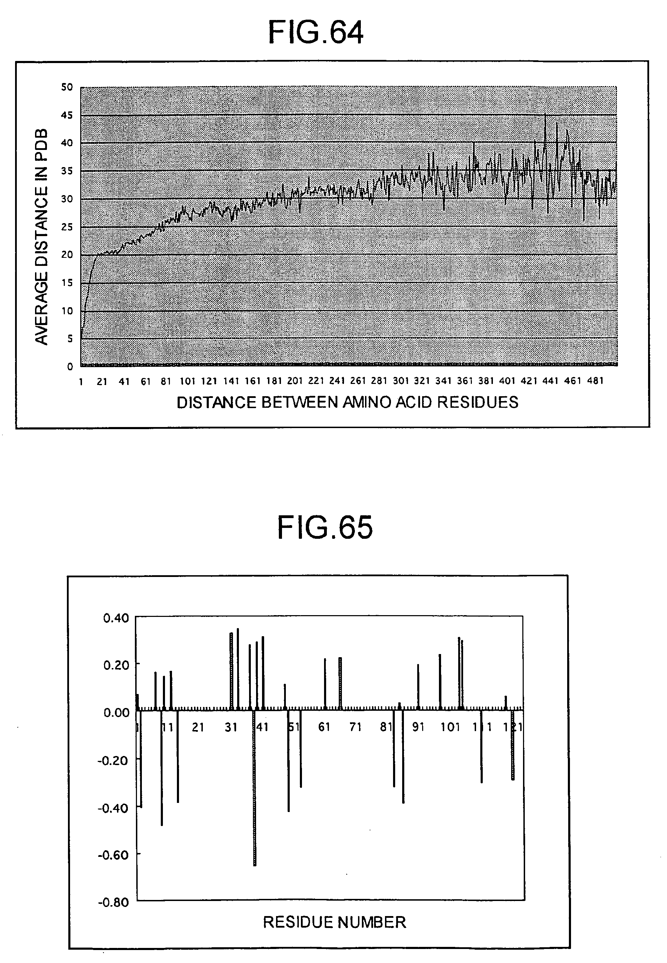

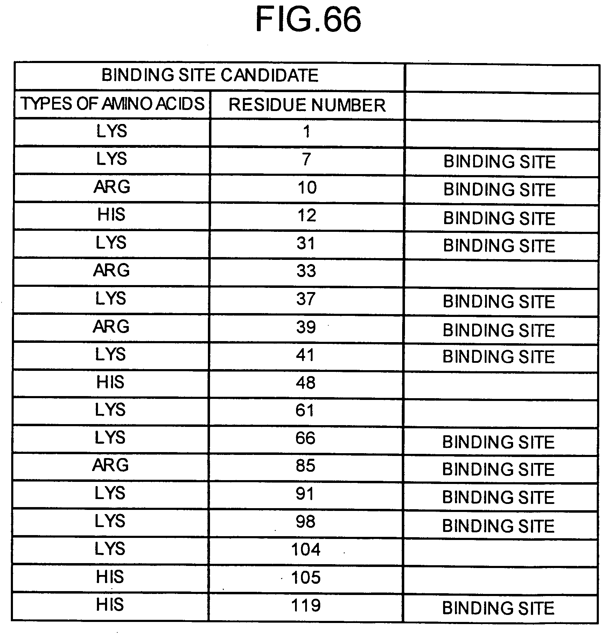

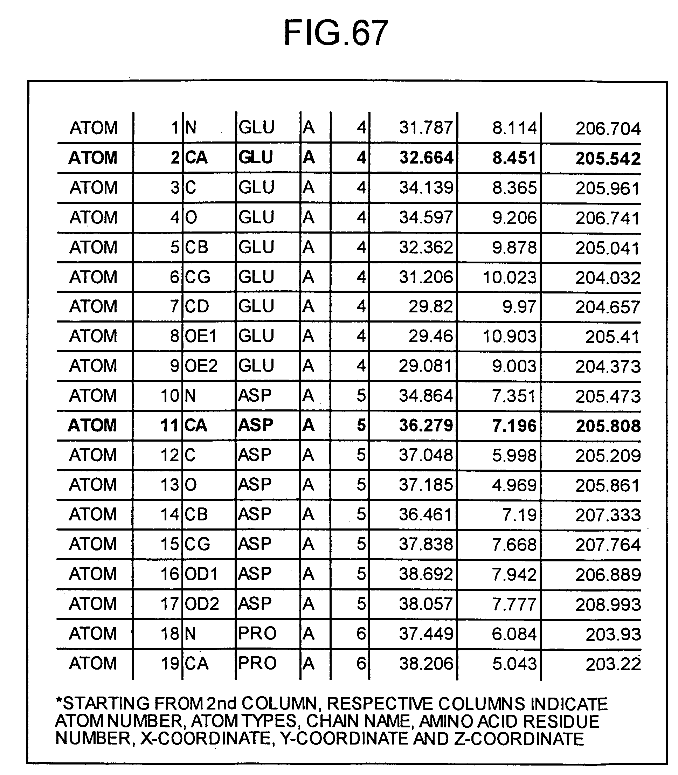

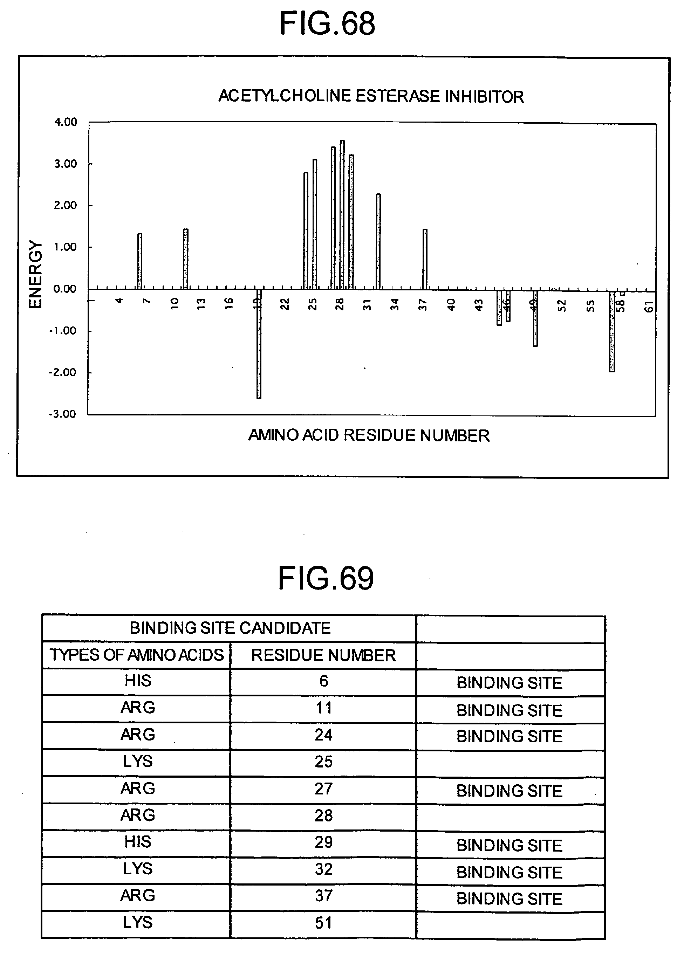

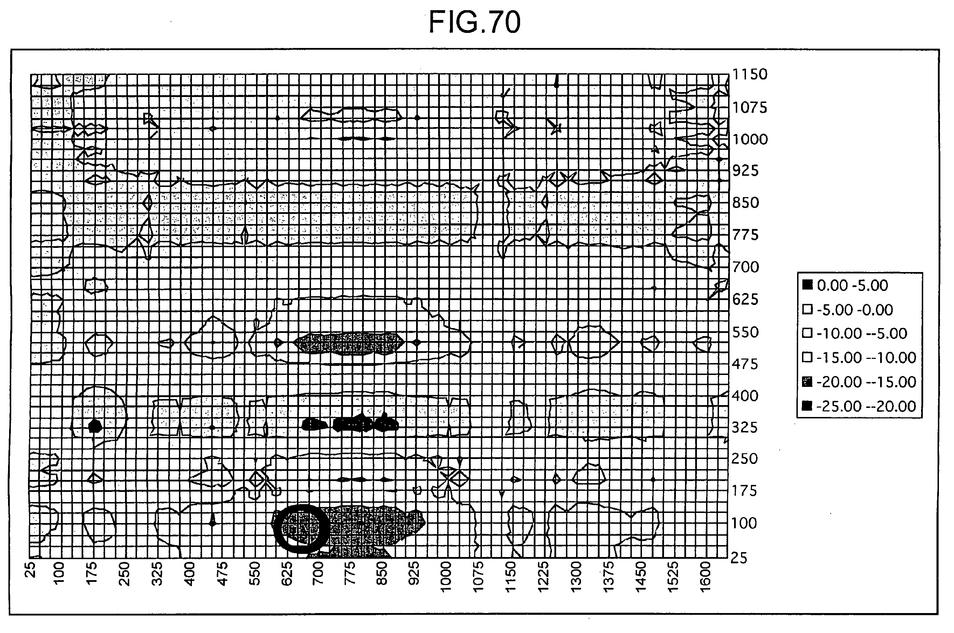

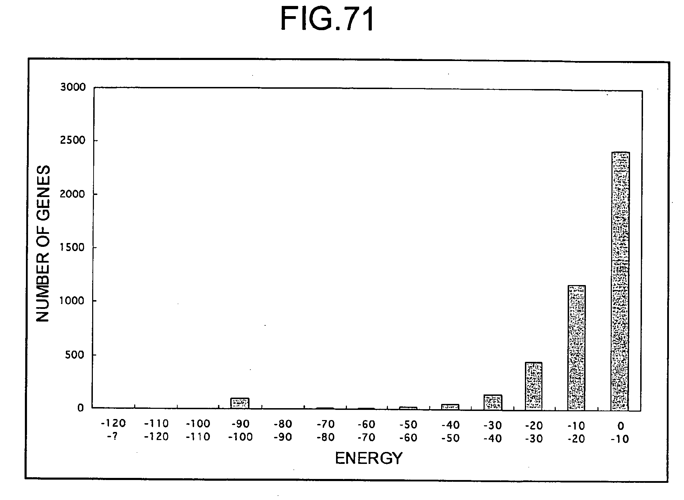

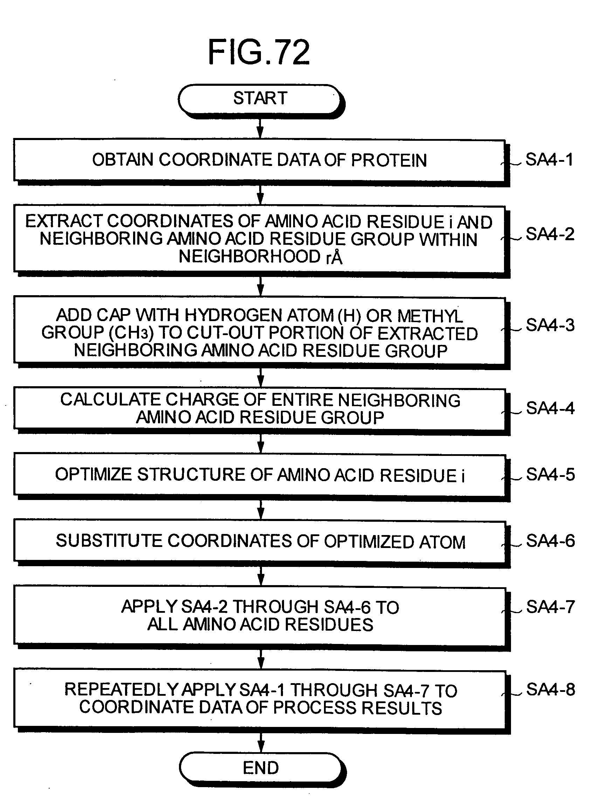

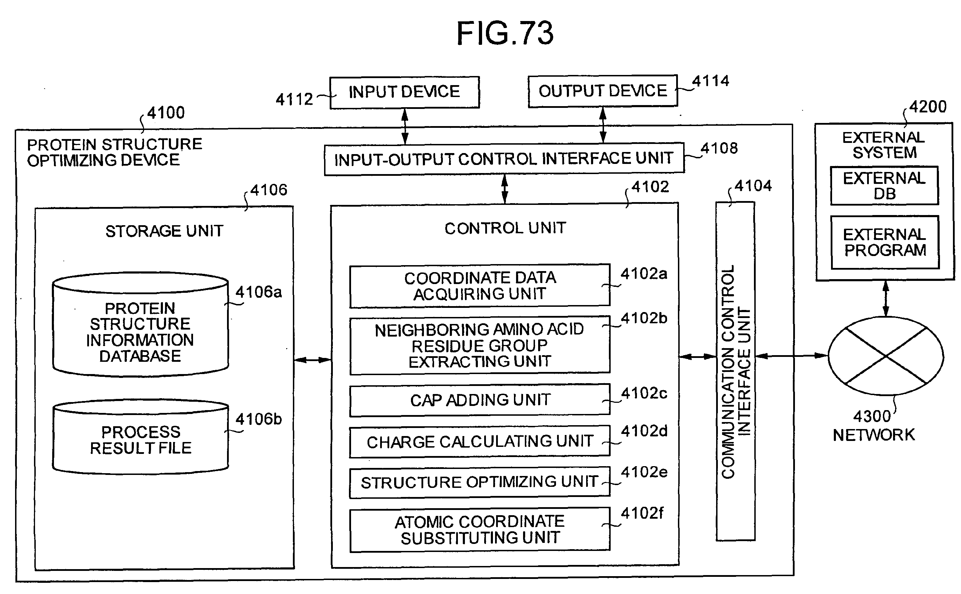

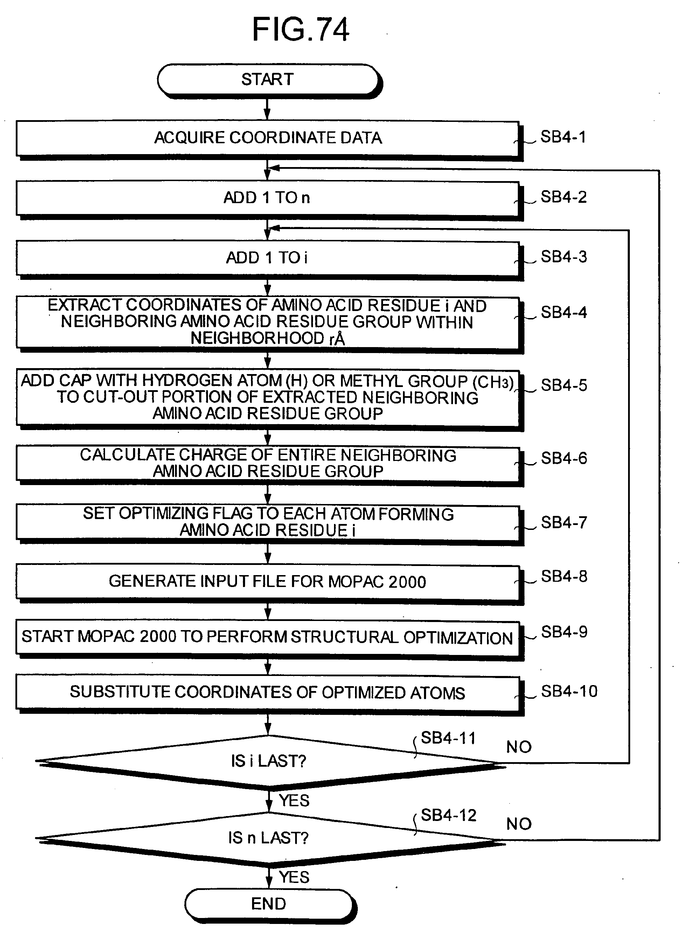

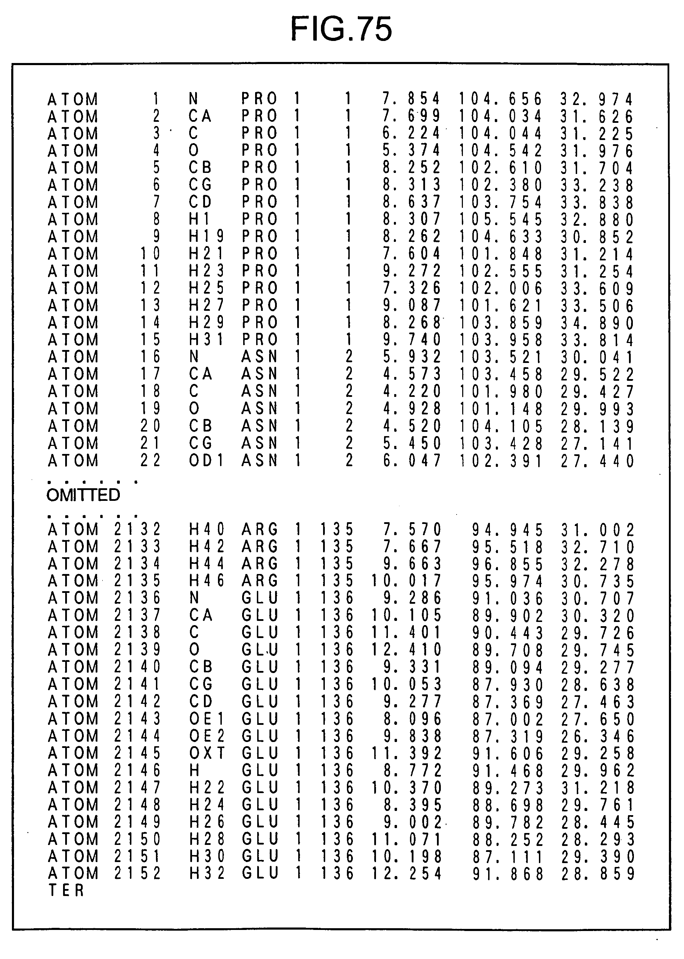

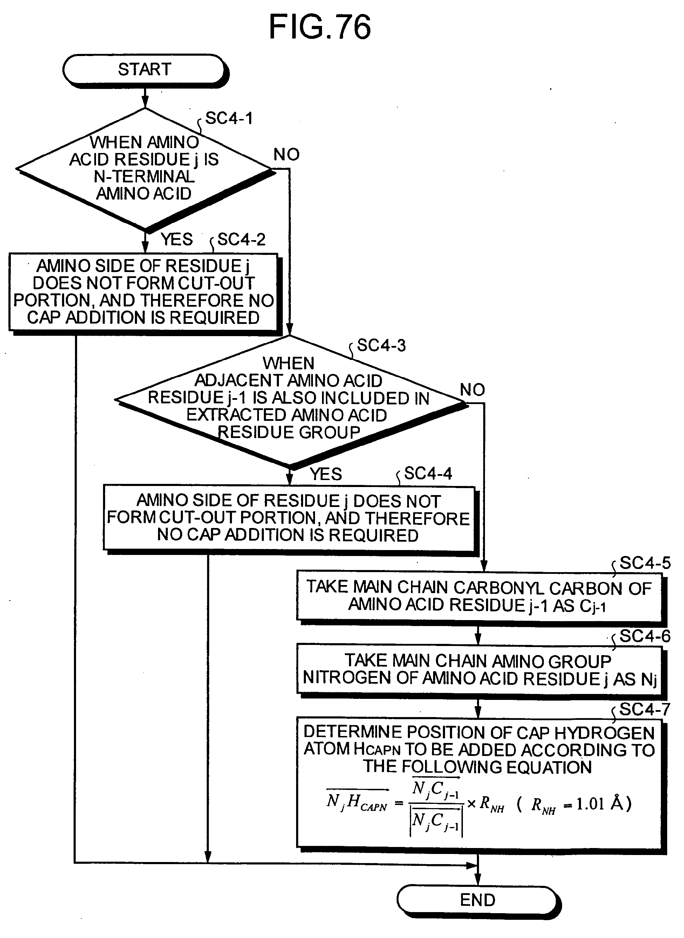

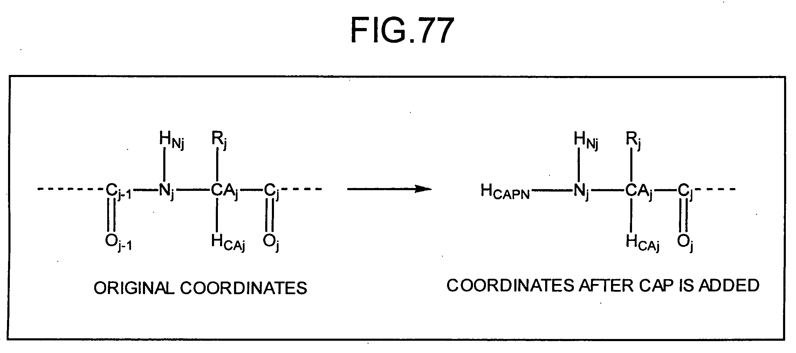

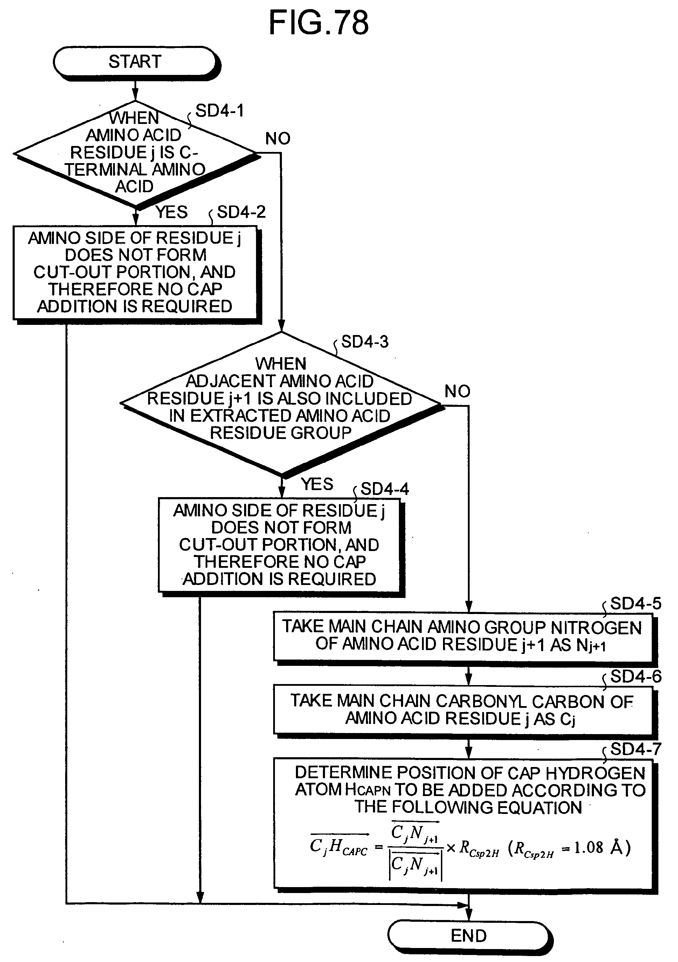

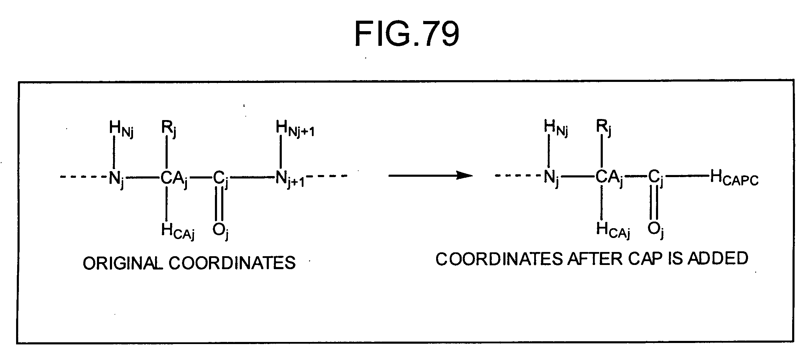

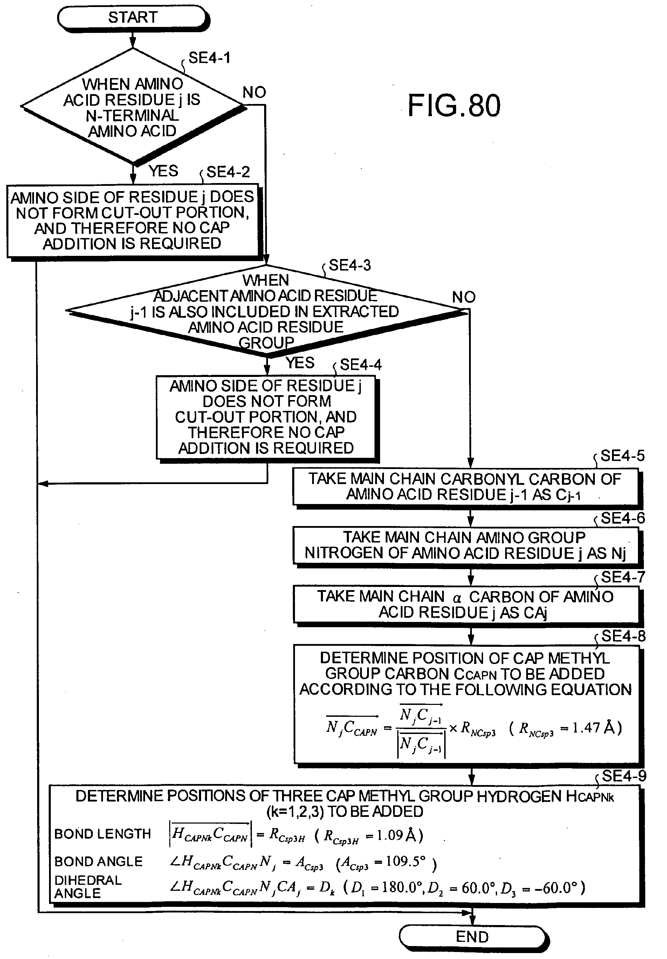

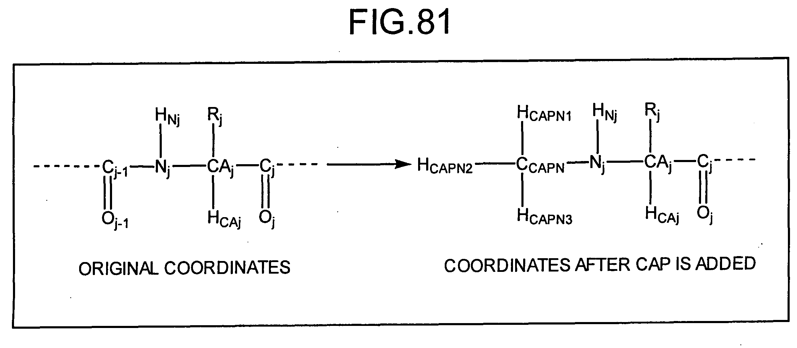

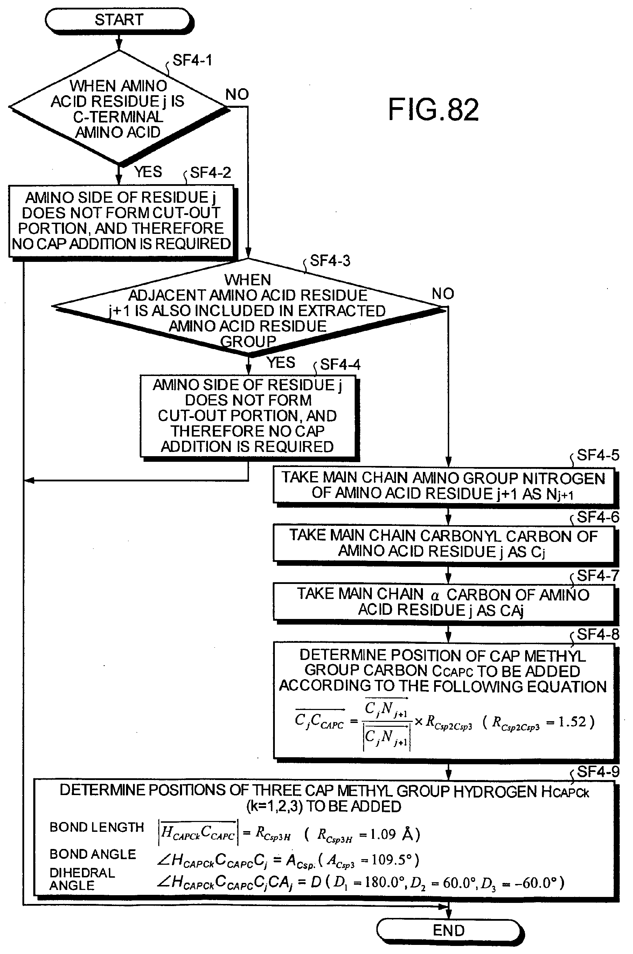

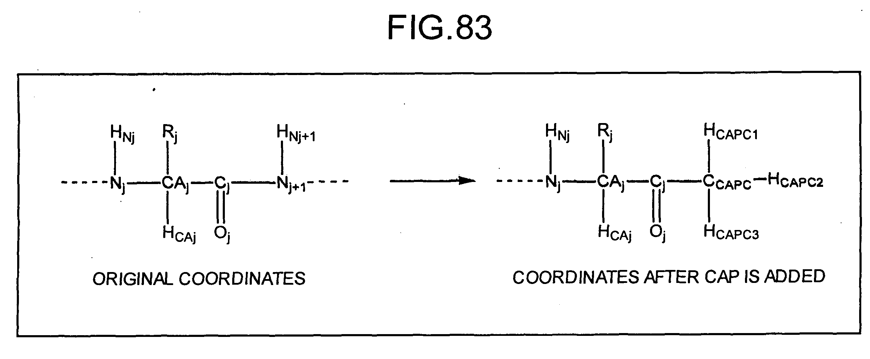

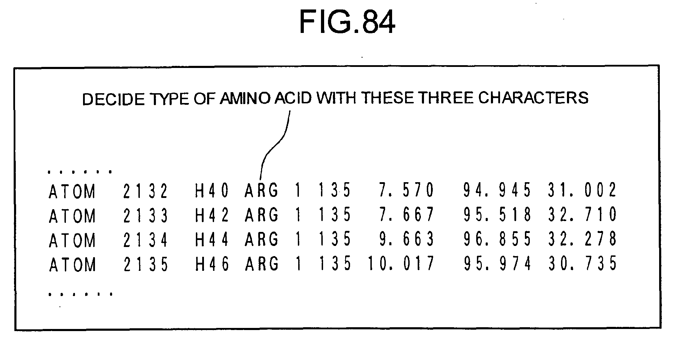

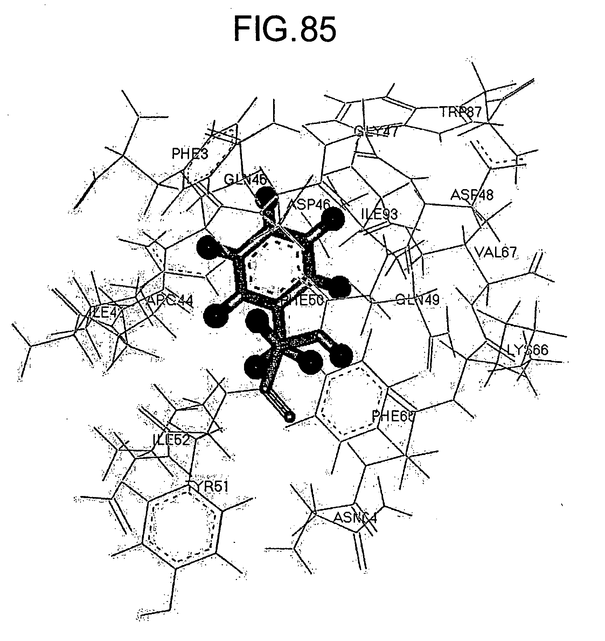

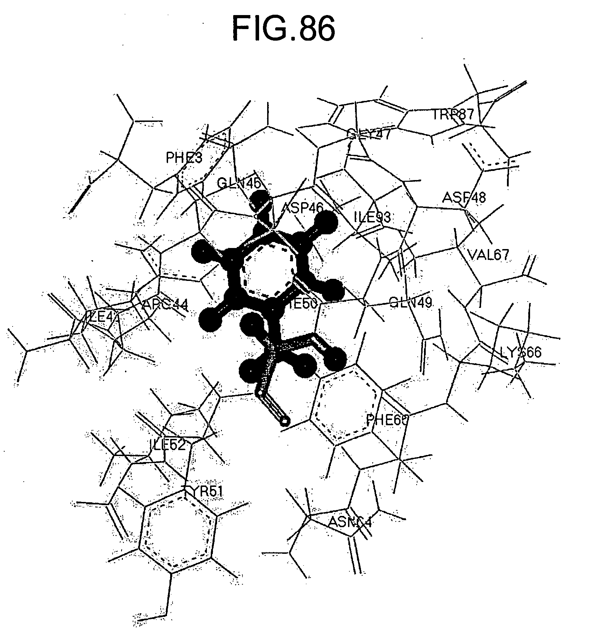

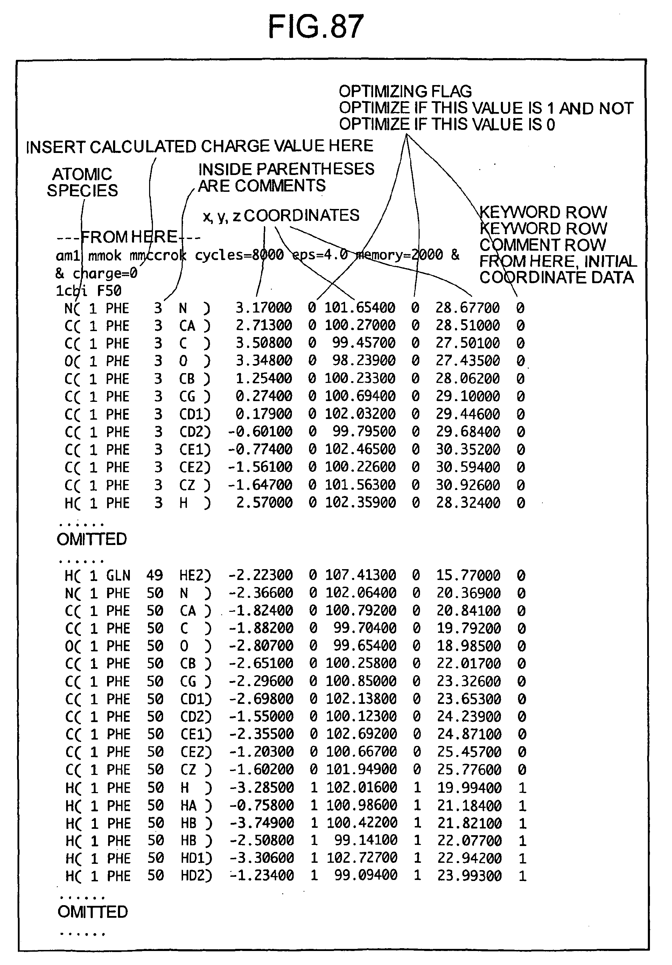

Fig. 1 is a principle block diagram that depicts a basic principle of the present invention; Fig. 2 is a block diagram that depicts one example of a structure of the present system to which the present invention is applied; Fig. 3 is a drawing that depicts an example of information to be stored in a prediction result data base 106a; Fig. 4 is a flow chart that depicts one example of a main process of the present system according to the present embodiment; Fig. 5 is a flow chart that depicts one example of a secondary structure data acquiring process of the present system according to the present embodiment; Fig. 6 is a flow chart that depicts one example of a frustration executing process that is executed by a frustration calculating unit 102e; Fig. 7 is a drawing that depicts one example of a display screen indicating interaction site prediction results displayed on an output device 114 of an interaction site predicting device 100; Fig. 8 is a drawing that depicts one example of a processing result output screen of the present embodiment displayed on a monitor of the interaction site predicting device 100; Fig. 9 is a drawing that is used for confirming whether a portion, which has been predicted as a portion having a high frustration through a known docking simulation, is actually functioning as an interaction site; Fig. 10 is a principle block diagram that depicts a basic principle of the present invention; Fig. 11 is a block diagram that depicts one example of a structure of the present system to which the present invention is applied; Fig. 12 is a block diagram that depicts one example of a structure of a frontier orbital calculating unit 1102a; Fig. 13 is a block diagram that depicts one example of a structure of an active site predicting unit 1102g; Fig. 14 is a flow chart that depicts one example of a main process of the present system according to the present embodiment; Fig. 15 is a flow chart that depicts one example of a molecular orbital computing process of the present system according to the present embodiment; Fig. 16 is a flow chart that depicts one example of a candidate amino acid residue determining process based upon a frontier orbital and its peripheral orbital of the present system according to the present embodiment; Fig. 17 is a flow chart that depicts one example of an attribution information determining process of respective molecular orbitals to amino acid of the present system according to the present embodiment; Fig. 18 is a flow chart that depicts one example of a candidate amino acid residue comparing process of the present system according to the present embodiment; Fig. 19 is a flow chart that depicts one example of a candidate amino acid residue determining process based upon orbital energy that is localized in heavy atoms in a main chain of the present system according to the present embodiment; Fig. 20 is a drawing that depicts one example of computed results obtained through a molecular orbital computing process; Fig. 21 is a drawing that depicts one example of a display screen used for confirming which position in a three-dimensional structure of protein a candidate amino acid residue is located; Fig. 22 is a drawing that depicts one example of computed results obtained through a molecular orbital computing process; Fig. 23 is a table that selectively depicts amino acid residues in which frontier orbitals of ribonuclease T1 are distributed in a first embodiment; Fig. 24 is a drawing in which orbital energies of molecular orbitals distributed on nitrogen atoms in a main chain are plotted in association with residue numbers of amino acid in the first embodiment; Fig. 25 is a table in which amino acid residues having high orbital energies are extracted and shown together with the orbital energies in a first embodiment; Fig. 26 is a table that selectively depicts candidate amino acid residues based on the frontier orbital shown in Fig. 23 in a first embodiment, candidate amino acid residues based on orbital energies of main chain atoms shown in Figs. 24 and 25, and common portions extracted from these residues according to the first embodiment; Fig. 27 is a table that depicts amino acid residues in which frontier orbitals of ribonuclease A are distributed in a second embodiment; Fig. 28 is a graph in which orbital energies of molecular orbitals distributed on nitrogen atoms in a main chain are plotted in association with residue numbers of amino acid in the second embodiment; Fig. 29 is a table that selectively depicts amino acid residues having high orbital energies and the orbital energies in the second embodiment; Fig. 30 is a table that depicts candidate amino acid residues based on the frontier orbital shown in Fig. 27, candidate amino acid residues based on orbital energies of main chain atoms shown in Figs. 28 and 29, and common portions extracted from these residues according to the second embodiment; Fig. 31 is a principle block diagram that depicts a basic principle of the present invention; Fig. 32 is a block diagram that depicts one example of a structure of the present system to which the present invention is applied; Fig. 33 is a flow chart that depicts one example of a main process of the present system according to the present embodiment; Fig. 34 is a flow chart that depicts one example of a solvent contact face specifying process of the present system according to the present embodiment; Fig. 35 is a flow chart that depicts one example of a hydrophobic face specifying process of the present system according to the present embodiment; Fig. 36 is a flow chart that depicts one example of an electrostatic interaction site specifying process of the present system according to the present embodiment; Fig. 37 is a flow chart that depicts one example of an interaction site specifying process of the present system according to the present embodiment; Fig. 38 is a flow chart that depicts one example of an interaction site predicting process of the present system according to the present embodiment; Fig. 39 is a processing diagram in which a protein interaction information processing device 100 calculates a difference ∆S in solvent contact areas for each of amino acid residues with respect to barnase based upon a crystal structure of a barnase-barstar composite body through processes of a solvent contact face specifying unit 102b; Fig. 40 is a processing diagram in which the protein interaction information processing device 100 calculates a hydrophobic interaction energy for each of amino acid residues with respect to barnase based upon a crystal structure of barnase as a single substance through processes of a hydrophobic face specifying unit 102c; Fig. 41 is a processing diagram in which the protein interaction information processing device 100 calculates an electrostatic interaction energy for each of amino acid residues with respect to barnase based upon a crystal structure of barnase as a single substance through processes of an electrostatic interaction specifying unit 102d; Fig. 42 is a processing diagram in which a protein interaction information processing device 100 calculates a difference ∆S in solvent contact areas for each of amino acid residues with respect to barstar based upon a crystal structure of a barnase-barstar composite body through processes of the solvent contact face specifying unit 102b; Fig. 43 is a processing diagram in which the protein interaction information processing device 100 calculates a hydrophobic interaction energy for each of amino acid residues with respect to barstar based upon a crystal structure of barstar as a single substance through processes of the hydrophobic face specifying unit 102c; Fig. 44 is a processing diagram in which the protein interaction information processing device 100 calculates an electrostatic interaction energy for each of amino acid residues with respect to barstar based upon a crystal structure of barstar as a single substance through processes of the electrostatic interaction specifying unit 102d; Fig. 45 is a processing diagram in which the protein interaction information processing device 100 calculates a difference ∆S in solvent contact areas for each of amino acid residues with respect to Ribonuclease based upon a crystal structure of a Ribonuclease-inhibitor composite body through processes of the solvent contact face specifying unit 102b; Fig. 46 is a processing diagram in which the protein interaction information processing device 100 calculates a hydrophobic interaction energy for each of amino acid residues with respect to Ribonuclease based upon a crystal structure of Ribonuclease as a single substance through processes of the hydrophobic face specifying unit 102c; Fig. 47 is a processing diagram in which the protein interaction information processing device 100 calculates an electrostatic interaction energy for each of amino acid residues with respect to Ribonuclease based upon a crystal structure of Ribonuclease as a single substance through processes of the electrostatic interaction specifying unit 102d; Fig. 48 is a processing diagram in which the protein interaction information processing device 100 calculates a difference ΔS in solvent contact areas for each of amino acid residues with respect to inhibitor based upon a crystal structure of a Ribonuclease-inhibitor composite body through processes of the solvent contact face specifying unit 102b; Fig. 49 is a processing diagram in which the protein interaction information processing device 100 calculates a hydrophobic interaction energy for each of amino acid residues with respect to inhibitor based upon a crystal structure of inhibitor as a single substance through processes of the hydrophobic face specifying unit 102c; Fig. 50 is a processing diagram in which the protein interaction information processing device 100 calculates an electrostatic interaction energy for each of amino acid residues with respect to inhibitor based upon a crystal structure of inhibitor as a single substance through processes of the electrostatic interaction specifying unit 102d; Fig. 51 is a drawing that explains the concept by which the present invention predicts binding sites of a protein based upon the amino acid sequence information of the protein; Fig. 52 is a drawing that explains the concept by which the present invention predicts binding sites based upon the amino acid sequence information of a plurality of proteins when a composite body is formed by using those proteins; Fig. 53 is a block diagram that depicts one example of a structure of the present system to which the present invention is applied; Fig. 54 is a block diagram that depicts one example of a structure of a space distance determining unit 3102b to which the present invention is applied; Fig. 55 is a block diagram that depicts one example of a structure of an energy calculating unit 3102d to which the present invention is applied; Fig. 56 is a drawing that depicts the concept of a high-speed computing method according to the present invention; Fig. 57 is a drawing that depicts the concept to be used upon assuming a binding residue on a plurality of amino acid sequences; Fig. 58 is a drawing that explains the concept of a target residue; Fig. 59 is a flow chart that depicts one example of processes of the present system according to the present embodiment; Fig. 60 is a drawing that depicts one example of energy, etc. of candidate amino acid residues as the process results; Fig. 61 is a drawing that depicts one example of a case in which unstable portions are clustered in a three-dimensional structure; Fig. 62 is a drawing that depicts the concept to be used for forming a composite body structure by using docking simulations; Fig. 63 depicts one example of a drawing on which the total sum of energies is plotted in the case when respective amino acid residues of protein A and protein B are used as binding residues; Fig. 64 is a drawing that depicts a relationship between the sequential distance and the spatial distance between two glutamic acids; Fig. 65 is a drawing on which energies of respective amino acid residues of Ribonuclease A are plotted in association with amino acid residue numbers; Fig. 66 is a drawing in which those amino acid residues of Ribonuclease A having energy of not less than 0 are listed up as binding sites candidates; Fig. 67 is a drawing that depicts a part of three-dimensional structure information data of an acetylcholine-esterase-inhibitor stored in a PDB; Fig. 68 is a drawing that depicts an energy of an acetylcholine-esterase-inhibitor found by the present invention; Fig. 69 is a drawing that depicts the results of experiments in which ten of those acetylcholine-esterase-inhibitors having energy of not less than 0 are extracted as binding site candidates and examined as to whether those points actually form binding sites; Fig. 70 is a drawing in which amino acid residue numbers corresponding to binding sites of huntingtin-associated protein interacting protein are plotted on the axis of abscissa and amino acid residue numbers corresponding to binding sites of nitric oxide synthase 2A are plotted on the axis of ordinate so that the total sum of energies upon forming a composite body at the respective binding sites is indicated as contour lines; Fig. 71 is a histogram relating to interaction energies of respective candidate proteins and the number of genes; Fig. 72 is a flow chart that depicts a basic principle of the present invention; Fig. 73 is a block diagram that depicts one example of a structure of the present system to which the present invention is applied; Fig. 74 is a flowchart that depicts one example of main processes of the present system according to the present embodiment; Fig. 75 is a drawing that depicts one example of coordinate data of protein; Fig. 76 is a flow chart that depicts one example of a cap adding process in which hydrogen atoms are applied to a cut-out portion, according to the present embodiment; Fig. 77 is a drawing that depicts the concept of coordinates between the original coordinate and the coordinate after addition of a cap substituent; Fig. 78 is a flow chart that depicts one example of the cap adding process in which hydrogen atoms are applied to a cut-out portion, according to the present embodiment; Fig. 79 is a drawing that depicts the concept of coordinates between the original coordinate and the coordinate after addition of a cap substituent; Fig. 80 is a flow chart that depicts one example of a cap adding process in which a methyl group is applied to a cut-out portion, according to the present embodiment; Fig. 81 is a drawing that depicts the concept of coordinates between the original coordinate and the coordinate after addition of a cap substituent; Fig. 82 is a flow chart that depicts one example of the cap adding process in which a methyl group is applied to a cut-out portion, according to the present embodiment; Fig. 83 is a drawing that depicts the concept of coordinates between the original coordinate and the coordinate after addition of a cap substituent; Fig. 84 is a drawing that explains the concept that is used upon distinguishing the amino acid type by using a three-character notation of PDB format data (character of 18-20 columns); Fig. 85 is a drawing that depicts one example in which an optimizing flag is set to hydrogen atoms of an amino acid residue i; Fig. 86 is a drawing that depicts one example in which an optimizing flag is set to hydrogen atoms and side chain atoms of the amino acid residue i; Fig. 87 is a drawing that depicts one example of an input file of MOPAC 2000;

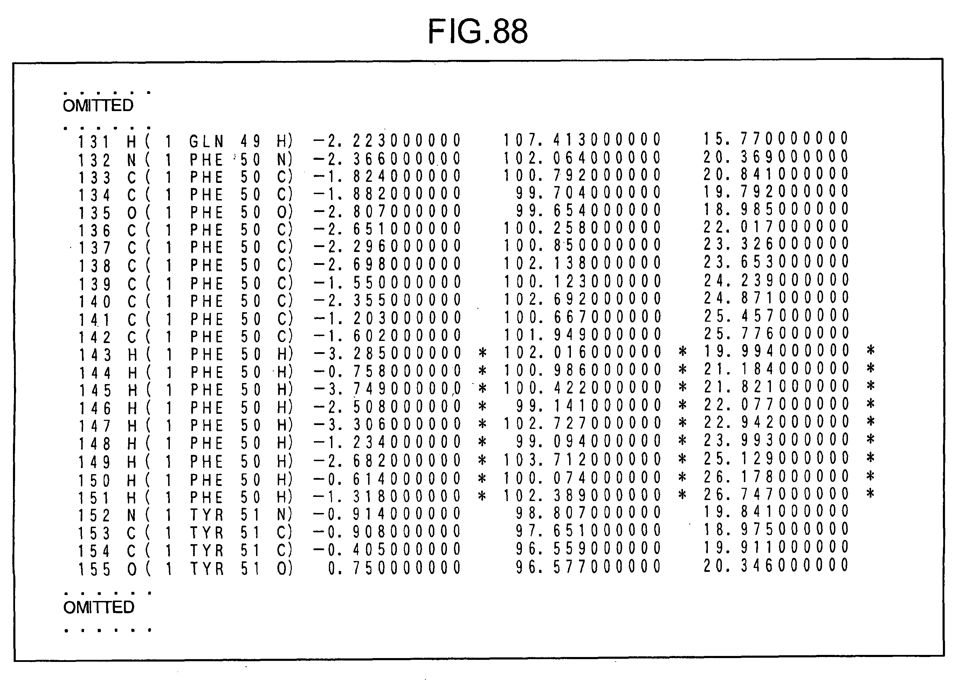

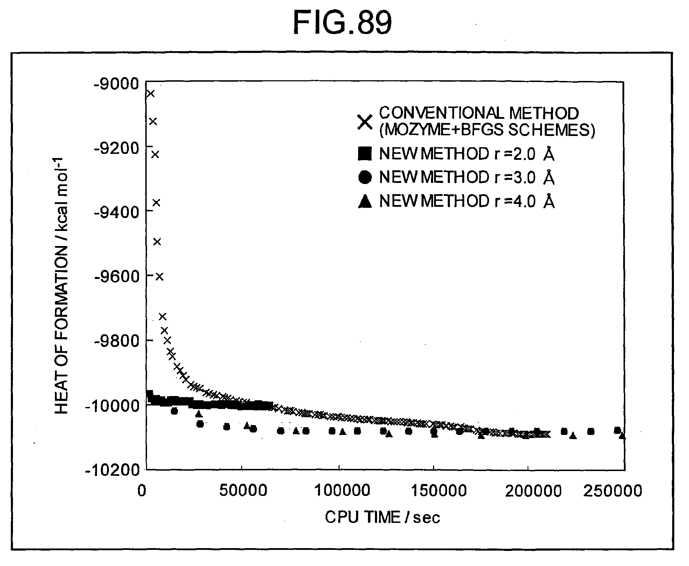

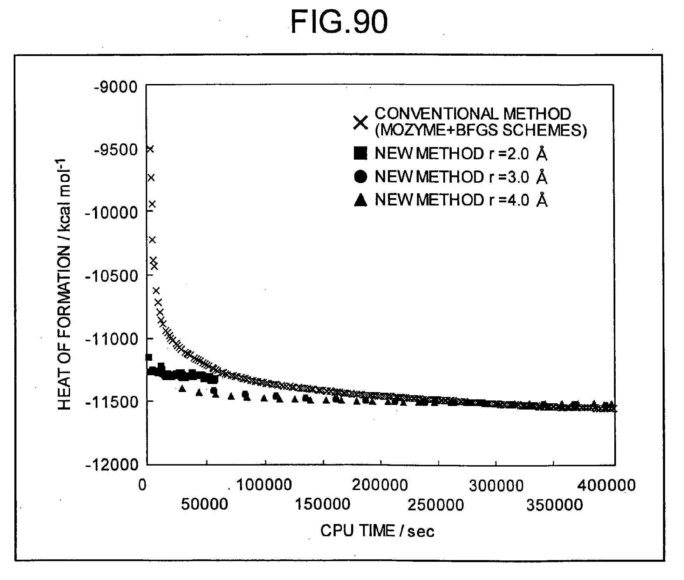

Fig. 88 is a drawing that depicts one example of an output file that indicates the results of a structure-optimizing process by MOPAC 2000; Fig. 89 is a drawing that depicts calculation results of cases in which a hydrogen structure is optimized through a conventional optimizing method (MOZYME method + BFGS method) and in which it is optimized by a method of the present invention; and Fig. 90 is a drawing that depicts calculation results of cases in which a side chain structure is optimized through a conventional optimizing method (MOZYME method + BFGS method) and in which it is optimized via a method of the present invention.

BEST MODE FOR CARRYING OUT THE INVENTION

(1) Referring to Figures, the following description will discuss embodiments of an interaction site predicting device, an interaction site predicting method, a program and a recording medium, according to the present invention, in detail. However, the present invention is not intended to be limited by these embodiments.

[Overview of the present invention]

The following description will first discuss the overview of the present invention, and the structure, processes and the like of the present invention will be explained later in detail. Fig. 1 is a principle block diagram that depicts a basic principle of the present invention.

Schematically, the present invention has the following basic features. First, the user inputs objective sequence data 10 that is primary sequence information of a target protein to an interaction site predicting device of the present invention. The user may input the objective sequence data 10, for example, by selecting primary sequence information registered in an external data base such as SWISS-PROT, PIR and TrEMBL, or may directly input desired primary sequence information.

Next, the interaction site predicting device of the present invention executes secondary structure predicting simulations on the objective sequence data 10 that have been inputted to secondary structure prediction programs 20a to 20d, which predict the secondary structure of the protein from the primary sequence information of the protein. Here, the secondary structure programs 20a to 20d execute the secondary structure predicting simulations by utilizing, for example, Chou-Fasman technique, a technique using a neural network, a technique using linear statistics and a technique using a nearest neighbor method.

Next, the interaction site predicting device of the present invention compares the secondary structure prediction results 30a to 30d of the respective secondary structure prediction programs 20a to 20d with each other (60). In other words, the execution results of the respective prediction programs corresponding to objective sequence data 61 are placed side by side and compared with each other (63 to 66).

Further, based upon these comparison results, the interaction site predicting device of the present invention calculates the frustration of localized portions of the primary sequence information of the target protein (70). In other words, localized portions that have predicted different secondary structures in the respective prediction result data (63 to 66) are extracted from the comparison results, and the frustration of these portions is calculated. In the known secondary structure prediction programs 20a to 20d, predicting processes are basically carried out by viewing one portion of the localized sequence of the primary sequence information; however, since the secondary structure is finally determined in association with the entire structure of the protein, in portions that have no matching between the entire portion and the localized portion, that is, in localized portions having a large frustration, the secondary structure prediction results tend to fail to hit the mark. Therefore, with respect to localized portions in which the prediction results fail to hit the mark in a plurality of programs, it is possible to estimate that these portions have a greater frustration.

With respect to the calculation method for frustration, the frustration may be increased or reduced according to the number of secondary structure prediction programs that have outputted different prediction result data, or the frustration may be increased or reduced according to the average value, the dispersion value or the like of the certainty factor in each of the structures having the different prediction results; alternatively, with respect to the amino acid sequence of the corresponding portion, a quantity of energy is found by using a technique derived from molecular dynamics or molecular kinetics so that the frustration may be calculated by using the quantity of energy.

Thus, the interaction site predicting device of the present invention predicts the interaction site of the target protein based upon calculated frustration of the localized portions (80). In other words, for example, the localized portions (67) having frustration exceeding a predetermined threshold value are predicted as interaction sites.

Moreover, when the secondary structure data of the target protein is registered in an external data base such as PDB and SCOP, the interaction site predicting device of the present invention acquires the secondary structure data 40, and uses the data upon comparing the prediction results (60). In other words, the secondary structure data 62 that actually correspond to the target protein are compared with the prediction result data 63 to 66 of the prediction programs.

With respect to portions in which the actual secondary structure data 62 and the prediction result data 63 to 66 of the prediction programs are different from each other, higher frustration is calculated. When the three-dimensional structure data of the protein has been known, that is, when the protein has its three-dimensional structure data registered in an existing PDB or the like, since the entire structure has been known, a localized portion (a portion having a higher probability to be an interaction site) having a frustration can be found more accurately by examining differences between the prediction results of various secondary structure predicting methods and the actual structure thereof. For example, the frustration may be increased or reduced according to the number of the secondary structure prediction programs that have outputted prediction result data that are different from the actual secondary structure data 62.

Moreover, the interaction site predicting device of the present invention is designed to set certainty factor information 50 that indicates the certainty factor with respect to the secondary structure predicting result data 30a to 30d of the secondary structure prediction programs 20a to 20d. In other words, the simulation precision of the secondary structure prediction programs 20a to 20d is set based upon actual secondary structure data and the like.

Furthermore, based upon the preset certainty factor information and the comparison results, the interaction site predicting device of the present invention calculates the frustration in the localized portion. In other words, by placing a higher weight on the secondary structure prediction result data derived from a program having higher certainty factor information (that is, higher precision in simulation), the certainty factor with respect to the simulation results can be reflected in the frustration calculation.

[System structure]

First, the following description will discuss the structure of the present system. Fig. 2, which is a block diagram that depicts one example of the structure of the present system to which the present invention is applied, conceptually indicates only the parts of the system relating to the present invention. Schematically, the present system includes an interaction site predicting device 100 and an external system 200 that provides external data bases relating to sequence information, three-dimensional structures and the like and external programs relating to homology retrieving, secondary structure predictions and the like, which are communicably connected to each other through a network 300.

In Fig. 2, the network 300, which has a function for mutually connecting the interaction site predicting device 100 and the external system 200, is provided as, for example, the Internet.

In Fig. 2, the external system 200, which is mutually connected to the interaction site predicting device 100 through the network 300, has functions for providing external data bases relating to sequence information, three-dimensional structures and the like and Web sites that execute external programs relating to homology retrieving, motif retrieving, secondary structure predictions and the like to the user.

Here, the external system 200 may be prepared as WEB servers, ASP servers and the like, and, in general, its hardware structure may be constituted by information processing apparatuses, such as commercially available work stations and personal computers with attached devices thereof. Moreover, the respective functions of the external system 200 can be achieved by a CPU, a disk device, a memory device, an input device, an output device, a communication controlling device and the like in the hardware structure in the external system 200 and programs and the like that control these devices.

In Fig. 2, schematically, the interaction site predicting device 100 includes a control unit 102 such as a CPU that systematically controls the entire interaction site predicting device 100, a communication control interface unit 104 that is connected to communication devices (not shown) such as routers that are connected to communication lines and the like, an input-output control interface unit 108 that is connected to an input device 112 and an output device 114, and a storage unit 106 that stores various data bases and tables (prediction result data base 106a to protein structure data base 106c), and these respective units are communicably connected to one another through communication paths. Moreover, the interaction site predicting device 100 is communicably connected to the network 300 through communication devices such as routers and wire or wireless communication lines such as dedicated lines.

In Fig. 2, various data bases and tables (prediction result data base 106a to protein structure data base 106c) to be stored in the storage unit 106 are prepared as storage units such as a fixed disk device, and store various programs used for various processes, files, data bases, files for use in Web pages and the like.

Among these constituent elements of the storage unit 106, the prediction result data base 106a serves as a prediction result information storage unit which stores information relating to prediction results of a secondary structure prediction program. Fig. 3 is a drawing that depicts one example of information to be stored in the prediction result data base 106a.

As shown in Fig. 3, pieces of information to be stored in the prediction result data base 106a include objective sequence data serving as primary sequence information (amino acid sequence information) of a target protein, secondary structure data of the objective sequence data obtained from the protein structure data base and prediction result data of respective secondary structure prediction programs, which are mutually associated with one another.

Moreover, a certainty factor information data base 106b serves as a prediction result information storage unit which stores certainty factor information that indicates the certainty factor with respect to the secondary structure prediction result data of the secondary structure prediction program. For example, provided that the certainty factor of a standard value of precision in the simulation result is 1 (for example, when simulation precision, which is a rate of coincidence between the secondary structure predicting result and the actual secondary structure data, is 60 %), when the precision is higher than the standard value, the value of the certainty factor may be made greater according to the precision, and when the precision is lower than the standard value, the value of the certainty factor may be made lower than the standard value according to the precision. Furthermore, the certainty factor may be set for each of the secondary structure programs, for each of the structures and for each of the sequences. In other words, for example, when a secondary structure prediction program predicts its secondary structure of a certain amino acid having a certain sequence, the certainty factor indicating the probability that the structure is an α-structure and the certainty factor indicating the probability that the structure is a β-structure may be respectively set differently.

Here, the protein structure data base 106c is a data base in which three-dimensional structure data of protein are stored. The protein structure data base 106c may be provided as an external protein structure data base that is accessed through the Internet, or may be prepared as an in-house data base that is formed by copying the data bases, storing original sequence information and adding original annotation information and the like.

Moreover, in Fig. 2, the communication control interface unit 104 carries out a communication control between the interaction site predicting device 100 and the network 300 (or communication devices such as routers). In other words, the communication control interface unit 104 has functions for carrying out data communications with other terminals through communication lines.

Furthermore, in Fig. 2, the input-output control interface unit 108 controls the input device 112 and the output device 114. Here, the output device 114 may be prepared as a speaker in addition to a monitor (including a home-use television)(in the following description, the output device is described as a monitor). The input device 112 may be prepared as a keyboard, a mouse, a microphone and the like. Here, the monitor is also allowed to function as a pointing device in cooperation with a mouse.

In Fig. 2, the control unit 102 is provided with an internal memory for storing control programs such as an OS (Operating System), programs that control various processing procedures, and required data, and these programs and the like are used to carry out information processes to execute various processes. Functionally, the control unit 102 is provided with an objective sequence input unit 102a, a secondary structure prediction program executing unit 102b, a secondary structure prediction program 102c, a prediction result comparing unit 102d, a frustration calculating unit 102e, an interaction site predicting unit 102f, a secondary structure data acquiring unit 102g and a certainty factor information setting unit 102h.

Among these, the objective sequence input unit 102a serves as an input unit used for inputting primary sequence information (objective sequence data) of a target protein. Here, the secondary structure prediction program executing unit 102b serves as a secondary structure prediction program executing unit used for executing secondary structure predicting simulations for the primary sequence information (objective sequence data) inputted to the secondary structure prediction program through the input unit. Moreover, the secondary structure prediction program 102c serves as a secondary structure prediction program used for predicting the secondary structure of the protein from the primary sequence information of the protein.

Furthermore, the prediction result comparing unit 102d serves as a prediction result comparing unit that compares the results of secondary structure prediction of the secondary structure prediction program, and also serves as a prediction result comparing unit that compares the secondary structure prediction results of the secondary structure prediction program with the secondary structure data acquired by the secondary structure data acquiring unit. Here, the frustration calculating unit 102e serves as a frustration calculating unit that calculates the frustration in localized portions in the primary sequence information (objective sequence data) of the target protein based upon the comparison results of the prediction result comparing unit, and also serves as a frustration calculating unit that calculates the frustration of localized portions based upon the certainty factor information set by the certainty factor information setting unit and the comparison results.

Here, the interaction site predicting unit 102f serves as an interaction site predicting unit that predicts an interaction site of the target protein based upon the frustration of the localized portions calculated by the frustration calculating unit. Moreover, the secondary structure data acquiring unit 102g serves as a secondary structure data acquiring unit that acquires the secondary structure data of the target protein. Furthermore, the certainty factor information setting unit 102h serves as a certainty factor information setting unit that sets certainty factor information indicating the certainty factor with respect to the secondary structure prediction results of the secondary structure prediction program. With respect to the processes to be carried out by these respective units, the detailed description thereof will be given later.

[System processes]

Next, referring to Figs. 4 to 7, the following description will discuss one example of processes of the present system according to the present embodiment having the above-mentioned arrangement.

[Main processes]

Referring to Fig. 4, the following description will discuss main processes in detail. Fig. 4 is a flow chart that depicts one example of main processes of the present system according to the present embodiment.

First, the interaction site predicting device 100 allows the user to input primary sequence information (objective sequence data) of a target protein through processes in the objective sequence input unit 102a (step SA-1).

Next, the interaction site predicting device 100 acquires secondary structure data of the objective sequence data inputted by the user through processes in the secondary structure data acquiring unit 102g (step SA-2).

Here, referring to Fig. 5, the following description will discuss the secondary structure data acquiring processes executed by the secondary structure data acquiring unit 102g in step SA-2 in detail. Fig. 5 is a flow chart that depicts one example of the secondary structure data acquiring processes of the present system according to the present embodiment.

First, referring to the protein structure data base 106c, the secondary structure data acquiring unit 102g determines whether the objective sequence data has been registered (step SB-1). In step SB-1, when the objective sequence date is registered in the protein structure data base 106c, the secondary structure data acquiring unit 102g acquires the secondary structure data of the objective sequence data from the protein structure data base 106c, and stores the acquired data in a predetermined storing area of the prediction result data base 106a (step SB-2).

In contrast, when, in step SB-1, the objective sequence data is not registered in the protein structure data base 106c, the secondary structure data acquiring unit 102g determines whether secondary structure data of a protein having a sequence similar to the objective sequence data is present in the protein structure data base 106c (step SB-3). In other words, by using, for example, a program for determining homology between the sequences, the secondary structure data acquiring unit 102g compares the objective sequence data with sequence data corresponding to protein having a known structure registered in the protein structure data base 106c, and determines whether there is sequence data (which may correspond to one portion of the objective sequence data) that has high homology to the target data.

At step SB-3, when secondary structure data of a protein having a sequence similar to the objective sequence data is present in the protein structure data base 106c, the secondary structure data acquiring unit 102g stores the secondary structure data of the similar portion in a predetermined storing area in the prediction result data base 106a (step SB-4). When the secondary structure data is present for one portion of the objective sequence data, the secondary structure data relating to the portion is stored in the prediction result data base 106a.

In contrast, at step SB-3, when no secondary structure data of a protein having a sequence similar to the objective sequence data is present in the protein structure data base 106c, the secondary structure data acquiring processes are completed.

Referring to Fig. 4 again, the interaction site predicting device 100 allows one or two or more secondary structure prediction programs 102c to execute the objective sequence data through processes of the secondary structure prediction program executing unit 102b (step SA-3). For example, the secondary structure prediction program executing unit 102b converts the objective sequence data to a predetermined format or adds predetermined header information and the like to the objective sequence data, so that the input formats of the respective secondary structure prediction programs 102c are matched with each other, and executes the secondary structure programs 102c. Here, the secondary structure prediction programs 102c may be programs located inside the interaction site predicting device 100, or external programs in the external system 200 that can be remote-controlled through the network 300.

Next, the secondary structure prediction program executing unit 102b stores the secondary structure prediction results that are simulation results of the respective secondary structure prediction programs 102c in a predetermined storing area in the prediction result data base 106a (step SA-4).

Next, the interaction site predicting device 100 compares the secondary structure prediction results of the respective secondary structure prediction programs 102c with respect to the objective sequence data stored in the prediction result data base 106a through processes in the prediction result comparing unit 102d (step SA-5). Specifically, the prediction result comparing unit 102d compares the respective prediction results from the leading portion to the last portion of the objective sequence data with respect to the secondary structure prediction results of the respective secondary structure prediction programs 102c. Here, at step SA-2, when the secondary structure prediction program executing unit 102b can acquire the secondary structure data corresponding to the objective sequence data, that is, when the secondary structure data of the objective sequence data is stored in the prediction result data base 106a, the secondary structure data is compared with the secondary structure prediction results of the respective secondary structure prediction programs 102c.

Next, the interaction site predicting device 100 calculates the score of frustration in localized portions of the objective sequence data through processes in the frustration calculating unit 102e (step SA-6). Here, Fig. 6 is a flow chart that depicts one example of frustration execution processes to be executed by the frustration calculating unit 102e of the present system.

As shown in Fig. 6, in the computing method of the score of frustration by the frustration calculating unit 102e, for example, with respect to the localized portions on which the secondary structure prediction programs have outputted different secondary structure prediction results, the score may be increased or reduced according to the number of secondary structure prediction programs that have outputted different prediction results, or the frustration may be increased or reduced according to the average value, the dispersion value or the like of the certainty factor in each of the structures having the different production results; alternatively, with respect to the localized portions on which the secondary structure prediction programs have outputted different secondary structure prediction results, a quantity of energy of the amino acid sequence may be found by using a technique derived from molecular dynamics or molecular kinetics so that the frustration may be calculated by using the quantity of energy (step SC-1).

Moreover, the frustration calculating unit 102e may calculate a high score in frustration with respect to portions on which the secondary structure data and the secondary structure prediction results of the prediction programs are different from each other (step SC-2). For example, the score may be increased or reduced according to the number of the secondary structure prediction programs that have outputted secondary structure prediction results different from the secondary structure data.

Furthermore, referring to the certainty factor information data base 106b, the frustration calculating unit 102e may acquire the certainty factor information of the respective secondary structure prediction programs 102c previously stored through the processes by the certainty factor information setting unit 102h, and may calculate the score of frustration based upon the certainty factor information (step SC-3). In other words, the frustration calculating unit 102e places a higher weight on the secondary structure prediction results of the secondary structure prediction programs 102c having higher simulation precision on calculating the score of frustration.

One example of the setting of certainty factor information by the certainty factor information setting unit 102h will be described. First, the certainty factor information setting unit 102h compares the secondary structure prediction results of the respective secondary structure prediction programs 102c with the secondary structure data to calculate the precision (rate of coincidence) of the secondary structure prediction results of the respective secondary structure prediction programs 102c. Further, the certainty factor information setting unit 102h sets the average value of precisions of the respective secondary structure prediction programs 102c as standard certainty factor information (for example, 1), and with respect to precision of not less than the average value, a value higher than the standard certainty factor information (for example, a figure greater than 1) is set, while with respect to precision of not more than the average value, a value lower than the standard certainty factor information (for example, a figure smaller than 1) is set. Then the values are stored in a predetermined storing area in the certainty factor information data base 106b.

The certainty factor information setting unit 102h may set certainty factor information of each of the secondary structure prediction programs 102c for each of amino acids (residue) in the respective sequences. In other words, the certainty factor information of the secondary structure prediction programs 102c may be set for each of amino acids in the sequence with respect to the sequence prediction results by the respective secondary structure prediction programs 102c (for example, with respect to the first amino acid in a sequence, for program A the certainty factor information of $\alpha$-structure is set to 1.5, the certainty factor information of $\beta$-structure to 0.7, the certainty factor information of the other structures to 1.1, and so on).

Moreover, the certainty factor information setting unit 102h may set certainty factor information of the secondary structure prediction programs 102c for each of structures (such as $\alpha$-structure and $\beta$-structure). In other words, depending on the respective secondary structure prediction programs 102c, some of them may have high precision and others may have low precision with respect to a specific structure; therefore, the certainty factor information of the secondary structure prediction programs 102c may be set for each of the structures (for example, for program A the certainty factor information of $\alpha$-structure is set to 1.5, the certainty factor information of $\beta$-structure to 0.7, the certainty factor information of the other structures to 1.1 and so on).

Referring to Fig. 4 again, the interaction site predicting device 100 predicts localized portions to form interaction sites with respect to the objective sequence data based upon the calculated frustration score in the localized portions, through processes of the interaction site predicting unit 102f (step SA-7). In other words, for example, the interaction site predicting unit 102f predicts localized portions having a frustration score exceeding a predetermined threshold value as the interaction sites.

Next, the interaction site predicting device 100 outputs the prediction results of the interaction sites in the sequence data to the output device 114 (step SA-8).

Here, Fig. 7 is a drawing that depicts one example of a display screen having interaction site prediction results displayed on the output device 114 of the interaction site predicting device 100. As shown in this Figure, the display screen of the interaction site prediction results includes, for example, a display area MA-1 for sequence information of the objective sequence data, display areas MA-2 and MA-3 for localized portions to be predicted as interaction sites, and display areas MA-4 and MA-5 for frustration scores of the localized portions to be predicted as interaction sites. Thus, the main processes are completed.

[EMBODIMENTS]

Referring to Figs. 8 and 9, the following description will discuss embodiments of the present invention in detail.

The present embodiment exemplifies a case in which, with respect to amino acid sequences of Mammalian Adenylyl Cyclase (PDB ID: 1CJK)(referred to as "MAC" in the present specification), secondary structure predicting processes are carried out by using programs 1 and 2, and frustration values are calculated based upon the secondary structure prediction results so that interaction sites are predicted.

Fig. 8 is a drawing that depicts one example of a process-results output screen of the present embodiment displayed on the monitor of the interaction site predicting device 100. As shown in this Figure, the process-results output screen includes, for example, a display area MB-1 for a graph indicating the certainty factor when the amino acid sequence of MAC has a β-strand structure, a display area MB-2 for a graph indicating the certainty factor when the amino acid sequence of MAC has an α-helix structure, a display area MB-3 for a graph indicating the certainty factor when the amino acid sequence of MAC has another secondary structure, a display area MB-4 for amino acid sequences of MAC, a display area MB-5 that indicates a fragment area of amino acid sequences having a high frustration value (that is, an area having a high possibility of forming an interaction site), a display area MB-6 for secondary structure prediction results of program 1 and a display area MB-7 for secondary structure prediction results of program 2.

In the present embodiment, with respect to frustration calculations, two programs carry out different secondary structure predictions, and those structures that have comparatively long sequence portions and have high certainty factors in the prediction results are allowed to have greater frustration values. In addition to this arrangement, frustration calculations may be directly carried out by using a difference between predictions in the secondary structures, without using the certainty factor.

Fig. 9 is a drawing that is used for confirming whether a site, which has been predicted as a site having a high frustration through a known docking simulation, is actually functioning as an interaction site.

In Fig. 9, the predicted three-dimensional structure of MAC is illustrated as space fills. Sites having high frustration values are indicated by darker colors. Moreover, in Fig. 9, other proteins, which form composite bodies together with MAC, are illustrated as wire frames. As shown in Fig. 9, the sites having high frustration values have comparatively closer distances from other proteins, and it is indicated that these sites or a part of sequences that is connected to these have a high possibility of forming interaction sites.

[Other Embodiments]

While the invention has been described in detail and with reference to specific embodiments thereof, it will be apparent to one skilled in the art that various changes and modifications can be made therein without departing from the spirit and scope of the invention disclosed in claims.

For example, the above-mentioned embodiment has exemplified a case in which the interaction site predicting device 100 carries out interaction site predicting processes as a stand alone system; however, another arrangement may be used in which: interaction site predicting processes are carried out in response to a request from a client terminal that is arranged in a different housing from the interaction site predicting device 100, and the prediction results are returned to the client terminal.

Moreover, among those processes explained in the embodiment, all or a part of the processes that have been explained as automatic processes may be executed as manual processes, and all or a part of the processes that have been explained as manual processes may be executed as automatic processes by using a known method.

In addition to these, process procedures, control procedures, specific names, information including parameters such as various registered data and retrieving conditions, screen examples and data base structures, described in the above and figures, may be desirably modified, unless otherwise indicated.

Furthermore, with respect to the interaction site predicting device 100, the respective constituent elements shown in the Figures are based upon functional concept, and need not be physically formed in the same manner as shown in the Figures.

For example, with respect to processing functions possessed by the respective servers of the interaction site predicting device 100, in particular, the respective processing functions to be carried out by the control unit, all or a desired part thereof may be achieved by a CPU (Central Processing Unit) and programs that are interpreted and executed in the CPU, or may be achieved as hardware based upon wired logic. The programs are recorded in a recording medium, which will be described later, and read mechanically by the interaction site predicting device 100 as necessary.

Moreover, these programs may be recorded in an application program server that is connected to the interaction site predicting device 100 through a desired network, and all or a part thereof may be downloaded, if necessary.

Furthermore, the various data bases and the like (prediction results data base 106a to protein structure data base 106c), stored in the storage unit 106, are prepared as storage units such as memory devices like RAM and ROM, fixed disk devices like hard disks, flexible disks and optical disks, and these units store various programs used for various processes and Web site supplies, tables, files, data bases, files for use in Web pages and the like.

The interaction site predicting device 100 may be achieved by connecting peripheral devices such as a printer, a monitor and an image scanner to an information processing apparatus such as an information processing terminal like a personal computer and a work station that have been known, and by installing software (including programs, data and the like) used for achieving the method of the present invention in the information processing apparatus.

Moreover, the specific mode of dispersed or integrated structures of the interaction site predicting device 100, is not limited to the mode shown in Figures, all or a part thereof may be functionally or physically dispersed or integrated based upon a desired unit determined according to various loads and the like to form the system. For example, the respective data bases may be individually prepared as independent data base devices, and a part of the processes may be achieved by using a CGI (Common Gateway Interface).

Furthermore, the programs relating to the present invention may be stored in a recording medium that can be read by a computer. Here, the term "recording medium" includes a desired "portable physical medium", such as a flexible disk, a magneto-optical disk, ROM, EPROM, EEPROM, CD-ROM, MO, and DVD; a desired "fixed physical medium", such as ROM, RAM and HD installed in various computer systems; and a "communication medium" for holding programs in a short period, such as communication lines and carrier waves to be used upon transferring programs through a network typically represented by LAN, WAN and Internet.

Here, the term, "program" refers to a data processing method described in a desired language and description method, irrespective of formats such as source codes and binary codes. In addition, not limited to a single structure, "program" may be constituted in a dispersed manner as a plurality of modules and libraries, or may achieve its functions in cooperation with a different program typically prepared as an OS (Operating System). With respect to a specific structure used for reading from a recording medium, reading procedure or installing procedure after the reading process in the respective devices shown in the present embodiment, known structures and sequences can be utilized.

Moreover, the network 300, which has a function for mutually connecting the interaction site predicting device 100 and the external system 200, may include any of networks such as the Internet, Intranet, LAN (including both of wire/wireless systems), VAN, personal computer communication network, public telephone network (including both of analog/digital systems), dedicated line network (including both of analog/digital systems), CATV network, portable line exchange network/portable packet exchange network such as IMT2000 system, GSM system or PDC/PDC-P system, wireless call network, local wireless network such as Bluetooth, PHS network; and satellite communication networks such as CS, BS or ISDB. In other words, the present system can transmit and receive various data through any desired network regardless of wire or wireless system.

As described above in detail, according to the present invention, primary sequence information of a target protein is inputted, and with respect to the primary sequence information inputted to a secondary structure prediction program that predicts the secondary structure of the protein from the primary sequence information of the protein, secondary structure predicting simulating processes are executed so that the secondary structure prediction results of the secondary structure prediction program are compared with each other, and based upon the comparison results, frustration values of localized portions of the primary sequence information of the target protein are calculated so that an interaction site of the target protein is predicted from the calculated frustration values of the localized portions; thus, it becomes possible to provide an interaction site predicting device which can effectively predict an interaction site by finding out localized portions having frustration in the primary sequence information of a protein, such an interaction site predicting method, a program and a recording medium for such a method.

Moreover, according to the present invention, primary sequence information of a target protein is inputted, and secondary structure data of the target protein is acquired, and with respect to the primary sequence information inputted to a secondary structure prediction program that predicts the secondary structure of the protein from the primary sequence information of the protein, secondary structure predicting simulating processes are executed so that the secondary structure prediction results of the secondary structure prediction program are compared with the acquired secondary structure data, and based upon the comparison results, frustration values of localized portions of the primary sequence information of the target protein are calculated so that an interaction site of the target protein is predicted from the calculated frustration values of the localized portions; thus, it becomes possible to provide an interaction site predicting device which can find out an interaction site (that is, a site having a high possibility of forming an interaction site) more accurately by reviewing a difference between the prediction results of the secondary structure prediction program and the actual secondary structure of the target protein, such an interaction site predicting method, a program and a recording medium for such a method.

Moreover, according to the present invention, certainty factor information, which indicates a certainty factor with respect to the secondary structure prediction results of the secondary structure prediction program, is set, and based upon the set certainty factor information and the comparison results, frustration values of localized portions are calculated; thus, it becomes possible to provide an interaction site predicting device in which by placing a higher weight on the secondary structure prediction results data derived from a program having high certainty factor information (that is, having high precision in simulation), the certainty factor with respect to the simulation results is reflected to frustration calculations, such an interaction site predicting method, a program and a recording medium for such a method.

(II) Referring to the Figures, the following description will discuss embodiments of an active site predicting device, an active site predicting method, a program and a recording medium, according to the present invention, in detail. However, the present invention is not intended to be limited by these embodiments. The present embodiments will exemplify a case relating to an active site prediction of protein; however, it will be apparent to one skilled in the art that the present invention can be easily applied to physiologically active polypeptide based upon the description of the present embodiments.

[Overview of the present invention]

The following description will first discuss the overview of the present invention, and the structure, processes and the like of the present invention will be explained later in detail. Fig. 10 is a principle block diagram that depicts a basic principle of the present invention.

Schematically, the present invention has the following basic features. First, the user acquires three-dimensional structure data of a target protein from an external data base such as PDB (Protein Data Bank)(step S1).

Further, based upon the three-dimensional structure data of the protein, molecular orbital calculations are carried out to find out a frontier orbital (highest occupied orbital (HOMO) or the lowest unoccupied orbital (LUMO)) and/or orbital energy of main chain atoms based upon three-dimensional structure data of the target protein (step S2).

Here, the orbital energy of the highest occupied orbital (HOMO) or the lowest unoccupied orbital (LUMO) can be calculated through an AM1 Hamiltonian method or the like using a commercially-available program MOPAC2000 (J.J.P. Stewart, Fujitsu Limited, Tokyo, Japan (1999)) and the like (step S21).

Moreover, with respect to the molecular orbital calculations, in addition to semi empirical molecular orbital calculation and non-empirical molecular orbital calculation, density-generalized functional calculation may be used. Under the processing capability of the current computers, the semi empirical molecular orbital calculation is preferably used; however, in the future, a method with higher precision may be adopted.

Here, as a result of extensive research efforts on the calculating conditions, the inventors have successively found three calculating conditions required for the prediction (step S3). The first condition is to allow the calculations to include water molecules. In order to take the hydrogen bond between water molecules and protein and the charge transfer between water molecules and protein into account, it is necessary to generate water molecules around the protein of the inputted data. Since information about water molecules is included in crystal structure data, such information can be utilized, but in most cases, the number of pieces of such information is too small. Therefore, by using a method in which water molecules are placed in a position to allow them to be hydrogen-bonded to protein, molecular orbital calculations are carried out with water molecules being generated around the protein of the inputted data (step S31).

The second condition is to take dielectric effects of water molecules into consideration (step S32). Various methods are proposed to achieve this condition. For example, a method in which a continuous dielectric material is placed around protein (typically exemplified by COSMO method developed by Klamt et al.) or the like may be used.

In the third condition, in an attempt to apply the present invention to a very large molecule, it is expected that when the effects from a solvent are taken into consideration, the limit of processing capability of a computer might be exceeded. In such a case, dissociative amino acid residues on the protein surface are turned into a non-charged state (for example, glutamic acid is protonated) so that dissociative amino acids embedded therein are turned into a charged state (for example, glutamic acid is deprotonated); thus, calculated results in which a solvent is taken into consideration can be found in an approximate manner (step S33).

In this manner, in the present invention, by setting the three calculating conditions appropriately, the molecular orbital calculations can be executed effectively and the precision in active site prediction can be greatly improved.

Here, the term "peripheral orbitals of the frontier orbital" in the present invention is defined as follows: In general, "frontier orbital" refers to two orbitals, that is, "highest occupied orbital (HOMO)" and "lowest unoccupied orbital (LUMO)". However, in the case of a system of a giant molecule such as protein, in most cases, molecular orbitals, which have virtually no change from the frontier orbital in terms of energy, tend to give great effects to the functions in the same manner as the frontier orbital. After extensive researches by the inventors, it has been found that, in the case of a slight difference in energies (for example, 1 to 2 eV), the molecular orbital gives the same effects as the frontier orbital. Therefore, in the present invention, the frontier orbital is expanded to its peripheral area. For example, all the occupied orbitals having an energy gap from the highest occupied orbital (HOMO) that is within a predetermined threshold value (for example, 2 eV or the like) and all the orbitals having an energy gap from the lowest unoccupied orbital within a predetermined threshold value (for example, 2 eV or the like) are defined as "peripheral orbitals" of the frontier orbital. This expansion in definition is one of features of the present invention.

Next, the present invention attributes the frontier orbital and peripheral orbitals thus found to a specific amino acid residue in the amino sequence of protein (step S4). The attribution of molecular orbitals to an amino acid residue is carried out in the following manner.

Each of molecular orbitals is indicated by a linear bond of a basis function as shown below:

$$\varphi = \Sigma c_i \Phi_i$$

(where i is the number of a basis function, $\Phi_i$ is the basis function and $c_i$ is a coefficient)

Each basis function belongs to an atom, and each atom belongs to an amino acid residue. Therefore, each basis function belongs to one of amino acid residues. Accordingly, the distribution rate for each atom and for each amino acid residue is found.

$$D(K) = \Sigma c_i^2$$

(i represents all the basis functions belonging to an atom or an amino acid residue K)

Thus, it is possible to obtain an amino acid residue having the greatest distribution rate or an amino acid residue having an atom having the greatest distribution rate, for each of molecular orbitals. These are defined as amino acid residues in which the respective molecular orbitals are distributed. This definition gives one-to-one correspondence as to which amino acid a molecular orbital is distributed on. In general, since the molecular orbital has an expansion to a certain degree, the idea that a molecular orbital is distributed on one amino acid residue is not generally turned in the field of quantum chemistry; however, the inventors have found the fact that, when limited to orbitals relating to functions, the orbital is localized on almost one amino acid. Giving a one-to-one correspondence between the molecular orbital and amino acid provides easy understanding to people other than the technicians, and allows people other than the technicians to easily utilize the present invention. The present invention is also advantageous in this point.

As described above, an amino acid residue on which the frontier orbital and peripheral orbitals of protein are distributed is found, and in the present invention, this amino acid residue is determined as an amino acid residue that is a candidate for an active site (hereinafter, referred to as "candidate amino acid residue" or simply as "candidate") (step S4).

Next, in the present invention, candidates that are not allowed to form active sites are deleted, and an active site is predicted (step S5). For example, an amino acid residue containing an aromatic ring, such as tryptophan and phenylalanine, tends to form a frontier orbital and peripheral orbitals in its nature. However, it has been found that in most cases, these fail to form active sites. In the same manner, it has been found that although cystine and methionine, having a disulfide bond, also tend to have a frontier orbital and peripheral orbitals distributed thereon, these seldom form active sites. Among the frontier orbital and peripheral orbitals, those orbitals belonging to these amino acid residues are excluded from candidates for the active site.

The amino acid residues on which the remaining frontier orbitals and peripheral orbitals are distributed are candidates for the active site; however, there is hardly any case in which the active site is made from one amino acid residue, and in most cases, it is made from a plurality of amino acid residues. Therefore, when a three-dimensional structure is actually displayed from three-dimensional structure data of the target protein by using known graphic software so that the frontier orbitals and peripheral orbitals are observed, in most cases, there are portions in which the frontier orbitals and peripheral orbitals are present in a closely concentrated manner. Those candidate amino acid residues corresponding to the portion forming localized clusters in the three-dimensional structure tend to have a high possibility of forming active sites; therefore, such candidates are selected and predicted as active sites.

Moreover, when the orbital energy of main chain atoms is also used, calculations are carried out under the same calculating conditions as the case in which the above-mentioned frontier orbital is used; however, there is a difference in that the molecular orbitals are attributed not to amino acids but to molecules (step S22). The orbital energy of molecular orbitals distributed on an atom (for example, nitrogen, carbon and the like) of a main chain of an amino acid is noted. Since there are a plurality of such molecular orbitals, the orbital energy of the occupied orbital having the highest energy, which is the most characteristic, is noted. In this case also, the amino acid and the orbital energy have a one-to-one correspondence.

This method in which each amino acid is made correspondent with the orbital energy of molecular orbitals distributed on atoms of a main chain of the amino acid to carry out an analysis is a unique method different from conventional methods. For example, when the numbers of amino acids and orbital energies are plotted, relative sizes of the orbital energies are obtained. A portion of an amino acid residue in which atoms having comparatively high orbital energies are present has a high possibility of forming an active site. Moreover, an amino acid residue on which molecular orbitals having an orbital energy exceeding a predetermined value are distributed has a high possibility of forming an active site. The threshold value may be determined based upon an orbital energy of the active site of protein having the similar functions.

The above-mentioned two methods (step S21 and step S22) are in common in that the active site is predicted and in that the molecular orbital calculation is utilized. However, the prediction results by the respective predicting methods are not completely the same. It is supposed that the respective methods have respective advantages and disadvantages. Therefore, by combining these methods to compare the respective candidates, the precision can be further improved. For example, amino acid residues may be classified as those which are predicted as active sites through all the prediction results by the different methods and those which are predicted as active sites through one method or more; thus, it is possible to more accurately indicate the likelihood of being the active site.

[System structure]

First, referring to Figs. 11 to 13, the following description will discuss the structure of the present system. Fig. 11, which is a block diagram that depicts one example of the structure of the present system to which the present invention is applied, conceptually indicates only the parts of the system relating to the present invention. Schematically, the present system includes a protein active site predicting device 1100 and an external system 1200 that provides external data bases relating to structure information and the like of protein and external programs relating to homology retrieving and the like, which are communicably connected to each other through a network 1300.

In Fig. 11, the network 1300, which has a function for mutually connecting the protein active site predicting device 1100 and the external system 1200, is provided as, for example, the Internet.

In Fig. 11, the external system 1200, which is mutually connected to the protein active site predicting device 1100 through the network 1300, has a function for providing external data bases relating to protein structure information and the like and Web sites that execute external programs relating to homology retrieving, motif retrieving and the like to the user.

Here, the external system 1200 may be prepared as WEB servers, ASP servers and the like, and, in general, its hardware structure may be constituted by information processing apparatuses, such as commercially available work stations and personal computers with attached devices thereof. Moreover, the respective functions of the external system 1200 can be achieved by a CPU, a disk device, a memory device, an input device, an output device, a communication controlling device and the like in the hardware structure in the external system 1200 and programs and the like that control these devices.

In Fig. 11, schematically, the protein active site predicting device 1100 includes a control unit 1102 such as a CPU that systematically controls the entire protein active site predicting device 1100, a communication control interface unit 1104 that is connected to communication devices (not shown) such as routers that are connected to communication lines and the like, an input-output control interface unit 1108 that is connected to an input device 1112 and an output device 1114, and a storage unit 1106 that stores various data bases and tables, and these respective units are communicably connected to one another through communication paths. Moreover, the protein active site predicting device 1100 is communicably connected to the network 1300 through communication devices such as routers and wire or wireless communication lines such as dedicated lines.

Various data bases and tables (protein structure data base 1106a and processing result data 1106b) to be stored in the storage unit 1106 are prepared as storage units such as a fixed disk device, and store various programs used for various processes, files, data bases, files for use in Web pages and the like.

Among these constituent elements of the storage unit 1106, the protein structure data base 1106a serves as a data base that stores protein structure data (including amino acid sequence data, three-dimensional structure data, various annotation data and the like). The protein structure data base 1106a may be an external data base that is accessed through the Internet, or may be prepared as an in-house data base that is formed by copying these data bases, storing original sequence information and adding original annotation information and the like.

Here, the processing result data 1106b serves as a processing result data storage unit that stores information or the like relating to processing results by the control unit 1102.

Moreover, in Fig. 11, the communication control interface unit 1104 carries out a communication control between the protein active site predicting device 1100 and the network 1300 (or communication devices such as routers). In other words, the communication control interface unit 1104 has functions for carrying out data communications with other terminals through communication lines.

Furthermore, in Fig. 11, the input-output control interface unit 1108 controls the input device 1112 and the output device 1114. Here, the output device 1114 may be prepared as a speaker in addition to a monitor (including a home-use television)(in the following description, the output device 1114 is sometimes described as a monitor). The input device 1112 may be prepared as a keyboard, a mouse, a microphone and the like. Here, the monitor is also allowed to function as a pointing device in cooperation with a mouse.

In Fig. 11, the control unit 1102 is provided with an internal memory for storing control programs such as an OS (Operating System) programs that control various processing procedures and required data, and these programs and the like are used to carry out information processes to execute various processes. From the viewpoint of functions, the control unit 1102 is constituted by a frontier orbital calculating unit 1102a, a peripheral orbital determining unit 1102b, a water molecule setting unit 1102c, a dielectric setting unit 1102d, a charge setting unit 1102e, a candidate amino acid residue determining unit 1102f, an active site predicting unit 1102g, an orbital energy calculating unit 1102h and a structure data acquiring unit 1102p.

Among these, the frontier orbital calculating unit 1102a serves as a frontier orbital calculating unit that finds out an electron state of protein through molecular orbital calculations based upon the structure data to specify the frontier orbital. Here, as shown in Fig. 12, the frontier orbital calculating unit 1102a is constituted by a highest occupied orbital calculating unit 1102i and a lowest unoccupied orbital calculating unit 1102j.

Here, the peripheral orbital determining unit 1102b serves as a peripheral orbital determining unit that determines a molecular orbital having a predetermined energy gap from the frontier orbital as a peripheral orbital of the frontier orbital.

The water molecule setting unit 1102c serves as a water molecule setting unit that generates water molecules around protein to carry out quantum chemical calculations such as molecular orbital calculations.

Further, the dielectric setting unit 1102d serves as a dielectric setting unit that places a continuous dielectric material around the protein to carry out quantum chemical calculations such as molecular orbital calculations.

Moreover, the charge setting unit 1102e serves as a charge setting unit that turns a dissociative amino acid residue on the surface of protein into a non-charged state so that the dissociative amino acids embedded inside thereof are turned into a charged state, thereby carrying out quantum chemical calculations such as molecular orbital calculations.

Furthermore, the candidate amino acid residue determining unit 1102f serves as a candidate amino acid determining unit that determines those amino acid residues on which the frontier orbital and peripheral orbitals are distributed and/or those amino acid residues on which molecule orbitals having an orbital energy exceeding a predetermined value and/or molecule orbitals having relatively high orbital energy among orbital energies are distributed, as candidate amino acid residues.

Here, the active site predicting unit 1102g serves as an active site predicting unit that selects an active site from the candidate amino acid residues determined by the candidate amino acid residue determining unit 1102f to predict an active site. As shown in Fig. 13, the active site predicting unit 1102g is constituted by a specific amino acid residue excluding unit 1102k that deletes those candidates that cannot form active sites, a localized amino acid residue selecting unit 1102m that selects a candidate amino acid residue in a portion that is localized in the three-dimensional structure to form clusters, and a candidate comparing unit 1102n that compares candidates selected by the respective methods, and selects the overlapped candidates.

Moreover, the structure data acquiring unit 1102p serves as a structure data acquiring unit that acquires structure data of the target protein.

Additionally, with respect to the processes to be carried out by these respective units, the description thereof will be given later in detail.

[System processes]

Next, referring to Figs. 14 to 21, the following description will discuss one example of processes of the present system according to the present embodiment having the above-mentioned arrangement.

[Main processes]

Referring to Fig. 14, the following description will discuss main processes in detail. Fig. 14 is a flow chart that depicts one example of main processes of the present system according to the present embodiment.

The protein active site predicting device 1100 first acquires three-dimensional structure data of a target protein from an external data base such as PDB (Protein Data Bank) through processes in the structure data acquiring unit 1102p (step SA1-1).

Next, the protein active site predicting device 1100 carries out molecular orbital calculations through quantum chemical calculations based upon the three-dimensional structure data of the protein through processes of the control unit 1102 (step SA1-2). Here, referring to Fig. 15, the following description will discuss the molecular orbital calculation processes in detail. Fig. 15 is a flow chart that depicts one example of the molecular orbital calculation processes of the present system according to the present embodiment.

First, after acquiring coordinates of the protein (step SB1-1), the protein active site predicting device 1100 carries out molecular orbital calculations. Here, with respect to the molecular orbital calculations, the detailed description thereof is given, for example, in "Introduction to Computer Chemistry" (edited by Minoru Sakurai and Atsushi Inokai, published by Maruzen in 1999). The following description will discuss one example of the molecular orbital calculation processes. First, a Fock equation is solved (step SB1-2 to step SB1-7). Since this equation is "non-linear", it is solved by repeating calculations until the solution has been converged.

$$FC = SC\varepsilon$$

In this equation, F represents a Fock matrix, C represents a matrix in which LCAO coefficients form factors, S represents a matrix in which overlapping integrations form factors and $\varepsilon$ represents a vector in which orbital energies form factors. The Fock matrix can be associated with a density matrix D, for example, as shown by $F=h+G\cdot D$. The density matrix can be calculated from the LCAO coefficients. The respective steps of generation of F (step SB1-4), diagonalization of F (step SB1-5) and generation of a density matrix (step SB1-6) are repeatedly carried out until the density matrix has been converged.

Further, the protein active site detecting device 1100 acquires orbital energies and coefficients of molecular orbitals (step SB1-8) to find out the energy of the system (step SB1-9). Thus, the molecular orbital calculation processes are completed.

Referring to Fig. 14 again, the protein active site predicting device 1100 determines candidate amino acid residues from the frontier orbit and its peripheral orbitals based upon information such as molecular orbitals found in step SA1-2 (step SA1-3). Here, referring to Fig. 16, the following description will discuss the candidate amino acid residue determining processes by using the frontier orbital and its peripheral orbitals in detail. Fig. 16 is a flow chart that depicts one example of the candidate amino acid residue determining processes by using the frontier orbital and its peripheral orbitals of the system of the present embodiment.

First, the protein active site predicting device 1100 attributes the calculated molecular orbital to the corresponding distribution on amino acid residue in the amino acid sequence of the protein (step SC1-1). Here, when the molecular orbital calculations are carried out, two pieces of information, "state of distribution" and "orbital energies", are obtained as outputs with respect to the respective molecular orbitals, and in this case, based upon the information, "state of distribution", it is specified which atom (amino acid residue) each of the molecular orbitals is distributed on. Referring to Fig. 17, the attribution information determining process of each of the molecular orbitals to the corresponding amino acid is explained in detail. Fig. 17 is a flow chart that depicts one example of the attribution information determining process of each of the molecular orbitals to the corresponding amino acid in the present system according to the present embodiment.

First, the N-numbered molecular orbital is acquired (step SD1-1), and each of coefficients of a basis function belonging to each atom is squared and the resulting values are added for each atom (step SD1-2), and squared sums of the coefficients of the basis function belonging to each of atoms belonging to an amino acid are then added to one another for each amino acid (step SD1-3).

Then, the amino acid having the greatest sum is specified as the amino acid to which the N-numbered molecular orbital belongs (step SD1-4).

Moreover, Fig. 20 is a drawing that depicts one example of the calculation results obtained through the molecular orbital calculations. In the example shown in Fig. 20, oligopeptide (REWTY) composed of five residues is explained as an example. In this Figure, molecular orbital 1 attributes to amino acid residue R, molecular orbital 2 attributes to amino acid residue T, molecular orbital 3 attributes to amino acid residue E, molecular orbital 4 attributes to amino acid residue W, molecular orbital 5 attributes to amino acid residue R, molecular orbital 6 attributes to amino acid residue Y and molecular orbital 7 attributes to amino acid residue E, respectively.

Thus, the attribution information determining process of each of the molecular orbitals to the corresponding amino acid is completed.

Referring to Fig. 16 again, the protein active site predicting device 1100 defines the frontier orbit and its peripheral orbitals. In other words, the frontier orbital calculating unit 1102a determines molecular orbital 4 as the highest occupied orbital (HOMO) and molecular orbital 5 as the lowest unoccupied orbital (LUMO), through processes of the highest occupied orbital calculating unit 1102i and the lowest unoccupied orbital calculating unit 1102j. Moreover, in the present embodiment, when molecular orbitals having an orbital energy of not more than 2eV are defined as peripheral orbitals of the frontier orbital, the peripheral orbital determining unit 1102b determines molecular orbitals 2, 3, 4, 5 and 6 as peripheral orbitals. Therefore, the candidate amino acid residue determining unit 1102f determines the amino acid residues corresponding to the molecular orbitals 2, 3, 4, 5 and 6 as candidate amino acid residues for active sites (step SC1-2).

Next, the active site predicting unit 1102g excludes residues which are inappropriate as functional site candidates through processes of the specific amino acid residue excluding unit 1102k (step SC1-3). In this example, the specific amino acid residue excluding unit 1102k excludes molecular orbital 4 since molecular orbital 4 is distributed on tryptophan that is an amino acid residue that has a low possibility of forming an active site. As a result, the candidate amino acid residues are limited to those having molecular orbitals 2, 3, 5 and 6.

Next, the active site predicting unit 1102g examines how each of the candidates is present in space through processes in the localized amino acid residue selecting unit 1102m, and selects localized amino acid residues (step SC1-4). Fig. 21 is a drawing that depicts one example of a display screen used for confirming which position a candidate amino acid residue is located in the three-dimensional structure of protein.

As shown in Fig. 21, the structure data of the protein is graphic-displayed in either one of models including a wire model, a ribbon model, a pipe model, a ball and stick model and a space fill model by a known graphic display program so that each of candidate amino acid residues is displayed. In this Figure, since there is a cluster biased rightward, three candidates forming the cluster have a high possibility of being functional sites.

Thus, the candidate amino acid residue determining processes by the use of the frontier orbital and its peripheral orbitals are completed.

Referring to Fig. 14 again, based upon information of the molecular orbital and the like obtained in step SA1-2, the protein active site predicting device 1100 determines candidate amino acid residues from orbital energies that are localized on heavy atoms in a main chain (step SA1-4). Referring to Fig. 19, the following description will discuss the candidate amino acid residue determining processes based upon orbital energies that are localized on heavy atoms in a main chain, in detail. Fig. 19 is a flow chart that depicts one example of the candidate amino acid residue determining processes based upon orbital energies that are localized on heavy atoms in a main chain in the present system according to the present embodiment.

First, the protein active site predicting device 1100 attributes the calculated molecular orbital to the corresponding distribution on atoms that constitute an amino acid sequence of protein (step SF1-1). In step SC1-1, the distribution for each amino acid is found; however, this step is different in that the distribution is found for each of atoms.

Fig. 22 is a drawing that depicts one example of calculation results obtained from molecular orbital calculations. According to this Figure, molecular orbital 1 is attributed to atom number 1, molecular orbital 2 is attributed to atom number 4, molecular orbital 5 is attributed to atom number 1, molecular orbital 6 is attributed to atom number 4, molecular orbital 7 is attributed to atom number 2, molecular orbital 8 is attributed to atom number 3, molecular orbital 9 is attributed to atom number 1 and molecular orbital 10 is attributed to atom number 4, respectively.

Next, the orbital energy calculating unit 1102h extracts only molecular orbitals that are attributed to specific heavy atoms of a main chain (step SF1-2). In the example of Fig. 22, when the main chain N atoms are examined, molecular orbitals 1, 5 and 9 are distributed on the main chain N atom (atom number 1) of R, and molecular orbitals 2, 6 and 10 are distributed on the main chain N atom (atom number 4) of E.

Next, the orbital energy calculating unit 1102h selects the occupied orbital that has the highest energy among those orbitals that have been noted (step SF1-3). In the example shown in Fig. 22, after molecular orbitals 9 and 10 have been excluded since these are unoccupied orbitals, the orbital energy calculating unit 1102h respectively select molecular orbital 5 in the main chain N atom (atom number 1) of R and molecular orbital 6 in the main chain N atom (atom number 4) of E, since these have the highest energy respectively. In other words, typical energies are -6 eV in the orbital energy of R and -5 eV in the orbital energy of E.

Next, the orbital energy calculating unit 1102h forms a plot in which typical energies are plotted, with amino acid residue numbers being set on the axis of abscissas and typical energies being set on the axis of ordinates (step SF1-4), and specifies peripheral portions of the peak position in the graph as candidate amino acid residues (step SF1-5).

Thus, the candidate amino acid residue determining processes by the use of orbital energies localized on heavy atoms in the main chain are completed.

Referring to Fig. 14 again, the protein active site predicting device 1100 selects an active site from the candidate amino acid residues to predict the active site through processes in the active site predicting unit 1102g (step SA1-5). Here, referring to Fig. 18, candidate amino acid residue comparison processes will be explained in detail. Fig. 18 is a flow chart that depicts one example of the candidate amino acid residue comparison processes of the present system according to the present embodiment.

As shown in Fig. 18, a plurality of candidate amino acid residues are generated by using the above-mentioned methods using the frontier orbital and the orbital energy in the main chain atom (step SE1-1), and the active site predicting unit 1102g determines whether the candidates derived from the respective methods are coincident with each other (step SE1-2) through the processes of a candidate comparing unit 1102n, and when no coincidence is found, amino acids located before and after are also added to the candidates (when no coincidence is found, the next amino acids are further added), and the candidate determining method is again executed (step SE1-3).

In contrast, at step SE1-2, when the candidates derived from the respective methods are coincident with each other, the active site predicting unit 1102g predicts these candidates as active sites (step SE1-4). Thus, the candidate amino acid residue comparison processes are completed.

Consequently, the main process is completed.

[First Example of the present invention; Ribonuclease T1]

Referring to Figs. 23 to 26, the following description will discuss the first example of the present invention in detail.

Ribonuclease T1, which is a hydrolytic enzyme, has been fully examined through experiments, and it has been experimentally proven that essential amino acid residues are His40, Glu58, Arg77 and His92.

Hydrogen molecules were added to Ribonuclease T1 based upon X-ray crystal structure data by using a commercial program Insightll so that coordinates required for molecular orbital calculations were completed. After an optimized structure had been found by using a commercial program MOPAC2000, an electron state was obtained. Water molecules were placed around protein, and the effects of a solvent were further taken into consideration by using continuous dielectric approximation (COSMO method).

Here, a table in Fig. 23 depicts amino acid residues on which the frontier orbital of Ribonuclease T1 is distributed in the first example.

As shown in Fig. 23, with respect to amino acid residues that would become active site candidates, Glu58 distributed as the second one from HOMO, His40 distributed as the third one from HOMO, His92 distributed as the fourth one from LUMO and Arg77 distributed as the third one from LUMO are listed. Since these four amino residues are aggregated closely together, these are easily predicted as active sites. These are well coincident with experimental data. Here, it is predicted that His40 and Glu58 function in a nucleophilic manner and that Arg77 and His92 function in an electrophilic manner. In other words, different from conventional techniques, this method makes it possible to analyze not only the active site positions, but also the mechanisms of reactions.

Next, the nitrogen atoms in a main chain are considered. Fig. 24 is a graph in which orbital energies of the molecular orbitals distributed on the nitrogen atoms in the main chain are plotted in association with the residue numbers of amino acids in the first example. As shown in this Figure, a portion having a high orbital energy appears in the vicinity of each of the amino acid residue numbers 40, 60, 80 and 90. Moreover, in the present first example, Fig. 25 depicts a table in which amino acid residues having high orbital energies and the orbital energies thereof in the present first example are extracted. The amino acid residues located on the periphery of each of the amino acid residues having high orbital energies form candidates for the active sites.

Moreover, Fig. 26 is a table on which common portions of the candidate amino acid residues derived from the frontier orbital shown in Fig. 23, the candidate amino acid residues derived from the orbital energies of the main chain atom shown in Figs. 24 and 25 and common portions extracted from these are shown. For example, based upon the method using the frontier orbital, four candidates of nucleophilic groups and four candidates of electrophilic groups are listed. Moreover, based upon the method using the orbital energy of the main chain atom, respective two residues before and after an amino acid residue forming a peak (with peaks up to the fifth peak being taken into consideration) are selected as candidates. Further, five common residues, 40, 57, 58, 77 and 92, are listed.

All the amino acid residues, extracted as common portions in Fig. 26, are well matched with amino acid residues (40, 58, 77 and 92) required for activation, which are found through experiments (57 is erroneously predicted as an active site because it is close to 58).

[Second Example; Ribonuclease A]

Referring to Figs. 27 to 30, the following description will discuss a second example of the present invention in detail.

Ribonuclease A, which is a hydrolytic enzyme, has been fully examined through experiments, and it has been experimentally proven that essential amino acid residues are His12 and His119.

Hydrogen molecules were added to Ribonuclease A based upon X-ray crystal structure data by using a commercial program Insightll so that a coordinate required for molecular orbital calculations was completed. After an optimized structure had been found by using a commercial program MOPAC2000, an electron state was obtained. Water molecules were placed around protein, and the effects of a solvent were further taken into consideration by using continuous dielectric approximation (COSMO method).

Here, a table in Fig. 27 depicts amino acid residues on which the frontier orbital of Ribonuclease A is distributed in the present example.

Next, the nitrogen atoms in a main chain are considered. Fig. 28 is a graph in which orbital energies of the molecular orbitals distributed on the nitrogen atoms in the main chain are plotted in association with the residue numbers of amino acids in the second example. As shown in this Figure, a portion having a high orbital energy appears in the vicinity of each of the amino acid residue numbers 12, 47, 117, 76 and 53. Moreover, Fig. 29 depicts a table in which amino acid residues having high orbital energies and the orbital energies thereof are extracted. The amino acid residues located on the periphery of each of the amino acid residues having high orbital energies form candidates for the active sites.

Moreover, Fig. 30 is a table on which common portions of the candidate amino acid residues derived from the frontier orbital shown in Fig. 27, the candidate amino acid residues derived from the orbital energies of the main chain atom shown in Figs. 28 and 29 and common portions extracted from these are shown. For example, based upon the method using the frontier orbital, four candidates of nuleophilic groups and four candidates of electrophilic groups are listed. Moreover, based upon the method using the orbital energy of the main chain atom, respective two residues before and after an amino acid residue forming a peak (with peaks up to the fifth peak being taken into consideration) are selected as candidates. Further, three common residues, 12, 14 and 119 are listed.

All the amino acid residues, extracted as common portions in Fig. 30, are well matched with amino acid residues (12, 119) required for activation, which are found through experiments (14 is erroneously predicted as an active site because it is close to 12).

[Other Embodiments]

While the invention has been described in detail and with reference to specific examples thereof, it will be apparent to one skilled in the art that various changes and modifications can be made therein without departing from the spirit and scope of the invention disclosed in claims.

For example, the above-mentioned embodiment has exemplified a case in which the protein active site predicting device 1100 carries out processes as a stand alone system; however, another arrangement may be used in which: the processes are carried out in response to a request from a client terminal that is provided in a different housing from the protein active site predicting device 1100, and the prediction results are returned to the client terminal.

Moreover, among those processes explained in the embodiment, all or a part of the processes that have been explained as automatic processes may be executed as manual processes, or all or a part of the processes that have been explained as manual processes may be executed as automatic processes by using a known method.

In addition to these, process procedures, control procedures, specific names, information including parameters such as various registered data and retrieving conditions, screen examples and data base structures, described in the above document and figures, may be desirably modified, unless otherwise indicated.

Furthermore, with respect to the protein active site predicting device 1100, the respective constituent elements shown in the Figures are based upon functional concept, and need not be physically formed in the same manner as shown in the Figures.

For example, with respect to processing functions possessed by the respective units or devices of the protein active site predicting device 1100, in particular, the respective processing functions to be carried out by the control unit 1102, all or a desired part thereof may be achieved by a CPU (Central Processing Unit) and programs that are interpreted and executed in the CPU, or may be achieved as hardware based upon wired logic. Here, the programs are recorded in a recording medium, which will be described later, and read mechanically by the protein active site predicting device 1100 as necessary.

In other words, computer programs, which give instructions to the CPU in cooperation with the OS (Operation System) and are used for carrying out various processes, are stored in the storage unit 1106 such as a ROM or a HD. These computer programs are loaded in a RAM or the like to be executed, and form a control unit 1102 in cooperation with the CPU. Here, these computer programs may be recorded in an application program server that is connected to the protein active site predicting device 1100 through a desired network 1300, and all or a part thereof may be downloaded, if necessary.

Moreover, the programs relating to the present invention may be stored in a recording medium that can be read by a computer. Here, the term "recording medium" includes a desired "portable physical medium", such as a flexible disk, a magneto-optical disk, ROM, EPROM, EEPROM, CD-ROM, MO, and DVD; a desired "fixed physical medium", such as ROM, RAM and HD installed in various computer systems; and a "communication medium" for holding programs in a short period, such as communication lines and carrier waves to be used upon transferring programs through a network typically represented by LAN, WAN and Internet.

Here, the term, "program" refers to a data processing method described in a desired language and description method, irrespective of formats such as source codes and binary codes. In addition, not limited to a single structure, "program" may be constituted in a dispersed manner as a plurality of modules and libraries, or may achieve its functions in cooperation with a different program typically prepared as an OS (Operating System). With respect to a specific structure used for reading a recording medium, reading procedure or installing procedure after the reading process in the respective devices shown in the present embodiment, known structures and procedures can be utilized.

Furthermore, the various data bases and the like (protein structure data base 1106a and process result data 1106b), stored in the storage unit 1106, are prepared as storage units such as memory devices like RAM and ROM, fixed disk devices like hard disks, flexible disks and optical disks, and these units store various programs used for various processes and Web site supplies, tables, files, data bases, files for use in Web pages and the like.

Here, the protein active site predicting device 1100 may be achieved by connecting peripheral devices such as a printer, a monitor and an image scanner to an information processing apparatus such as an information processing terminal like a personal computer and a work station that have been known, and by installing software (including programs, data and the like) used for achieving the method of the present invention in the information processing apparatus.

Moreover, with respect to the specific mode of dispersed or integrated structures of the protein active site predicting device 1100, not limited to the mode shown in Figures, all or a part thereof may be functionally or physically dispersed or integrated based upon a desired unit determined according to various loads and the like to form the system. For example, the respective data bases may be individually prepared as independent data base devices, and a part of the processes may be achieved by using a CGI (Common Gateway Interface).

Moreover, the network 1300, which has a function for mutually connecting the protein active site predicting device 1100 and the external system 1200, may include any of networks such as the Internet, Intranet, LAN (including both of wire/wireless systems), VAN, personal computer communication network, public telephone network (including both of analog/digital systems), dedicated line network (including both of analog/digital systems), CATV network, portable line exchange network/portable packet exchange network such as IMT2000 system, GSM system or PDC/PDC-P system, wireless call network, local wireless network such as Bluetooth, PHS network, and satellite communication networks such as CS, BS or ISDB. In other words, the present system can transmit and receive various data through any desired network regardless of wire or wireless system.

As described above in detail, according to the present invention, an electron state of protein or physiologically active polypeptide is found out through molecular orbital calculations to specify the frontier orbital and its peripheral orbitals and/or orbital energies localized on heavy atoms in a main chain so that based upon the positions of the frontier orbital and its peripheral orbitals and/or the orbital energies, an amino acid residue to form an active site of the protein or the physiologically active polypeptide is predicted; therefore, it becomes possible to provide an active site predicting device which can effectively predict an active site with high precision by utilizing molecular orbital calculations that are considered to have high precision so that the relationship between the position of the frontier orbital or the position having high orbital energy and the reactive site is applied to the system of the protein or physiologically active polypeptide, such an active site predicting method, a program and a recording medium for such a method.

Moreover, according to the present invention, the structure data of the target protein or physiologically active polypeptide is acquired, and based upon the acquired structure data, an electron state of protein or physiologically active polypeptide is found out through molecular orbital calculations to specify the frontier orbital, and a molecular orbital that has a predetermined energy gap from the frontier orbital is determined as a peripheral orbital of the frontier orbital while an amino acid residue on which the frontier orbital and the peripheral orbital are distributed is determined as a candidate amino acid residue for an active site so that the active site is predicted by selecting an active site from the candidate amino acid residues thus determined; thus, it becomes possible to provide an active site predicting device which can predict an active site with high precision by utilizing molecular orbital calculations that are considered to have high precision so that the relationship between the position of the frontier orbital and the reactive site is applied to the system of the protein or physiologically active polypeptide, such an active site predicting method, a program and a recording medium for such a method.

Furthermore, according to the present invention, the structure data of the target protein or physiologically active polypeptide is acquired, and based upon the acquired structure data, an electron state of protein or physiologically active polypeptide is found out through molecular orbital calculations to specify orbital energies that are localized on heavy atoms in a main chain, and an amino acid residue on which a molecular orbital having an orbital energy exceeding a predetermined value and/or a molecular orbital having a relatively high orbital energy among the specified orbital energies are distributed is determined as a candidate amino acid residue for an active site; therefore, it becomes possible to provide an active site predicting device which can predict an active site with high precision by utilizing molecular orbital calculations that are considered to have high precision so that the relationship between the position having a high orbital energy and the reactive site is applied to the system of.the protein or physiologically active polypeptide, such an active site predicting method, a program and a recording medium for such a method.

According to the present invention, the structure data of the target protein or physiologically active polypeptide is acquired, and based upon the acquired structure data, an electron state of protein or physiologically active polypeptide is found out through molecular orbital calculations to specify the frontier orbital; based upon the acquired structure data, an electron state of protein or physiologically active polypeptide is found out through molecular orbital calculations to specify orbital energies that are localized on heavy atoms in a main chain; a molecular orbital that has a predetermined energy gap from the frontier orbital is determined as a peripheral orbital of the frontier orbital; and an amino acid residue on which the frontier orbital and the peripheral orbital are distributed and/or an amino acid residue on which a molecular orbital having an orbital energy exceeding a predetermined value and/or a molecular orbital having a relatively high orbital energy among the specified orbital energies are distributed is determined as a candidate amino acid residue for an active site, so that the active site is predicted by selecting an active site from the candidate amino acid residues thus determined; therefore, it becomes possible to provide an active site predicting device which can predict an active site with high precision by utilizing molecular orbital calculations that are considered to have high precision so that the relationship between the position of the frontier orbital or the position having a high orbital energy and the reactive site is applied to the system of the protein or physiologically active polypeptide, such an active site predicting method, a program and a recording medium for such a method.

Moreover, according to the present invention, at least one of the following three calculating conditions is taken in the molecular orbital calculations, and by appropriately setting the three calculating conditions, it is possible to effectively execute molecular orbital calculations; consequently, it becomes possible to provide an active site predicting device which can greatly improve the precision of active site predicting processes, such an active site predicting method, a program and a recording medium for such a method.

The three conditions are:

1) Water molecules are generated around protein or physiologically active polypeptide.
2) A continuous dielectric material is placed around protein or physiologically active polypeptide.
3) Dissociative amino acid residues on the surface of protein or physiologically active polypeptide are made into a non-charge state so that dissociative amino acid embedded therein is changed into a charged state.

(III) Referring to Figures, the following description will discuss embodiments of a protein interaction information processing device, a protein interaction information processing method and a program and a recording medium for such a method, according to the present invention, in detail. However, the present invention is not intended to be limited by these embodiments.

[Overview of the present invention]

The following description will first discuss the overview of the present invention, and the structure, processes and the like of the present invention will be explained later in detail. Fig. 31 is a principle block diagram that depicts a basic principle of the present invention.

Schematically, the present invention has the following basic features.

The present invention specifies a site having high instability based upon hydrophobic interaction of a solvent contact face. In other words, in the present invention, first, with respect to a plurality of proteins that are interactive with one another, the solvent contact area (the area of a molecule surface with which solvent molecules are made in contact, also referred to as "solvent exposure surface area") as a single substance and the solvent contact area upon formation of a composite body are respectively calculated, and by finding a difference from these, the solvent contact face of the interaction site is found. In other words, the site having a great difference between the solvent contact area as a single substance and the solvent contact area upon formation of a composite body indicates the fact that, when a composite body is formed, the area that contacts the solvent becomes smaller; therefore, such a site is highly possible to form an interaction site so that an amino acid residue site having such a great difference is specified as a solvent contact face of the interaction site. Here, when no structure data at the time of formation of a composite body is available, the present processes are not carried out.

Further, the present invention specifies a site that is a solvent face and also forms a hydrophobic face in an amino acid residue forming a primary structure of protein by finding hydrophobic interaction energy with respect to the solvent contact face of protein. It is considered that such a site is highly instable as a single substance, and is also stabilized when formed into a composite body with the hydrophobic face being covered with the composite body; thus, this site is highly possible to form an interaction site.

Moreover, the present invention specifies a site that is highly instable by specifying a site having high electrostatic interaction energy in protein. In other words, based upon an atomic charge (partial charge) found through a molecular orbital method and the like, the present invention calculates a site having a high electrostatic interaction energy. Such a site is highly instable as a single substance, and is also stabilized in terms of energy when formed into a composite body; thus, this site is highly possible to form an interaction site. Here, the atomic charge may be found through various calculating methods such as a molecular orbital method, or a value of atomic charge, given as various parameter values obtained through techniques derived from molecular dynamics or molecular kinetics, may be adopted.

Thus, the present invention specifies an interaction site by specifying a site that is highly instable based upon the solvent contact face, hydrophobic interaction energy and electrostatic interaction energy.

[System structure]

First, the following description will discuss the structure of the present system. Fig. 32, which is a block diagram that depicts one example of the structure of the present system to which the present invention is applied, conceptually indicates only the parts of the system relating to the present invention. Schematically, the present system is constituted by a protein interaction information processing device 2100 and an external system 2200 that provides external data bases relating to sequence information and the like and external programs relating to homology retrieving and the like, which are communicably connected to each other through a network 2300.

In Fig. 32, the network 2300, which has a function for mutually connecting the protein interaction information processing device 2100 and the external system 2200, is provided as, for example, the Internet.

In Fig. 32, the external system 2200, which is mutually connected to the protein interaction information processing device 2100 through the network 2300, has a function for providing external data bases relating to sequence information of DNA and the like and structure information such as protein and the like and Web sites that execute external programs relating to homology retrieving, motif retrieving and the like to the user.

Here, the external system 2200 may be prepared as WEB servers, ASP servers and the like, and, in general, its hardware structure may be constituted by information processing apparatuses, such as commercially available work stations and personal computers with attached devices thereof. Moreover, the respective functions of the external system 2200 can be achieved by a CPU, a disk device, a memory device, an input device, an output device, a communication controlling device and the like in the hardware structure in the external system 2200 and programs and the like that control these devices.

In Fig. 32, schematically, the protein interaction information processing device 2100 includes a control unit 2102 such as a CPU that systematically controls the entire protein interaction information processing device 2100, a communication control interface unit 2104 that is connected to communication devices (not shown) such as routers that are connected to communication lines and the like, an input-output control interface unit 2108 that is connected to an input device 2112 and an output device 2114, and a storage unit 2106 that stores various data bases and tables, and these respective units are communicably connected to one another through communication paths. Moreover, the protein interaction information processing device 2100 is communicably connected to the network 2300 through communication devices such as routers and wire or wireless communication lines such as dedicated lines.

Various data bases and tables (protein structure data base 2106a and processing result data 2106b) to be stored in the storage unit 2106 are prepared as storage units such as a fixed disk device, and store various programs used for various processes, files, data bases, files for use in Web pages and the like.

Among these constituent elements of the storage unit 2106, the protein structure data base 2106a serves as a data base that stores amino acid sequence information of protein (primary structure data), three-dimensional structure data (three-dimensional coordinate data of constituent atoms, and the like), various annotation information and the like. The protein structure data base 2106a may be an external data base that is accessed through the Internet, or may be prepared as an in-house data base that is formed by copying these data bases, storing original sequence information and adding original annotation information and the like.

Here, the processing result data 2106b serves as a processing result data storage unit that stores information or the like relating to processing results.

Moreover, in Fig. 32, the communication control interface unit 2104 carries out a communication control between the protein interaction information processing device 2100 and the network 2300 (or communication devices such as routers). In other words, the communication control interface unit 2104 has functions for carrying out data communications with other terminals through communication lines.

Furthermore, in Fig. 32, the input-output control interface unit 2108 controls the input device 2112 and the output device 2114. Here, the output device 2114 may be prepared as a speaker in addition to a monitor (including a home-use television)(in the following description, the output device 2114 is sometimes described as a monitor). The input device 2112 may be prepared as a keyboard, a mouse, a microphone and the like. Here, the monitor is also allowed to function as a pointing device in cooperation with a mouse.

In Fig. 32, the control unit 2102 is provided with an internal memory for storing control programs such as an OS (Operating System), programs that control various processing procedures and required data, and these programs and the like are used to carry out information processes to execute various processes. From the viewpoint of functions, the control unit 2102 includes a structure data acquiring unit 2102a, a solvent contact face specifying unit 2102b, a hydrophobic face specifying unit 2102c, an electrostatic interaction site specifying unit 2102d, an interaction site specifying unit 2102e and an interaction site predicting unit 2102f.

Among these, the structure data acquiring unit 2102a serves as a structure data acquiring unit that acquires structure data including primary structure data of a plurality of proteins that interact with one another and three-dimensional structure data as a single substance and/or as a composite body. Moreover, the solvent contact face specifying unit 2102b serves as a solvent contact face specifying unit that specifies a solvent contact face for each of amino acid residues that constitute primary structure data based upon the structure data acquired by the structure data acquiring unit.

Moreover, the hydrophobic face specifying unit 2102c serves as a hydrophobic face specifying unit that specifies hydrophobic interaction energy for each of amino acid residues that constitute primary structure data based upon the structure data acquired by the structure data acquiring unit. Furthermore, the electrostatic interaction site specifying unit 2102d serves as an electrostatic interaction site specifying unit that specifies electrostatic interaction energy for each of amino acid residues that constitute primary structure data based upon the structure data acquired by the structure data acquiring unit.

Here, the interaction site specifying unit 2102e serves as an interaction site specifying unit that specifies an interaction site by specifying a site of an amino acid residue that is highly instable based upon the solvent contact face specified by the solvent contact face specifying unit, the hydrophobic interaction energy specified by the hydrophobic face specifying unit and the electrostatic interaction energy specified by the electrostatic interaction site specifying unit.

Moreover, the interaction site predicting unit 2102f is provided with a candidate protein retrieving unit 2102g that specifies a primary sequence serving as a partner that interacts with the interaction site specified by the interaction site specifying unit to retrieve a candidate protein having a primary structure containing the primary sequence, and operates the structure data acquiring unit, the solvent contact face specifying unit, the hydrophobic face specifying unit, the electrostatic interaction site specifying unit and the interaction site specifying unit to confirm whether the primary sequence site on the partner side is specified as the interaction site of the candidate protein. Additionally, the processes to be carried out by these units will be described later in detail.

[System processes]

Next, referring to Figs. 33 to 42, the following description will discuss one example of processes of the present system according to the present embodiment having the above-mentioned arrangement.

[Main processes]

Referring to Fig. 33, the following description will discuss main processes in detail. Fig. 33 is a flow chart that depicts one example of main processes of the present system according to the present embodiment.

The protein interaction information processing device 2100 accesses the protein structure data base 2106a or the external data base of the external system 2200 (for example, PDB (Protein Data Bank)) through processes in the structure data acquiring unit 2102a, and acquires structure data including primary structure data of a plurality of proteins that interact with one another and three-dimensional structure data as a single substance and/or as a composite body (step SA2-1). Here, the structure data to be acquired may include both of structure data as a single substance of a plurality of proteins that interact with one another and structure data as a composite body, or may have only the structure data as a single substance of a plurality of proteins that interact with one another.

Next, in the case when the structure data as a composite body is available, as will be described later by reference to Fig. 34, the protein interaction information processing device 2100 specifies a solvent contact face for each of amino acid residues constituting primary structure data according to both of the structure data as a single substance of a plurality of proteins that interact with one another and the structure data as a composite body, through processes of the solvent contact face specifying unit 2102b (step SA2-2). Here, referring to Fig. 34, the following description will discuss the solvent contact face specifying process in detail. Fig. 34 is a flow chart that depicts one example of the solvent contact face specifying process of the present system according to the present embodiment.

First, the solvent contact face specifying unit 2102b calculates the solvent contact area $S_{isolated}$ with respect to each of the residues as a single substance (step SB2-1). Here, with respect to the method for obtaining the solvent contact area in the present invention, for example, any one of the following known methods, for example, may be used: Document 1 ("Numerical Calculation of Molecular Surface Area. I. Assessment of Errots" A.A. Bliznyuk and J.E. Gready, J. Comput. Chem., 17, 962-969 (1996).) and Document 2 ("Numerical Calculation of Molecular Surface Area. II. Assessment of Errots" A.A. Bliznyuk and J.E. Gready, J. Comput. Chem., 17, 970-975 (1996).)

Next, the solvent contact face specifying unit 2102b calculates the solvent contact area $S_{composite\ body}$ with respect to each of the residues as a composite body (step SB2-2).

Further, with respect to each of the residues, the solvent contact face specifying unit 2102b calculates a difference between the solvent contact area $S_{isolated}$ as a single substance and the solvent contact area $S_{composite\ body}$ as a composite body (step SB2-3). Thus, the solvent contact face specifying processes are completed.

Referring to Fig. 33 again, as will be described later with reference to Fig. 35, the protein interaction information processing device 2100 calculates the hydrophobic interaction energy for each of the residues and for each of atoms based upon hydrophobic parameters and the like for each of the amino acid residues and for each of atoms that constitute the primary structure of protein, according to both of the structure data as a single substance of a plurality of proteins that interact with one another and the structure data as a composite body, through processes of the hydrophobic face specifying unit 2102c, to specify the hydrophobic face (step SA2-3). For example, when the amino acid residue is represented by Lys, the nitrogen atom N at ε position and the hydrogen atom H bonded thereto are regarded as hydrophilic, while the carbon atoms C at β, γ and δ positions and the hydrogen atoms H bonded thereto are regarded as hydrophobic.

Here, referring to Fig. 35, the following description will discuss the hydrophobic face specifying process in detail. Fig. 35 is a flow chart that depicts one example of the hydrophobic face specifying process of the present system according to the present embodiment. The present example will discuss a case in which protein A and protein B interact with each other.

First, the hydrophobic face specifying unit 2102c calculates an amount of reduction in the hydrophobic face by using equation 1 (step SC2-1).

$$\Delta S_{hydrophobic} = S_{hydrophobicA} + S_{hydrophobicB} - S_{hydrophobicAB} \qquad \text{Equation 1}$$

Here, $\Delta S_{hydrophobic}$ represents an amount of reduction in the hydrophobic face, $S_{hydrophobicA}$ represents an area of the hydrophobic face of protein A as a single substance, $S_{hydrophobicB}$ represents an area of the hydrophobic face of protein B as a single substance and $S_{hydrophobicAB}$ represents an area of the hydrophobic face of protein A and protein B formed into a composite body.

Further, the hydrophobic face specifying unit 2102c calculates the hydrophobic interaction energy $E_{hydrophobic}$ based upon equation 2 (SC2-2).

$$E_{hydrophobic} = k \times \Delta S_{hydrophobic} \qquad \text{Equation 2}$$

Here, $k = 24$ cal/mol·Å$^2$.

(Reference "Quantification of the hydrophobic interaction by simulations of the aggregation of small hydrophobic solutions in water", T.M. Raschke, JTsai and M. Levitt, PNAS, 98, 5965-5969 (2001)).

Further, the hydrophobic face specifying unit 2102c specifies an amino acid residue site having a hydrophobic interaction energy exceeding a predetermined threshold value as the hydrophobic face (step SC2-3). Thus, the hydrophobic face specifying processes are completed.

Referring to Fig. 33 again, as will be described later by reference to Fig. 36, the protein interaction information processing device 2100 specifies an electrostatic interaction energy for each of the amino acid residues that constitute the primary structure data, according to both of the structure data as a single substance of a plurality of proteins that interact with one another and the structure data as a composite body, through processes of the electrostatic interaction site specifying unit 2102d (step SA2-4). Referring to Fig. 36, the following description will discuss the electrostatic interaction site specifying process in detail. Fig. 36 is a flow chart that depicts one example of the electrostatic interaction site specifying process of the present system according to the present embodiment.

First, the electrostatic interaction site specifying unit 2102d calculates an electrostatic interaction energy $E_n$ with respect to each of the residues by using equation 3 (step SD2-1).

[Equation 3]

$$E_n = \frac{1}{4\pi\varepsilon} \sum_{i \in n} \sum_{j \notin n} \frac{q_i q_j}{R_{ij}}$$

Here, ε represents a dielectric constant inside a molecule, q represents a partial charge, i and j are subscripts indicating atoms, and R represents a distance between atom i and atom j. $E_n$ represents electrostatic interaction, which approximates interaction between a polar site inside a molecule and a site that is ionized and charged, by placing a partial charge on the atomic nucleus. Thus, the electrostatic interaction site specifying processes are completed.

Referring to Fig. 33 again, as will be described later by reference to Fig. 37, the protein interaction information processing device 2100 specifies a highly unstable portion of the amino acid residue based upon the solvent contact face, the hydrophobic interaction energy and the electrostatic interaction energy so that the interaction site is specified through processes of interaction site specifying unit 2102e (step SA2-5). Here, referring to Fig. 37, the following description will discuss the interaction site specifying process in detail. Fig. 37 is a flow chart that depicts one example of the interaction site specifying process of the present system according to the present embodiment.

First, the interaction site specifying unit 2102e specifies a site having a difference $\Delta S$ in the solvent contact areas that exceeds a predetermined threshold value (step SE2-1).

Next, the interaction site specifying unit 2102e specifies a site in which the hydrophobic interaction energy $E_{hydrophobic}$ exceeds a predetermined threshold value (step SE2-2).

Next, the interaction site specifying unit 2102e specifies a site in which the electrostatic interaction energy $E_n$ exceeds a predetermined threshold value (step SE2-3). Thus, the interaction site specifying processes are completed. Consequently, the main processes are completed.

[Interaction site predicting processes]

Referring to Fig. 38, the following description will discuss the interaction site predicting processes in detail. Fig. 38 is a flow chart that depicts one example of the interaction site predicting processes of the present system according to the present embodiment.

First, the protein interaction information processing device 2100 specifies an interaction site through the main processes (step SF2-1).

Next, the interaction site predicting unit 2102f specifies a primary sequence (including a sequence in the same protein) serving as a partner that interacts with the interaction site specified at step SF2-1 (step SF2-2), and retrieves for a candidate protein having a primary structure including the corresponding primary sequence through processes of the candidate protein retrieving unit 2102g (step SF2-3).

Next, with respect to the candidate proteins, the interaction site predicting unit 2102f executes the structure data acquiring process, the solvent contact face specifying process (when the structure data as a composite body is available), the hydrophobic face specifying process, the electrostatic interaction site specifying process and the interaction site specifying process to confirm whether the portion of the primary sequence on the partner side is specified as an interaction site of the candidate protein (step SF2-4). Thus, the interaction site predicting processes are completed.

[First Example]

Referring to Figs. 39 to 44, the following description will discuss the first example in detail. The first example explains a case in which "barnase" and "barstar" are used as proteins and the interaction site is specified.

Fig. 39 depicts a processing diagram in which the protein interaction information processing device 100 calculates a difference $\Delta S$ in the solvent contact areas for each of amino acid residues with respect to the barnase based upon the crystal structure of a barnase-barstar composite body through processes of the solvent contact face specifying unit 102b. As shown in this Figure, in the primary structure of the barnase, the difference $\Delta S$ in each of the 38th, 59th, 83rd and 102nd amino acid residues is large so that it is specified that the barnase interacts with the barstar in these sites.

Further, Fig. 40 depicts a processing diagram in which the protein interaction information processing device 100 calculates the hydrophobic interaction energy of each of the amino acid residues with respect to the barnase based upon the crystal structure of a barnase single substance through processes of the hydrophobic face specifying unit 102c. As shown in this Figure, the hydrophobic interaction energy of the 82nd amino acid residue is high to show a possibility of an interaction at this site.

Moreover, Fig. 41 depicts a processing diagram in which the protein interaction information processing device 100 calculates the electrostatic interaction energy of each of the amino acid residues with respect to the barnase based upon the crystal structure of a barnase single substance through processes of the electrostatic interaction specifying unit 102d. As shown in this Figure, the electrostatic interaction energy in each of the 59th, 66th, 83rd and 102nd amino acid residues is high to show a possibility of an interaction at these sites.

Here, Fig. 42 depicts a processing diagram in which the protein interaction information processing device 100 calculates a difference $\Delta S$ in the solvent contact areas for each of amino acid residues with respect to the barstar based upon the crystal structure of a barnase-barstar composite body through processes of the solvent contact face specifying unit 102b. As shown in this Figure, in the primary structure of the barstar, the difference $\Delta S$ in each of the 30th, 36th, 40th, 45th, 47th and 77th amino acid residues is large so that it is specified that the barstar interacts with the barnase in these sites.

Further, Fig. 43 depicts a processing diagram in which the protein interaction information processing device 100 calculates the hydrophobic interaction energy of each of the amino acid residues with respect to the barstar based upon the crystal structure of a barstar single substance through processes of the hydrophobic face specifying unit 102c. As shown in this Figure, the hydrophobic interaction energy of the 30th amino acid residue is high to show a possibility of an interaction at this site.

Moreover, Fig. 44 depicts a processing diagram in which the protein interaction information processing device 100 calculates the electrostatic interaction energy of each of the amino acid residues with respect to the barstar based upon the crystal structure of a barstar single substance through processes of the electrostatic interaction specifying unit 102d. As shown in this Figure, the electrostatic interaction energy in each of the 35th, 39th, 58th, 65th, 77th and 80th amino acid residues is high to show a possibility of an interaction at these sites.

Based upon the results shown in Figs. 40 and 41, the protein interaction information processing device 100 specifies the 59th, 66th, 82nd, 83rd and 102nd amino acid residues as interaction candidate sites with respect to the barnase through processes of the interaction site specifying unit 102e. These are well coincident with the results of known information in the interaction sites of a composite body shown in Fig. 39, thereby indicating that, upon forming a composite body, it is possible to predict the binding sites from the protein single substance structure. Moreover, based upon the results shown in Figs. 43 and 44, the protein interaction information processing device 100 specifies the 30th, 35th, 39th, 58th 65th, 77th and 80th amino acid residues as interaction candidate sites with respect to the barstar through processes of the interaction site specifying unit 102e. These are well coincident with the results of known information in the interaction sites of a composite body shown in Fig. 42, thereby also indicating that, upon forming a composite body, it is possible to predict the binding sites from the protein single substance structure. Thus, the processes of the first example are completed.


[Second Example]

Referring to Figs. 45 to 50, the following description will discuss the second example in detail. The second example explains a case in which Ribonuclease and its Inhibitor are used as proteins and the interaction site is specified.

Fig. 45 depicts a processing diagram in which the protein interaction information processing device 100 calculates a difference $\Delta S$ in the solvent contact areas for each of amino acid residues with respect to the Ribonuclease based upon the crystal structure of a Ribonuclease-inhibitor composite body through processes of the solvent contact face specifying unit 102b. As shown in this Figure, in the primary structure of the Ribonuclease, the difference $\Delta S$ in the 39th amino acid residue is large so that it is specified that the Ribonuclease interacts with the inhibitor in this site.

Further, Fig. 46 depicts a processing diagram in which the protein interaction information processing device 100 calculates the hydrophobic interaction energy of each of the amino acid residues with respect to the Ribonuclease based upon the crystal structure of a Ribonuclease single substance through processes of the hydrophobic face specifying unit 102c. As shown in this Figure, with respect to the hydrophobic interaction energy, no particular peak is recognized.

Moreover, Fig. 47 depicts a processing diagram in which the protein interaction information processing device 100 calculates the electrostatic interaction energy of each of the amino acid residues with respect to the Ribonuclease based upon the crystal structure of a Ribonuclease single substance through processes of the electrostatic interaction specifying unit 102d. As shown in this Figure, the electrostatic interaction energy in each of the 1st, 7th and 39th amino acid residues is high to show a possibility of an interaction at these parts.

Here, Fig. 48 depicts a processing diagram in which the protein interaction information processing device 100 calculates a difference $\Delta S$ in the solvent contact areas for each of amino acid residues with respect to the inhibitor based upon the crystal structure of a Ribonuclease-inhibitor composite body through processes of the solvent contact face specifying unit 102b. As shown in this Figure, in the primary structure of the inhibitor, the difference $\Delta S$ in the 433rd amino acid residue is large so that it is specified that the inhibitor interacts with the Ribonuclease at this site.

Further, Fig. 49 depicts a processing diagram in which the protein interaction information processing device 100 calculates the hydrophobic interaction energy of each of the amino acid residues with respect to the inhibitor based upon the crystal structure of an inhibitor single substance through processes of the hydrophobic face specifying unit 102c. As shown in this Figure, the hydrophobic interaction energy of the 433rd amino acid residue is high to show a possibility of an interaction at this site.

Moreover, Fig. 50 depicts a processing diagram in which the protein interaction information processing device 100 calculates the electrostatic interaction energy of each of the amino acid residues with respect to the inhibitor based upon the crystal structure of an inhibitor single substance through processes of the electrostatic interaction specifying unit 102d. As shown in this Figure, the electrostatic interaction energy in the 433rd amino acid residue is high to show a possibility of an interaction at this site.

Based upon the results shown in Figs. 46 and 47, the protein interaction information processing device 100 specifies the 1st, 7th and 39th amino acid residues as interaction candidate sites with respect to the Ribonuclease through processes of the interaction site specifying unit 102e. These are well coincident with the results of known information in the interaction sites of a composite body shown in Fig. 45, thereby indicating that, upon forming a composite body, it is possible to predict the binding sites from the protein single substance structure. Moreover, based upon the results shown in Figs. 49 and 50, the protein interaction information processing device 100 specifies the 433rd amino acid residue as an interaction candidate site with respect to the inhibitor through processes of the interaction site specifying unit 102e. This is well coincident with the results of known information in the interaction sites of a composite body shown in Fig. 48, thereby also indicating that, upon forming a composite body, it is possible to predict the binding sites from the protein single substance structure. Thus, the processes of the second example are completed.

[Other Embodiments]

While the invention has been described in detail and with reference to specific examples thereof, it will be apparent to one skilled in the art that various changes and modifications can be made therein without departing from the spirit and scope of the invention disclosed in claims.

The present embodiment indicates that there is a correlation between the results obtained by specifying the solvent contact face by the use of the structure data as a single substance of proteins that interact with one another and structure data as a composite body and the results obtained by finding the hydrophobic interaction and the electrostatic interaction by the use of the structure data as a single substance. However, it is self-evident that, even in the case when the hydrophobic interaction and the electrostatic interaction are found by using only the structure data as a single substance, the same effects as those of the present invention can be obtained.

For example, the above-mentioned embodiment has exemplified a case in which the protein interaction information processing device 2100 carries out processes as a stand alone system; however, another arrangement may be used in which: the processes are carried out in response to a request from a client terminal that is provided in a different housing from the protein interaction information processing device 2100, and the processing results are returned to the client terminal.

Moreover, among those processes explained in the embodiment, all or a part of the processes that have been explained as automatic processes may be executed as manual processes, or all or a part of the processes that have been explained as manual processes may be executed as automatic processes by using a known method.

In addition to these, process procedures, control procedures, specific names, information including parameters such as various registered data and retrieving conditions, screen examples and data base structures, described in the above and figures, may be desirably modified, unless otherwise indicated.

Furthermore, with respect to the protein interaction information processing device 2100, the respective constituent elements shown in the Figures are explained based upon functional concept, and need not be physically formed in the same manner as shown in the Figures.

For example, with respect to processing functions possessed by the respective units or devices of the protein interaction information processing device 2100, in particular, the respective processing functions to be carried out by the control unit 2102, all or a desired part thereof may be achieved by a CPU (Central Processing Unit) and programs that are interpreted and executed in the CPU, or may be achieved as hardware based upon wired logic. Here, the programs are recorded in a recording medium, which will be described later, and read mechanically by the protein interaction information processing device 2100 as necessary.

In other words, computer programs, which give instructions to the CPU in cooperation with the OS (Operation System) and are used for carrying out various processes, are stored in the storage unit 2106 such as a ROM or a HD. These computer programs are loaded in a RAM or the like to be executed, and form a control unit 2102 in cooperation with the CPU. Here, these computer programs may be recorded in an application program server that is connected to the protein interaction information processing device 2100 through a desired network 2300, and all or a part thereof may be downloaded, if necessary.

Moreover, the programs according to the present invention may be stored in a recording medium that can be read by a computer. Here, the term "recording medium" includes a desired "portable physical medium", such as a flexible disk, a magneto-optical disk, ROM, EPROM, EEPROM, CD-ROM, MO, and DVD; a desired "fixed physical medium", such as ROM, RAM and HD installed in various computer systems; and a "communication medium" for holding programs in a short period, such as communication lines and carrier waves to be used upon transferring programs through a network typically represented by LAN, WAN and Internet.

Here, the term, "program" refers to a data processing method described in a desired language and description method, irrespective of formats such as source codes and binary codes. In addition, not limited to a single structure, "program" may be constituted in a dispersed manner as a plurality of modules and libraries, or may achieve its functions in cooperation with a different program typically prepared as an OS (Operating System). With respect to a specific structure used for reading from a recording medium, reading procedure or installing procedure after the reading process in the respective devices shown in the present embodiment, known structures and procedures can be utilized.

Furthermore, the various data bases and the like (protein structure data base 2106a and process result data 2106b), stored in the storage unit 2106, are prepared as storage units such as memory devices like RAM and ROM, fixed disk devices like hard disks, flexible disks and optical disks, and these units store various programs used for various processes and Web site supplies, tables, files, data bases, files for use in Web pages and the like.

Here, the protein interaction information processing device 2100 may be achieved by connecting peripheral devices such as a printer, a monitor and an image scanner to an information processing apparatus such as an information processing terminal like a personal computer and a work station that have been known and by installing software (including programs, data and the like) used for achieving the method of the present invention in the information processing apparatus.

Moreover, with respect to the specific mode of dispersed or integrated structures of the protein interaction information processing device 2100, not limited to the mode shown in Figures, all or a part thereof may be functionally or physically dispersed or integrated based upon a desired unit determined according to various loads and the like to form the system. For example, the respective data bases may be individually prepared as independent data base devices, and a part of the processes may be achieved by using a CGI (Common Gateway Interface).

Moreover, the network 2300, which has a function for mutually connecting the protein interaction information processing device 2100 and the external system 2200, may be prepared as any of networks such as the Internet, Intranet, LAN (including both of wire/wireless systems), VAN, personal computer communication network, public telephone network (including both of analog/digital systems), dedicated line network (including both of analog/digital systems), CATV network, portable line exchange network/portable packet exchange network such as IMT2000 system, GSM system or PDC/PDC-P system, wireless call network, local wireless network such as Bluetooth, PHS network, and satellite communication networks such as CS, BS or ISDB. In other words, the present system can transmit and receive various data through any desired network regardless of wire or wireless system.

As described above in detail, according to the present invention, the structure data including primary structure data of a plurality of proteins that interact with one another and three-dimensional structure data as a single substance and/or as a composite body is acquired; based upon the structure data thus acquired, hydrophobic interaction energy for each of amino acid residues that constitute primary structure data is specified; based upon the structure data thus acquired, electrostatic interaction energy for each of amino acid residues that constitute primary structure data is specified; and based upon the specified hydrophobic interaction energy and electrostatic interaction energy, an interaction site is specified by specifying a site of an amino acid residue that is highly instable; therefore, it becomes possible to provide a protein interaction information processing device which can easily specify an interaction site of protein by using the structure data, such a protein interaction information processing method and a program and a recording medium for such a method.

Moreover, according to the present invention, based upon the structure data acquired, a solvent contact for each of amino acid residues that constitute primary structure data is specified, and based upon the specified solvent contact face, hydrophobic interaction energy and electrostatic interaction energy, an interaction site is specified by specifying a site of an amino acid residue that is highly instable; therefore, it becomes possible to provide a protein interaction information processing device which, in the case when the structure data as a composite body is available, can more easily specify an interaction site of protein more accurately, such a protein interaction information processing method and a program and a recording medium for such a method.

Furthermore, according to the present invention, with respect to the interaction site specified by the interaction site specifying unit, a primary sequence on the partner side for the interaction is specified, and a candidate protein having a primary structure including the corresponding primary sequence is retrieved, and with respect to the candidate protein thus retrieved, processes of the structure data acquiring unit, the solvent contact face specifying unit (when the structure data as a composite body is available), the hydrophobic face specifying unit, the electrostatic interaction site specifying unit and the interaction site specifying unit are executed to confirm whether the primary sequence portion on the partner side is specified as an interaction site of a candidate protein; therefore, it becomes possible to provide a protein interaction information processing device which easily predicts an unknown interaction, such a protein interaction information processing method and a program and a recording medium for such a method.

(IV) Referring to Figures, the following description will discuss embodiments of a binding site predicting device, a binding site predicting method, a program and a recording medium, according to the present invention, in detail. However, the present invention is not intended to be limited by these embodiments.

The present embodiments will exemplify a case in which the present invention is applied to an amino acid sequence of protein, and the like; however, not limited to this case, the present invention is also applied to a case in which an amino acid sequence of physiologically active polypeptide is used.

[Overview of the present invention]

The following description will first discuss the overview of the present invention, and the structure, processes and the like of the present invention will be explained later in detail. Figs. 51 and 52 are principle block diagrams that depict a basic principle of the present invention. Schematically, the present invention has the following basic features.

Fig. 51 is a drawing that is used for explaining the concept of an arrangement in which from amino acid sequence information of a protein, binding sites of the protein are predicted by the present invention.

As shown in Fig. 51, in the present invention, spatial distance data between the respective amino acid residues in a three-dimensional structure of a protein is found from amino acid sequence data of protein or physiologically active polypeptide (step SA3-1).

With respect to the method for obtaining the spatial distance data, for example, the following three methods are proposed.

1) High-speed calculating method

In this method, the distance on the sequence between amino acids is converted to a spatial distance. Fig. 56 is a drawing that depicts the concept of a high-speed calculating method of the present invention. Supposing that the three-dimensional structure of protein has Gaussian chains, the distance on the amino acid sequence of protein and the spatial distance in the three-dimensional structure of protein are made in association with each other based upon the following equation.

$$r = k \, d^n \ (0 < n < 1)$$

Here, r represents a spatial distance, d represents a distance on the sequence, and k is a proportional constant. In other words, it is possible to calculate the spatial distance r, if the distance on the sequence d is found. The values of k and n may be set to appropriate values by statistically processing the relationship between the distance on the sequence between amino acids and the spatial distance based upon three-dimensional structure information data collected in a protein structure data base, for example, PDB (Protein Data Bank). In this case, n is set in a range from 0 to 1, preferably, from 0.5 to 0.6. Moreover, k is set in a range from 2.8Å to 4.8Å, preferably, from 3.3Å to 4.3Å. This method, which needs only a simple algorithm with very small calculating loads, makes it possible to provide a helpful method for processing large amount of protein data, for example, proteins of not less than several tens of thousand.

2) Calculation method using structure data

This method finds the spatial distance between actual amino acid residues accurately by utilizing three-dimensional structure information data registered in a protein structure data base. For example, when the three-dimensional structure information data of an objective protein is stored in a protein structure data base such as PDB, the three-dimensional structure information data, registered in the data base, is acquired so that the spatial distance is calculated accurately through the following processes.

For example, supposing that the coordinates of the center of gravity of amino acid residue number I, an atom in a specific chain and the like are indicated by $(x_I, y_I, z_I)$ and that the coordinates of the center of gravity of amino acid residue number J, an atom in a specific chain and the like are indicated by $(x_J, y_J, z_J)$, the spatial distance $R_{IJ}$ between amino acid residue number I and amino acid residue number J is calculated based upon the following equation.

$$R_{IJ}{}^2 = (x_I - x_J)^2 + (y_I - Y_J)^2 + (z_I - z_J)^2$$

(where $R_{IJ} > 0$)

3) Calculation method using simulation data

In this method, with respect to a protein having an unknown structure, the structure simulation process is carried out on the protein by using a known structure simulation method, and by using the simulation data (predicted three-dimensional structure information data), the spatial distance is found. With respect to the three-dimensional structure predicting simulation method, various methods, such as a homology modeling method, may be used. These methods have been introduced in, for example, "Practice Bioinformatics" (written by C. Gibas and P. Jambeck, O'Reilly Japan, 2002), etc. in detail.

Although this method is disadvantageous in that a large calculation load is imposed in comparison with method 1 and method 2, it is advantageous in that the spatial distance can be obtained virtually accurately with respect to a protein having an unknown structure.

One of the features of the present invention is to make a plurality of calculation methods applicable to the respective steps. In particular, to compensate for the disadvantage that the three-dimensional structure predicting method using a known simulation technique takes long time, method 1 in which methods for determining the spatial distance data between the respective amino acid residues from amino acid sequence data are simply combined is used so that high-speed calculating processes are prepared to achieve a predicting method capable of processing a large amount of data used for bonding-partner prediction and the like.

Next, in the present invention, the entire energy of a protein is calculated according to the distance data and the charge of each amino acid (step SA3-2).

Here, various charge-determining methods for amino acids are proposed. For example, in some methods, the charge of a chargeable amino acid (lysine, arginine) positively charged is defined as 1, the charge of a chargeable amino acid (glutamic acid, aspartic acid) negatively charged is defined as - 1 and the charge of the other amino acid is defined as 0. Moreover, the charge of each of amino acid residues may be determined by using a known quantum chemical calculation method based upon three-dimensional structure information of proteins registered in a protein structure data base and three-dimensional structure information obtained through simulation techniques.

Moreover, with respect to calculations for the entire energy of a protein, various methods are proposed and, for example, energy calculation techniques based upon molecular dynamics, molecular kinetics, molecular orbital method, density generalized function method and the like, which are explained in "Introduction to Computational Chemistry" (written by Frank Jensen, John Wiley & Sons Co., Ltd., 1999), etc., may be used; and selection is appropriately made from those techniques depending on required prediction precision and calculation environments of the user. In addition to these techniques, the energy of each of amino acid residues can be found by using a Fragment MO method (Chemical Physics Letters, Volume 336, Issues 1-2, 9 March 2001, Pages 163-170). Although this method requires a long calculation time, high prediction precision is expected.

In addition to these, the following method for calculating electrostatic energy is proposed as a method that does not require a long calculation time.

$$E_{total} = 1/2 \; \Sigma\Sigma \; q_i q_j / r_{ij}$$

(where i and j represent desired amino acid residue numbers of all the amino acid residues, and i is not j)

In this equation, $E_{total}$ represents the entire energy of a protein, $q_i$ represents a partial charge of amino acid residue i, $q_j$ represents a partial charge of amino acid residue j, and $r_{ij}$ represents a spatial distance between amino acid residue i and amino acid residue j.

Since this method requires a very small calculation load in comparison with other methods, it is particularly effective upon carrying out composite body calculation processes.

Next, the present invention carries out calculations on the interaction energy between a specific amino acid and another amino acid residue in a protein based upon the following equations to examine to what extent each of the amino acid residues stabilizes the entire energy of the protein (step SA3-3).

$$E_{interaction} (N) = q_N \; \Sigma \; q_j / r$$

$$E_{total} = 1/2 \; \Sigma \; E_{interaction} (N)$$

In these equations, N represents a desired amino acid residue number, $E_{interaction}$ (N) represents interaction energy between amino acid residue N and another amino acid residue, j represents an amino acid residue number other than N, $q_N$ represents a partial charge of amino acid residue N, $q_j$ represents a partial charge of amino acid residue j, r represents a spatial distance between amino acid residue N and amino acid residue j. Here, the half of the sum of interaction energies of the total amino acid residues corresponds to the total protein energy $E_{total}$.

Next, the present invention predicts a binding site by specifying the amino acid residue having a relatively high interaction energy found in step SA3-3 and the amino acid residue having an interaction energy exceeding a predetermined threshold value as instable amino acid residues in terms of energy (step SA3-4).

Here, Fig. 52 is a drawing that explains the concept of the method of the present invention in which, in the case when based upon amino acid sequence information of a plurality of proteins, a composite body is formed by using the proteins.

First, the present invention assumes an amino acid residue (binding residue) to form a binding site on a plurality of amino acid sequences (step SB3-1). Here, Fig. 57 is a drawing that depicts the concept of the assumption of a binding residue on the amino acid sequences. In the example shown in Fig. 57, it is assumed that the 50th amino acid residue of amino acid sequence A and the 100th amino acid residue of amino acid sequence B form binding residues. Here, with respect to the binding residue, amino acid residues, predicted as binding sites in amino acid sequences through the method of the present invention as described by reference to Fig. 51, may be used.

Next, the present invention determines the spatial distance between two amino acid residues located on different amino acid sequences (step SB3-2). The above-mentioned three methods can be used as the spatial distance determining method, and the following description will discuss a case 1) in which a high-speed calculation method which effectively carries out calculations with the least calculation loads is used.

First, the sequence distance between two amino acid residues located on different amino acid sequences is defined in the following manner.

(Distance d between attention residues on sequences) =

(|Distance between attention residue on sequence A and binding

residue on sequence| + |Distance between attention residue on

sequence B and binding residue on sequence|)

Fig. 58 is a drawing that explains the concept of the attention residue. As shown in Fig. 58, the binding residue of two amino acid sequences (A and B) and desired attention residues other than the binding residue are defined.

Next, the present invention estimates the spatial distance r in the three-dimensional structure of a composite body based upon the sequence distance d between two amino acid residues located on different amino acid sequences (step SB3-3).

$$r = k\, d^{n}\ (0 < n < 1)$$

Here, r represents the spatial distance, d represents the sequence distance, and k represents a proportional constant. Here, n is set from 0 to 1, preferably, from 0.5 to 0.6. Moreover, k is set in a range from 2.8Å to 4.8Å, preferably, from 3.3Å to 4.3Å. In other words, if the distance d on sequences is found, the spatial distance r can be calculated.

In addition to this method, when the three-dimensional structure of a composite body has been known, the above-mentioned 2) calculation method using structure data is used for accurately obtain the spatial distance between amino acid residues.

Moreover, by using the above-mentioned 3) calculation method using simulation data, the three-dimensional structure of the composite body is predicted so that the spatial distance between amino acid residues can be found accurately to a certain degree. Fig. 62 is a drawing that depicts the concept of the formation of a composite body structure by using docking simulation processes. As shown in Fig. 62, the docking simulation processes for forming a structure of the composite body are carried out by using a plurality of pieces of three-dimensional structure information. With respect to the docking simulation processes, various known simulation techniques may be used. For example, as shown in Fig. 62, in those techniques, in general, the distance and orientation of two proteins are changed. In a specific example, with one of structures being fixed, two degrees of freedom in rotation and two degrees of freedom in translation motion are given to the other structure so that various structures are generated. When the structure satisfying the condition that the two structures are made in contact with each other without being overlapped is extracted, structures that can be taken by the composite body are prepared.

Next, the present invention calculates the entire energy of the protein based upon the spatial distance and charges of the respective amino acids (step SB3-4).

Here, various charge-determining methods for amino acids are proposed. For example, as described earlier, in some methods, the charge of a chargeable amino acid (lysine, arginine) positively charged is defined as 1, the charge of a chargeable amino acid (glutamic acid, aspartic acid) negatively charged is defined as - 1 and the charge of the other amino acid is defined as 0. Moreover, as described earlier, the charge of each of amino acid residues may be determined by using a known quantum chemical calculation method based upon three-dimensional structure information of proteins registered in a protein structure data base and three-dimensional structure information obtained through simulation techniques.

Furthermore, with respect to calculations for the entire energy of a protein, as described earlier, various methods are proposed and, for example, energy calculation techniques based upon molecular dynamics, molecular kinetics, molecular orbital method, density generalized function method and the like, which are explained in "Introduction to Computational Chemistry" (written by Frank Jensen, John Wiley & Sons Co., Ltd., 1999), etc., may be used; and selection is appropriately made from those techniques depending on required prediction precision and calculation environments of the user. In addition to these techniques, as described earlier, the energy of each of amino acid residues can be found by using a Fragment MO method (Chemical Physics Letters, Volume 336, Issues 1-2, 9 March 2001, Pages 163-170). Although this method requires a long calculation time, high prediction precision is expected.

In addition to these, as described earlier, the following method for calculating electrostatic energy is proposed as a method that does not require a long calculation time.

$$E_{total} = 1/2 \; \Sigma\Sigma \; q_i q_j / r_{ij}$$

(where i and j represent desired amino acid residue numbers of all the amino acid residues, and i is not j)

In this equation, $E_{total}$ represents the entire energy of a protein, $q_i$ represents a partial charge of amino acid residue i, $q_j$ represents a partial charge of amino acid residue j, and $r_{ij}$ represents a spatial distance between amino acid residue i and amino acid residue j. In this manner, the processes of the present method basically proceed through the same sequence as that of the processing flow indicated by the double line, and repeated while the amino acid sequence of the candidate protein is changed. Among those candidates, it is predicted that the one which can form the most stable composite body has a high possibility of serving as the interaction partner.

Next, in the present invention, the procedure returns to step SB3-1, and $E_{total}$ is calculated with respect to all the combinations while the amino acid residue (binding residue) for interaction being changed so that the binding residue obtained when $E_{total}$ is the lowest is predicted as a binding site (step SB3-5).

[System structure]

First, the following description will discuss the structure of the present system. Fig. 53, which is a block diagram that depicts one example of the structure of the present system to which the present invention is applied, conceptually indicates only the parts of the system relating to the present invention. Schematically, the present system is constituted by a binding site predicting device 3100 and an external system 3200 that provides external data bases relating to sequence information and the like and external programs relating to homology retrieving and the like, which are communicably connected to each other through a network 3300.

In Fig. 53, the network 3300, which has a function for mutually connecting the binding site predicting device 3100 and the external system 3200, is provided as, for example, the Internet and the like.

In Fig. 53, the external system 3200, which is mutually connected to the binding site predicting device 3100 through the network 3300, has functions for providing external data bases relating to amino acid sequence information, protein three-dimensional structure information and the like and Web sites that execute external programs relating to homology retrieving, motif retrieving and the like to the user.

Here, the external system 3200 may be prepared as WEB servers, ASP servers and the like, and, in general, its hardware structure may be constituted by information processing apparatuses, such as commercially available work stations and personal computers with attached devices thereof. Moreover, the respective functions of the external system 3200 can be achieved by a CPU, a disk device, a memory device, an input device, an output device, a communication controlling device and the like in the hardware structure in the external system 3200 and programs and the like that control these devices.

In Fig. 53, schematically, the binding site predicting device 3100 is constituted by a control unit 3102 such as a CPU that systematically controls the entire binding site predicting device 3100, a communication control interface unit 3104 that is connected to communication devices (not shown) such as routers that are connected to communication lines and the like, an input-output control interface unit 3108 that is connected to an input device 3112 and an output device 3114, and a storage unit 3106 that stores various data bases and tables; and these respective units are communicably connected to one another through predetermined communication paths. Moreover, the binding site predicting device 3100 is communicably connected to the network 3300 through communication devices such as routers and wire or wireless communication lines such as dedicated lines.

Various data bases and tables (amino acid sequence data base 3106a to processing result file 3106g) to be stored in the storage unit 3106 are prepared as storage units such as a fixed disk device, and store various programs used for various processes, tables, files, data bases, files for use in Web pages and the like.

Among these constituent elements of the storage unit 3106, the amino acid sequence data base 3106a serves as a data base for storing amino acid sequences. The amino acid sequence data base 3106a may be prepared as an external amino acid sequence data base that is accessed through the Internet, or may be prepared as an in-house data base that is formed by copying these data bases, storing original sequence information and adding original annotation information and the like.

Moreover, the protein structure data base 3106b is a data base that stores three-dimensional structure information of proteins. The protein structure data base 3106b may be provided as an external three-dimensional structure information data base that is accessed through the Internet, or may be prepared as an in-house data base that is formed by copying these data bases, storing original three-dimensional structure information and adding original annotation information and the like.

Here, a distance data file 3106c serves as a distance information storage unit that stores information and the like relating to the distance (distance on sequences, spatial distance) between amino acid residues contained in amino acid sequences.

Further, a entire energy data file 3106d serves as a entire energy data storage unit that stores information and the like relating to the entire energy of a protein.

Moreover, an interaction energy data file 3106e serves as an interaction energy data storage unit that stores information and the like relating to interaction energy of each of amino acid residues.

Furthermore, a composite body structure data file 3106f serves as a composite body structure data storage unit that stores information and the like relating to the composite body structure of each of proteins.

The processing result file 3106g serves as a processing result storage unit that stores information and the like relating to various processing results given by the binding site predicting device 3100.

Moreover, in Fig. 53, the communication control interface unit 3104 carries out a communication control between the binding site predicting device 3100 and the network 3300 (or communication devices such as routers). In other words, the communication control interface unit 3104 has functions for carrying out data communications with other terminals through communication lines.

Furthermore, in Fig. 53, the input-output control interface unit 3108 controls the input device 3112 and the output device 3114. Here, the output device 3114 may be prepared as a speaker in addition to a monitor (including a home-use television) (in the following description, the output device 3114 is described as a monitor). The input device 3112 may be prepared as a keyboard, a mouse, a microphone and the like. Here, the monitor is also allowed to function as a pointing device in cooperation with a mouse.

In Fig. 53, the control unit 3102 is provided with an internal memory for storing control programs such as an OS (Operating System), programs that control various processing procedure and required data, and these programs and the like are used to carry out information processes to execute various processes. From the viewpoint of functions, the control unit 3102 is constituted by an amino acid sequence data acquiring unit 3102a, a spatial distance determining unit 3102b, a charge determining unit 3102c, an energy calculating unit 3102d, a candidate amino acid residue determining unit 3102e, a composite body structure generating unit 3102f, an energy minimizing unit 3102g, a bonding candidate data acquiring unit 3102h, a binding site predicting unit 3102i and a bonding partner candidate determining unit 3102j.

Among these, the amino acid sequence data acquiring unit 3102a serves as an amino acid sequence data acquiring unit that acquires amino acid sequence data of an objective protein or physiologically active polypeptide, an amino acid sequence data acquiring unit that acquires amino acid sequence data of a plurality of objective proteins or physiologically active polypeptides and an amino acid sequence data acquiring unit that acquires amino acid sequence data of an objective protein or physiologically active polypeptide and amino acid sequence data of a plurality of proteins or physiologically active polypeptides that form bonding candidates.

Moreover, the spatial distance determining unit 3102b serves as a spatial distance determining unit that determines a spatial distance between respective amino acid residues contained in amino acid sequence data obtained by the amino acid sequence data acquiring unit, a spatial distance determining unit that determines a spatial distance between respective amino acid residues contained in a plurality of amino acid sequence data obtained by the amino acid sequence data acquiring unit according to the three-dimensional structure information of a composite body generated by the composite body structure generating unit, and a spatial distance determining unit that determines a spatial distance between respective amino acid residues contained in amino acid sequence data of objective amino acid and amino acid sequence data of bonding candidates obtained by the amino acid sequence data acquiring unit, according to the three-dimensional structure information of a composite body generated by the composite body structure generating unit. Here, as shown in Fig. 54, the spatial distance determining unit 3102b is constituted by a high-speed calculating unit 3102k, a calculating unit 3102m using structure data and a calculating unit 3102n using simulation data. In this case, the high-speed calculating unit 3102k serves as a high-speed calculating unit that determines a spatial distance by using a high-speed calculating technique. Moreover, structure data use calculating unit 3102m serves as a calculating unit using structure data that determines a spatial distance by the use of a structure data use calculating unit. Furthermore, simulation data use calculating unit 3102n serves as a calculating unit using simulation data that determines a spatial distance by the use of a simulation data use calculating unit.

Here, the charge determining unit 3102c serves as a charge determining unit that determines a charge possessed by each of amino acid residues contained in amino acid sequence data, a charge determining unit that determines a charge possessed by each of amino acid residues contained in amino acid sequence data of a plurality of amino acids and a charge determining unit that determines a charge possessed by each of amino acid residues contained in amino acid sequence data of objective amino acid and amino acid sequence data of bonding candidates.

Further, the energy calculating unit 3102d serves an energy calculating unit that calculates energy of each of amino acid residues according to the spatial distance between the amino acid residues determined by the spatial distance determining unit and the charge possessed by each of the amino acid residues determined by the charge determining unit. As shown in Fig. 55, the energy calculating unit 3102d is constituted by a entire energy calculating unit 3102p and an interaction energy calculating unit 3102q. Here, the entire energy calculating unit 3102p serves as a entire energy calculating unit that calculates the entire energy of a protein. Moreover, the interaction energy calculating unit 3102q serves as an interaction energy calculating unit that calculates interaction energy of each of amino acid residues.

Here, the candidate amino acid residue determining unit 3102e serves as a candidate amino acid residue determining unit that determines a candidate amino acid residue to form a binding site based upon the energy calculated by the energy calculating unit and a candidate amino acid residue determining unit that determines a binding site at which the sum of energies is made the smallest by the energy minimizing unit as a candidate amino acid residue for the binding site.

Further, the composite body structure generating unit 3102f serves as a composite body structure generating unit that generates three-dimensional structure information of a composite body in which a plurality of objective proteins or physiologically active polypeptides are combined with one another, and a composite body structure generating unit that generates three-dimensional structure information of a composite body in which an objective protein or physiologically active polypeptide and a protein or physiologically active polypeptide to form a bonding candidate are combined with each other.

The energy minimizing unit 3102g serves as an energy minimizing unit that generates three-dimensional structure information of a composite body by changing a binding site with respect to a composite body using the composite body structure generating unit, calculates energy of each of amino acid residues using the energy calculating unit, and finds a binding site at which the sum of the energies is minimized.

Further, the bonding candidate data acquiring unit 3102h serves as a bonding candidate data acquiring unit that acquires amino acid sequence data or the like of a protein to form a bonding candidate.

Moreover, the binding site predicting unit 3102i serves as a binding site predicting unit that predicts an amino acid residue of the binding site from candidate amino acid residues for the binding site.

Furthermore, the bonding partner candidate determining unit 3102j serves as a bonding candidate determining unit which, after having allowed the energy minimizing unit to execute its processes on all the bonding candidates, determines a bonding candidate having a binding site at which the sum of energies is minimized.

The processes to be carried out by these units will be described later in detail.

[System processes]

Next, referring to Figs. 53 to 71, the following description will discuss one example of processes of the present system in detail according to the present embodiment having the above-mentioned arrangement.

Fig. 59 is a flow chart that depicts one example of the processes of the present system according to the present embodiment. In Fig. 59, the procedure of processes indicated by a dot line depicts a procedure of processes in which a binding site in a protein sequence is predicted by the present system, the procedure of processes indicated by a double line depicts a procedure of processes in which a binding site is predicted by using amino acid sequences of a plurality of proteins that have been known to interact with one another according to the present system, and the procedure of processes indicated by a solid line depicts a procedure of processes in which a candidate protein on the partner side that is best combined with an objective protein is predicted by the present system. With respect to these three procedures of processes, the basic idea and calculation processes are almost the same. Further, these procedures of processes have the same major objective, that is, to analyze interaction information.

[Process in which a binding site in one protein sequence is predicted]

Next, referring to Fig. 59, the following description will discuss the process in which a binding site in one protein sequence is predicted by the present system in detail. In Fig. 59, the procedure of processes indicated by the dot line is a flow chart that depicts one example of processes in which a binding site in one protein sequence is predicted by the present system in the present embodiment.

First, the binding site predicting device 3100 accesses an external data base and an amino acid sequence data base 3106a of the external system 3200 such as Genbank through processes of an amino acid sequence data acquiring unit 3102a to acquire amino acid sequence data of an objective protein or physiologically active polypeptide (step SC3-1).

Further, the binding site predicting device 3100 determines a spatial distance between respective amino acid residues contained in the amino acid sequence data acquired at step SC3-1, through processes of a spatial distance determining unit 3102b (step SC3-2).

Here, the spatial distance determining unit 3102b may determine the spatial distance based upon the distance on sequences between the respective amino acid residues by using the high-speed calculating technique through processes of the high-speed calculating unit 3102k, or may determine the spatial distance between the respective amino acid residues based upon known structure data by using the calculation technique using structure data through processes of the calculating unit 3102m using structure data, or may also determine the spatial distance between the respective amino acid residues by using the predicted structure based upon the processing results of a known structure simulation program by the use of the calculation technique using simulation data through processes of the calculating unit 3102n using simulation data.

Next, the binding site predicting device 3100 determines a charge possessed by each of amino acid residues contained in amino acid sequence data through processes of the charge determining unit 3102c (step SC3-3). Here, various charge determining methods for amino acids are proposed. In general, a method is used in which the charge of a chargeable amino acid (lysine, arginine) positively charged is defined as 1, the charge of a chargeable amino acid (glutamic acid, aspartic acid) negatively charged is defined as - 1 and the charge of the other amino acid is defined as 0. Further, the charge may be determined by using a known quantum chemical calculation method based upon the resulting three-dimensional structure information. Moreover, in the case when experimental data relating to the charge of each of amino acid residues have been known through experiments, it is preferable to utilize the data.

Next, the binding site predicting device 3100 calculates the energy of each of amino acid residues based upon the determined spatial distance between the amino acid residues and charge possessed by each of the amino acid residues through processes of the energy calculating unit 3102d (step SC3-4).

Here, various techniques are proposed with respect to the energy calculation, and the following method for calculating electrostatic energy is proposed as a method that does not require a long calculation time.

First, the entire energy of a protein is calculated based upon the following equation through processes of the entire energy calculating unit 3102p.

$$E_{total} = 1/2 \; \Sigma\Sigma \; q_i q_j/r_{ij}$$

(where i and j represent desired amino acid residue numbers of all the amino acid residues, and i is not j)

In this equation, $E_{total}$ represents the entire energy of a protein, $q_i$ represents a partial charge of amino acid residue i, $q_j$ represents a partial charge of amino acid residue j, and $r_{ij}$ represents a spatial distance between amino acid residue i and amino acid residue j.

Next, the interaction energy calculating unit 3102q carries out calculations on the interaction energy between a specific amino acid and another amino acid residue in a protein based upon the following equations to examine to what extent each of the amino acid residues stabilizes the entire energy of the protein.

$$E_{interaction} \; (N) = q_N \; \Sigma \; q_j/r$$

$$E_{total} = 1/2 \; \Sigma \; E_{interaction} \; (N)$$

In these equations, N represents a desired amino acid residue number, $E_{interaction}$ (N) represents interaction energy between amino acid residue N and an amino acid residue other than N, j represents an amino acid residue number other than N, $q_N$ represents a partial charge of amino acid residue N, $q_j$ represents a partial charge of amino acid residue j, r represents a spatial distance between amino acid residue N and amino acid residue j. Here, the half of the sum of interaction energies of the total amino acid residues corresponds to the total protein energy $E_{total}$.

Further, the binding site predicting device 3100 determines a candidate amino acid residue to form a binding site according to the calculated interaction energy through processes of the candidate amino acid residue determining unit 3102e (step SC3-5). In other words, the candidate amino acid residue determining unit 3102e determines the candidate amino acid residue to form a binding site by specifying the amino acid residue having a relatively high interaction energy and the amino acid residue having an interaction energy exceeding a predetermined threshold value as instable amino acid residues in terms of energy.

Moreover, the binding site predicting device 3100 predicts a binding site by removing those candidates that do not form binding sites in terms of space or energy from the candidate amino acid residues through processes of the binding site predicting unit 3102i. For example, if the results shown in Fig. 60 are obtained as the processing results with respect to candidate amino acid residue energy and the like, the binding site predicting unit 3102i predicts glutamic acid (GLU) having the highest energy in Fig. 60 as the first candidate for a binding site. Moreover, the binding site predicting unit 3102i also predicts that a portion at which unstable portions in the three-dimensional structure are clustered (amino acid residue portion indicated by a black circle) as shown in Fig. 61 has a high possibility of forming a binding site.

Thus, the process in which a binding site in one protein sequence is predicted by using the present system is completed.

[Process in which a binding site is predicted by using amino acid sequences of a plurality of proteins that are known to interact with one another]

Next, referring to Fig. 59 and the like, the following description will discuss the process in which a binding site is predicted by using amino acid sequences of a plurality of proteins that are known to interact with one another according to the present system in detail. In Fig. 59, the procedure of processes indicated by the double line is a flow chart that depicts one example of processes in which a binding site is predicted by using amino acid sequences of a plurality of proteins that are known to interact with one another according to the present system of the present embodiment.

First, the binding site predicting device 3100 accesses an external data base and an amino acid sequence data base 3106a of the external system 3200 such as Genbank through processes of an amino acid sequence data acquiring unit 3102a to acquire amino acid sequence data of an objective protein or physiologically active polypeptide (step SC3-1).

Further, the binding site predicting device 3100 generates three-dimensional structure information of a composite body in which a plurality of objective proteins or physiologically active polypeptides are combined with one another through processes of the composite body structure generating unit 3102f (step SC3-7). Here, as described above by reference to Fig. 62, the composite body structure generating unit 3102f may predict a three-dimensional structure of the composite body by using the calculation technique using simulation data. Moreover, when the three-dimensional structure of the composite body has been known, the composite body structure generating unit 3102f may acquire the three-dimensional structure information of the composite body.

Moreover, by assuming an amino acid residue (binding residue) to form a binding site on a plurality of amino acid sequences as described earlier, the composite body structure generating unit 3102f may carry out processes without actually generating the composite body structure. Here, Fig. 57 is a drawing that depicts the concept of the assumption of a binding residue on the amino acid sequences. In the example shown in Fig. 57, it is assumed that the 50th amino acid residue of amino acid sequence A and the 100th amino acid residue of amino acid sequence B form binding residues. Here, with respect to the binding residue, amino acid residues, predicted as binding sites in amino acid sequences through the above-mentioned method of the present invention, may be used.

Next, the binding site predicting device 3100 determines the spatial distance between respective amino acid residues contained in acquired sequence data of a plurality of amino acids through processes of the spatial distance determining unit 3102b based upon three-dimensional structure information of the composite body (step SC3-2).

Here, with respect to the determining method for the spatial distance, the aforementioned three methods may be used, and when the three-dimensional structure of the composite body has been known or when docking simulation processes are carried out, the spatial distance determining unit 3102b is allowed to find the spatial distance between amino acid residues accurately. The following description will discuss a case 1) in which a high-speed calculation method which effectively carries out calculations with the least calculation loads is used.

First, the spatial distance determining unit 3102b defines a sequence distance between two amino acid residues located on different amino acid sequences in the following manner.

(Distance d between attention residues on sequences) =

(|Distance between attention residue on sequence A and binding

residue on sequence| + |Distance between attention residue on

sequence B and binding residue on sequence|)

Fig. 58 is a drawing that explains the concept of the attention residue. The binding residue of two amino acid sequences (A and B) and desired attention residues other than the binding residue are defined as shown in Fig. 58.

Next, the spatial distance determining unit 3102b estimates the spatial distance r in the three-dimensional structure of a composite body based upon the sequence distance d between two amino acid residues located on different amino acid sequences.

$$r = k\, d^n \quad (0 < n < 1)$$

Here, r represents the spatial distance, d represents the sequence distance, and k represents a proportional constant. Here, n is set in a range from 0 to 1, preferably, from 0.5 to 0.6. Moreover, k is set in a range from 2.8Å to 4.8Å, preferably, from 3.3Å to 4.3Å.

Next, the binding site predicting device 3100 determines a charge possessed by each of amino acid residues contained in sequence data of a plurality of amino acids through processes of the charge determining unit 3102c (step SC3-3).

Next, the binding site predicting device 3100 calculates the energy of each of amino acid residues based upon the spatial distance between the amino acid residues determined at step SC3-2 and a charge possessed by each of the amino acid residues determined at step SC3-3, by processes of the energy calculating unit 3102d (step SC3-4).

Further, the binding site predicting device 3100 determines a candidate amino acid residue to form a binding site according to the calculated interaction energy through processes of the candidate amino acid residue determining unit 3102e (step SC3-5).

The binding site predicting device 3100 generates three-dimensional structure information of a composite body by changing binding sites with respect to the composite body at step SC3-7 through processes of the energy minimizing unit 3102g, and calculates energies of respective amino acid residues at step SC3-4 to find a binding site at which the sum of the energies is minimized (steps from step SC3-7 to step SC3-5 are repeated on demand).

Further, the binding site predicting device 3100 determines the binding site at which the sum of the energies is finally minimized as a candidate amino acid residue for the binding site through processes of the candidate amino acid residue determining unit 3102e (step SC3-5). Here, the candidate amino acid residue determining unit 3102e may form a graph in which the sum of protein energies are plotted with respect to amino acid sequences, and output the graph to the output device 3114. Fig. 63 depicts one example of a graph in which the sum of energies is plotted when amino acid residues of protein A and protein B are used as binding residues. By forming this plot graph, it becomes possible to visually confirm which amino acid residues of the two amino acid sequences should be selected as binding residues to minimize the sum of energies.

Thus, the processes in which a binding site is predicted by using amino acid sequences of a plurality of proteins that are known to interact with one another through the present system are completed.

[Process in which a candidate protein on the partner side that is best combined with an objective protein is predicted]

Next, referring to Fig. 59 and the like, the following description will discuss the process in which a candidate protein on the partner side that is best combined with an objective protein is predicted according to the present system in detail. In Fig. 59, the procedure of processes indicated by the solid line is a flow chart that depicts one example of processes in which a candidate protein on the partner side that is best combined with an objective protein is predicted according to the present system of the present embodiment.

First, the binding site predicting device 3100 accesses an external data base and an amino acid sequence data base 3106a of the external system 3200 such as Genbank through processes of an amino acid sequence data acquiring unit 3102a to acquire amino acid sequence data of an objective protein or physiologically active polypeptide (step SC3-1). Further, the binding site predicting device 3100 accesses an external data base and an amino acid sequence data base 3106a of the external system 3200 such as Genbank through processes of a bonding candidate data acquiring unit 3102h to acquire amino acid sequence data of one or a plurality of proteins or physiologically active polypeptide to form bonding candidates of the objective protein (step SC3-6).

Next, the binding site predicting device 3100 generates three-dimensional structure information of a composite body in which an objective protein or physiologically active polypeptide is combined with a protein or physiologically active polypeptide that forms a bonding candidate through processes of the composite body structure generating unit 3102f (step SC3-7).

The binding site predicting device 3100 determines the spatial distance between respective amino acid residues contained in objective amino acid sequence data obtained at step SC3-1 and bonding-candidate amino acid sequence data obtained at step SC3-6 through processes of the spatial distance determining unit 3102b, according to the three-dimensional structure information of the composite body generated at step SC3-7 (step SC3-2).

Next, the binding site predicting device 3100 determines a charge possessed by each of amino acid residues contained in the objective amino acid sequence data and bonding-candidate amino acid sequence data through processes of the charge-determining unit 3102c (step SC3-3).

Further, the binding site predicting device 3100 calculates energies of the respective amino acid residues based upon the spatial distance between the amino acid residues determined at step SC3-2 and the charge possessed by each of the amino acid residues determined at step SC3-3 through processes of the energy calculating unit 3102d (step SC3-4).

Next, the binding site predicting device 3100 generates three-dimensional structure information of a composite body by changing binding sites with respect to the composite body at step SC3-7 through processes of the energy minimizing unit 3102g, and calculates energies of respective amino acid residues at step SC3-4 to find a binding site at which the sum of the energies is minimized (steps from step SC3-7 to step SC3-5 are repeated on demand).

Further, the binding site predicting device 3100 repeats steps from step SC3-6 to SC3-5 with respect to all the bonding candidates through processes of candidate amino acid residue determining unit 3102e so that the energy minimizing process is executed; consequently, the bonding candidate having a binding site at which the sum of the energies is minimized is determined (step SC3-8).

Thus, the processes in which a candidate protein on the partner side that is best combined with an objective protein is predicted through the present system are completed.

[Examples of the present invention]

Referring to Figs. 64 to 71, the following description will discuss examples of the present invention in detail.

[First Example of the present invention; Ribonuclease A]

Referring to Figs. 64 to 66, etc., the following description will discuss the first example of the present invention in detail. The first example relates to binding site predicting processes for a protein as a single substance.

Ribonuclease A, which is a hydrolytic enzyme, is a protein that has been fully examined through experiments. With respect to Ribonuclease A, since the structure of a composite body formed with its inhibitor has been known, binding sites on amino acid sequences are specified.

First the amino acid sequence data of Ribonuclease A is acquired from the protein sequence data base Genbank.

Then, the distance information of amino acid is estimated by the following method from the amino acid sequence data of Ribonuclease A. First, based upon three-dimensional structure information of all the proteins or polypeptides registered in the PDB (Protein Data Bank), the relationship between the distance on sequences and the spatial distance is found for each kind of amino acids. For example, Fig. 64 is a drawing that depicts the relationship between the distance on sequences and the spatial distance of two glutamic acids. As shown in Fig. 64, for example, the fact that the average spatial distance is 20 Å when a glutamic acid and another glutamic acid are apart from each other by 20 residues on the sequences is found through known statistical techniques. Thus, the information indicating the relationship between the distance between amino acid residues on sequences and the spatial distance is obtained.

Further, the charge of amino acid is determined. In this case, charges are assigned to respective amino acid residues in the following manner: - 1 to glutamic acid and aspartic acid; + 1 to each of arginine, lysine and histidine; and 0 to the others.

Then, the interaction energy of each of the amino acid residues is calculated from the following equation:

$$E_{interaction} (K) = q_K \, \Sigma \, q_j/r$$

(In this equation, K represents an amino acid residue number, $E_{interaction}$ (K) represents interaction energy between amino acid residue K and another amino acid residue, j represents a desired amino acid residue other than K, and r represents a spatial distance between amino acid residue K and amino acid residue j).

Thus, based upon the above-mentioned equation, the energy of each of amino acid residues of Ribonuclease A is calculated, and the energies of the respective amino acid residues of Ribonuclease A are plotted in association with the amino acid residue numbers. Fig. 65 is a graph in which the energies of the respective amino acid residues of Ribonuclease A are plotted in association with the amino acid residue numbers.

Further, those amino acid residues of Ribonuclease A having energies of not less than 0 are listed in a table as binding site candidates (Fig. 66). As shown in Fig. 66, among the eighteen binding site candidates, twelve of them actually formed binding sites (binding sites found through experiments). In this manner, the present invention makes it possible to predict the binding site with high precision at high speeds by using only the amino acid sequence information of Ribonuclease A.

Thus, processes of the first example of the present invention are completed.

[Second example of the present invention; Acetylcholine-esterase-inhibitor]

Referring to Figs. 67 to 69, etc., the following description will discuss the second example of the present invention in detail. The second example also relates to binding site predicting processes for a protein as a single substance.

In the second example, the binding site is estimated based upon amino acid sequences of acetylcholine-esterase-inhibitor. In this case, existing three-dimensional structure information data contained in the PDB is utilized without predicting the three-dimensional structure.

Fig. 67 is a drawing that depicts a part of the three-dimensional structure information data of acetylcholine-esterase-inhibitor stored in the PDB. Starting from the second column in Fig. 67, the respective columns indicate atom number, atom kind, chain name, amino acid residue number, X-coordinate, Y-coordinate and Z-coordinate.

For example, supposing that the coordinates of the center of gravity of amino acid residue number I, an atom in a specific main chain and the like are indicated by $(x_I, y_I, z_I)$ and that the coordinates of the center of gravity of amino acid residue number J, an atom in a specific main chain and the like are indicated by $(x_J, y_J, z_J)$, the spatial distance $R_{IJ}$ between amino acid residue number I and amino acid residue number J is calculated based upon the following equation.

$$R_{IJ}{}^2 = (x_I - x_J)^2 + (y_I - y_J)^2 + (z_I - z_J)^2$$

(where $R_{IJ} > 0$)

More specifically, in Fig. 67, the spatial distance between the glutamic acid of amino acid residue number 4 and the aspartic acid of amino acid residue number 5 is calculated based upon the distance between $\alpha$ carbon atoms in the following manner:

$$R_{45}{}^2 = (32.664 - 36.279)^2$$
$$+ (8.451 - 7.196)^2$$
$$+ (205.542 - 205.808)^2$$
$$= 14.714$$

$$R_{45} = 3.835884$$

Fig. 68 is a graph that depicts energies of acetylcholine-esterase-inhibitor found by the present invention. In Fig. 68, ten of those amino acid residues of the acetylcholine-esterase-inhibitor having energies of not less than 0 are extracted as binding site candidates, and after experiments have been carried out to find out whether those sites actually form binding sites, the results show that seven of them are actually binding sites (Fig. 69).

As described above, it is possible to predict the binding site with very high precision. The second example is different from the first example in that the known three-dimensional structure information is utilized. In other words, although the first example and the second example use respectively different spatial-distance determining techniques, both of them provide superior results; thus, whichever spatial-distance determining technique may be used, it becomes possible to obtain the effects of the present invention.

Thus, processes of the second example of the present invention are completed.

[Third example of the present invention; Composite body between "huntingtin-associated protein interacting protein" and "nitric oxide synthase 2A"]

Referring to Fig. 70, etc., the following description will discuss a third example of the present invention in detail. The third example relates to binding site predicting processes at the time when two proteins are bonded to each other. It has been found through experiments that "huntingtin-associated protein interacting protein" is combined with "nitric oxide synthase 2A". Further, it has been known that the binding site in "huntingtin-associated protein interacting protein" is near amino acid residue number 600 while the binding site in "nitric oxide synthase 2A" is near amino acid residue number 100.

Here, in the present example also, in the same manner as the first example, the sequence information was obtained, the three-dimensional structure was predicted and the charge was determined. With respect to the method for converting the distance on sequences between amino acids to the spatial distance, however, supposing that the three-dimensional structure of protein has Gaussian chains, the distance on sequences and the spatial distance are made in association with each other by using the following equation:

$$r = 3.8 \, d^{0.5}$$

Here, r represents a spatial distance, d represents a distance on the sequences.

Moreover, the composite body structure was generated by using the aforementioned high-speed calculating method. In other words, the following equation was used.

$$\text{(Spatial distance)} = k \, (|\text{Distance between attention residue on sequence}$$

$$\text{A and binding residue on sequence}| + |\text{Distance between attention}$$

$$\text{residue on sequence B and binding residue on sequence}|)^n$$

Further, energies of a composite body with respective binding sites being assumed are calculated so that Fig. 70 is formed. Here, in Fig. 70, amino acid residue numbers of the binding sites of huntingtin-associated protein interacting protein are plotted on the axis of abscissas and amino acid residue numbers of the binding sites of nitric oxide synthase 2A are plotted on the axis of ordinates so that upon formation of a composite body by using the respective binding sites, the sum of energies is displayed as contour lines.

According to Fig. 70, energies for the respective binding sites are found in such a manner that, for example, when the binding sites are the 500th amino acid residue in huntingtin-associated protein interacting protein and the 150th amino acid residue in nitric oxide synthase 2A, the energy of the composite body is -10.

As shown in Fig. 70, there are two minimum points in energy, that is, one is a case in which the bonding is made with a binding site in the vicinity of the 600th to 950th amino acid residues in huntingtin-associated protein interacting protein and a binding site in the vicinity of the 25th to 100th amino acid residues in nitric oxide synthase 2A, and the other is a case in which the bonding is made with a binding site in the vicinity of the 650th to 900th amino acid residues in huntingtin-associated protein interacting protein and a binding site in the vicinity of the 475th to 500th amino acid residues in nitric oxide synthase 2A.

Here, the former case corresponds to the actual binding site (portion surrounded by a black circle). Thus, it is possible to predict the binding sites of the two proteins accurately.

Thus, processes of the third example of the present invention are completed.

[Fourth Example of the present invention; E2F transcription factor 1]

Referring to Fig. 71, etc., the following description will discuss a fourth example of the present invention in detail.

The fourth Example relates to bonding-partner predicting processes. Here, E2F transcription factor 1 (hereinafter, referred to as E2F1) is a protein in which information for its interaction partners has been well known under experiments.

Here, the gene data base of Homo Sapiens is retrieved for interaction partners with E2F1 (6600 genes are extracted at random) to form candidate protein amino acid sequence data.

Further, by using the same procedure as that of the third example, a binding site with E2F1 is found for each of partner candidate proteins. Thus, the energy at the binding site having the most stable energy (smallest energy) is defined as an interaction energy. Fig. 71 depicts a histogram that indicates the interaction energy of each of candidate proteins and the number of genes.

As shown in Fig. 71, relative interaction energies can be calculated. For example, there are 100 proteins having interaction energies greater than 90 (energies smaller than -90), and these have a high possibility of forming interaction partners. This method makes it possible to calculate the interaction systematically at very high speeds.

Thus, processes of the fourth example of the present invention are completed.

[Other Embodiments]

While the present invention has been described in detail and with reference to specific examples thereof, it will be apparent to one skilled in the art that various changes and modifications can be made therein without departing from the spirit and scope of the invention disclosed in claims.

For example, the above-mentioned embodiment has exemplified a case in which the binding site predicting device 3100 carries out interaction site predicting processes as a stand alone system; however, another arrangement may be used in which: interaction site predicting processes are carried out in response to a request from a client terminal that is constituted by a device other than the binding site predicting device 3100, and the prediction results are returned to the client terminal.

Moreover, among those processes explained in the embodiment, all or a part of the processes that have been explained as automatic processes may be executed as manual processes, or all or a part of the processes that have been explained as manual processes may be executed as automatic processes by using a known method.

In addition to these, process procedures, control procedures, specific names, information including parameters such as various registered data and retrieving conditions, screen examples and data base structures, described in the above document and figures, may be desirably modified, unless otherwise indicated.

Furthermore, with respect to the binding site predicting device 3100, the respective constituent elements shown in the Figures are explained based upon functional concept, and need not be physically formed in the same manner as shown in the Figures.

For example, with respect to processing functions possessed by the respective servers of the binding site predicting device 3100, in particular, the respective processing functions to be carried out by the control unit 3102, all or a desired part thereof may be achieved by a CPU (Central Processing Unit) and programs that are interpreted and executed in the CPU, or may be achieved as hardware based upon wired logic. Here, the programs are recorded in a recording medium, which will be described later, and read mechanically by the binding site predicting device 3100 on demand.

In other words, computer programs, which give instructions to the CPU in cooperation with the OS (Operating System) and are used to carry out various processes, are recorded in the storage unit 3106 or the like, such as ROM or HD. These computer programs, which are loaded in RAM or the like and executed, constitute a control unit 3102 in cooperation with the CPU. Moreover, these programs may be recorded in an application program server that is connected to the binding site predicting device 3100 through a desired network 3300, and all or a part thereof may be downloaded, if necessary.

Furthermore, the programs relating to the present invention may be stored in a recording medium that can be read by a computer. Here, the term "recording medium" includes a desired "portable physical medium", such as a flexible disk, a magneto-optical disk, ROM, EPROM, EEPROM, CD-ROM, MO, and DVD; a desired "fixed physical medium", such as ROM, RAM and HD installed in various computer systems; and a "communication medium" for holding programs in a short period, such as communication lines and carrier waves to be used upon transferring programs through a network typically represented by LAN, WAN and Internet.

Here, the term, "program" refers to a data processing method described in a desired language and description method, irrespective of formats such as source codes and binary codes. In addition, not limited to a single structure, "program" may be constituted in a dispersed manner as a plurality of modules and libraries, or may achieve its functions in cooperation with a different program typically prepared as an OS (Operating System). With respect to a specific structure used for reading a recording medium, reading procedure or installing procedure after the reading process in the respective devices shown in the present embodiment, known structures and procedures can be utilized.

Furthermore, the various data bases and the like (amino acid sequence data base 3106a to process result files 3106g), stored in the storage unit 3106, are prepared as storage units such as memory devices like RAM and ROM, fixed disk devices like hard disks, flexible disks and optical disks, and these units store various programs used for various processes and Web site supplies, tables, files, data bases, files for use in Web pages and the like.

Here, the binding site predicting device 3100 may be achieved by connecting peripheral devices such as a printer, a monitor and an image scanner to an information processing apparatus such as an information processing terminal' like a personal computer and a work station that have been known, and by installing software (including programs, data and the like) used for achieving the method of the present invention in the information processing apparatus.

Moreover, with respect to the specific mode of dispersed or integrated structures of the binding site predicting device 3100, not limited to the mode shown in Figures, all or a part thereof may be functionally or physically dispersed or integrated based upon a desired unit determined according to various loads and the like to form the system. For example, the respective data bases may be individually prepared as independent data base devices, and a part of the processes may be achieved by using a CGI (Common Gateway Interface).

Moreover, the network 3300, which has a function for mutually connecting the binding site predicting device 3100 and the external system 3200, may be prepared as any of networks such as the Internet, Intranet, LAN (including both of wire/wireless systems), VAN, personal computer communication network, public telephone network (including both of analog/digital systems), dedicated line network (including both of analog/digital systems), CATV network, portable line exchange network/portable packet exchange network such as IMT2000 system, GSM system or PDC/PDC-P system, wireless call network, local wireless network such as Bluetooth, PHS network, and satellite communication networks such as CS, BS or ISDB. In other words, the present system can transmit and receive various data through any desired network regardless of wire or wireless system.

As described above in detail, according to the present invention, spatial distance data between amino acid residues in a three-dimensional structure of a protein or a physiologically active polypeptide from amino acid sequence data of the protein or the physiologically active polypeptide is obtained; and by specifying an electrostatically instable amino acid residue based upon the distance data and the charge of each of amino acids, a binding site is predicted; thus, it becomes possible to provide a binding site predicting device which can effectively predict a binding site with high precision at high speeds, by utilizing the fact that an amino acid residue that is likely to become electrostatically instable on amino acid sequences of a protein or a physiologically active polypeptide tends to form a binding site, such a binding site predicting method and a program and a recording medium for such a method.

Moreover, according to the present invention, amino acid sequence data of an objective protein or a physiologically active polypeptide is acquired so that the spatial distance between amino acid residues contained in the acquired amino acid sequence data is determined, and the charge possessed by each of the amino acid residues contained in the acquired amino acid sequence data is determined; based upon the determined spatial distance between amino acid residues and the determined charge possessed by each of the amino acid residues, energies of the amino acid residues are calculated; and based upon the calculated energies, a candidate amino acid residue to form a binding site is determined; thus, it becomes possible to provide a binding site predicting device which can effectively predict a binding site with high precision at high speeds by utilizing the fact that an amino acid residue that is likely to become electrostatically instable on amino acid sequences of a protein or a physiologically active polypeptide tends to form a binding site, such a binding site predicting method and a program and a recording medium for such a method.

Furthermore, according to the present invention, amino acid sequence data of a plurality of objective proteins or physiologically active polypeptides is acquired so that three-dimensional structure information of a composite body in which the objective proteins or physiologically active polypeptides are combined with one another is generated; the spatial distance between amino acid residues contained in the acquired sequence data of amino acids is determined based upon the three-dimensional structure information of the composite body thus generated, and the charge possessed by each of the amino acid residues contained in the acquired sequence data of amino acids is determined; based upon the determined spatial distance between amino acid residues and the determined charge possessed by each of the amino acid residues, energies of the amino acid residues are calculated; and three-dimensional structure information of the composite body is generated by changing the binding sites of the composite body, and energies of the amino acid residues are calculated to find a binding site at which the sum of the energies is minimized so that the binding site at which the sum of the energies is minimized is determined as a candidate amino acid residue for a binding site; thus, it becomes possible to provide a binding site predicting device which can effectively predict a binding site with high precision at high speeds, by utilizing the fact that an amino acid residue that is likely to become electrostatically instable on amino acid sequences of a protein or a physiologically active polypeptide tends to form a binding site, such a binding site predicting method and a program and a recording medium for such a method.

Furthermore, according to the present invention, amino acid sequence data of an objective protein or physiologically active polypeptide is acquired and amino acid sequence data of one or a plurality of proteins or physiologically active polypeptides to form bonding candidates are acquired so that three-dimensional structure information of a composite body in which the objective protein or physiologically active polypeptide is combined with proteins or physiologically active polypeptides to form bonding candidates are combined with each other is generated; the spatial distance between amino acid residues contained in the acquired sequence data of objective amino acid and the sequence data of the bonding candidate amino acid sequence data is determined according to the generated three-dimensional structure information of the composite body, and the charge possessed by each of the amino acid residues contained in the sequence data of the objective amino acid and the sequence data of the bonding candidate amino acid is determined; based upon the determined spatial distance between amino acid residues and the determined charge possessed by each of the amino acid residues, energies of the amino acid residues are calculated; and three-dimensional structure information of the composite body is generated by changing the binding sites of the composite body, and energies of the amino acid residues are calculated to find a binding site at which the sum of the energies is minimized so that a bonding candidate having the binding site at which the sum of the energies is minimized is determined after having executed an energy-minimizing process on all the bonding candidates; thus, it becomes possible to provide a binding site predicting device which can effectively predict a protein as a binding site with high precision at high speeds, by utilizing the fact that an amino acid residue that is likely to become electrostatically instable on amino acid sequences of a protein or a physiologically active polypeptide tends to form a binding site, such a binding site predicting method and a program and a recording medium for such a method.

(V) Moreover, referring to Figures, the following description will discuss embodiments of a protein structure optimizing device, a protein structure optimizing method, and a program and a recording medium for such a method, according to the present invention. However, the present invention is not intended to be limited by these embodiments.

The following embodiments exemplify a system in which the present invention is applied to "MOPAC2000 ver. 1.0"(product name) made by Fujitsu Ltd (company name); however, not limited to this, the present invention may be applied to any other programs in the same manner.

[Summary of the present invention]

A summary of the present invention is described below, and then the structure, process, and others of the present invention are described in detail. Fig. 72 is a flowchart depicting a basic principle of the present invention.

The present invention generally has the following basic features. Firstly, the present invention acquires coordinate data of protein (step SA4-1). Here, the coordinate data of protein to be acquired may be any coordinate data of protein, such as coordinate data obtained through X-ray crystal analysis with hydrogen being added thereto by known modeling software (for example, "WebLab Viewer Pro 4.2" (product name) of Accelrys Inc. (company name), "Insight II" (product name) (www.accelrys.com), "SYBYL 6.7" of Tripos, Inc. (company name), "Chem3D 7.0" (product name) of CambridgeSoft Corporation (company name) (www.camsoft.com)) and coordinate data registered in a known protein-structure database, such as PDB (Protein Data Base).

The present invention then extracts, as for coordinate data of protein, coordinates of a neighboring amino acid residue group within a predetermined distance (for example, r angstrom(Å)) from a specific amino acid residue i (step SA4-2). That is, an amino acid residue group including atoms within a predetermined distance from all atoms included in the amino acid residue i is a neighboring amino acid residue group, and coordinates of all atoms included in this neighboring amino acid residue group are extracted. When the extracted neighboring amino acid residue group includes cysteine (CYS) that has a disulfide bond with another cysteine (CYS), this other CYS may also be included as the neighboring amino acid residue group.

When coordinates are automatically cut out with the operation of step SA4-2, its section becomes radical, thereby causing an inconvenience. To solve the inconvenience, the present invention adds a cap substituent (for example, hydrogen atom (H) or methyl group ($CH_3$)) to a section of the neighboring amino acid residue group (step SA4-3).

The present invention then calculates the entire charge of the neighboring amino acid residue group with the cap substituent being added thereto (step SA4-4). The charge calculation may be performed using any known charge calculating scheme, for example, by subtracting the number of acidic amino acid residues from the number of basic amino acid residues for high-speed calculation.

The present invention then uses the charge to perform structural optimization on the neighboring amino acid residue group with the cap substituent being added thereto by using a known molecular orbital computation program (for example, a semi empirical molecular orbital computation program, such as "MOPAC 2000 ver. 1.0" (product name)) or the like. (step SA4-5)

The present invention then substitutes the optimized atomic coordinates for the corresponding atomic coordinates on the initial coordinate data of protein (step SA4-6).

The present invention then applies step SA4-2 to step SA4-6 to all amino acid residues i (performing a loop process by incrementing i from the first amino acid residue to the last amino acid residue) to optimize all amino acid residues (step SA4-7).

The present invention then takes the structural data obtained at step SA4-7 as an initial structure to perform a plurality of procedures (n times) from step SA4-1 to step SA4-7, thereby further increasing the accuracy in structure optimization (step SA4-8).

[System Configuration]

First, the configuration of the system is described. Fig. 73 is a block diagram of one example of the configuration of the system to which the present invention is applied, only conceptually depicting a part of the configuration related to the present invention. The system schematically has a structure in which a protein-structure optimizing device 4100 and an external system 4200 that provides external databases related to protein-structure information and the like and external programs for homology retrieving and the like are communicably connected to each other via a network 4300.

In Fig. 73, the network 4300 has a function of mutually connecting the protein-structure optimizing device 4100 and the external system 4200 to each other, and exemplified by the Internet.

In Fig. 73, the external system 4200 is mutually connected to the protein-structure optimizing device 4100 via the network 4300, and has a function of providing users with a web site for executing an external database regarding protein-structure information or the like and an external program for homology retrieving, motif retrieving, or the like.

Here, the external system 4200 may be configured as a WEB server, an ASP server, or the like, and its hardware structure may be configured by an generally- and commercially-available information processing device, such as a work station and a personal computer, and its attached device. Also, each function of the external system 4200 is achieved by a CPU, a disk device, a memory device, an input device, an output device, a communication control device, and the like included in the hardware structure of the external system 4200, a program controlling these devices, and the like.

In Fig. 73, the protein-structure optimizing device 4100 generally includes a control unit 4102 that performs centralized control over the entire protein-structure optimizing device 4100, such as a CPU, a communication control interface unit 4104 connected to a communication device (not shown), such as a router connected to a communication line or the like, an input/output control interface unit 4108 connected to an input device 4112 and an output device 4114, and a storage unit 4106 that stores various database, tables, and the like. These components are communicably connected to each other via an arbitrary communication channel. Furthermore, this protein-structure optimizing device 4100 is communicably connected to the network 4300 via a communication device, such as a router, and a wired or wireless communication line, such as a dedicated line.

Various databases, tables, and the like (protein-structure information database 4106a and process result files 4106b) stored in the storage unit 4106 each are a storage unit, such as a fixed disk device, that stores various programs, tables, files, databases, files for web pages, and others for various processes.

Of these components of the storage unit 4106, the protein-structure information database 4106a is a coordinate-data storage unit that stores coordinate data of a three-dimensional structure of protein or the like. The protein-structure information database 4106a may be an external database, such as a PDB to be accessed via the Internet, or may be an in-house database created by, for example, copying such an external database, storing original information, or further adding unique annotation information and the like.

The process result file 4106b is a process result storage unit that stores information regarding the process result of each process performed by the control unit 4102 of the protein-structure optimizing device 4100.

In Fig. 73, the communication control interface unit 4104 controls communication between the protein-structure optimizing device 4100 and the network 4300 (or the communication device, such as a router). That is, the communication control interface unit 4104 has a function of communicating data with another terminal via a communication line.

In Fig. 73, the input/output control interface unit 4108 controls the input device 4112 and the output device 4114. Here, as the output device 4114, a monitor (including a home-use television) and also a loudspeaker can be used (in the following, the output device 4114 may be described as a monitor). Also, as the input device 4112, a keyboard, a mouse, a microphone, or the like can be used. Furthermore, the monitor also achieves a pointing-device function in cooperation with a mouse.

In Fig. 73, the control unit 4102 has an internal memory for storing a control program, such as an Operating System (OS), a program in which various procedures and the like are defined, and predetermined data and, with these programs and the like, performs various information processing for executing various processes. The control unit 4102 functionally and conceptually, includes a coordinate-data acquiring unit 4102a, a neighboring amino acid residue group extracting unit 4102b, a cap adding unit 4102c, a charge calculating unit 4102d, a structure optimizing unit 4102e, and an atomic coordinate substituting unit 4102f.

Of these components, the coordinate data acquiring unit 4102a is a coordinate data acquiring unit that acquires coordinate data of protein. The neighboring amino acid residue group extracting unit 4102b is an neighboring amino acid residue group extracting unit that extracts, from the coordinate data of protein, coordinates of a neighboring amino acid residue group included within a predetermined distance from a specific amino acid residue. The cap adding unit 4102c is a cap adding unit that adds a cap substituent to a section of the neighboring amino acid residue group. The charge calculating unit 4102d is a charge calculating unit that calculates the entire charge of the neighboring amino acid residue group with the cap substituent being added thereto by the cap adding unit. The structure optimizing unit 4102e is a structure optimizing unit that performs, as for the neighboring amino acid residue group with the cap substituent being added thereto by the cap adding unit, structure optimization on the atomic coordinates of the specific amino acid residue by using the charge calculated by the charge calculating unit. The atomic coordinate substituting unit 4102f is an atomic coordinate substituting unit that substitutes the atomic coordinates optimized by the structure optimizing unit for the corresponding atomic coordinates on the coordinate data of protein. Details of the processes performed by these components are described further below.

[Process of the System]

Next, one example of a process of the present system according to the embodiment structured as mentioned above is described in detail below with reference to Figs. 74 to 90.

[Main Process]

First, details of main processes are described with reference to Fig. 74. Fig. 74 is a flowchart depicting one example of the main processes of the present system according to the present invention.

The protein-structure optimizing device 4100 acquires, with the process of the coordinate data acquiring unit 4102a, coordinate data of desired protein from the protein-structure information database 4106a or an external database of the external system 4200 (step SB4-1), Here, the coordinate data of protein to be acquired may be any coordinate data of protein, such as coordinate data obtained through X-ray crystal analysis with hydrogen being added thereto by known modeling software (for example, "WebLab Viewer Pro 4.2" (product name) of Accelrys Inc. (company name), "Insight II" (product name) (www.accelrys.com), "SYBYL 6.7" of Tripos, Inc. (company name), "Chem3D 7.0" (product name) of CambridgeSoft Corporation (company name) (www.camsoft.com)) and coordinate data registered in a known protein-structure database, such as PDB (Protein Data Bank).

Fig. 75 is a drawing that depicts one example of coordinate data of protein. In the example shown in Fig. 75, coordinate data in PDB format is used. Also, with a commercially available program, hydrogen is added to structure information obtained through X-ray crystal analysis.

Referring back to Fig. 74, with the process of the control unit 4102, the protein-structure optimizing device 4100 adds 1 to a counter n (its initial value is 0) representing the number of processes (step SB4-2).

Also, with the process of the control unit 4102, the protein-structure optimizing device 4100 adds 1 to a counter i (its initial value is 0) representing an amino acid residue number (step SB4-3).

With the process of the neighboring amino acid residue group extracting unit 4102b, the protein-structure optimizing device 4100 extracts, as for coordinate data of protein to be processed, coordinates of the neighboring amino acid residue group included within a predetermined distance (for example, r angstrom) from the specific amino acid residue i (step SB4-4). That is, an amino acid residue k (k is not i) group including atoms 1 within a predetermined distance from all atoms j included in the amino acid residue i is a neighboring amino acid residue group, and coordinates of all atoms m included in this neighboring amino acid residue group are extracted.

When the extracted neighboring amino acid residue group includes cysteine (CYS) that has a disulfide bond with another cysteine (CYS), this other CYS may also be included as the neighboring amino acid residue group. That is, when the extracted neighboring amino acid residue group includes cysteine (CYS), the neighboring amino acid residue group extracting unit 4102b determines whether that cysteine (CYS) has a disulfide bond with another cysteine (CYS) not included in the neighboring amino acid residue group. If such another cysteine (CYS) is present, this cysteine (CYS) is also included as the neighboring amino acid residue group.

When the coordinates are automatically cut out with the operation at step SB4-4, its section becomes radical, thereby causing an inconvenience. To solve the inconvenience, with the process of the cap adding unit 4102c, the protein-structure optimizing device 4100 adds a cap substituent (for example, a hydrogen atom (H) or a methyl group $(CH_3)$) to a section of the neighboring amino acid residue group (step SB4-5). Which of a hydrogen or a methyl group is to be used as the cap substituent is determined by the user depending on the purpose.

Details of a cap adding process performed by the cap adding unit 4102c are described with reference to Figs. 76 to 83.

Fig. 76 is a flowchart depicting one example of a cap adding process according to the present embodiment in which a hydrogen atom is added to a section. Fig. 77 is a drawing that depicts the concept of the original coordinates and the coordinates after addition of a cap substituent. Fig. 76 depicts one example of a process in which, to the original coordinates shown in Fig. 77 (at left), a cap is added (shown at right). An arbitrary residue of the neighboring amino acid residue group is denoted as j.

When the amino acid residue j is N-terminal amino acid (step SC4-1), the amino side of the amino acid residue j does not form a section. Therefore, the cap adding unit 4102c regards cap addition as not being required (step SC4-2).

When the amino acid residue j is not N-terminal amino acid (step SC4-1) and an adjacent amino acid residue j-1 is also included in the extracted amino acid residue group (step SC4-3), the amino side of the residue j does not form a section. Therefore, the cap adding unit 4102c regards cap addition as not being required (step SC4-4).

On the other hand, when the adjacent amino acid residue j-1 is also not included in the extracted amino acid residue group (step SC4-3), the cap adding unit 4102c takes main chain carbonyl carbon of the amino acid residue j-1 as $C_{j-1}$ (step SC4-5).

The cap adding unit 4102c then takes main chain amino group nitrogen of the amino acid residue j as $N_j$ (step SC4-6).

The cap adding unit 4102c then determines, according to the following equation (1), the position of a cap hydrogen atom $H_{CAPN}$ to be added (step SC4-7).

[Equation (1)]

$$\overrightarrow{N_j H_{CAPN}} = \frac{\overrightarrow{N_j C_{j-1}}}{\left|\overrightarrow{N_j C_{j-1}}\right|} \times R_{NH} \qquad (R_{NH} = 1.01\text{Å})$$

Fig. 78 is a flowchart depicting one example of the cap adding process according to the present embodiment in which a hydrogen atom is added to the section. Fig. 79 is a drawing that depicts the concept of the original coordinates and the coordinates after addition of a cap substituent. Fig. 78 depicts one example of a process in which, to the original coordinates shown in Fig. 79 (at left), a cap is added to the calboxyl side (shown at right). An arbitrary residue of the neighboring amino acid residue group is denoted as j.

When the amino acid residue j is C-terminal amino acid (step SD4-1), the amino side of the amino acid residue j does not form a section. Therefore, the cap adding unit 4102c regards cap addition as not being required (step SD4-2).

When the amino acid residue j is not C-terminal amino acid (step SD4-1) and an adjacent amino acid residue j+1 is also included in the extracted amino acid residue group (step SD4-3), the amino side of the residue j does not form a section. Therefore, the cap adding unit 4102c regards cap addition as not being required (step SD4-4).

On the other hand, when the adjacent amino acid residue j+1 is also not included in the extracted amino acid residue group (step SD4-3), the cap adding unit 4102c takes main chain amino group nitrogen of the amino acid residue j+1 as $N_{j+1}$ (step SD4-5).

The cap adding unit 4102c then takes main chain carbonyl carbon of the amino acid residue j as $C_j$ (step SD4-6).

The cap adding unit 4102c then determines, according to the following equation (2), the position of a cap hydrogen atom $H_{CAPC}$ to be added (step SD4-7).

[Equation (2)]

$$\overrightarrow{C_jH_{CAPC}} = \frac{\overrightarrow{C_jN_{j+1}}}{\left|\overrightarrow{C_jN_{j+1}}\right|} \times R_{Csp2H} \qquad (R_{Csp2H} = 1.08\text{Å})$$

Fig. 80 is a flowchart depicting one example of the cap adding process according to the present embodiment in which a methyl group is added to the section. Fig. 81 is a drawing that depicts the concept of the original coordinates and the coordinates after addition of a cap substituent. Fig. 80 depicts one example of a process in which, to the original coordinates shown in Fig. 81 (at left), a cap is added to the amino group side (shown at right). An arbitrary residue of the neighboring amino acid residue group is denoted as j.

When the amino acid residue j is N-terminal amino acid (step SE4-1), the amino side of the amino acid residue j does not form a section. Therefore, the cap adding unit 4102c regards cap addition as not being required (step SE4-2).

When the amino acid residue j is not N-terminal amino acid (step SE4-1) and an adjacent amino acid residue j-1 is also included in the extracted amino acid residue group (step SE4-3), the amino side of the residue j does not form a section. Therefore, the cap adding unit 4102c regards cap addition as not being required (step SE4-4).

On the other hand, when the adjacent amino acid residue j-1 is also not included in the extracted amino acid residue group (step SE4-3), the cap adding unit 4102c takes main chain carbonyl carbon of the amino acid residue j-1 as $C_{j-1}$ (step SE4-5).

The cap adding unit 4102c then takes main chain amino group nitrogen of the amino acid residue j as $N_j$ (step SE4-6).

The cap adding unit 4102c then takes main chain $\alpha$ carbon of the amino acid residue j as $CA_j$ (step SE4-7).

The cap adding unit 4102c then determines, according to the following equation (3), the position of cap methyl group carbon $C_{CAPN}$ to be added (step SE4-8).

[Equation (3)]

$$\overrightarrow{N_jC_{CAPN}} = \frac{\overrightarrow{N_jC_{j-1}}}{\left|\overrightarrow{N_jC_{j-1}}\right|} \times R_{NCsp3} \qquad (R_{NCsp3} = 1.47\text{Å})$$

The cap adding unit 4102c then determines, according to the following conditions (equations (4)), the positions of three cap methyl group hydrogen $H_{CAPNk}$ (k=1, 2, 3) to be added (step SE4-9).

[Equations (4)]

Bond length $\qquad \left|\overrightarrow{H_{CAPNk}C_{CAPN}}\right| = R_{Csp3H} \qquad (R_{Csp3H} = 1.09\text{Å})$

Bond angle $\qquad \angle H_{CAPNk}C_{CAPN}N_j = A_{Csp3} \qquad (A_{Csp3} = 109.5°)$

Dihedral angle $\qquad \angle H_{CAPNk}C_{CAPN}N_jCA_j = D_k \qquad (D_1 = 180.0°, D_2 = 60.0°, D_3 = -60.0°)$

Fig. 82 is a flowchart depicting one example of the cap adding process according to the present embodiment in which a methyl group is added to the section. Fig. 83 is a drawing that depicts the concept of the original coordinates and the coordinates after addition of a cap substituent. Fig. 82 depicts one example of a process in which, to the original coordinates shown in Fig. 83 (at left), a cap is added to the carboxyl group side (shown at right). An arbitrary residue of the neighboring amino acid residue group is denoted as j.

When the amino acid residue j is C-terminal amino acid (step SF4-1), the amino side of the amino acid residue j does not form a section. Therefore, the cap adding unit 4102c regards cap addition as not being required (step SF4-2).

When the amino acid residue j is not C-terminal amino acid (step SF4-1) and an adjacent amino acid residue j+1 is also included in the extracted amino acid residue group (step SF4-3), the amino side of the residue j does not form a section. Therefore, the cap adding unit 4102c regards cap addition as not being required (step SF4-4).

On the other hand, when the adjacent amino acid residue j+1 is also not included in the extracted amino acid residue group (step SF4-3), the cap adding unit 4102c takes main chain amino group nitrogen of the amino acid residue j+1 as $N_{j+1}$ (step SF4-5).

The cap adding unit 4102c then takes main chain carbonyl carbon of the amino acid residue j as $C_j$ (step SF4-6).

The cap adding unit 4102c then takes main chain $\alpha$ carbon of the amino acid residue j as $CA_j$ (step SF4-7).

The cap adding unit 4102c then determines, according to the following equation (5), the position of a cap methyl group carbon $C_{CAPC}$ to be added (step SF4-8).

[Equation (5)]

$$\overrightarrow{C_j C_{CAPC}} = \frac{\overrightarrow{C_j N_{j+1}}}{\left|\overrightarrow{C_j N_{j+1}}\right|} \times R_{Csp2Csp3} \qquad (R_{Csp2Csp3} = 1.52)$$

The cap adding unit 4102c then determines, according to the following conditions (equations (6)), the position of three cap methyl group hydrogen $H_{CAPCk}$ (k=1, 2, 3) to be added (step SF4-9).

[Equations (6)]

Bond length $\qquad \left|\overrightarrow{H_{CAPCk} C_{CAPC}}\right| = R_{Csp3H} \qquad (R_{Csp3H} = 1.09\text{Å})$

Bond angle $\qquad \angle H_{CAPCk} C_{CAPC} C_j = A_{Csp3} \qquad (A_{Csp3} = 109.5°)$

Dihedral angle $\qquad \angle H_{CAPCk} C_{CAPC} C_j CA_j = D_k \qquad (D_1 = 180.0°,\ D_2 = 60.0°,\ D_3 = -60.0°)$

Here, in equations (1) to (6), R, A, and D are a standard bond length, a standard bond angle, a standard dihedral angle, respectively, and are their numerical values under the conditions mentioned above are merely examples (refer to Tsuneo Hirano and Kazutoshi Tanabe, "Molecular Orbital Method MOPAC guidebook (third revision)", Kaibundo Publishing, 1999).

With this, the cap adding process ends.

Referring back to Fig. 74, upon adding a cap to the section of every neighboring amino acid residue group, the protein-structure optimizing device 4100 performs charge calculation on the entire amino acid residue group extracted at step SB4-4. That is, in not only MOPAC 2000 but molecular orbital computation in general, a charge of the entire system to be processed is given as input data. Therefore, with the process of the charge calculating unit 4102d, the protein-structure optimizing device 4100 calculates the entire charge of the neighboring amino acid residue group with a cap substituent being added thereto (step SB4-6).

The charge computation may be performed by any known charge computation scheme. For example, by using the following equation (7), the number of acidic amino acid residues can be subtracted from the number of basic amino acid residues for high-speed calculation.

(entire charge)=(the number of basic amino acid residues)-(the

number of acidic amino acid residues) $\hspace{4cm}$ Equation (7)

Here, the basic amino acid residues are ARG, LYS, and the like, while the acidic amino acid residues are ASP, GLU, and the like. A type of amino acid is decided with three characters notation of data in PDB format (characters of 18 to 20 columns) to be given as input data, as shown in Fig. 84 (refer to "PDB File Format Contents Guide Version 2.2" (20 December 1996)). Also, neutral amino acid residues (for example, ARG, LYS, ASP, and GLU) and protonated HIS (charge of +1) are represented, according to molecular dynamics calculating program "Amber 7" (University of California, 2002.), as ARN, LYN, ASH, GLH, and HIP in inputted PDB data for discrimination. Also, charges of unnatural amino acid residues, user-defined amino acid, and ligand molecules can also be individually set. For example, it is set with a program such that phosphorylated THR is defined as TPO and its amino acid is provided with a charge of -2.

Then, with the process of the structure optimizing unit 4102e, to generate an input file in MOPAC 2000, the protein-structure optimizing device 4100 then sets, to each atom forming the amino acid residue i, an "optimizing flag" representing the atom that is subjected to an optimizing process (step SB4-7). When structure optimization is performed with a general chemical computation scheme (such as a molecular orbital scheme and a molecular-mechanical scheme) not restricted to MOPAC 2000, an atom to be moved to an optimum position and an atom to be fixed in coordinate and not to be moved in position are set for partial structure optimization. Here, setting an atom to be moved to an optimum position so that the atom can be discriminated as input data is referred herein as "setting an optimizing flag" according to the convention in MOPAC 2000 (refer to "MOPAC 2000 Manual", Fujitsu Limited, Tokyo, 2000).

Specifically, when performing structure optimization of hydrogen, the structure optimizing unit 4102e sets an optimizing flag to a hydrogen atom of the amino acid residue i. Fig. 85 is a drawing that depicts one example in which an optimizing flag is set to a hydrogen atom of the amino acid residue i. Fig. 85 depicts, for input PDB data with hydrogen added to protein having a PDB code of "1CBI", an adjacent amino acid residue group when the specific amino acid residue is a 50-th amino acid residue (i=50) and the distance is 3.0 angstroms (r=3.0 angstroms). Also, with the scheme described above, a cap substituent (hydrogen atom) is added to the section of the amino acid residue group. Furthermore, at step SB4-6 described above, charge computation is performed in consideration of all atoms shown in the drawing. In Fig. 85, a portion represented by bold lines and balls is PHE50 (phenylalanine of an amino acid residue of i=50), which is a center residue for computation. Of PHE50, hydrogen atoms to each of which an optimizing flag is added are represented by balls.

Also, when performing structure optimization of a side chain, the structure optimizing unit 4102e sets an optimizing flag to a hydrogen atom and a side-chain atom of the amino acid residue i. Fig. 86 is a drawing that depicts one example in which an optimizing flag is set to a hydrogen atom and a side-chain atom of the amino acid residue i. Fig. 86 depicts, for input PDB data with hydrogen added to protein having a PDB code of "1CBI", an adjacent amino acid residue group when the specific amino acid residue is a 50-th amino acid residue (i=50) and the distance is 3.0 angstroms (r=3.0 angstroms). Also, with the scheme described above, a cap substituent (hydrogen atom) is added to each section of the amino acid residue group. Furthermore, at step SB4-6 described above, charge computation is performed in consideration of all atoms shown in the drawing. In Fig. 86, a portion represented by bold lines and balls is PHE50 (phenylalanine of an amino acid residue of i=50), which is a center residue for computation. Of PHE50, hydrogen atoms and side-chain atoms each of which an optimizing flag is added are represented by balls.

Furthermore, when performing structure optimization of all atoms, the structure optimizing unit 4102e sets an optimizing flag to every atom of the amino acid residue i. However, in the current molecular orbital theories including MOPAC 2000, it is difficult to reproduce the secondary structure of the main chain structure, and therefore, optimization of the main chain atom is generally not performed. If a theory allowing the secondary structure to be reproduced with high accuracy is constructed, optimization of the entire structure will be effective.

Referring back to Fig. 74, with the process of the structure optimizing unit 4102e, the protein-structure optimizing device 4100 generates an input file for MOPAC 2000 (step SB4-8). Fig. 87 is a drawing that depicts one example of an input file of MOPAC 2000. As shown in Fig. 87, an input file including a charge, coordinate data of the adjacent amino acid residue group, the optimizing flags, and the like is generated.

With the process of the structure optimizing unit 4102e, the protein-structure optimizing device 4100 then performs structure optimization on the adjacent amino acid residue group with the cap substituents being added thereto by using the charge and by using MOPAC 2000 for atomic coordinates of a specific amino acid residue (step SB4-9). Fig. 88 is a drawing that depicts one example of an output file indicating the results of a structure optimizing process by MOPAC 2000. As shown in Fig. 88, the coordinate data after structure optimization is outputted. Note that, in Fig. 88, coordinates with "*" marks are optimized portions.

With the process of the atomic-coordinate substituting unit 4102f, the protein-structure optimizing device 4100 then substitutes the optimized atomic coordinates for the corresponding atomic coordinates on the initial coordinate data of protein (step SB4-10). That is, since the coordinates with "*" marks in the process results of MOPAC 2000 (output file) are an optimized portion, the protein-structure optimizing device 4100 extracts this portion and substitutes this portion for the portion of the corresponding coordinates in the coordinate data prepared at step SB4-1.

The protein-structure optimizing device 4100 then applies steps SB4-3 to SB4-10 to all amino acid residues i (performing a loop process by incrementing i from the first amino acid residue to the last amino acid residue) to optimize all amino acid residues (step SB4-11).

The protein-structure optimizing device 4100 then takes the structural data obtained at step SB4-10 as an initial structure to perform a procedure from step SB4-2 to step SB4-7 a predetermined plurality number of times (n times), thereby further increasing the accuracy in structure optimization (step SA4-12). That is, with the process at step SB4-4 to step SB4-10 being performed on the N-residue to the C-terminal residue, coordinate data in PDB format with a partial structure of all amino acid residues being optimized can be obtained. With this coordinate data as being an input, energy calculation is performed through MOPAC with the coordinates being fixed (without setting an optimizing flag to all atoms). Also, the loop process including the operations from step SB4-4 to step SB4-10 may be performed by using, for example, a script program.

With this, the main processes end.

[Calculation Example According to the Invention]

Next, details of a calculation example according to the present invention are described with reference to Figs. 89, 90, and others. In this calculation example, "Japanese Pear S3-Ribonuclease (PDB ID:1IQQA) is used as a sample molecule, and the 200-th amino acid residue (3262 atom C1047H1619 N285 0300 S11) is taken as the specific amino acid residue. Also, the type of the calculator used in this calculation example is "AlphaServer ES40 (CPU Alpha 21264 833MHz)" (product name) of COMPAQ (company name). Fig. 89 is a drawing that depicts calculation results when a hydrogen structure is optimized by using a conventional optimizing method (MOZYME scheme+BFGS scheme) and when the structure is optimized by using the method of the present invention. Fig. 90 is a drawing that depicts calculation results when a side chain structure is optimized by using a conventional optimizing method (MOZYME scheme+BFGS scheme) and when the structure is optimized by using the method of the present invention. In Figs. 89 and 90, the vertical axis represents Heat of Formation (kcal mol$^{-1}$), while the horizontal axis represents CPU time (seconds). Also, a value of Heat of Formation in the initial structure is -1044.53571 kcal·mol$^{-1}$.

In the calculation example, the relation between the calculation time and energy (heat of formation) is such that, in the method according to the present invention, convergence in energy is quick with respect to the calculation time. It can be seen that energy is converged by repeating the entire loop three to fifth times (n=3 to 5). Also, r may be set to be small if the calculation time is more prioritized than calculation accuracy, while r may be set to be large if, by contrast, calculation accuracy is more prioritized than the calculation time.

Also, as for the maximum memory capacity required for the calculation example, in the conventional scheme, 506 megabytes are required for hydrogen structure optimization, and 667 megabytes are required for side chain structure optimization. On the other hand, in the method according to the present invention, 301 megabytes are required for hydrogen structure optimization, and 301 megabytes are required for side chain structure optimization. As such, in the method according to the present invention, memory saving can be achieved.

[Other Embodiments]

Although the embodiments according to the present embodiment has been described so far, the present invention can be achieved with various different embodiments other than that described above within the technical idea disclosed in the claims described above.

For example, although the example has been described in which the protein-structure optimizing device 4100 performs processes on a stand-alone basis, these processes may be performed upon request from a client terminal formed of a box other than the protein-structure optimizing device 4100 and the process results may be returned to the client terminal.

Also, in the embodiment described above, the example has been described in which MOPAC 2000, which is a semi empirical molecular orbital computation program, is used. Alternatively, another known computation scheme or program may be used. For example, a molecular orbital computation program, such as "Gaussian 98 Rev. A. 11.3" (product name) (Gaussian, Inc. (company name), Pittsburg, PA, 2002) or "Gamess June 20 2002 R2" (product name) (Iowa State University, 2002) can be substituted, thereby allowing structure optimization through ab-initio molecular orbital method. Furthermore, when "Amber 7" (product name) (University of California, 2002), "Tinker 3.7" (product name) (Washington University School of Medicine, 2001), or the like is substituted, molecular mechanical computation can also be possible at high speed. Input/output data of these programs are different from the input file only in arrangement of coordinate parameters, for example, and therefore can be substituted for the input/output data of MOPAC 2000 by using a program such as "Babel version 1.6" (product name) (Pat Walters and Matt Stahl, 1996). MOPAC 2000 is called a semi empirical molecular orbital computation program, and semi quantitative results can be obtained therefrom. On the other hand, programs, such as Gaussian or Gamess, are called ab-initio molecular orbital computation programs, and their results are more quantitative than those from the semi empirical methods, but the calculation time is generally much larger than that in the semi empirical methods.

Of the processes described in the embodiment, all or part of the processes described as being automatically performed may be performed manually, or all or part of the processes described as being manually performed may be automatically performed with a known structure.

Furthermore, the process procedures, the control procedures, the specific names, the information including various registration data and parameters such as conditions, the screen examples, and the database structure in this document and the attached drawings can be arbitrarily changed unless otherwise particularly specified.

Still further, as for the protein-structure optimizing device 4100, the components in the drawings are merely functional and conceptual representations, and are not necessarily configured physically as shown in the drawings.

For example, all or part of the processing functions of each component or each device of the protein-structure optimizing device 4100, particularly the processing functions performed in the control unit 4102, can be performed by a Central Processing Unit (CPU) and a program interpreted by the CPU, or can be implemented as hardware under wired logic control. Here, the program is recorded on a recording medium, which will be described further below, and is read as required to the protein-structure optimizing device 4100.

That is, a computer program for providing an instruction to the CPU in cooperation with an Operating System (OS) and performing various processes is recorded on the storage unit 4106, such as a ROM or an HD. The computer program is executed as being loaded to the RAM, etc., to configure the control unit 4102 in cooperation with the CPU. Also, the computer program may be recorded on an application program server connected to the protein-structure optimizing device 4100 via the arbitrary network 4300, and all or part of the computer program can be downloaded as required.

Furthermore, the program according to the present invention can be stored in a computer-readable recording medium. Here, the "recording medium" includes an arbitrary "portable physical medium", such as a flexible disk, a magneto-optical disk, a ROM, an EPROM, an EEPROM, a CD-ROM, an MO, and a DVD, a "fixed physical medium", such as a ROM, a RAM, and an HD incorporated in various computer systems, and a "communication medium" retaining a program for a short period of time, such as a communication line and carrier wave for use in transmitting the program via a network typified by a LAN, a WAN, and the Internet.

Still further, the "program" is a data processing method described in an arbitrary language or an arbitrary method irrespectively of source code or binary code. Here, the "program" is not restricted to the one singly configured, but includes the one configured in a distributed manner as a plurality of modules or a library and the one achieving its function in cooperation with another program, such as an Operating System (OS). Here, a specific structure for reading a recording medium in each device shown in the embodiment, a reading procedure, an installing procedure after reading, and others are achieved by using any known structure or procedure.

Still further, the protein-structure optimizing device 4100 may further include, as additional components, an input device (not shown) including a various pointing device exemplified by a mouse, a keyboard, an image scanner, a digitizer, and the like; a display device (not shown) for use as an input data monitor; a clock generating unit (not shown) that generates a system clock, and an output device (not shown) that outputs various process results and other data. Also, the input device, the display device, and the output device may be connected to the control unit 4102 via an input/output interface.

Various database and the like stored in the storage unit 4106 (the protein-structure information database 4106a and the process result files 4106b) are storage units, such as memory devices exemplified by a RAM and a ROM, fixed disk devices exemplified by hard disk, a flexible disk, and an optical disk, and store various programs, tables, files, databases, and web-page files for use in various processes and web-site provision.

Still further, the protein-structure optimizing device 4100 may be implemented by software (including programs, data, etc.) for connecting a peripheral device, such as a printer, a monitor, and an image scanner, to an information processing device, such as an information processing terminal of a work station, to cause the information processing device to achieve the method according to the present invention.

Still further, the specific patterns of distribution and integration of the protein-structure optimizing device 4100 are not restricted to those in the drawings, but can be achieved by functionally or physically distributing and integrating all or part of the patterns in arbitrary units according to various loads or the like. For example, each database may be independently structured as an independent database device. Also, part of the processes may be achieved by using a CGI (Common Gateway Interface).

Still further, the network 4300 may have a function of mutually connecting the protein-structure optimizing device 4100 and the external system 4200 to each other, may include, for example, one of the Internet, an intranet, a LAN (inclusive of both wired and wireless networks), a VAN, a personal-computer communication network, a public telephone line (inclusive of both analog and digital), a dedicated-line network (inclusive of both analog and digital), a CATV network, a portable line switched network/portable packet switched network in IMT 2000, GSM, or PDC/PDC-P scheme, a radio-paging network, a local wireless network such as Bluetooth, a PHS network, and a satellite communication network such as CS, BS, or ISDB. That is, the present system can transmit and receive various data via an arbitrary network, irrespectively of whether the network is wired or wireless.

As has been described in detail above, according to the present invention, coordinate data of protein is obtained; of the coordinate data of protein, coordinates of a neighboring amino acid residue group included within a predetermined distance from a specific amino acid residue are extracted; a cap substituent is added to a section of the neighboring amino acid residue group; the entire charge of the neighboring amino-acid-residue group with the cap substituent being added thereto is calculated; for the neighboring amino acid residue group with the cap, structure optimization is performed on atomic coordinate of the specific amino acid residue by using the calculated charge value; and the optimized atomic coordinates are substituted for the corresponding atomic coordinates on the coordinate data of protein. Therefore, a protein-structure optimizing device, and a method, program, and recording medium for protein-structure optimization can be provided that can solve problems regarding determination the position of hydrogen and packing by using practical calculation resources.

Also, according to the present invention, a protein-structure optimizing device, and a method, program, and recording medium for protein-structure optimization can be provided that can achieve a high-speed optimizing process without manipulating the existing calculation program. That is, the present device can be executed by using input/output files of the existing molecular orbital computation program and molecular mechanical computation program. Also, the algorithm of the present device can be incorporated in the existing molecular orbital computation program and molecular mechanical computation program.

Furthermore, according to the present invention, a protein-structure optimizing device, and a method, program, and recording medium for protein-structure optimization can be provided that allow protein structure optimization in consideration of solvent effects that cannot be achieved in the conventional scheme.

Still further, according to the present invention, the cap substituent is a hydrogen atom (H) or a methyl group ($CH_3$). Therefore, a protein-structure optimizing device, and a method, program, and recording medium for protein-structure optimization can be provided that can easily solve the problem in which the section formed when the neighboring amino acid residue group is automatically cut out becomes radical and causes an inconvenience for calculation.

Still further, according to the present invention, when cysteine (CYS) is included in the extracted neighboring amino acid residue group, it is determined whether the cysteine (CYS) has a disulfide bond with another cysteine (CYS) not included in the neighboring amino acid reside group. If such a cysteine (CYS) is present, this cysteine (CYS) is also included as the neighboring amino acid residue group.

Therefore, a protein-structure optimizing device, and a method, program, and recording medium for protein-structure optimization can be provided that can perform structure optimization in consideration of a disulfide bond between cysteines.

Industrial Applicability

(I) As described above, in the interaction-site predicting device and the method, program, and recording medium for interaction-site prediction, an interaction site can be effectively predicted by finding a local site where a frustration is present in the primary sequence of protein.

That is, in the interaction-site predicting device and the method, program, and recording medium for interaction-site prediction according to the present invention, an interaction site can be predicated based on a frustration of a local site.

With this, the interaction-site predicting device and the method, program, and recording medium for interaction-site prediction according to the present invention are quite useful in the field of bioinformatics for analysis of protein and others. Also, the present invention can be widely implemented in many industrial fields, particularly in the fields such as pharmaceuticals, foods, cosmetics, medical-care, genetic expression analysis, and protein's three-dimensional structure analysis, and therefore is quite useful.

(II) Also, in the active-site predicting device and the method, program, and recording medium for active-site prediction, an active site of protein can be predicted from information on energy and expansion of a molecular orbital obtained from molecular orbital computation.

That is, the active-site predicting device and the method, program, and recording medium for active-site prediction according to the present invention, an active site of physiologically-active polypeptide or protein can be estimated with high accuracy.

With this, the active-site predicting device and the method, program, and recording medium for active-site prediction according to the present invention are quite useful in the field of bioinformatics for analysis of protein and others. Also, the present invention can be widely implemented in many industrial fields, particularly in the fields such as pharmaceuticals, foods, cosmetics, medical-care, genetic expression analysis, and protein's three-dimensional structure analysis, and therefore is quite useful.

(III) Furthermore, in the protein interaction information processing device and the method, program, and recording medium for protein interaction information processing, a highly unstable part of a protein unit is specified based on hydrophobic interaction and electrostatic interaction found from the structure data of protein, thereby specifying an interaction site.

**EP 1 510 943 A1**

With this, the protein interaction information processing device and the method, program, and recording medium for protein interaction information processing according to the present invention are quite useful in the field of bioinformatics for analysis of protein and others. Also, the present invention can be widely implemented in many industrial fields, particularly in the fields such as pharmaceuticals, foods, cosmetics, medical-care, genetic expression analysis, and protein's three-dimensional structure analysis, and therefore is quite useful.

(IV) Still further, in the bonding-site predicting device and the method, program, and recording medium for bonding-site prediction, electrostatically unstable portion is predicted by using experimentally-found three-dimensional structure information (distance information in space between amino acid residues) and charge information, thereby efficiently predicting a bonding site of protein or physiologically-active polypeptide, for example.

That is, in the bonding-site predicting device and the method, program, and recording medium for bonding-site prediction according to the present invention, calculation for predicting interaction of protein through bioinformatics can be performed in an extremely short period of time, thereby allowing an exhaustive analysis.

With this, the bonding-site predicting device and the method, program, and recording medium for bonding-site prediction according to the present invention are quite useful in the field of bioinformatics for analysis of protein and others. Also, the present invention can be widely implemented in many industrial fields, particularly in the fields such as pharmaceuticals, foods, cosmetics, medical-care, genetic expression analysis, and protein's three-dimensional structure analysis, and therefore is quite useful.

(V) Still further, in the protein-structure optimizing device, and the method, program, and recording medium for protein-structure optimization, desired atomic coordinates can be optimized while the structure of protein is divided.

With this, the interaction predicting device and the method, program, and recording medium for interaction prediction are quite useful in the field of bioinformatics for analysis of protein and others. Also, the present invention can be widely implemented in many industrial fields, particularly in the fields such as pharmaceuticals, foods, cosmetics, medical-care, genetic expression analysis, and protein's three-dimensional structure analysis, and therefore is quite useful.

**Claims**

1. An interaction site predicting device comprising:

   an inputting unit that inputs primary sequence information of an objective protein;
   a secondary structure prediction program executing unit that makes a secondary structure prediction program to execute a secondary structure prediction simulation for the primary sequence information inputted by the inputting unit, the secondary structure prediction program predicting a secondary structure of a protein from primary sequence information of the protein;
   a prediction result comparing unit that compares prediction results of secondary structure obtained by the secondary structure prediction program executed by the secondary structure prediction program executing unit;
   a frustration calculating unit that calculates frustration of a local portion of the primary sequence information of the objective protein based on a comparison result made by the prediction result comparing unit; and
   an interaction site predicting unit that predicts an interaction site of the objective protein from the frustration of the local portion calculated by the frustration calculating unit.

2. An interaction site predicting device comprising:

   an inputting unit that inputs primary sequence information of an objective protein;
   an secondary structure data acquiring unit that acquires secondary structure data of the objective protein;
   a secondary structure prediction program executing unit that makes a secondary structure prediction program to execute a secondary structure prediction simulation for the primary sequence information inputted by the inputting unit, the secondary structure prediction program predicting a secondary structure of a protein from primary sequence information of the protein;
   a prediction result comparing unit that compares a prediction result of secondary structure obtained by the secondary structure prediction program executed by the secondary structure prediction program executing unit, with the secondary structure data acquired by the secondary structure data acquiring unit;
   a frustration calculating unit that calculates frustration of a local portion of the primary sequence information of the objective protein based on a comparison result made by the prediction result comparing unit; and
   an interaction site predicting unit that predicts an interaction site of the objective protein from the frustration of the local portion calculated by the frustration calculating unit.

**3.** The interaction site predicting device according to claim 1 or 2, further comprising:

a certainty factor information setting unit that sets certainty factor information representing certainty factor for the prediction result of secondary structure obtained by the secondary structure prediction program,

wherein the frustration calculating unit calculates the frustration of the local portion based on the certainty factor information set by the certainty factor information setting unit and the comparison result.

**4.** An interaction site predicting method comprising:

an inputting step that inputs primary sequence information of an objective protein;
a secondary structure prediction program executing step that makes a secondary structure prediction program to execute a secondary structure prediction simulation for the primary sequence information inputted by the inputting step, the secondary structure prediction program predicting a secondary structure of a protein from primary sequence information of the protein;
a prediction result comparing step that compares prediction results of secondary structure obtained by the secondary structure prediction program executed by the secondary structure prediction program executing step;
a frustration calculating step that calculates frustration of a local portion of the primary sequence information of the objective protein based on a comparison result made by the prediction result comparing step; and
an interaction site predicting step that predicts an interaction site of the objective protein from the frustration of the local portion calculated by the frustration calculating step.

**5.** An interaction site predicting method comprising:

an inputting step that inputs primary sequence information of an objective protein;
an secondary structure data acquiring step that acquires secondary structure data of the objective protein;
a secondary structure prediction program executing step that makes a secondary structure prediction program to execute a secondary structure prediction simulation for the primary sequence information inputted by the inputting step, the secondary structure prediction program predicting a secondary structure of a protein from primary sequence information of the protein;
a prediction result comparing step that compares a prediction result of secondary structure obtained by the secondary structure prediction program executed by the secondary structure prediction program executing step, with the secondary structure data acquired by the secondary structure data acquiring step;
a frustration calculating step that calculates frustration of a local portion of the primary sequence information of the objective protein based on a comparison result made by the prediction result comparing step; and
an interaction site predicting step that predicts an interaction site of the objective protein from the frustration of the local portion calculated by the frustration calculating step.

**6.** The interaction site predicting method according to claim 4 or 5, further comprising:

a certainty factor information setting step that sets certainty factor information representing certainty factor for the prediction result of secondary structure obtained by the secondary structure prediction program,

wherein the frustration calculating step calculates the frustration of the local portion based on the certainty factor information set by the certainty factor information setting step and the comparison result.

**7.** A program that makes a computer to execute an interaction site predicting method which comprises:

an inputting step that inputs primary sequence information of an objective protein;
a secondary structure prediction program executing step that makes a secondary structure prediction program to execute a secondary structure prediction simulation for the primary sequence information inputted by the inputting step, the secondary structure prediction program predicting a secondary structure of a protein from primary sequence information of the protein;
a prediction result comparing step that compares prediction results of secondary structure obtained by the secondary structure prediction program executed by the secondary structure prediction program executing step;
a frustration calculating step that calculates frustration of a local portion of the primary sequence information of the objective protein based on a comparison result made by the prediction result comparing step; and
an interaction site predicting step that predicts an interaction site of the objective protein from the frustration of the local portion calculated by the frustration calculating step.

8. A program that makes a computer to execute an interaction site predicting method which comprises:

an inputting step that inputs primary sequence information of an objective protein;
an secondary structure data acquiring step that acquires secondary structure data of the objective protein;
a secondary structure prediction program executing step that makes a secondary structure prediction program to execute a secondary structure prediction simulation for the primary sequence information inputted by the inputting step, the secondary structure prediction program predicting a secondary structure of a protein from primary sequence information of the protein;
a prediction result comparing step that compares a prediction result of secondary structure obtained by the secondary structure prediction program executed by the secondary structure prediction program executing step, with the secondary structure data acquired by the secondary structure data acquiring step;
a frustration calculating step that calculates frustration of a local portion of the primary sequence information of the objective protein based on a comparison result made by the prediction result comparing step; and
an interaction site predicting step that predicts an interaction site of the objective protein from the frustration of the local portion calculated by the frustration calculating step.

9. The program according to claim 7 or 8, further comprising:

a certainty factor information setting step that sets certainty factor information representing certainty factor for the prediction result of secondary structure obtained by the secondary structure prediction program,

wherein the frustration calculating step calculates the frustration of the local portion based on the certainty factor information set by the certainty factor information setting step and the comparison result.

10. A recording medium readable by a computer, on which a program according to any one of claims 7 to 9 is recorded.

11. An active site predicting method wherein a electron state of a protein or physiologically active polypeptide is calculated by molecular orbital calculation to determine a frontier orbital and its peripheral orbital, and/or an orbital energy localized in a heavy atom of a main chain, and based on the frontier orbital and its peripheral orbital, and/or the orbital energy, an amino acid residue which serves as an active site of the protein or physiologically active polypeptide is predicted.

12. An active site predicting method comprising:

a structure data acquiring step that acquires structure data of an objective protein or physiologically active polypeptide;
a frontier orbital calculating step that calculates an electron state of the protein or physiologically active polypeptide by molecular orbital calculation based on the structure data acquired by the structure data acquiring step to determine a frontier orbital;
a peripheral orbital determining step that determines a molecular orbital having a predetermined energy gap from the frontier orbital, as a peripheral orbital of the frontier orbital;
a candidate amino acid residue determining step that determines as candidate amino acid residues for an active site, amino acid residues in which the frontier orbital and the peripheral orbital distribute; and
an active site predicting step that predicts an active site by selecting an active site from the candidate amino acid residues determined by the candidate amino acid residue determining step.

13. An active site predicting method comprising:

a structure data acquiring step that acquires structure data of an objective protein or physiologically active polypeptide;
an orbital energy calculating step that calculates an electron state of the protein or physiologically active polypeptide by molecular orbital calculation based on the structure data acquired by the structure data acquiring step to determine an orbital energy localized in a heavy atom of a main chain; and
a candidate amino acid residue determining step that determines as a candidate amino acid residue for an active site, amino acid residues in which a molecular orbital having an orbital energy exceeding a predetermined level and/or a molecular orbital having a relatively high orbital energy in the orbital energy determined by the orbital energy calculating step distributes.

14. An active site predicting method comprising:

a structure data acquiring step that acquires structure data of an objective protein or physiologically active polypeptide;

a frontier orbital calculating step that calculates an electron state of the protein or physiologically active polypeptide by molecular orbital calculation based on the structure data acquired by the structure data acquiring step to determine a frontier orbital;

an orbital energy calculating step that calculates an electron state of the protein or physiologically active polypeptide by molecular orbital calculation based on the structure data acquired by the structure data acquiring step to determine an orbital energy localized in a heavy atom of a main chain;

a peripheral orbital determining step that determines a molecular orbital having a predetermined energy gap from the frontier orbital, as a peripheral orbital of the frontier orbital;

a candidate amino acid residue determining step that determines as a candidate amino acid residue for an active site, amino acid residues in which the frontier orbital and the peripheral orbital distribute and/or amino acid residues in which a molecular orbital having an orbital energy exceeding a predetermined level and/or a molecular orbital having a relatively high orbital energy in the orbital energy determined by the orbital energy calculating step distributes; and

an active site predicting step that predicts an active site by selecting an active site from the candidate amino acid residues determined by the candidate amino acid residue determining step.

15. The active site predicting method according to any one of claims 12 to 14, further comprising:

a calculating condition setting step that sets at least one of the following calculating conditions 1) to 3) in the molecular orbital calculation:

1) generating water molecules around the protein or physiologically active polypeptide;
2) placing continuous dielectric materials around the protein or physiologically active polypeptide; and
3) bringing dissociative amino acid residues on a surface of the protein or physiologically active polypeptide into a non-charged state while bringing embedded inside dissociative amino acids into a charged state.

16. An active site predicting device comprising:

a structure data acquiring unit that acquires structure data of an objective protein or physiologically active polypeptide;

a frontier orbital calculating unit that calculates an electron state of the protein or physiologically active polypeptide by molecular orbital calculation based on the structure data acquired by the structure data acquiring unit to determine a frontier orbital;

a peripheral orbital determining unit that determines a molecular orbital having a predetermined energy gap from the frontier orbital, as a peripheral orbital of the frontier orbital;

a candidate amino acid residue determining unit that determines as candidate amino acid residues for an active site, amino acid residues in which the frontier orbital and the peripheral orbital distribute; and

an active site predicting unit that predicts an active site by selecting an active site from the candidate amino acid residues determined by the candidate amino acid residue determining unit.

17. An active site predicting device comprising:

a structure data acquiring unit that acquires structure data of an objective protein or physiologically active polypeptide;

an orbital energy calculating unit that calculates an electron state of the protein or physiologically active polypeptide by molecular orbital calculation based on the structure data acquired by the structure data acquiring unit to determine an orbital energy localized in a heavy atom of a main chain; and

a candidate amino acid residue determining unit that determines as a candidate amino acid residue for an active site, amino acid residues in which a molecular orbital having an orbital energy exceeding a predetermined level and/or a molecular orbital having a relatively high orbital energy in the orbital energy determined by the orbital energy calculating unit distributes.

**18.** An active site predicting device comprising:

a structure data acquiring unit that acquires structure data of an objective protein or physiologically active polypeptide;

a frontier orbital calculating unit that calculates an electron state of the protein or physiologically active polypeptide by molecular orbital calculation based on the structure data acquired by the structure data acquiring unit to determine a frontier orbital;

an orbital energy calculating unit that calculates an electron state of the protein or physiologically active polypeptide by molecular orbital calculation based on the structure data acquired by the structure data acquiring unit to determine an orbital energy localized in a heavy atom of a main chain;

a peripheral orbital determining unit that determines a molecular orbital having a predetermined energy gap from the frontier orbital, as a peripheral orbital of the frontier orbital;

a candidate amino acid residue determining unit that determines as a candidate amino acid residue for an active site, amino acid residues in which the frontier orbital and the peripheral orbital distribute and/or amino acid residues in which a molecular orbital having an orbital energy exceeding a predetermined level and/or a molecular orbital having a relatively high orbital energy in the orbital energy determined by the orbital energy calculating unit distributes; and

an active site predicting unit that predicts an active site by selecting an active site from the candidate amino acid residues determined by the candidate amino acid residue determining unit.

**19.** The active site predicting device according to any one of claims 16 to 18, further comprising:

a calculating condition setting unit that sets at least one of the following calculating conditions 1) to 3) in the molecular orbital calculation:

1) generating water molecules around the protein or physiologically active polypeptide;
2) placing continuous dielectric materials around the protein or physiologically active polypeptide; and
3) bringing dissociative amino acid residues on a surface of the protein or physiologically active polypeptide into a non-charged state while bringing embedded inside dissociative amino acids into a charged state.

**20.** A program that makes a computer to execute an active site predicting method which comprises:

a structure data acquiring step that acquires structure data of an objective protein or physiologically active polypeptide;

a frontier orbital calculating step that calculates an electron state of the protein or physiologically active polypeptide by molecular orbital calculation based on the structure data acquired by the structure data acquiring step to determine a frontier orbital;

a peripheral orbital determining step that determines a molecular orbital having a predetermined energy gap from the frontier orbital, as a peripheral orbital of the frontier orbital;

a candidate amino acid residue determining step that determines as candidate amino acid residues for an active site, amino acid residues in which the frontier orbital and the peripheral orbital distribute; and

an active site predicting step that predicts an active site by selecting an active site from the candidate amino acid residues determined by the candidate amino acid residue determining step.

**21.** A program that makes a computer to execute an active site predicting method which comprises:

a structure data acquiring step that acquires structure data of an objective protein or physiologically active polypeptide;

an orbital energy calculating step that calculates an electron state of the protein or physiologically active polypeptide by molecular orbital calculation based on the structure data acquired by the structure data acquiring step to determine an orbital energy localized in a heavy atom of a main chain; and

a candidate amino acid residue determining step that determines as a candidate amino acid residue for an active site, amino acid residues in which a molecular orbital having an orbital energy exceeding a predetermined level and/or a molecular orbital having a relatively high orbital energy in the orbital energy determined by the orbital energy calculating step distributes.

**22.** A program that makes a computer to execute an active site predicting method which comprises:

a structure data acquiring step that acquires structure data of an objective protein or physiologically active polypeptide;

a frontier orbital calculating step that calculates an electron state of the protein or physiologically active polypeptide by molecular orbital calculation based on the structure data acquired by the structure data acquiring step to determine a frontier orbital;

an orbital energy calculating step that calculates an electron state of the protein or physiologically active polypeptide by molecular orbital calculation based on the structure data acquired by the structure data acquiring step to determine an orbital energy localized in a heavy atom of a main chain;

a peripheral orbital determining step that determines a molecular orbital having a predetermined energy gap from the frontier orbital, as a peripheral orbital of the frontier orbital;

a candidate amino acid residue determining step that determines as a candidate amino acid residue for an active site, amino acid residues in which the frontier orbital and the peripheral orbital distribute and/or amino acid residues in which a molecular orbital having an orbital energy exceeding a predetermined level and/or a molecular orbital having a relatively high orbital energy in the orbital energy determined by the orbital energy calculating step distributes;

an active site predicting step that predicts an active site by selecting an active site from the candidate amino acid residues determined by the candidate amino acid residue determining step.

**23.** The program according to any one of claims 20 to 22, wherein a computer is made to execute an active site predicting method further comprising:

a calculating condition setting step that sets at least one of the following calculating conditions 1) to 3) in the molecular orbital calculation:

1) generating water molecules around the protein or physiologically active polypeptide;
2) placing continuous dielectric materials around the protein or physiologically active polypeptide; and
3) bringing dissociative amino acid residues on a surface of the protein or physiologically active polypeptide into a non-charged state while bringing embedded inside dissociative amino acids into a charged state.

**24.** A recording medium readable by a computer, on which a program according to any one of claims 20 to 23 is recorded.

**25.** A protein interaction information processing device comprising:

a structure data acquiring unit that acquires structure data including primary structure data of a plurality of interacting proteins and three-dimensional structure data thereof when they are single protein molecules and/or when they form a composite body;

a hydrophobic surface determining unit that determines a hydrophobic interaction energy for each of amino acid residues constituting the primary structure data, according to the structure data acquired by the structure data acquiring unit;

an electrostatic interaction determining unit that determines an electrostatic interaction energy for each of amino acid residues constituting the primary structure data, according to the structure data acquired by the structure data acquiring unit; and

an interaction site determining unit that determines an interaction site by determining a site in the amino acid residues which is highly unstable, based on the hydrophobic interaction energy determined by the hydrophobic surface determining unit and the electrostatic interaction energy determined by the electrostatic interaction site determining unit.

**26.** The protein interaction information processing device according to claim 25, further comprising:

a solvent contact face determining unit that determines a solvent contact face for each of amino acid residues constituting the primary structure data, according to the structured data acquired by the structure data acquiring unit;

wherein the interaction site determining unit determines an interaction site by determining a site in the amino acid residues which is highly unstable, based on the solvent contact face determined by the solvent contact face determining unit, the hydrophobic interaction energy determined by the hydrophobic surface determining unit and the electrostatic interaction energy determined by the electrostatic interaction site determining unit.

27. The protein interaction information processing device according to claim 25 or 26, further comprising:

a candidate protein retrieving unit that determines a primary sequence of an interacting partner for the interaction site determined by the interaction site determining unit and retrieves for a candidate protein having a primary structure including the determined primary sequence,

wherein with respect to the candidate protein retrieved out by the candidate protein retrieving unit, whether a part of the primary sequence of the partner is identified as an interaction site of the candidate protein is confirmed.

28. A protein interaction information processing method comprising:

a structure data acquiring step that acquires structure data including primary structure data of a plurality of interacting proteins and three-dimensional structure data thereof when they are single protein molecules and/or when they form a composite body;
a hydrophobic surface determining step that determines a hydrophobic interaction energy for each of amino acid residues constituting the primary structure data, according to the structure data acquired by the structure data acquiring step;
an electrostatic interaction determining step that determines an electrostatic interaction energy for each of amino acid residues constituting the primary structure data, according to the structure data acquired by the structure data acquiring step; and
an interaction site determining step that determines an interaction site by determining a site in the amino acid residues which is highly unstable, based on the hydrophobic interaction energy determined by the hydrophobic surface determining step and the electrostatic interaction energy determined by the electrostatic interaction site determining step.

29. The protein interaction information processing method according to claim 28, further comprising:

a solvent contact face determining step that determines a solvent contact face for each of amino acid residues constituting the primary structure data, according to the structured data acquired by the structure data acquiring step;

wherein the interaction site determining step determines an interaction site by determining a site in the amino acid residues which is highly unstable, based on the solvent contact face determined by the solvent contact face determining step, the hydrophobic interaction energy determined by the hydrophobic surface determining step and the electrostatic interaction energy determined by the electrostatic interaction site determining step.

30. The protein interaction information processing device according to claim 28 or 29, further comprising:

a candidate protein retrieving step that determines a primary sequence of an interacting partner for the interaction site determined by the interaction site determining step and retrieves for a candidate protein having a primary structure including the determined primary sequence,

wherein with respect to the candidate protein retrieved out by the candidate protein retrieving step, whether a part of the primary sequence of the partner is identified as an interaction site of the candidate protein is confirmed.

31. A program that makes a computer to execute a protein interaction information processing method which comprises:

a structure data acquiring step that acquires structure data including primary structure data of a plurality of interacting proteins and three-dimensional structure data thereof when they are single protein molecules and/or when they form a composite body;
a hydrophobic surface determining step that determines a hydrophobic interaction energy for each of amino acid residues constituting the primary structure data, according to the structure data acquired by the structure data acquiring step;
an electrostatic interaction determining step that determines an electrostatic interaction energy for each of amino acid residues constituting the primary structure data, according to the structure data acquired by the structure data acquiring step; and

an interaction site determining step that determines an interaction site by determining a site in the amino acid residues which is highly unstable, based on the hydrophobic interaction energy determined by the hydrophobic surface determining step and the electrostatic interaction energy determined by the electrostatic interaction site determining step.

32. The program according to claim 31, further comprising:

a solvent contact face determining step that determines a solvent contact face for each of amino acid residues constituting the primary structure data, according to the structured data acquired by the structure data acquiring step,

wherein the interaction site determining step determines an interaction site by determining a site in the amino acid residues which is highly unstable, based on the solvent contact face determined by the solvent contact face determining step, the hydrophobic interaction energy determined by the hydrophobic surface determining step and the electrostatic interaction energy determined by the electrostatic interaction site determining step.

33. The program according to claim 31 or 32, further comprising:

a candidate protein retrieving step that determines a primary sequence of an interacting partner for the interaction site determined by the interaction site determining step and retrieves for a candidate protein having a primary structure including the determined primary sequence,

wherein with respect to the candidate protein retrieved out by the candidate protein retrieving step, whether a part of the primary sequence of the partner is identified as an interaction site of the candidate protein is confirmed.

34. A recording medium readable by a computer, on which a program according to any one of claims 31 to 33 is recorded.

35. A binding site predicting method, wherein from amino acid sequence data of a protein or physiologically active polypeptide, spatial distance data between each amino acid residue in three-dimensional structure of the protein or physiologically active polypeptide is calculated, and a binding site is predicted by determining an amino acid residue which is electrostatically unstable according to the distance data and an electric charge of each amino acid.

36. A binding site predicting method comprising:

an amino acid sequence data acquiring step that acquires amino acid sequence data of an objective protein or physiologically active polypeptide;
a spatial distance determining step that determines a spatial distance between each amino acid residue contained in the amino acid sequence data acquired by the amino acid sequence data acquiring step;
an electric charge determining step that determines an electric charge possessed by each amino acid residue included in the amino acid sequence data;
an energy calculating step that calculates an energy of each amino acid residue, according to the spatial distance of each amino acid residue determined by the spatial distance determining step and an electric charge possessed by each amino acid residue determined by the electric charge determining step; and
a candidate amino acid residue determining step that determines a candidate amino acid residue which serves as a binding site, according to the energy calculated by the energy calculating step.

37. A binding site predicting method comprising:

an amino acid sequence data acquiring step that acquires amino acid sequence data of a plurality of objective proteins or physiologically active polypeptides;
a composite body structure generating step that generates three-dimensional structure information of a composite body resulting from binding of the objective proteins or physiologically active polypeptides;
a spatial distance determining step that determines a spatial distance between each amino acid residue contained in the amino acid sequence data acquired by the amino acid sequence data acquiring step, according to the three-dimensional structure information of the composite body generated by the composite body structure generating step;

an electric charge determining step that determines an electric charge possessed by each amino acid residue contained in the amino acid sequence data;

an energy calculating step that calculates an energy of each amino acid residue, according to the spatial distance of each amino acid residue determined by the spatial distance determining step and an electric charge possessed by each amino acid residue determined by the electric charge determining step;

an energy minimization step that generates three-dimensional structure information of the composite body while changing the binding site for the composite body by the composite body structure generating step, calculates an energy of each amino acid residue by the energy calculating step, and determines a binding site where a sum total of the energies is minimum; and

a candidate amino acid residue determining step that determines a binding site where a sum total of energies is determined as being minimum by the energy minimization step, as a candidate amino acid residue of a binding site.

**38.** A binding site predicting method comprising:

an amino acid sequence data acquiring step that acquires amino acid sequence data of an objective protein or physiologically active polypeptide and amino acid sequence data of one or more candidate protein(s) or physiologically active polypeptide(s) for a binding site;

a composite body structure generating step that generates three-dimensional structure information of a composite body resulting from binding of the objective protein or physiologically active polypeptide and the candidate protein or physiologically active polypeptide;

a spatial distance determining step that determines a spatial distance between each amino acid residue contained in the objective amino acid sequence data and the candidate amino acid sequence data acquired by the amino acid sequence data acquiring step, according to the three-dimensional structure information of the composite body generated by the composite body structure generating step;

an electric charge determining step that determines an electric charge possessed by each amino acid residue contained in the objective amino acid sequence data and the candidate amino acid sequence data;

an energy calculating step that calculates an energy of each amino acid residue, according to the spatial distance of each amino acid residue determined by the spatial distance determining step and an electric charge possessed by each amino acid residue determined by the electric charge determining step;

an energy minimization step that generates three-dimensional structure information of the composite body while changing the binding site for the composite body by the composite body structure generating step, calculates an energy of each amino acid residue by the energy calculating step, and determines a binding site where a sum total of the energies is minimum; and

a binding candidate determining step that determines a binding candidate having a binding site where a sum total of energies is minimum as a result of execution of the energy minimization step for every binding candidate.

**39.** A binding site predicting device comprising:

an amino acid sequence data acquiring unit that acquires amino acid sequence data of an objective protein or physiologically active polypeptide;

a spatial distance determining unit that determines a spatial distance between each amino acid residue contained in the amino acid sequence data acquired by the amino acid sequence data acquiring unit;

an electric charge determining unit that determines an electric charge possessed by each amino acid residue included in the amino acid sequence data;

an energy calculating unit that calculates an energy of each amino acid residue, according to the spatial distance of each amino acid residue determined by the spatial distance determining unit and an electric charge possessed by each amino acid residue determined by the electric charge determining unit; and

a candidate amino acid residue determining unit that determines a candidate amino acid residue which serves as a binding site, according to the energy calculated by the energy calculating unit.

**40.** A binding site predicting device comprising:

an amino acid sequence data acquiring unit that acquires amino acid sequence data of a plurality of objective proteins or physiologically active polypeptides;

a composite body structure generating unit that generates three-dimensional structure information of a composite body resulting from binding of the objective proteins or physiologically active polypeptides;

a spatial distance determining unit that determines a spatial distance between each amino acid residue contained in the amino acid sequence data acquired by the amino acid sequence data acquiring unit, according to the three-dimensional structure information of the composite body generated by the composite body structure generating unit;

an electric charge determining unit that determines an electric charge possessed by each amino acid residue contained in the amino acid sequence data;

an energy calculating unit that calculates an energy of each amino acid residue, according to the spatial distance of each amino acid residue determined by the spatial distance determining unit and an electric charge possessed by each amino acid residue determined by the electric charge determining unit;

an energy minimization unit that generates three-dimensional structure information of the composite body while changing the binding site for the composite body by the composite body structure generating unit, calculates an energy of each amino acid residue by the energy calculating unit, and determines a binding site where a sum total of the energies is minimum; and

a candidate amino acid residue determining unit that determines a binding site where a sum total of energies is determined as being minimum by the energy minimization unit, as a candidate amino acid residue of a binding site.

**41.** A binding site predicting device comprising:

an amino acid sequence data acquiring unit that acquires amino acid sequence data of an objective protein or physiologically active polypeptide and amino acid sequence data of one or more candidate protein(s) or physiologically active polypeptide(s) for a binding site;

a composite body structure generating unit that generates three-dimensional structure information of a composite body resulting from binding of the objective protein or physiologically active polypeptide and the candidate protein or physiologically active polypeptide;

a spatial distance determining unit that determines a spatial distance between each amino acid residue contained in the objective amino acid sequence data and the candidate amino acid sequence data acquired by the amino acid sequence data acquiring unit, according to the three-dimensional structure information of the composite body generated by the composite body structure generating unit;

an electric charge determining unit that determines an electric charge possessed by each amino acid residue contained in the objective amino acid sequence data and the candidate amino acid sequence data;

an energy calculating unit that calculates an energy of each amino acid residue, according to the spatial distance of each amino acid residue determined by the spatial distance determining unit and an electric charge possessed by each amino acid residue determined by the electric charge determining unit;

an energy minimization unit that generates three-dimensional structure information of the composite body while changing the binding site for the composite body by the composite body structure generating unit, calculates an energy of each amino acid residue by the energy calculating unit, and determines a binding site where a sum total of the energies is minimum; and

a binding candidate determining unit that determines a binding candidate having a binding site where a sum total of energies is minimum as a result of execution of the energy minimization unit for every binding candidate.

**42.** A program that makes a computer to execute a binding site predicting method which comprises:

an amino acid sequence data acquiring step that acquires amino acid sequence data of an objective protein or physiologically active polypeptide;

a spatial distance determining step that determines a spatial distance between each amino acid residue contained in the amino acid sequence data acquired by the amino acid sequence data acquiring step;

an electric charge determining step that determines an electric charge possessed by each amino acid residue included in the amino acid sequence data;

an energy calculating step that calculates an energy of each amino acid residue, according to the spatial distance of each amino acid residue determined by the spatial distance determining step and an electric charge possessed by each amino acid residue determined by the electric charge determining step; and

a candidate amino acid residue determining step that determines a candidate amino acid residue which serves as a binding site, according to the energies calculated by the energy calculating step.

**43.** A program that makes a computer to execute a binding site predicting method which comprises:

an amino acid sequence data acquiring step that acquires amino acid sequence data of a plurality of objective proteins or physiologically active polypeptides;

a composite body structure generating step that generates three-dimensional structure information of a composite body resulting from binding of the objective proteins or physiologically active polypeptides;

a spatial distance determining step that determines a spatial distance between each amino acid residue contained in the amino acid sequence data acquired by the amino acid sequence data acquiring step, according to the three-dimensional structure information of the composite body generated by the composite body structure generating step;

an electric charge determining step that determines an electric charge possessed by each amino acid residue contained in the amino acid sequence data;

an energy calculating step that calculates an energy of each amino acid residue, according to the spatial distance of each amino acid residue determined by the spatial distance determining step and an electric charge possessed by each amino acid residue determined by the electric charge determining step;

an energy minimization step that generates three-dimensional structure information of the composite body while changing the binding site for the composite body by the composite body structure generating step, calculates an energy of each amino acid residue by the energy calculating step, and determines a binding site where a sum total of the energies is minimum; and

a candidate amino acid residue determining step that determines a binding site where a sum total of energies is determined as being minimum by the energy minimization step, as a candidate amino acid residue of a binding site.

44. A program that makes a computer to execute a binding site predicting method which comprises:

an amino acid sequence data acquiring step that acquires amino acid sequence data of an objective protein or physiologically active polypeptide and amino acid sequence data of one or more candidate protein(s) or physiologically active polypeptide(s) for a binding site;

a composite body structure generating step that generates three-dimensional structure information of a composite body resulting from binding of the objective protein or physiologically active polypeptide and the candidate protein or physiologically active polypeptide;

a spatial distance determining step that determines a spatial distance between each amino acid residue contained in the objective amino acid sequence data and the candidate amino acid sequence data acquired by the amino acid sequence data acquiring step, according to the three-dimensional structure information of the composite body generated by the composite body structure generating step;

an electric charge determining step that determines an electric charge possessed by each amino acid residue contained in the objective amino acid sequence data and the candidate amino acid sequence data;

an energy calculating step that calculates an energy of each amino acid residue, according to the spatial distance of each amino acid residue determined by the spatial distance determining step and an electric charge possessed by each amino acid residue determined by the electric charge determining step;

an energy minimization step that generates three-dimensional structure information of the composite body while changing the binding site for the composite body by the composite body structure generating step, calculates an energy of each amino acid residue by the energy calculating step, and determines a binding site where a sum total of the energies is minimum; and

a binding candidate determining step that determines a binding candidate having a binding site where a sum total of energies is minimum as a result of execution of the energy minimization step for every binding candidates.

45. A recording medium readable by a computer, on which a program according to any one of claims 42 to 44 is recorded.

46. A protein structure optimizing device comprising:

a coordinate data acquiring unit that acquires coordinate data of a protein;

a neighboring amino acid residue group extracting unit that extracts a coordinate of neighboring amino acid residue group located within a certain distance from a specific amino acid residue, with respect to the coordinate data of a protein;

a cap adding unit that adds a capping substituent for a cutting portion of the neighboring amino acid residue group;

an electric charge calculating unit that calculates an electric charge of the whole of the neighboring amino acid residue group for which the capping substituent is added by the cap adding unit;

a structure optimizing unit that executes structure optimization on an atomic coordinate of the specific amino acid residue using the electric charge calculated by the electric charge calculating unit for the neighboring amino acid residue group to which the capping substituent is added by the cap adding unit; and

an atomic coordinate substituting unit that substitutes the atomic coordinate optimized by the structure optimizing unit for a corresponding atomic coordinate on the coordinate data of the protein.

47. The protein structure optimizing device according to claim 46, wherein the capping substituent is a hydrogen atom (H) or a methyl group ($CH_3$).

48. The protein structure optimizing device according to claim 46 or 47, wherein the neighboring amino acid residue group extracting unit judges whether there is another cysteine (CYS) that forms a disulfide bond with the cysteine (CYS) but not included in the neighboring amino acid residue group, when cysteine (CYS) is included in the extracted neighboring amino acid residue group, and when there is another cysteine (CYS), the cysteine (CYS) is added to the neighboring amino acid residue group.

49. A protein structure optimizing method comprising:

a coordinate data acquiring step that acquires coordinate data of a protein;
a neighboring amino acid residue group extracting step that extracts a coordinate of neighboring amino acid residue group located within a certain distance from a specific amino acid residue, with respect to the coordinate data of a protein;
a cap adding step that adds a capping substituent for a cutting portion of the neighboring amino acid residue group;
an electric charge calculating step that calculates an electric charge of the whole of the neighboring amino acid residue group for which the capping substituent is added by the cap adding step;
a structure optimizing step that executes structure optimization on an atomic coordinate of the specific amino acid residue using the electric charge calculated by the electric charge calculating step for the neighboring amino acid residue group to which the capping substituent is added by the cap adding step; and
an atomic coordinate substituting step that substitutes the atomic coordinate optimized by the structure optimizing step for a corresponding atomic coordinate on the coordinate data of the protein.

50. The protein structure optimizing method according to claim 49, wherein the capping substituent is a hydrogen atom (H) or a methyl group ($CH_3$).

51. The protein structure optimizing method according to claim 49 or 50, wherein the neighboring amino acid residue group extracting step judges whether there is another cysteine (CYS) that forms a disulfide bond with the cysteine (CYS) but not included in the neighboring amino acid residue group, when cysteine (CYS) is included in the extracted neighboring amino acid residue group, and when there is another cysteine (CYS), the cysteine (CYS) is added to the neighboring amino acid residue group.

52. A program that makes a computer to execute a protein structure optimizing method which comprises:

a coordinate data acquiring step that acquires coordinate data of a protein;
a neighboring amino acid residue group extracting step that extracts a coordinate of neighboring amino acid residue group located within a certain distance from a specific amino acid residue, with respect to the coordinate data of a protein;
a cap adding step that adds a capping substituent for a cutting portion of the neighboring amino acid residue group;
an electric charge calculating step that calculates an electric charge of the whole of the neighboring amino acid residue group for which the capping substituent is added by the cap adding step;
a structure optimizing step that executes structure optimization on an atomic coordinate of the specific amino acid residue using the electric charge calculated by the electric charge calculating step for the neighboring amino acid residue group to which the capping substituent is added by the cap adding step; and
an atomic coordinate substituting step that substitutes the atomic coordinate optimized by the structure optimizing step for a corresponding atomic coordinate on the coordinate data of the protein.

53. The program according to claim 52, wherein the capping substituent is a hydrogen atom (H) or a methyl group ($CH_3$).

**54.** The program according to claim 52 or 53, wherein the neighboring amino acid residue group extracting step judges whether there is another cysteine (CYS) that forms a disulfide bond with the cysteine (CYS) but not included in the neighboring amino acid residue group, when cysteine (CYS) is included in the extracted neighboring amino acid residue group, and when there is another cysteine (CYS), the cysteine (CYS) is added to the neighboring amino acid residue group.

**55.** A recording medium readable by a computer, on which a program according to any one of claims 52 to 54 is recorded.

# FIG.1

(BASIC PRINCIPLE OF THE INVENTION)

OBJECTIVE
SEQUENCE DATA
10

Glu—Lys—Asp—Tyr—Arg · · ·

20a SECONDARY STRUCTURE PREDICTION PROGRAM A

20b SECONDARY STRUCTURE PREDICTION PROGRAM B

20c SECONDARY STRUCTURE PREDICTION PROGRAM C

20d SECONDARY STRUCTURE PREDICTION PROGRAM D

30a SECONDARY STRUCTURE PREDICTION RESULT DATA A

30b SECONDARY STRUCTURE PREDICTION RESULT DATA B

30c SECONDARY STRUCTURE PREDICTION RESULT DATA C

30d SECONDARY STRUCTURE PREDICTION RESULT DATA D

40 SECONDARY STRUCTURE DATA

COMPARE PREDICTION RESULTS

OBJECTIVE SEQUENCE DATA — 61

SECONDARY STRUCTURE DATA — 62 | $\alpha$ | $\beta$ | OTHERS | 60

PREDICTION RESULT DATA

PROGRAM A | $\alpha$ | $\beta$ | OTHERS | 63

PROGRAM B | $\alpha$ | $\beta$ | OTHERS | 64

PROGRAM C | $\alpha$ | $\beta$ | OTHERS | 65

PROGRAM D | $\alpha$ | $\beta$ | OTHERS | 66

67

50 CERTAINTY FACTOR INFORMATION

70 EXTRACT LOCALIZED PORTION WHOSE FRUSTRATION IS RELATIVELY LARGE

80 PREDICT PORTION HAVING HIGH PROBABILITY THAT THE PORTION IS INTERACTION SITE

# FIG.2

INPUT DEVICE 112

OUTPUT DEVICE 114

INTERACTION SITE PREDICTION 100

INPUT-OUTPUT CONTROL INTERFACE UNIT 108

STORAGE UNIT 106

PREDICTION RESULT DB 106a

CERTAINTY FACTOR INFORMATION DB 106b

PROTEIN STRUCTURE DB 106c

CONTROL UNIT 102

OBJECTIVE SEQUENCE INPUT UNIT 102a

SECONDARY STRUCTURE PREDICTION PROGRAM EXECUTING UNIT 102b

SECONDARY STRUCTURE PREDICTION PROGRAM 102c

PREDICTION RESULT COMPARING UNIT 102d

FRUSTRATION CALCULATING UNIT 102e

INTERACTION SITE PREDICTING UNIT 102f

SECONDARY STRUCTURE DATA ACQUIRING UNIT 102g

CERTAINTY FACTOR INFORMATION SETTING UNIT 102h

COMMUNICATION CONTROL INTERFACE UNIT 104

EXTERNAL SYSTEM 200

EXTERNAL DB

EXTERNAL PROGRAM

NETWORK 300

EP 1 510 943 A1

77

# FIG.3

PREDICTION RESULT DATA BASE
106a

| OBJEC-TIVE SE-QUENCE DATA | SECOND-ARY STRUC-TURE DATA | PREDICTION RESULT DATA | | | | |
|---|---|---|---|---|---|---|
| | | PROGRAM A | PROGRAM B | PROGRAM C | PROGRAM D | ... |
| | | | | | | |
| | | | | | | |
| | | | | | | |
| ⋮ | ⋮ | ⋮ | ⋮ | ⋮ | ⋮ | ⋮ |

# FIG.4

```
        ┌─────────────┐
        │    START    │
        └─────────────┘
               │
               ▼
  ┌──────────────────────────────┐
  │    USER INPUTS OBJECTIVE     │───── SA-1
  │      SEQUENCE DATA           │
  └──────────────────────────────┘
               │
               ▼
  ┌──────────────────────────────┐
  │  ACQUIRE SECONDARY STRUCTURE │───── SA-2
  │ DATA OF OBJECTIVE SEQUENCE DATA │
  └──────────────────────────────┘
               │
               ▼
  ┌──────────────────────────────┐
  │    EXECUTE EACH SECONDARY    │
  │ STRUCTURE PREDICTION PROGRAM │───── SA-3
  │   ON OBJECTIVE SEQUENCE DATA │
  └──────────────────────────────┘
               │
               ▼
  ┌──────────────────────────────┐
  │ STORE RESPECTIVE RESULT DATA IN │───── SA-4
  │ PREDICTION RESULT DATA BASE  │
  └──────────────────────────────┘
               │
               ▼
  ┌──────────────────────────────┐
  │     COMPARE RESPECTIVE       │───── SA-5
  │   PREDICTION RESULT DATA     │
  └──────────────────────────────┘
               │
               ▼
  ┌──────────────────────────────┐
  │  CALCULATE FRUSTRATION SCORES │───── SA-6
  │    OF RESPECTIVE PORTIONS    │
  └──────────────────────────────┘
               │
               ▼
  ┌──────────────────────────────┐
  │    PREDICT INTERACTION SITE  │───── SA-7
  └──────────────────────────────┘
               │
               ▼
  ┌──────────────────────────────┐
  │    OUTPUT PREDICTION RESULTS │───── SA-8
  └──────────────────────────────┘
               │
               ▼
        ┌─────────────┐
        │     END     │
        └─────────────┘
```

# FIG.5

START

SB-1

IS OBJECTIVE SEQUENCE DATA PRESENT IN PROTEIN STRUCTURE DATA BASE?

Yes

No

SB-2

STORE SECONDARY STRUCTURE DATA IN PREDICTION RESULT DB

SB-3

IS SIMILAR SEQUENCE PRESENT IN PROTEIN STRUCTURE DATA BASE ?

Yes

No

SB-4

STORE SECONDARY STRUCTURE DATA OF SIMILAR SITE IN PREDICTION RESULT DB

END

# FIG.6

START

CALCULATE SCORE BASED ON COMPARISON RESULTS OF PREDICTION RESULT DATA OF RESPECTIVE PROGRAMS — SC-1

CALCULATE SCORE BASED ON SECONDARY STRUCTURE DATA — SC-2

CALCULATE SCORE BASED ON CERTAINTY FACTOR INFORMATION — SC-3

END

# FIG.7

<DISPLAY SCREEN OF INTERACTION SITE PREDICTION RESULTS>

OBJECTIVE SEQUENCE DATA — MA-1

A — MA-2

B — MA-3

FRUSTRATION

INTERACTION SITE A — MA-4

FRUSTRATION

INTERACTION SITE B — MA-5

# FIG.8

# FIG.9

# FIG.10

(BASIC PRINCIPLE OF THE INVENTION)

```
                                           ⌐S1
  ┌─────────────────────────────┐
  │   ACQUIRE THREE-DIMENSIONAL  │
  │  STRUCTURE DATA OF PROTEIN   │
  └─────────────────────────────┘
```

(CALCULATION CONDITIONS) ⌐S3

```
                        ⌐S2
  ┌──────────────────────────────────┐         ┌─────────────┐
  │  MOLECULAR ORBITAL CALCULATION    │◄───────│   WATER     │ ~S31
  │  ⌐S21                  ⌐S22        │         │  MOLECULE   │
  │ ┌──────────────┐ ┌──────────────┐ │         │  SETTING    │
  │ │FRONTIER ORBITAL│ │ORBITAL ENERGY OF│◄──────│  DIELECTRIC │ ~S32
  │ │(HOMO, LUMO)  │ │MAIN CHAIN ATOM│ │         │  SETTING    │
  │ └──────────────┘ └──────────────┘ │◄──────│   CHARGE    │ ~S33
  └──────────────────────────────────┘         │  SETTING    │
                                                └─────────────┘
           ⌐S4
  ┌─────────────────────────────┐
  │   DETERMINE CANDIDATE        │
  │   AMINO ACID RESIDUE         │
  └─────────────────────────────┘
           ⌐S5
  ┌─────────────────────────────┐
  │    PREDICT ACTIVE SITE       │
  └─────────────────────────────┘
```

# FIG.11

PROTEIN ACTIVE SITE PREDICTING DEVICE `1100`

INPUT DEVICE `1112`

OUTPUT DEVICE `1114`

INPUT-OUTPUT CONTROL INTERFACE UNIT `1108`

STORAGE UNIT `1106`

PROTEIN STRUCTURE DB `1106a`

PROCESS RESULT DATA `1106b`

CONTROL UNIT `1102`

STRUCTURE DATA ACQUIRING UNIT `1102p`

WATER MOLECULE SETTING UNIT `1102c`

DIELECTRIC SETTING UNIT `1102d`

CHARGE SETTING UNIT `1102e`

FRONTIER ORBITAL CALCULATING UNIT `1102a`

PERIPHERAL ORBITAL DETERMINING UNIT `1102b`

ORBITAL ENERGY CALCULATING UNIT `1102h`

CANDIDATE AMINO ACID RESIDUE DETERMINING UNIT `1102f`

ACTIVE SITE PREDICTING UNIT `1102g`

COMMUNICATION CONTROL INTERFACE UNIT `1104`

EXTERNAL SYSTEM `1200`

EXTERNAL DB

EXTERNAL PROGRAM

NETWORK `1300`

EP 1 510 943 A1

# FIG.12

FRONTIER ORBITAL CALCULATING UNIT

1102a

| HIGHEST OCCUPIED ORBITAL CALCULATING UNIT | ~1102i |

| LOWEST UNOCCUPIED ORBITAL CALCULATING UNIT | ~1102j |

# FIG.13

ACTIVE SITE PREDICTING UNIT

1102g

| SPECIFIC AMINO ACID RESIDUE EXCLUDING UNIT | ~1102k |

| LOCALIZED AMINO ACID RESIDUE SELECTING UNIT | ~1102m |

| CANDIDATE COMPARING UNIT | ~1102n |

# FIG.14

```
                    START

        ┌──────────────────────────┐
        │  THREE-DIMENSIONAL       │── SA1-1
        │  STRUCTURE OF PROTEIN    │
        └──────────────────────────┘

        ┌──────────────────────────┐
        │  QUANTUM CHEMISTRY       │
        │  CALCULATION             │── SA1-2
        │  (MOLECULE ORBITAL       │
        │  CALCULATION)            │
        └──────────────────────────┘
```

SA1-3                                          SA1-4

DETERMINE CANDIDATE
AMINO ACID RESIDUE FROM
FRONTIER ORBITAL AND
PERIPHERAL ORBITALS

DETERMINE CANDIDATE AMINO
ACID RESIDUE FROM ORBITAL
ENERGY OF MAIN CHAIN ATOM

SA1-5

PREDICTION BASED ON
ACTIVE SITE CANDIDATE
AMINO ACID RESIDUE

END

EP 1 510 943 A1

# FIG.15

START

COORDINATES OF PROTEIN — SB1-1

GENERATE INITIAL DENSITY MATRIX — SB1-2

CALCULATION OF 2 ELECTRON INTEGRATION — SB1-3

GENERATE FOCK MATRIX — SB1-4

DIAGONIZE FOCK MATRIX — SB1-5

GENERATE NEW DENSITY MATRIX — SB1-6

IS DIFFERENCE SUFFICIENTLY SMALL ? — SB1-7

No

REPETITION

Yes

ORBITAL ENERGY, COEFFICIENT OF MOLECULAR ORBITAL — SB1-8

SYSTEM ENERGY — SB1-9

END

# FIG.16

START

ATTRIBUTE MOLECULAR ORBITAL TO CORRESPONDING
DISTRIBUTION ON AMINO ACID RESIDUE ⎯SC1-1

DEFINE AMINO ACID RESIDUE (CANDIDATE FOR ACTIVE SITE)
ON WHICH FRONTIER ORBITAL AND PERIPHERAL ORBITALS ARE
DISTRIBUTED AMONG THESE ⎯SC1-2

EXCLUDE FROM CANDIDATES AMINO ACID RESIDUE THAT IS LESS
POSSIBLE TO FORM ACTIVE SITE, BUT TENDS TO HAVE FRONTIER
ORBITAL, SUCH AS TRIPTOPHANE AND PHENYL ALANINE, AS WELL
AS FRONTIER ORBITAL DISTRIBUTED ON METHIONINE AND CYSTINE
HAVING DISULFIDE BOND, FOR THE SAME REASON ⎯SC1-3

PORTION IN WHICH CANDIDATE AMINO ACID RESIDUES ARE
LOCATED CLOSELY TOGETHER FORMS CANDIDATE FOR ACTIVE SITE ⎯SC1-4

END

# FIG.17

START

N-NUMBERED MOLECULAR ORBITAL ～SD1-1

ADD SQUARES OF COEFFICIENTS OF BASIS FUNCTION BELONGING TO ATOM FOR EACH ATOM ～SD1-2

ADD SUMS OF SQUARES OF COEFFICIENTS OF ATOM BELONGING TO AMINO ACID FOR EACH AMINO ACID ～SD1-3

DEFINE AMINO ACID HAVING HIGHEST VALUE AS AMINO ACID TO WHICH MOLECULAR ORBITAL BELONGS FOR EXAMPLE, EACH MOLECULAR ORBITAL IS ATTRIBUTED TO AMINO ACID AS FOLLOWS:

MOLECULAR ORBITAL NO. 1  HISTIDINE 40
MOLECULAR ORBITAL NO. 2  GLUTAMIC ACID 58
MOLECULAR ORBITAL NO. 3 ....

～SD1-4

END

# FIG.18

SE1-1

**GENERATE CANDIDATE BASED ON FRONTIER ORBITAL**

SE1-1

**GENERATE CANDIDATE BASED ON ORBITAL ENERGY OF MAIN CHAIN ATOM**

SE1-2

**COINCIDE WITH EACH OTHER ?**

Yes

No

SE1-3

**FURTHER ADD AMINO ACIDS LOCATED BEFORE AND AFTER TO CANDIDATE**

SE1-3

**FURTHER ADD NEXT AMINO ACID TO CANDIDATE**

SE1-4

**ACTIVE SITE**

# FIG.19

```
              ┌─────────────┐
              │    START    │
              └─────────────┘
                     │
                     ▼
```

ATTRIBUTE MOLECULAR ORBITAL TO ATOM
ON WHICH IT IS DISTRIBUTED — SF1-1

EXTRACT ONLY MOLECULAR ORBITAL DISTRIBUTED ON
HEAVY ATOMS IN MAIN CHAIN — SF1-2

AMONG MOLECULAR ORBITALS ATTRIBUTED TO
RESPECTIVE HEAVY ATOMS, DETERMINE ORBITAL
ENERGY OF MOLECULAR ORBITAL THAT IS
UNOCCUPIED ORBITAL AND HAS HIGHEST
ENERGY AS TYPICAL ENERGY OF RESPECTIVE
AMINO ACID RESIDUES — SF1-3

PLOT AMINO ACID RESIDUE NUMBERS AND TYPICAL
ENERGY ON AXIS OF ABSCISSAS AND AXIS OF
ORDINATES RESPECTIVELY — SF1-4

IN PLOTTED GRAPH, PERIPHERAL PART OF
PEAK FORMS FUNCTIONAL SITE — SF1-5

```
              ┌─────────────┐
              │     END     │
              └─────────────┘
```

# FIG.20

| MOLECULAR ORBITAL | ORBITAL ENERGY | TYPES | DISTRIBUTION RATE | | | | |
|---|---|---|---|---|---|---|---|
| | | | R | E | W | T | Y |
| 1 | -10 | OCCUPIED | 90% | 1% | 9% | | |
| 2 | -8.5 | OCCUPIED | 1% | 4% | 5% | 65% | 25% |
| 3 | -8 | OCCUPIED | 2% | 95% | 3% | | |
| 4 | -7 | OCCUPIED | | 2% | 90% | 8% | |
| 5 | 1 | UNOCCUPIED | 99% | | 1% | | |
| 6 | 2 | UNOCCUPIED | | | | 10% | 90% |
| 7 | 4 | UNOCCUPIED | | 100% | | | |

# FIG.21

CANDIDATE

THREE DIMENSIONAL STRUCTURE OF PROTEIN

# FIG.22

| MOLECULAR ORBITAL | ORBITAL ENERGY | TYPES | R MAIN CHAIN N ATOM NUMBER 1 | R SIDE CHAIN C ATOM NUMBER 2 | R SIDE CHAIN N ATOM NUMBER 3 | E MAIN CHAIN N ATOM NUMBER 4 | OTHERS |
|---|---|---|---|---|---|---|---|
| 1 | -10 | OCCUPIED | <u>90</u> | 10 | | | |
| 2 | -9 | OCCUPIED | 1 | | | <u>66</u> | 33 |
| 3 | -8 | OCCUPIED | | | | | 100 |
| 4 | -7 | OCCUPIED | | | 5 | 5 | 90 |
| 5 | -6 | OCCUPIED | <u>80</u> | | 20 | | |
| 6 | -5 | OCCUPIED | | | | <u>60</u> | 40 |
| 7 | -4 | OCCUPIED | 1 | <u>99</u> | | | |
| 8 | -3 | OCCUPIED | | | <u>99</u> | 1 | |
| 9 | 2 | UNOCCUPIED | <u>99</u> | | | | 1 |
| 10 | 1 | UNOCCUPIED | | 45 | | <u>50</u> | 5 |

# FIG.23

| NAME | ORBITAL ENERGY | AMINO ACID RESIDUE | NOTE | ACTIVE SITE |
|---|---|---|---|---|
| HOMO-6 | -8.734 | Tyr 57 | ④ | |
| HOMO-5 | -8.574 | Glu 58 | ③ | ○ |
| HOMO-4 | -8.543 | Trp 59 | × | |
| HOMO-3 | -8.533 | Tyr 11 | × | |
| HOMO-2 | -8.305 | His 40 | ② | ○ |
| HOMO-1 | -8.176 | Glu 58 | ① | ○ |
| HOMO | -8.013 | Trp 59 | × | |
| LUMO | -1.876 | Cys 2 | × | |
| LUMO+1 | -1.486 | Cys 6 | × | |
| LUMO+2 | -0.328 | Arg 77 | ① | ○ |
| LUMO+3 | -0.186 | His 92 | ② | ○ |
| LUMO+4 | -0.186 | Tyr 68 | ③ | |
| LUMO+5 | -0.186 | His 27 | ④ | |

# FIG.24

# FIG.25

| CANDIDATE | AMINO ACID RESIDUE | ORBITAL ENERGY |
|---|---|---|
| HIGHEST | 39 | -9.508 |
| SECOND HIGHEST | 60 | -9.563 |
| THIRD HIGHEST | 91 | -9.665 |
| FOURTH HIGHEST | 58 | -9.673 |
| FIFTH HIGHEST | 79 | -9.681 |

# FIG.26

| CANDIDATE BASED ON FRONTIER ORBITAL | CANDIDATE BASED ON ORBITAL ENERGY OF MAIN CHAIN ATOM | COMMON | ACTIVE SITE |
|---|---|---|---|
| 27 | 77-81 | 40 | 40 |
| 40 | 56-60 | 57 | 58 |
| 57 | 89-93 | 58 | 77 |
| 58 | 58-62 | 77 | 92 |
| 77 | 37-41 | 92 | |
| 68 | | | |
| 92 | | | |

# FIG.27

| NAME | ORBITAL ENERGY | AMINO ACID RESIDUE | NOTE | ACTIVE SITE |
|---|---|---|---|---|
| HOMO-5 | -8.699 | Tyr 73 | ④ | |
| HOMO-4 | -8.604 | His 48 | ③ | |
| HOMO-3 | -8.545 | Met 13 | ✕ | |
| HOMO-2 | -8.424 | His 12 | ② | ◯ |
| HOMO-1 | -8.420 | Asp 14 | ① | |
| HOMO | -8.353 | Met 79 | ✕ | |
| LUMO | -2.457 | Cys 84 | ✕ | |
| LUMO+1 | -2.233 | Cys 72 | ✕ | |
| LUMO+2 | -2.122 | Cys 40 | ✕ | |
| LUMO+3 | -1.575 | Cys 58 | ✕ | |
| LUMO+4 | -0.489 | Tyr 97 | ① | |
| LUMO+5 | -0.428 | Cys 26 | ✕ | |
| LUMO+6 | -0.274 | Tyr 97 | ① | |
| LUMO+7 | -0.124 | Cys 26 | ✕ | |
| LUMO+8 | -0.085 | Tyr 25 | ② | |
| LUMO+9 | -0.075 | Arg 85 | ③ | |
| LUMO+10 | -0.047 | Cys 95 | ✕ | |
| LUMO+11 | -0.024 | His 119 | ④ | ◯ |

# FIG.28

ORBITAL ENERGY (eV) vs AMINO ACID RESIDUE NUMBER

# FIG.29

| CANDIDATE | AMINO ACID RESIDUE | ORBITAL ENERGY |
|---|---|---|
| HIGHEST | 12 | -9.533 |
| SECOND HIGHEST | 47 | -9.59 |
| THIRD HIGHEST | 117 | -9.616 |
| FOURTH HIGHEST | 76 | -9.669 |
| FIFTH HIGHEST | 53 | -9.715 |

# FIG.30

| CANDIDATE BASED ON FRONTIER ORBITAL | CANDIDATE BASED ON ORBITAL ENERGY OF MAIN CHAIN ATOM | COMMON | ACTIVE SITE |
|---|---|---|---|
| 12 | 10-14 | 12 | 12 |
| 14 | 45-49 | 14 | 119 |
| 25 | 115-119 | 119 | |
| 48 | 74-78 | | |
| 73 | 51-55 | | |
| 85 | | | |
| 97 | | | |
| 119 | | | |

# FIG.31

(BASIC PRINCIPLE OF THE INVENTION)

① SPECIFY PORTION THAT IS HIGHLY INSTABLE DUE TO HYDROPHOBIC INTERACTION

PROTEIN A    PROTEIN B

SOLVENT CONTACT FACE AS WELL AS HYDROPHOBIC FACE (HIGHLY INSTABLE)

PROTEIN A    PROTEIN B

STABILIZED WITH HYDROPHOBIC FACE COVERED WITH COMPOSITE BODY FORMATION

② SPECIFY PORTION THAT IS HIGHLY INSTABLE DUE TO ELECTROSTATIC INTERACTION

PROTEIN A    PROTEIN B

PORTION THAT IS INSTABLE AS SINGLE SUBSTANCE

PROTEIN A    PROTEIN B

STABILIZED IN TERMS OF ENERGY BY COMPOSITE BODY FORMATION

③ THROUGH BOTH OF PROCESSES, SPECIFY PORTION THAT IS HIGHLY INSTABLE AS SINGLE SUBSTANCE AND STABILIZED UPON FORMATION OF COMPOSITE BODY AS INTERACTION SITE

EP 1 510 943 A1

# FIG.32

INPUT DEVICE `2112`

OUTPUT DEVICE `2114`

`2100`

PROTEIN INTERACTION INFORMATION PROCESSING DEVICE

INPUT-OUTPUT CONTROL INTERFACE UNIT `2108`

`2106`

STORAGE UNIT

`2106a`

PROTEIN STRUCTURE DB

`2106b`

PROCESS RESULT DATA

`2102`

CONTROL UNIT

STRUCTURE DATA ACQUIRING UNIT `2102a`

SOLVENT CONTACT FACE SPECIFYING UNIT `2102b`

HYDROPHOBIC FACE SPECIFYING UNIT `2102c`

ELECTROSTATIC INTERACTION SPECIFYING UNIT `2102d`

INTERACTION SITE SPECIFYING UNIT `2102e`

INTERACTION PREDICTING UNIT `2102f`

CANDIDATE PROTEIN RETRIEVING UNIT `2102g`

`2104`

COMMUNICATION CONTROL INTERFACE UNIT

`2200`

EXTERNAL SYSTEM

EXTERNAL DB

EXTERNAL PROGRAM

NETWORK `2300`

EP 1 510 943 A1

# FIG.33

```
          ┌──────────┐
          │  START   │
          └────┬─────┘
               │
               ▼
  ┌──────────────────────────────────┐
  │  ACQUIRE PROTEIN STRUCTURE DATA   │─── SA2-1
  └────────────────┬─────────────────┘
                   │
                   ▼
  ┌──────────────────────────────────┐
  │   SPECIFY SOLVENT CONTACT FACE    │─── SA2-2
  └────────────────┬─────────────────┘
                   │
                   ▼
  ┌──────────────────────────────────┐
  │     SPECIFY HYDROPHOBIC FACE      │─── SA2-3
  └────────────────┬─────────────────┘
                   │
                   ▼
  ┌──────────────────────────────────┐
  │  SPECIFY ELECTROSTATIC INTERACTION │─── SA2-4
  └────────────────┬─────────────────┘
                   │
                   ▼
  ┌──────────────────────────────────┐
  │     SPECIFY INTERACTION SITE      │─── SA2-5
  └────────────────┬─────────────────┘
                   │
                   ▼
             ┌──────────┐
             │   END    │
             └──────────┘
```

# FIG.34

START

CALCULATE SOLVENT CONTACT FACE $S_{isolated}$ WITH RESPECT TO EACH RESIDUE AS SINGLE SUBSTANCE ⟋ SB2-1

CALCULATE SOLVENT CONTACT FACE $S_{complex}$ WITH RESPECT TO EACH RESIDUE UPON FORMATION OF COMPOSITE BODY ⟋ SB2-2

CALCULATE DIFFERENCE $\Delta S$ WITH RESPECT TO EACH RESIDUE $\Delta S = S_{isolated} - S_{complex}$ ⟋ SB2-3

END

# FIG.35

```
┌─────────────┐
│    START    │
└─────────────┘
       │
       ▼
┌──────────────────────────────────────┐
│ CALCULATE AMOUNT OF REDUCTION IN      │  ── SC2-1
│ HYDROPHOBIC FACE                      │
└──────────────────────────────────────┘
       │
       ▼
┌──────────────────────────────────────┐
│ CALCULATE HYDROPHOBIC INTERACTION     │  ── SC2-2
│ ENERGY $E_{hydrophobic}$              │
└──────────────────────────────────────┘
       │
       ▼
┌──────────────────────────────────────┐
│ SPECIFY SITE HAVING HYDROPHOBIC       │
│ INTERACTION ENERGY $E_{hydrophobic}$  │  ── SC2-3
│ EXCEEDING THRESHOLD VALUE AS          │
│ HYDROPHOBIC FACE                      │
└──────────────────────────────────────┘
       │
       ▼
┌─────────────┐
│     END     │
└─────────────┘
```

# FIG.36

START

CALCULATE ELECTROSTATIC INTERACTION
ENERGY $E_n$ WITH RESPECT TO EACH RESIDUE
SD2-1

$$E_n = \frac{1}{4\pi\varepsilon} \sum_{i\in n} \sum_{j\notin n} \frac{q_i q_j}{R_{ij}}$$

END

# FIG.37

START

SPECIFY SITE HAVING SOLVENT
CONTACT FACE $\triangle S$ EXCEEDING
THRESHOLD VALUE
SE2-1

SPECIFY SITE HAVING HYDROPHOBIC
INTERACTION ENERGY $E_{hydrophobic}$ EXCEEDING
THRESHOLD VALUE
SE2-2

SPECIFY SITE HAVING ELECTROSTATIC
INTERACTION ENERGY $E_n$ EXCEEDING
THRESHOLD VALUE
SE2-3

END

# FIG.38

START

**SF2-1**

WITH RESPECT TO OBJECTIVE PROTEIN A, ELECTROSTATIC INTERACTION ENERGY AND HYDROPHOBIC FACE ARE ANALYZED TO ESTIMATE OLIGO PEPTIDE CHAINS $(a_1, a_2, a_3, ...)$ AS CANDIDATES FOR INTERACTION

$a_4$:TEHYQ
$a_2$:SNREG
$a_3$:GFRNS
$a_1$:VASKG

**SF2-2**

RETRIEVE OLIGO PEPTIDE CHAINS $(b_{i1}, b_{i2}, b_{i3}, ...)$ TO FORM PAIRS WITH $a_i$ INSIDE THE SAME PROTEIN OR BETWEEN PROTEINS FROM PDB DATA

$a_1$:VASKG

$b_{11}$:AKRSE   $b_{12}$:LDEET   $b_{13}$:WPYKN   $b_{14}$:PPYWN   $\cdots\cdots$

**SF2-3**

RETRIEVE PROTEINS $(B_{ij1}, B_{ij2}, B_{ij3}, ...)$ HAVING $b_{ij}$ AS PARTIAL SEQUENCE FROM PDB DATA

$B_{ij1}$   $B_{ij2}$   $B_{ij3}$   $B_{ij4}$

$b_{ij}$

**SF2-4**

WITH RESPECT TO PROTEIN $B_{ijk}$, ELECTROSTATIC INTERACTION ENERGY AND HYDROPHOBIC FACE ARE ANALYZED TO ESTIMATE INTERACTION CANDIDATE SITE. EXAMINE WHETHER OR NOT THIS SITE IS COINCIDES WITH $b_{ij}$.

END

# FIG.39

EP 1 510 943 A1

# FIG.40

barnase 1a2p

F82

EP 1 510 943 A1

FIG.41

# FIG.42

EP 1 510 943 A1

# FIG.43

EP 1 510 943 A1

FIG.44

EP 1 510 943 A1

# FIG.45

EP 1 510 943 A1

FIG.46

EP 1 510 943 A1

FIG.47

EP 1 510 943 A1

# FIG.48

EP 1 510 943 A1

EP 1 510 943 A1

# FIG.49

FIG.50

# FIG.51

(BASIC PRINCIPLE 1 OF THE INVENTION: METHOD FOR SEARCHING FOR BINDING SITE OF PROTEIN FROM ONE PIECE OF SEQUENCE INFORMATION)

SA3-1

ESTIMATE SPATIAL DISTANCE r IN SPACE OF THREE-DIMENSIONAL STRUCTURE OF PROTEIN FROM DISTANCE d ON AMINO ACID SEQUENCE, RESULTS OF THREE-DIMENSIONAL STRUCTURE PREDICTION SIMULATION, THREE-DIMENSIONAL STRUCTURE COORDINATE DATA OBTAINED FROM PROTEIN STRUCTURE DB AND THE LIKE

SA3-2

CALCULATE TOTAL ENERGY $E_{total}$ OF PROTEIN FROM SPATIAL DISTANCE r

EXAMPLE) $E_{total} = 1/2 \ \Sigma\Sigma \ q_i q_j / r$

SA3-3

BY CALCULATING INTERACTION ENERGY BETWEEN SPECIFIC AMINO ACID AND OTHER AMINO ACID RESIDUE INSIDE PROTEIN BASED ON FOLLOWING EQUATION, FIND OUT HOW EACH OF AMINO ACID RESIDUES CONTRIBUTES TO STABILIZE TOTAL ENERGY OF PROTEIN

$E_{interaction}(N) = q_N \Sigma q_j / r$

SA3-4

PREDICT AN AREA HAVING AMINO ACID RESIDUE THAT MAKES PROTEIN INSTABLE IN TERMS OF ENERGY AS AN AREA OF BINDING SITE

BINDING SITE

AREA THAT MAKES PROTEIN INSTABLE (BINDING SITE)

# FIG.52

(BASIC PRINCIPLE 2 OF THE INVENTION: METHOD WHICH, IN THE CASE WHEN PIECES OF SEQUENCE INFORMATION SHOW BINDING SITES, PREDICTS RESPECTIVE BINDING SITES)

SB3-1

ESTIMATE AMINO ACID TO BE USED FOR INTERACTION WHEN A PLURALITY OF SEQUENCES ARE BOUND

SB3-2

DEFINE "DISTANCE ON SEQUENCES" BETWEEN TWO AMINO ACID RESIDUES LOCATED ON DIFFERENT SEQUENCES

IN GENERAL, FOLLOWING EQUATION HOLDS

(DISTANCE BETWEEN ATTENTION RESIDUES ON SEQUENCES) = (|DISTANCE BETWEEN ATTENTION RESIDUE ON SEQUENCE A AND BINDING RESIDUE| + |DISTANCE BETWEEN ATTENTION RESIDUE ON SEQUENCE B AND BINDING RESIDUE|)

SB3-3

ESTIMATE SPATIAL DISTANCE r OF THREE-DIMENSIONAL STRUCTURE OF PROTEIN FROM DISTANCE d ON AMINO ACID SEQUENCE BETWEEN AMINO ACID RESIDUES

SB3-4

CALCULATE TOTAL ENERGY Etotal OF PROTEIN FROM INFORMATION ON SPATIAL DISTANCE

SB3-5

RETURN TO SB3-1, CHANGE AMINO ACID USED FOR INTERACTION, AND AFTER TRYING OUT ALL COMBINATIONS, PREDICT AMINO ACID TO INTERACT AT THE LOWEST ENERGY AS BINDING SITE

# FIG.53

INPUT DEVICE ~3112    OUTPUT DEVICE ~3114

3100

BINDING SITE
PREDICTING DEVICE ~3106

INPUT-OUTPUT CONTROL INTERFACE UNIT ~3108

EXTERNAL
SYSTEM ~3200

STORAGE UNIT

AMINO ACID
SEQUENCE DB ~3106a

PROTEIN
STRUCTURE DB ~3106b

DISTANCE DATA FILE ~3106c

ENTIRE ENERGY
DATA FILE ~3106d

INTERACTION
ENERGY DATA FILE ~3106e

COMPOSITE BODY
STRUCTURE DATA
FILE ~3106f

PROCESSING
RESULT DATA ~3106g

CONTROL UNIT ~3102

AMINO ACID SEQUENCE
DATA ACQUIRING UNIT ~3102a

SPACE DISTANCE
DETERMINING UNIT ~3102b

CHARGE
DETERMINING UNIT ~3102c

ENERGY
CALCULATING UNIT ~3102d

CANDIDATE AMINO ACID
RESIDUE DETERMINING UNIT

COMPOSITE BODY
STRUCTURE GENERATING
UNIT ~3102e ~3102f

ENERGY MINIMIZING UNIT ~3102g

BINDING CANDIDATE
DATA ACQUIRING UNIT ~3102h

BINDING SITE
PREDICTING UNIT ~3102i

BINDING PARTNER
CANDIDATE
DETERMINING UNIT ~3102j

COMMUNICATION CONTROL INTERFACE UNIT ~3104

EXTERNAL DB

EXTERNAL
PROGRAM

NETWORK
~3300

EP 1 510 943 A1

# FIG.54

3102b

SPACE DISTANCE DETERMINING UNIT

| HIGH-SPEED CALCULATING UNIT | ~3102k |

| STRUCTURE DATA USE CALCULATING UNIT | ~3102m |

| SIMULATION DATA USE CALCULATING UNIT | ~3102n |

# FIG.55

3102d

ENERGY CALCULATING UNIT

| ENTIRE ENERGY CALCULATING UNIT | ~3102p |

| INTERACTION ENERGY CALCULATING UNIT | ~3102q |

# FIG.56

d
100 RESIDUES

30  130

AMINO ACID SEQUENCE

EXAMPLE) $r = kd^n$ $(0 < n < 1)$

$r$
20Å

THREE-DIMENSIONAL STRUCTURE

# FIG.57

BINDING SITE a OF SEQUENCE A: 50TH

BOUND AT THIS POINT

BINDING SITE b OF SEQUENCE B: 100TH

# FIG.58

30 RESIDUES    BINDING RESIDUE

ATTENTION RESIDUE ON SEQUENCE A

ATTENTION RESIDUE ON SEQUENCE B

200 RESIDUES

# FIG.59

START

ACQUIRE SEQUENCE DATA OF PROTEIN — SC3-1

ACQUIRE SEQUENCE DATA OF CANDIDATE PROTEIN — SC3-6

GENERATE COMPOSITE BODY STRUCTURE BY USING DOCKING SIMULATION AND THE LIKE — SC3-7

DETERMINE SPATIAL DISTANCE — SC3-2

DETERMINE CHARGE FOR EACH OF AMINO ACID RESIDUES — SC3-3

CALCULATE ENERGY — SC3-4

DETERMINE BINDING SITE CANDIDATE — SC3-5

DETERMINE BINDING PARTNER CANDIDATE — SC3-8

END

DOT LINE: PREDICT BINDING SITE IN SEQUENCE OF ONE PROTEIN

DOUBLE LINE: PREDICT BINDING SITE IN EACH OF SEQUENCES OF A PLURALITY OF PROTEINS

SOLID LINE: PREDICT PARTNER CANDIDATE PROTEIN TO BE BEST BOUND TO OBJECTIVE PROTEIN

# FIG.60

| TYPES OF AMINO ACIDS | AMINO ACID NUMBER | CHARGE | INTERACTION ENERGY |
|---|---|---|---|
| GLU | 1 | -1 | 0.14 |
| ALA | 2 | 0 | 0 |
| ASP | 3 | -1 | -0.34 |
| ARG | 4 | 1 | -1.52 |
| TOTAL | | | -1.57 |

# FIG.61

BINDING SITE

# FIG.62

# FIG.63

# FIG.64

DISTANCE BETWEEN AMINO ACID RESIDUES

(Y-axis: AVERAGE DISTANCE IN PDB)

# FIG.65

RESIDUE NUMBER

# FIG.66

| BINDING SITE CANDIDATE | | |
|---|---|---|
| TYPES OF AMINO ACIDS | RESIDUE NUMBER | |
| LYS | 1 | |
| LYS | 7 | BINDING SITE |
| ARG | 10 | BINDING SITE |
| HIS | 12 | BINDING SITE |
| LYS | 31 | BINDING SITE |
| ARG | 33 | |
| LYS | 37 | BINDING SITE |
| ARG | 39 | BINDING SITE |
| LYS | 41 | BINDING SITE |
| HIS | 48 | |
| LYS | 61 | |
| LYS | 66 | BINDING SITE |
| ARG | 85 | BINDING SITE |
| LYS | 91 | BINDING SITE |
| LYS | 98 | BINDING SITE |
| LYS | 104 | |
| HIS | 105 | |
| HIS | 119 | BINDING SITE |

# FIG.67

| ATOM | 1 | N | GLU | A | 4 | 31.787 | 8.114 | 206.704 |
|------|------|------|------|------|------|--------|--------|---------|
| **ATOM** | **2** | **CA** | **GLU** | **A** | **4** | **32.664** | **8.451** | **205.542** |
| ATOM | 3 | C | GLU | A | 4 | 34.139 | 8.365 | 205.961 |
| ATOM | 4 | O | GLU | A | 4 | 34.597 | 9.206 | 206.741 |
| ATOM | 5 | CB | GLU | A | 4 | 32.362 | 9.878 | 205.041 |
| ATOM | 6 | CG | GLU | A | 4 | 31.206 | 10.023 | 204.032 |
| ATOM | 7 | CD | GLU | A | 4 | 29.82 | 9.97 | 204.657 |
| ATOM | 8 | OE1 | GLU | A | 4 | 29.46 | 10.903 | 205.41 |
| ATOM | 9 | OE2 | GLU | A | 4 | 29.081 | 9.003 | 204.373 |
| ATOM | 10 | N | ASP | A | 5 | 34.864 | 7.351 | 205.473 |
| **ATOM** | **11** | **CA** | **ASP** | **A** | **5** | **36.279** | **7.196** | **205.808** |
| ATOM | 12 | C | ASP | A | 5 | 37.048 | 5.998 | 205.209 |
| ATOM | 13 | O | ASP | A | 5 | 37.185 | 4.969 | 205.861 |
| ATOM | 14 | CB | ASP | A | 5 | 36.461 | 7.19 | 207.333 |
| ATOM | 15 | CG | ASP | A | 5 | 37.838 | 7.668 | 207.764 |
| ATOM | 16 | OD1 | ASP | A | 5 | 38.692 | 7.942 | 206.889 |
| ATOM | 17 | OD2 | ASP | A | 5 | 38.057 | 7.777 | 208.993 |
| ATOM | 18 | N | PRO | A | 6 | 37.449 | 6.084 | 203.93 |
| ATOM | 19 | CA | PRO | A | 6 | 38.206 | 5.043 | 203.22 |

*STARTING FROM 2nd COLUMN, RESPECTIVE COLUMNS INDICATE ATOM NUMBER, ATOM TYPES, CHAIN NAME, AMINO ACID RESIDUE NUMBER, X-COORDINATE, Y-COORDINATE AND Z-COORDINATE

# FIG.68

ACETYLCHOLINE ESTERASE INHIBITOR

# FIG.69

| BINDING SITE CANDIDATE | | |
|---|---|---|
| TYPES OF AMINO ACIDS | RESIDUE NUMBER | |
| HIS | 6 | BINDING SITE |
| ARG | 11 | BINDING SITE |
| ARG | 24 | BINDING SITE |
| LYS | 25 | |
| ARG | 27 | BINDING SITE |
| ARG | 28 | |
| HIS | 29 | BINDING SITE |
| LYS | 32 | BINDING SITE |
| ARG | 37 | BINDING SITE |
| LYS | 51 | |

## FIG.70

Legend:
- 0.00 -5.00
- -5.00 -0.00
- -10.00 --5.00
- -15.00 --10.00
- -20.00 --15.00
- -25.00 --20.00

EP 1 510 943 A1

# FIG.71

# FIG.72

START

OBTAIN COORDINATE DATA OF PROTEIN — SA4-1

EXTRACT COORDINATES OF AMINO ACID RESIDUE i AND NEIGHBORING AMINO ACID RESIDUE GROUP WITHIN NEIGHBORHOOD $r$Å — SA4-2

ADD CAP WITH HYDROGEN ATOM (H) OR METHYL GROUP ($CH_3$) TO CUT-OUT PORTION OF EXTRACTED NEIGHBORING AMINO ACID RESIDUE GROUP — SA4-3

CALCULATE CHARGE OF ENTIRE NEIGHBORING AMINO ACID RESIDUE GROUP — SA4-4

OPTIMIZE STRUCTURE OF AMINO ACID RESIDUE i — SA4-5

SUBSTITUTE COORDINATES OF OPTIMIZED ATOM — SA4-6

APPLY SA4-2 THROUGH SA4-6 TO ALL AMINO ACID RESIDUES — SA4-7

REPEATEDLY APPLY SA4-1 THROUGH SA4-7 TO COORDINATE DATA OF PROCESS RESULTS — SA4-8

END

# FIG.73

INPUT DEVICE — 4112

OUTPUT DEVICE — 4114

PROTEIN STRUCTURE OPTIMIZING DEVICE — 4100

INPUT-OUTPUT CONTROL INTERFACE UNIT — 4108

EXTERNAL SYSTEM — 4200

EXTERNAL DB

EXTERNAL PROGRAM

STORAGE UNIT — 4106

CONTROL UNIT — 4102

COMMUNICATION CONTROL INTERFACE UNIT — 4104

PROTEIN STRUCTURE INFORMATION DATABASE — 4106a

PROCESS RESULT FILE — 4106b

COORDINATE DATA ACQUIRING UNIT — 4102a

NEIGHBORING AMINO ACID RESIDUE GROUP EXTRACTING UNIT — 4102b

CAP ADDING UNIT — 4102c

CHARGE CALCULATING UNIT — 4102d

STRUCTURE OPTIMIZING UNIT — 4102e

ATOMIC COORDINATE SUBSTITUTING UNIT — 4102f

4300 NETWORK

# FIG.74

```
                      START

ACQUIRE COORDINATE DATA                          SB4-1

ADD 1 TO n                                       SB4-2

ADD 1 TO i                                       SB4-3

EXTRACT COORDINATES OF AMINO ACID RESIDUE i AND  SB4-4
NEIGHBORING AMINO ACID RESIDUE GROUP WITHIN
NEIGHBORHOOD rÅ

ADD CAP WITH HYDROGEN ATOM (H) OR METHYL GROUP (CH3)  SB4-5
TO CUT-OUT PORTION OF EXTRACTED NEIGHBORING
AMINO ACID RESIDUE GROUP

CALCULATE CHARGE OF ENTIRE NEIGHBORING           SB4-6
AMINO ACID RESIDUE GROUP

SET OPTIMIZING FLAG TO EACH ATOM FORMING         SB4-7
AMINO ACID RESIDUE i

GENERATE INPUT FILE FOR MOPAC 2000               SB4-8

START MOPAC 2000 TO PERFORM STRUCTURAL OPTIMIZATION  SB4-9

SUBSTITUTE COORDINATES OF OPTIMIZED ATOMS        SB4-10

            IS i LAST?          SB4-11    NO

              YES

            IS n LAST?          SB4-12    NO

              YES

                      END
```

# FIG.75

```
ATOM      1   N     PRO  1    1    7. 854  104. 656  32. 974
ATOM      2   CA    PRO  1    1    7. 699  104. 034  31. 626
ATOM      3   C     PRO  1    1    6. 224  104. 044  31. 225
ATOM      4   O     PRO  1    1    5. 374  104. 542  31. 976
ATOM      5   CB    PRO  1    1    8. 252  102. 610  31. 704
ATOM      6   CG    PRO  1    1    8. 313  102. 380  33. 238
ATOM      7   CD    PRO  1    1    8. 637  103. 754  33. 838
ATOM      8   H1    PRO  1    1    8. 307  105. 545  32. 880
ATOM      9   H19   PRO  1    1    8. 262  104. 633  30. 852
ATOM     10   H21   PRO  1    1    7. 604  101. 848  31. 214
ATOM     11   H23   PRO  1    1    9. 272  102. 555  31. 254
ATOM     12   H25   PRO  1    1    7. 326  102. 006  33. 609
ATOM     13   H27   PRO  1    1    9. 087  101. 621  33. 506
ATOM     14   H29   PRO  1    1    8. 268  103. 859  34. 890
ATOM     15   H31   PRO  1    1    9. 740  103. 958  33. 814
ATOM     16   N     ASN  1    2    5. 932  103. 521  30. 041
ATOM     17   CA    ASN  1    2    4. 573  103. 458  29. 522
ATOM     18   C     ASN  1    2    4. 220  101. 980  29. 427
ATOM     19   O     ASN  1    2    4. 928  101. 148  29. 993
ATOM     20   CB    ASN  1    2    4. 520  104. 105  28. 139
ATOM     21   CG    ASN  1    2    5. 450  103. 428  27. 141
ATOM     22   OD1   ASN  1    2    6. 047  102. 391  27. 440

. . . . . .
OMITTED
. . . . . .

ATOM   2132   H40   ARG  1  135    7. 570   94. 945  31. 002
ATOM   2133   H42   ARG  1  135    7. 667   95. 518  32. 710
ATOM   2134   H44   ARG  1  135    9. 663   96. 855  32. 278
ATOM   2135   H46   ARG  1  135   10. 017   95. 974  30. 735
ATOM   2136   N     GLU  1  136    9. 286   91. 036  30. 707
ATOM   2137   CA    GLU  1  136   10. 105   89. 902  30. 320
ATOM   2138   C     GLU  1  136   11. 401   90. 443  29. 726
ATOM   2139   O     GLU  1  136   12. 410   89. 708  29. 745
ATOM   2140   CB    GLU  1  136    9. 331   89. 094  29. 277
ATOM   2141   CG    GLU  1  136   10. 053   87. 930  28. 638
ATOM   2142   CD    GLU  1  136    9. 277   87. 369  27. 463
ATOM   2143   OE1   GLU  1  136    8. 096   87. 002  27. 650
ATOM   2144   OE2   GLU  1  136    9. 838   87. 319  26. 346
ATOM   2145   OXT   GLU  1  136   11. 392   91. 606  29. 258
ATOM   2146   H     GLU  1  136    8. 772   91. 468  29. 962
ATOM   2147   H22   GLU  1  136   10. 370   89. 273  31. 218
ATOM   2148   H24   GLU  1  136    8. 395   88. 698  29. 761
ATOM   2149   H26   GLU  1  136    9. 002   89. 782  28. 445
ATOM   2150   H28   GLU  1  136   11. 071   88. 252  28. 293
ATOM   2151   H30   GLU  1  136   10. 198   87. 111  29. 390
ATOM   2152   H32   GLU  1  136   12. 254   91. 868  28. 859
TER
```

# FIG.76

START

SC4-1

WHEN AMINO
ACID RESIDUE j IS
N-TERMINAL
AMINO ACID

NO →

YES → SC4-2

AMINO SIDE OF RESIDUE j
DOES NOT FORM CUT-OUT
PORTION, AND THEREFORE NO
CAP ADDITION IS REQUIRED

SC4-3

WHEN
ADJACENT AMINO ACID
RESIDUE j-1 IS ALSO INCLUDED IN
EXTRACTED AMINO ACID
RESIDUE GROUP

NO →

YES → SC4-4

AMINO SIDE OF RESIDUE j DOES NOT
FORM CUT-OUT PORTION, AND THEREFORE
NO CAP ADDITION IS REQUIRED

SC4-5

TAKE MAIN CHAIN CARBONYL CARBON OF
AMINO ACID RESIDUE j-1 AS $C_{j-1}$

SC4-6

TAKE MAIN CHAIN AMINO GROUP
NITROGEN OF AMINO ACID RESIDUE j AS $N_j$

SC4-7

DETERMINE POSITION OF CAP HYDROGEN
ATOM $H_{CAPN}$ TO BE ADDED ACCORDING TO
THE FOLLOWING EQUATION

$$\overrightarrow{N_j H_{CAPN}} = \frac{\overrightarrow{N_j C_{j-1}}}{\left|\overrightarrow{N_j C_{j-1}}\right|} \times R_{NH} \quad ( R_{NH} = 1.01 \text{ Å})$$

END

# FIG.77

ORIGINAL COORDINATES　　　COORDINATES AFTER CAP IS ADDED

# FIG.78

START

WHEN AMINO ACID RESIDUE j IS C-TERMINAL AMINO ACID — SD4-1

NO →

YES — SD4-2

AMINO SIDE OF RESIDUE j DOES NOT FORM CUT-OUT PORTION, AND THEREFORE NO CAP ADDITION IS REQUIRED

WHEN ADJACENT AMINO ACID RESIDUE j+1 IS ALSO INCLUDED IN EXTRACTED AMINO ACID RESIDUE GROUP — SD4-3

NO →

YES — SD4-4

AMINO SIDE OF RESIDUE j DOES NOT FORM CUT-OUT PORTION, AND THEREFORE NO CAP ADDITION IS REQUIRED

TAKE MAIN CHAIN AMINO GROUP NITROGEN OF AMINO ACID RESIDUE j+1 AS $N_{j+1}$ — SD4-5

TAKE MAIN CHAIN CARBONYL CARBON OF AMINO ACID RESIDUE j AS $C_j$ — SD4-6

DETERMINE POSITION OF CAP HYDROGEN ATOM $H_{CAPN}$ TO BE ADDED ACCORDING TO THE FOLLOWING EQUATION — SD4-7

$$\overrightarrow{C_j H_{CAPC}} = \frac{\overrightarrow{C_j N_{j+1}}}{\left|\overrightarrow{C_j N_{j+1}}\right|} \times R_{Csp2H} \quad (R_{Csp2H} = 1.08 \text{ Å})$$

END

140

# FIG.79

ORIGINAL COORDINATES

COORDINATES AFTER CAP IS ADDED

EP 1 510 943 A1

# FIG.80

START

**SE4-1**
WHEN AMINO ACID RESIDUE j IS N-TERMINAL AMINO ACID — NO

YES

**SE4-2**
AMINO SIDE OF RESIDUE j DOES NOT FORM CUT-OUT PORTION, AND THEREFORE NO CAP ADDITION IS REQUIRED

**SE4-3**
WHEN ADJACENT AMINO ACID RESIDUE j-1 IS ALSO INCLUDED IN EXTRACTED AMINO ACID RESIDUE GROUP — NO

YES

**SE4-4**
AMINO SIDE OF RESIDUE j DOES NOT FORM CUT-OUT PORTION, AND THEREFORE NO CAP ADDITION IS REQUIRED

**SE4-5**
TAKE MAIN CHAIN CARBONYL CARBON OF AMINO ACID RESIDUE j-1 AS $C_{j-1}$

**SE4-6**
TAKE MAIN CHAIN AMINO GROUP NITROGEN OF AMINO ACID RESIDUE j AS $N_j$

**SE4-7**
TAKE MAIN CHAIN $\alpha$ CARBON OF AMINO ACID RESIDUE j AS $CA_j$

**SE4-8**
DETERMINE POSITION OF CAP METHYL GROUP CARBON $C_{CAPN}$ TO BE ADDED ACCORDING TO THE FOLLOWING EQUATION

$$\overrightarrow{N_j C_{CAPN}} = \frac{\overrightarrow{N_j C_{j-1}}}{\left|\overrightarrow{N_j C_{j-1}}\right|} \times R_{NCsp3} \quad (R_{NCsp3} = 1.47\,\text{Å})$$

**SE4-9**
DETERMINE POSITIONS OF THREE CAP METHYL GROUP HYDROGEN $H_{CAPNk}$ (k=1,2,3) TO BE ADDED

BOND LENGTH $\quad \left|\overrightarrow{H_{CAPNk} C_{CAPN}}\right| = R_{Csp3H} \quad (R_{Csp3H} = 1.09\,\text{Å})$

BOND ANGLE $\quad \angle H_{CAPNk} C_{CAPN} N_j = A_{Csp3} \quad (A_{Csp3} = 109.5°)$

DIHEDRAL ANGLE $\quad \angle H_{CAPNk} C_{CAPN} N_j CA_j = D_k \quad (D_1 = 180.0°, D_2 = 60.0°, D_3 = -60.0°)$

END

# FIG.81

ORIGINAL COORDINATES

COORDINATES AFTER CAP IS ADDED

EP 1 510 943 A1

**FIG.82**

```
                    ┌──────────────┐
                    │    START     │
                    └──────┬───────┘
                           │
                        SF4-1
                    ╱──────────╲
                   ╱ WHEN AMINO ╲        NO
                  ╱ ACID RESIDUE ╲──────────────┐
                  ╲ j IS C-TERMINAL ╱           │
                   ╲ AMINO ACID   ╱              │
                    ╲──────────╱                 │
                        │ YES   SF4-2            │
                ┌───────┴────────┐               │
                │ AMINO SIDE OF  │               │
                │ RESIDUE j DOES │               │
                │ NOT FORM CUT-  │               │
                │ OUT PORTION,   │               │
                │ AND THEREFORE  │               │
                │ NO CAP ADDITION│               │
                │ IS REQUIRED    │               │
                └───────┬────────┘               │
```

SF4-3 — WHEN ADJACENT AMINO ACID RESIDUE j+1 IS ALSO INCLUDED IN EXTRACTED AMINO ACID RESIDUE GROUP — NO

SF4-4 — AMINO SIDE OF RESIDUE j DOES NOT FORM CUT-OUT PORTION, AND THEREFORE NO CAP ADDITION IS REQUIRED

SF4-5 — TAKE MAIN CHAIN AMINO GROUP NITROGEN OF AMINO ACID RESIDUE j+1 AS $N_{j+1}$

SF4-6 — TAKE MAIN CHAIN CARBONYL CARBON OF AMINO ACID RESIDUE j AS $C_j$

SF4-7 — TAKE MAIN CHAIN $\alpha$ CARBON OF AMINO ACID RESIDUE j AS $CA_j$

SF4-8 — DETERMINE POSITION OF CAP METHYL GROUP CARBON $C_{CAPC}$ TO BE ADDED ACCORDING TO THE FOLLOWING EQUATION

$$\overrightarrow{C_j C_{CAPC}} = \frac{\overrightarrow{C_j N_{j+1}}}{\left| \overrightarrow{C_j N_{j+1}} \right|} \times R_{Csp2Csp3} \quad ( R_{Csp2Csp3} = 1.52 )$$

SF4-9 — DETERMINE POSITIONS OF THREE CAP METHYL GROUP HYDROGEN $H_{CAPCk}$ (k=1,2,3) TO BE ADDED

BOND LENGTH $\left| \overline{H_{CAPCk} C_{CAPC}} \right| = R_{Csp3H} \quad ( R_{Csp3H} = 1.09 \ \text{Å})$

BOND ANGLE $\angle H_{CAPCk} C_{CAPC} C_j = \dot{A}_{Csp.} ( A_{Csp3} = 109.5° )$

DIHEDRAL ANGLE $\angle H_{CAPCk} C_{CAPC} C_j CA_j = D ( D_1 = 180.0°, D_2 = 60.0°, D_3 = -60.0° )$

```
                    ┌──────────────┐
                    │     END      │
                    └──────────────┘
```

EP 1 510 943 A1

# FIG.83

ORIGINAL COORDINATES → COORDINATES AFTER CAP IS ADDED

# FIG.84

DECIDE TYPE OF AMINO ACID WITH THESE THREE CHARACTERS

```
. . . . . .
ATOM   2132   H40  ARG  1  135    7. 570    94. 945  31. 002
ATOM   2133   H42  ARG  1  135    7. 667    95. 518  32. 710
ATOM   2134   H44  ARG  1  135    9. 663    96. 855  32. 278
ATOM   2135   H46  ARG  1  135   10. 017    95. 974  30. 735
. . . . . .
```

# FIG.85

# FIG.86

# FIG.87

```
                                            OPTIMIZING FLAG
                                            OPTIMIZE IF THIS VALUE IS 1 AND NOT
                                            OPTIMIZE IF THIS VALUE IS 0
INSERT CALCULATED CHARGE VALUE HERE
                          INSIDE PARENTHESES
ATOMIC                    ARE COMMENTS
SPECIES
                      x, y, z COORDINATES                    KEYWORD ROW
  ---FROM HERE---                                           KEYWORD ROW
  am1 mmok mmccrok cycles=8000 eps=4.0 memory=2000 &        COMMENT ROW
  & charge=0                                                FROM HERE, INITIAL
  1cbi F50                                                  COORDINATE DATA
  N( 1 PHE   3  N  )   3.17000  0 101.65400  0  28.67700  0
  C( 1 PHE   3  CA )   2.71300  0 100.27000  0  28.51000  0
  C( 1 PHE   3  C  )   3.50800  0  99.45700  0  27.50100  0
  O( 1 PHE   3  O  )   3.34800  0  98.23900  0  27.43500  0
  C( 1 PHE   3  CB )   1.25400  0 100.23300  0  28.06200  0
  C( 1 PHE   3  CG )   0.27400  0 100.69400  0  29.10000  0
  C( 1 PHE   3  CD1)   0.17900  0 102.03200  0  29.44600  0
  C( 1 PHE   3  CD2)  -0.60100  0  99.79500  0  29.68400  0
  C( 1 PHE   3  CE1)  -0.77400  0 102.46500  0  30.35200  0
  C( 1 PHE   3  CE2)  -1.56100  0 100.22600  0  30.59400  0
  C( 1 PHE   3  CZ )  -1.64700  0 101.56300  0  30.92600  0
  H( 1 PHE   3  H  )   2.57000  0 102.35900  0  28.32400  0
  ......
  OMITTED
  ......
  H( 1 GLN  49  HE2)  -2.22300  0 107.41300  0  15.77000  0
  N( 1 PHE  50  N  )  -2.36600  0 102.06400  0  20.36900  0
  C( 1 PHE  50  CA )  -1.82400  0 100.79200  0  20.84100  0
  C( 1 PHE  50  C  )  -1.88200  0  99.70400  0  19.79200  0
  O( 1 PHE  50  O  )  -2.80700  0  99.65400  0  18.98500  0
  C( 1 PHE  50  CB )  -2.65100  0 100.25800  0  22.01700  0
  C( 1 PHE  50  CG )  -2.29600  0 100.85000  0  23.32600  0
  C( 1 PHE  50  CD1)  -2.69800  0 102.13800  0  23.65300  0
  C( 1 PHE  50  CD2)  -1.55000  0 100.12300  0  24.23900  0
  C( 1 PHE  50  CE1)  -2.35500  0 102.69200  0  24.87100  0
  C( 1 PHE  50  CE2)  -1.20300  0 100.66700  0  25.45700  0
  C( 1 PHE  50  CZ )  -1.60200  0 101.94900  0  25.77600  0
  H( 1 PHE  50  H  )  -3.28500  1 102.01600  1  19.99400  1
  H( 1 PHE  50  HA )  -0.75800  1 100.98600  1  21.18400  1
  H( 1 PHE  50  HB )  -3.74900  1 100.42200  1  21.82100  1
  H( 1 PHE  50  HB )  -2.50800  1  99.14100  1  22.07700  1
  H( 1 PHE  50  HD1)  -3.30600  1 102.72700  1  22.94200  1
  H( 1 PHE  50  HD2)  -1.23400  1  99.09400  1  23.99300  1
  ......
  OMITTED
  ......
```

# FIG.88

```
. . . . . .
OMITTED
. . . . . .
  131   H (  1   GLN  49   H)   -2.223000000        107.413000000        15.770000000
  132   N (  1   PHE  50   N)   -2.366000000        102.064000000        20.369000000
  133   C (  1   PHE  50   C)   -1.824000000        100.792000000        20.841000000
  134   C (  1   PHE  50   C)   -1.882000000         99.704000000        19.792000000
  135   O (  1   PHE  50   O)   -2.807000000         99.654000000        18.985000000
  136   C (  1   PHE  50   C)   -2.651000000        100.258000000        22.017000000
  137   C (  1   PHE  50   C)   -2.296000000        100.850000000        23.326000000
  138   C (  1   PHE  50   C)   -2.698000000        102.138000000        23.653000000
  139   C (  1   PHE  50   C)   -1.550000000        100.123000000        24.239000000
  140   C (  1   PHE  50   C)   -2.355000000        102.692000000        24.871000000
  141   C (  1   PHE  50   C)   -1.203000000        100.667000000        25.457000000
  142   C (  1   PHE  50   C)   -1.602000000        101.949000000        25.776000000
  143   H (  1   PHE  50   H)   -3.285000000  *     102.016000000  *     19.994000000  *
  144   H (  1   PHE  50   H)   -0.758000000  *     100.986000000  *     21.184000000  *
  145   H (  1   PHE  50   H)   -3.749000000  *     100.422000000  *     21.821000000  *
  146   H (  1   PHE  50   H)   -2.508000000  *      99.141000000  *     22.077000000  *
  147   H (  1   PHE  50   H)   -3.306000000  *     102.727000000  *     22.942000000  *
  148   H (  1   PHE  50   H)   -1.234000000  *      99.094000000  *     23.993000000  *
  149   H (  1   PHE  50   H)   -2.682000000  *     103.712000000  *     25.129000000  *
  150   H (  1   PHE  50   H)   -0.614000000  *     100.074000000  *     26.178000000  *
  151   H (  1   PHE  50   H)   -1.318000000  *     102.389000000  *     26.747000000  *
  152   N (  1   TYR  51   N)   -0.914000000         98.807000000        19.841000000
  153   C (  1   TYR  51   C)   -0.908000000         97.651000000        18.975000000
  154   C (  1   TYR  51   C)   -0.405000000         96.559000000        19.911000000
  155   O (  1   TYR  51   O)    0.750000000         96.577000000        20.346000000
. . . . . .
OMITTED
. . . . . .
```

EP 1 510 943 A1

# FIG.89

# FIG.90

## INTERNATIONAL SEARCH REPORT

| International application No. |
|---|
| PCT/JP03/06952 |

| A. CLASSIFICATION OF SUBJECT MATTER |
|---|
| Int.Cl<sup>7</sup> G06F17/30, C07K1/00 |

According to International Patent Classification (IPC) or to both national classification and IPC

| B. FIELDS SEARCHED |
|---|
| Minimum documentation searched (classification system followed by classification symbols) |
| Int.Cl<sup>7</sup> G06F17/30, C07K1/00 |

Documentation searched other than minimum documentation to the extent that such documents are included in the fields searched

| Jitsuyo Shinan Koho | 1922–1996 | Toroku Jitsuyo Shinan Koho | 1994–2003 |
|---|---|---|---|
| Kokai Jitsuyo Shinan Koho | 1971–2003 | Jitsuyo Shinan Toroku Koho | 1996–2003 |

Electronic data base consulted during the international search (name of data base and, where practicable, search terms used)
JICST FILE(JOIS), WPI, INSPEC(DIALOG)

| C. DOCUMENTS CONSIDERED TO BE RELEVANT |
|---|

| Category* | Citation of document, with indication, where appropriate, of the relevant passages | Relevant to claim No. |
|---|---|---|
| E,X | JP 2003-196290 A (Celestar Lexico-Sciences, Inc.), 11 July, 2003 (11.07.03), Claims (Family: none) | 1-10 |
| A | JP 5-282381 A (Fujitsu Ltd.), 29 October, 1993 (29.10.93), Claim 1 (Family: none) | 1-10 |
| A | FURUTA et al., "Tanpakushitsu no Niji Kozo Yosoku Shien Expert·Expert System", Information Processing Society of Japan Dai 33 Kai(Showa 61 Nen Koki) Zenkoku Taikai Koen Ronbunshu(II), 01 October, 1986 (01.10.86), pages 1197 to 1198 | 1-10 |

| ☒ Further documents are listed in the continuation of Box C. | ☐ See patent family annex. |
|---|---|

| * | Special categories of cited documents: |
|---|---|
| "A" | document defining the general state of the art which is not considered to be of particular relevance |
| "E" | earlier document but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 18 September, 2003 (18.09.03) | 14 October, 2003 (14.10.03) |

| Name and mailing address of the ISA/ | Authorized officer |
|---|---|
| Japanese Patent Office | |
| Facsimile No. | Telephone No. |

Form PCT/ISA/210 (second sheet) (July 1998)

**INTERNATIONAL SEARCH REPORT**

| International application No. |
| --- |
| PCT/JP03/06952 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X | JP 2000-143554 A   (The Institute of Physical and Chemical Research), 23 May, 2000 (23.05.00), Claims (Family: none) | 11-24 |
| Y | AKABANE, UMEYAMA, "Yakubutsu-Juyotai Sosui Sogo Sayo", Gendai Kagaku, special extra issue 13, Shin'yaku no Lead Generation -Saishin Drug Design-, 20 November, 1987 (20.11.87), pages 136 to 148, particularly, page 145 | 25-34 |
| Y | KOMATSU, UMEYAMA, "Yakubutsu-Juyotai Seiden Sogo Sayo", Gendai Kagaku, special extra issue 13, Shin'yaku no Lead Generation -Saishin Drug Design-, 20 November, 1987 (20.11.87), pages 119 to 135, particularly, page 126, right column | 25-34 |
| X | WO 93/20525 A   (Akiko ITAI), 14 October, 1993 (14.10.93), Page 3, upper left column, line 16 to upper right column, line 2 & EP 633534 A            & US 5642292 A | 35-45 |
| X | SATO et al., "Ab initio pair Kinjiho ni yoru Heiretsu Bunshi Kido Keisan Program ABINIT-MP no Sakusei to Seino Hyoka", Transactions of Information Processing Society of Japan, Vol.41, No.SIG5(HPS1), 15 August, 2000 (15.08.00), page 104 to 112, particularly, p.106 | 46-55 |

Form PCT/ISA/210 (continuation of second sheet) (July 1998)

## INTERNATIONAL SEARCH REPORT

International application No.

PCT/JP03/06952

**Box I    Observations where certain claims were found unsearchable (Continuation of item 2 of first sheet)**

This international search report has not been established in respect of certain claims under Article 17(2)(a) for the following reasons:

1. ☐ Claims Nos.:

because they relate to subject matter not required to be searched by this Authority, namely:

2. ☐ Claims Nos.:

because they relate to parts of the international application that do not comply with the prescribed requirements to such an extent that no meaningful international search can be carried out, specifically:

3. ☐ Claims Nos.:

because they are dependent claims and are not drafted in accordance with the second and third sentences of Rule 6.4(a).

**Box II    Observations where unity of invention is lacking (Continuation of item 3 of first sheet)**

This International Searching Authority found multiple inventions in this international application, as follows:

The technical feature of claims 1-10 is prediction of an interaction portion of a protein by calculation of frustration.

The technical feature of claims 11-24 is prediction of an active portion of a protein or a polypeptide by molecular orbit calculation.

The technical feature of claims 25-34 is determination of an interaction portion of a protein in consideration of the hydrophobic interaction and the electrostatic interaction.

The technical feature of claims 35-45 is prediction of a junction portion by identifying electrostatically unstable amino acid residues on the basis of data on the spatial distances    (Continued to extra sheet.)

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐    The additional search fees were accompanied by the applicant's protest.

☒    No protest accompanied the payment of additional search fees.

Form PCT/ISA/210 (continuation of first sheet (1)) (July 1998)

**INTERNATIONAL SEARCH REPORT**

International application No.

PCT/JP03/06952

Continuation of Box No. II of continuation of first sheet (1)

between amino acid residues of a protein or polypeptide.
    The technical feature of claims 46-55 is optimization of a protein structure by electric charge calculation.
    Therefore, the number of inventions of claims 1-10, 11-24, 25-34, 35-45, 46-55 is five.

Form PCT/ISA/210 (extra sheet) (July 1998)